# EUROPEAN PATENT SPECIFICATION

(11) **EP 2 646 043 B1**
(45) Date of publication and mention of the grant of the patent: **15.02.2017**
(21) Application number: 11845460.2
(22) Date of filing: 05.12.2011
(51) Int. Cl.: A61K 38/00, C07K 7/64, A61K 38/13, A61P 31/12, A61P 31/18, A61P 31/20

(54) **NOVEL CYCLOSPORIN DERIVATIVES FOR THE TREATMENT AND PREVENTION OF A VIRAL INFECTION**
NEUE CYCLOSPORINDERIVATE ZUR VORBEUGUNG UND BEHANDLUNG VON VIRUSINFEKTIONEN
NOUVEAUX DÉRIVÉS DE CICLOSPORINE DESTINÉS AU TRAITEMENT ET À LA PRÉVENTION D'UNE INFECTION VIRALE

(30) Priority: 03.12.2010 US 419326 P
(43) Date of publication of application: 09.10.2013
(73) Proprietor: S&T Global Inc., Woburn, MA 01801 (US)
(72) Inventor: SU, Zhuang, Andover MA 01810 (US); LONG, Zhengyu, Bolton MA 01740 (US); HUANG, Zhennian, Newton MA 02459 (US); YANG, Suizhou, Dracut MA 01826 (US)
(74) Representative: Camenisch, Andrew Richard
(86) International application number: PCT/US2011/063295
(87) International publication number: WO 2012/075494

(56) References cited:
- WO-A2-2012/021796
- US-A- 4 703 033
- US-A1- 2010 173 837
- US-B2- 7 718 767

## Description

The invention relates to novel cyclosporine derivatives, their pharmaceutical compositions comprising the same, and methods for treating or preventing a viral infection using the same.

### Background of the Invention

Naturally occurring cyclosporins are poly-N-methyl, cyclic undecapeptides, isolated from fungi. Cyclosporin A has an immunosuppressive activity and has been used for almost 40 years to prevent rejection in kidney, heart and liver transplant recipients. It has antiinflammatory property and is useful for treating rheumatoid arthritis, severe psoriasis, Behget's uveitis and dry eye disease. In addition, it is useful for treating severe ulcerative colitis, Crohn's disease, alopecia areata, aplastic anemia, HSV-1 stromal keratitis, systemic lupus erythematosus, and severe lupus nephritis.

The anti-HIV activity of cyclosporin A was discovered (Klatzmann, D., et al., 1986, C R Acad. Sci. III, 303(9):343-8; Wainberg, M. A., et al., 1988, Blood, 72, 1904-10; Luban, J., et al., 1993, Cell, 73, 1067-1078). Its non-immunosuppressive derivative, NIM-811 was reported to have potent anti HIV activity, due to its ability to inhibit cyclophilin A (Franke, E. K., et al., 1994, Nature, 372, 359-362; Thali, M., et al., 1994, Nature, 372, 363-365; Gamble, T. R., et al., 1996, Cell, 87, 1157-1159; Rosenwirth B., et al., 1994, Antimicrob. Agents Chemother., 38, 1763-1772).

Cyclosporin A and its non-immunosuppressive derivatives, as such as NIM-811 (N-MeIle-4-Cyclosporin), Debio-025, and SCY-635, inhibit cyclophilin, which interact with HCV protein NS5B and stimulate its RNA-binding activity. As a result, these compounds have an effective anti-HCV activity (Watashi, K., et al., 2007, Rev. Med. Virol., 17:245-252.37; Inoue, K., et al., 2001, Nippon Rinsho., 59, 1326-30; Inoue, K., et al., 2003, J. Gastroenterol., 38, 567-72; Watashi, K., et al., 2003, Hepatology, 38, 1282-8; Gaither, L. A., et al., 2010, Virology, 397, 43-55). Currently, NIM-811, Debio-025, and SCY-635 are undergoing clinical trials for treating HCV.

NIM-811 and Debio-025 have a chemical structure similar to cyclosporine A, and have poor pharmacokinetic profile and poor oral absorption. In addition, they are metabolized by P450 for inducing drug interactions (Lill, J., et al., 2000, Am J Health-Syst Pharm 57, 1579).

SCY-635 has an improved pharmacokinetic profile and low blood serum binding. In addition, it is less metabolized by P450 and has low potential for drug-drug interactions. SCY-635's *in vitro* anti-HCV activity (EC₅₀) was reported to be 0.10 µM by using the luciferase end point method developed by Hopkins, S. et al., 2010, Antimicrob. Agents Chemother., 54, 660-672. However, SCY-635 is not chemically stable according to testing results in our laboratory. SCY-635 is easily converted to its diastereoisomer by epimerization, which is expected to have poor binding activity with cyclophilin, and as a result, its anti-viral activity in vivo may be affected.

Cyclosporin A and its non-immunosuppressive derivatives were also found to possess anti-HBV activity through the inhibition of cyclophilins (Chokshi, S., et al., 2011, Abstract 190 (Poster Presentations), 46th Annual Meeting of the European Association for the Study of the Liver (EASL 2011), Berlin, March 30-April 3; Tian, X. C., et al., 2010, J. Virol., 84, 3373-3381; Xia, W. L., et al.,2004, Hepatobiliary Pancreat Dis Int., 4, 18-22; Michael, J., et al., 2003, J. Virol., 77, 7713-7719).

Furthermore, Cyclophilin were reported to regulate life cycles and pathogenesis of several viruses, including influenza A virus, severe acute respiratory syndrome coronavirus, and vaccinia virus (Castro, A. P., et al., 2003, J. Virol., 77, 9052-9068; Chen, Z., L., et al., 2005, J. Infect. Dis. 191, 755-760; Liu, X. L., et al., 2009, Cell Microbiol., 11, 73 0-74 1). Cyclosporin A and its non-immunosuppressive derivative also possess such anti viral-activities.

N-MeVal-4-Cyclosporin (SDZ 220-384), another non-immunosuppressive cyclosporine derivative, was reported to have similar biological activity to that of NIM-811 (Fliri, H., et al., 1993, Ann. N Y Acad Sci. 696, 47-53; Zenke, G., et al., 1993, Ann N Y Acad Sci. 23;685:330-5).

Hepatitis C virus (HCV) is a small (55-65 nm in size), enveloped, positive sense single strand RNA virus in the family Flaviviridae. HCV has a high rate of replication and has an exceptionally high mutation rate. Most people infected with HCV (about 80%) develop chronic, persistent infection. More than 4 million Americans have been infected with HCV and more than 200 million people are estimated to be infected chronically worldwide. About 35,000 new cases of hepatitis C are estimated to occur in the United States each year. HCV infection is responsible for about 50% of all chronic liver disease, 30% of all liver transplants, and 30% of all cirrhosis, end-stage liver disease, and liver cancer in the U.S. The peg-interferon and ribavirin combination is the standard treatment for chronic hepatitis C but has low efficacy against HCV infection. Recently, the FDA has approved Vertex's Incivek (telaprevir) and Merck's Victrelis (boceprevir) as an add-on to the current interferon/ribavirin therapy for treating HCV. Both drugs are HCV protease inhibitors and target virus to prevent its replication. However, due to the fast mutation of HCV, drug resistance can be developed in a short period of time for the new drugs. There exists a need for an effective therapeutic for HCV treatment.

Hepatitis B virus (HBV) is a 42 nm partially double stranded DNA virus, composed of a 27 nm nucleocapsid core (HBcAg), surrounded by an outer lipoprotein envelope containing the surface antigen (HBsAg). About a quarter of the world's population, more than 2 billion people, have been infected with the hepatitis B virus. This includes 350 million chronic carriers of the virus. The disease has caused epidemics in parts of Asia and Africa, and it is endemic in China. Chronic hepatitis B will cause liver cirrhosis and liver cancer-a fatal disease with very poor response to current chemotherapy. Although the infection is preventable by vaccination and HBV load and replication can be reduced by current antiviral drugs lamivudine (Epivir), adefovir (Hepsera), tenofovir (Viread), telbivudine (Tyzeka) and entecavir (Baraclude) and the two immune system modulators interferon alpha-2a and PEGylated interferon alpha-2a (Pegasys), none of the available drugs can clear the infection. There remains a need for an effective therapeutic for treating or preventing HBV infection.

The non-immunosuppressive cyclosporins derivatives bind to cyclophilin, a family of host proteins that catalyze cis-tans peptidyl-prolyl isomerization in protein folding, which is crucial for the processing, maturation of the viral proteins for viral replication. It is also different to current anti-HIV and anti-HCV drugs, the advantages of targeting host cofactors-cyclophilins by cyclosporine derivatives is the presumed higher genetic barrier to development of resistance (Rosenwirth, B., et al., 1994, Antimicrob. Agents Chemother., 38, 1763-1772; Tang, H. L. et al., 2010, Viruses, 2, 1621-1634; Hopkins, S. et al., 2010, Oral Presentation, Scynexis's SCY-635 Demonstrates Impressive Barrier to Resistance in HCV Treatment, the 45th Annual Meeting of the European Association for the Study of the Liver (EASL 2010), Vienna, Austria, April 14-18). Cyclosporine derivatives affect a new target-cyclophilin, and therefore represent a new mechanism of action against HCV.

Cyclophilins are a family of enzymes that assist in the folding and transportation of other proteins synthesized within a cell. Protein folding or misfolding plays a important role in the pathophysiology of a number of serious diseases, such as viral diseases (HCV, HIV, and herpes simplex virus), central nervous system disorders (mitochondrial protection for stroke, traumatic brain and spinal cord injury, Alzheimer, Parkinson's Disease, and Huntington's Diseases), cardiovascular diseases (reperfusion injury in myocardial infarction, heart attack, and chronic heart failure), cancer, inflammation (respiratory inflammation, asthma, rheumatoid arthritis, dry eye disease), and including inflammatory bowel disease (Crohn's Disease and Ulcerative Colitis), atopic dermatitis, anti fungal and anti-parasitic treatment, hair growth, as well as obesity, diabetes, muscular dystrophy, and NSAID-induced enteropathy.

Cyclosporin derivatives target cyclophilin and can play a crucial role for treatment of these diseases.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting.

### Summary of the Invention

In one aspect, the present invention provides a compound of Formula (I): 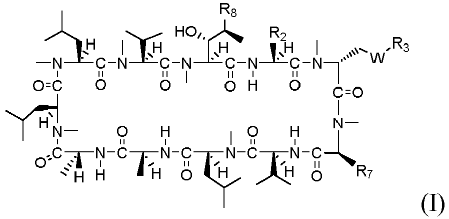 or pharmaceutically acceptable salt thereof, wherein:
R₈ is n-butyl, (*E*)-but-2-enyl or 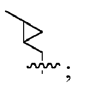
R₂ is ethyl, 1-hydroxyethyl, isopropyl or n-propyl;
W is O or S;
R₃ is:
   (C₁-C₆)alkyl, optionally substituted by one or more groups R₄ which may be the same or different;
   (C₂-C₆)alkenyl, optionally substituted by one or more groups which may be the same or different selected from halogen, hydroxy, amino, monoalkylamino and dialkylamino;
   (C₂-C₆)alkynyl, optionally substituted by one or one or more groups which may be the same or different selected from halogen, hydroxy, amino, monoalkylamino and dialkylamino;
   (C₃-C₇)cycloalkyl, optionally substituted by one or more groups which may be the same or different selected from halogen, hydroxy, amino, monoalkylamino and dialkylamino;
   phenyl or CH₂-phenyl, optionally substituted by one or more groups which may be the same or different selected from halogen, hydroxy, (C₁-C₆)alkyl;
R₇ is 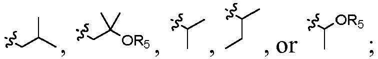
R₅ is: H;
(C₁-C₆)alkyl, optionally substituted by one or more groups R₆ which may be the same or different;
(C₂-C₆)alkenyl, optionally substituted by one or more groups which may be the same or different selected from hydroxy, (C₁-C₆)alkyl, aryl (e.g., phenyl), (CH₂)ₚOR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)ₚC(=O)OR_{A};
(C₂-C₆)alkynyl, optionally substituted by one or one or more groups which may be the same or different selected from halogen, hydroxy, amino, monoalkylamino and dialkylamino;
(C₃-C₇)cycloalkyl, optionally substituted by one or more groups which may be the same or different selected from halogen, hydroxy, amino, monoalkylamino and dialkylamino; phenyl or CH₂-phenyl, optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
   each occurrence of R₄ is independently halogen, hydroxy, aryl (e.g., phenyl), O(CH₂)ₘOR_{A}, O(CH₂)ₘO(CH₂)ₘOR_{A}, C(=O)(C₁-C₆)alkyl, C(=O)OR_{A}, C(=O)NR_{A}R_{B}, -NR_{A}R_{B},-NR_{C}CH₂(CH₂)ₚNR_{A}R_{B}, NR_{C}[CH₂(CH₂)ₚNR_{A}]ₘCH₂(CH₂)ₙNR_{A}R_{B}, O[CH₂(CH₂)ₚO]ₘCH₂(CH₂)ₙOR_{A}, OCH₂(CH₂)ₚNR_{A}R_{B}, or O[CH₂(CH₂)ₚO]ₘCH₂(CH₂)ₙNR_{A}R_{B};
   each occurrence of R₆ is independently halogen, hydroxy, aryl (e.g., phenyl), S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
   each occurrence of R_{A} and R_{B} is independently:
   hydrogen;
      (C₁-C₆)alkyl, optionally substituted by one or more groups R_{D} which may be the same or different;
      (C₂-C₆)alkenyl or (C₂-C₆)alkynyl;
      (C₃-C₇)cycloalkyl optionally substituted with (C₁-C₆)alkyl;
      phenyl optionally substituted with from one to five groups which may be the same or different selected from halogen, -O(C₁-C₆)alkyl, -C(=O)O(C₁-C₆)alkyl, amino, alkylamino and dialkylamino;
      or a heterocyclic ring which may be saturated or unsaturated containing five or six ring atoms and from one to three heteroatoms which may be the same or different selected from nitrogen, sulfur and oxygen;
      or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
   each occurrence of R_{C} is independently hydrogen or (C₁-C₆)alkyl;
   p is an integer of 0, 1, 2, 3, 4, or 5; and
   m is an integer of 1, 2, 3, 4 or 5.

In another aspect, the present invention provides a compound of Formulae (II) through (V): 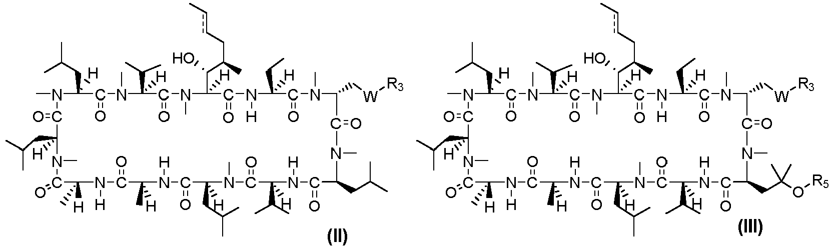 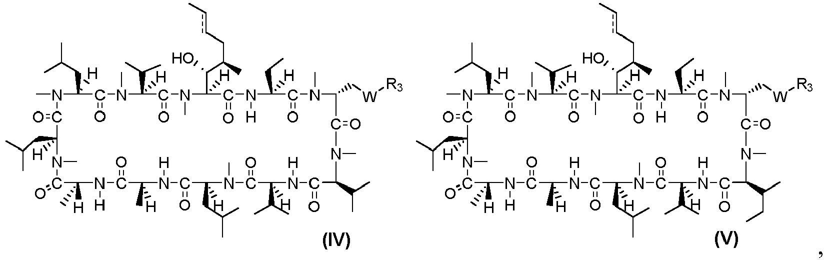 or pharmaceutically acceptable salt thereof, wherein: 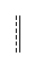
represents a single bond or double bond;
each W is independently O or S;
each R₃ is independently:
   (C₁-C₆)alkyl, optionally substituted by one or more groups R₄ which may be the same or different;
   (C₂-C₆)alkenyl, optionally substituted by one or more groups which may be the same or different selected from halogen, hydroxy, (C₁-C₆)alkyl, aryl (e.g., phenyl), (CH₂)ₚOR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{c}(CH₂)ₘNR_{c}(CH₂)ₘNR_{A}R_{B}, (CH₂)ₚC(=O)NR_{A}R_{B}, (CH₂)ₚC(=O)OR_{A};
   (C₂-C₆)alkynyl, optionally substituted by one or one or more groups which may be the same or different selected from halogen, hydroxy, amino, monoalkylamino and dialkylamino;
   (C₃-C₇)cycloalkyl, optionally substituted by one or more groups which may be the same or different selected from halogen, hydroxy, amino, monoalkylamino and dialkylamino;
   phenyl or CH₂-phenyl, optionally substituted by one or more groups which may be the same or different selected from halogen, hydroxy, (C₁-C₆)alkyl;
each R₅ is independently:
   H;
   (C₁-C₆)alkyl, optionally substituted by one or more groups R₆ which may be the same or different;
   (C₂-C₆)alkenyl, optionally substituted by one or more groups which may be the same or different selected from halogen, hydroxy, (C₁-C₆)alkyl, aryl (e.g., phenyl), (CH₂)ₚOR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{c}(CH₂)ₘNR_{c}(CH₂)ₘNR_{A}R_{B}, (CH₂)ₚC(=O)NR_{A}R_{B}, (CH₂)ₚC(=O)OR_{A};
   (C₂-C₆)alkynyl, optionally substituted by one or one or more groups which may be the same or different selected from halogen, hydroxy, amino, monoalkylamino and dialkylamino;
   (C₃-C₇)cycloalkyl, optionally substituted by one or more groups which may be the same or different selected from halogen, hydroxy, amino, monoalkylamino and dialkylamino;
   phenyl or CH₂-phenyl, optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₄ is independently halogen, hydroxy, aryl (e.g., phenyl), O(CH₂)ₘOR_{A}, O(CH₂)ₘO(CH₂)ₘOR_{A}, C(=O)(C₁-C₆)alkyl, C(=O)OR_{A}, C(=O)NR_{A}R_{B}, -NR_{A}R_{B},-NR_{C}CH₂(CH₂)ₚNR_{A}R_{B}, NR_{C}[CH₂(CH₂)ₚNR_{A}]ₘCH₂(CH₂)ₙNR_{A}R_{B}, O[CH₂(CH₂)ₚO]ₘCH₂(CH₂)ₙOR_{A}, OCH₂(CH₂)ₚNR_{A}R_{B}, or O[CH₂(CH₂)ₚO]ₘCH₂(CH₂)ₙNR_{A}R_{B};
each occurrence of R₆ is independently halogen, hydroxy, aryl (e.g., phenyl), S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl optionally substituted with from one to five groups which may be the same or different selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
   or R_{A} and R_{B}, together with the nitrogen atom to which they are attached, form -N=CH-NR_{F}R_{F'}, -N=CMe-NR_{F}R_{F'}, or -NR_{F}C(=NH)NR_{F}R_{F'};
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl;
each occurrence of R_{F} and R_{F'} is independently hydrogen, (C₁-C₆)alkyl, phenyl, benzyl, or R_{F} and R_{F'}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
p is an integer of 0, 1, 2, 3, 4, 5, or 6;
m is an integer of 1, 2, 3, 4, 5, or 6; and
n is an integer of 1, 2, 3, 4, 5 or 6.

In yet another aspect, the present invention provides a pharmaceutical composition comprising at least one compound as described herein and a pharmaceutically-acceptable carrier.

In a further aspect, the present invention provides a compound for use in treating or preventing a viral infection, hepatitis C virus infection or HIV infection in a mammalian species.

### Detailed Description of the Invention

### Definitions

The following are definitions of terms used in the present specification. The initial definition provided for a group or term herein applies to that group or term throughout the present specification individually or as part of another group, unless otherwise indicated.

The terms "alkyl" and "alk" refer to a straight or branched chain alkane (hydrocarbon) radical containing from 1 to 12 carbon atoms, preferably 1 to 6 carbon atoms. Exemplary "alkyl" groups include methyl, ethyl, propyl, isopropyl, n-butyl, t-butyl, isobutyl pentyl, hexyl, isohexyl, heptyl, 4,4-dimethylpentyl, octyl, 2,2,4-trimethylpentyl, nonyl, decyl, undecyl, dodecyl, and the like. The term "(C₁-C₄)alkyl" refers to a straight or branched chain alkane (hydrocarbon) radical containing from 1 to 4 carbon atoms, such as methyl, ethyl, propyl, isopropyl, n-butyl, t-butyl, and isobutyl. The term "(C₁-C₆)alkyl" refers to a straight or branched chain alkane (hydrocarbon) radical containing from 1 to 6 carbon atoms, such as n-hexyl, 2-methylpentyl, 3-methylpentyl, 4-methylpentyl, 2,3-dimethylbutyl, 2,2-dimethylbutyl, in addition to those exemplified for "(C₁-C₄)alkyl." "Substituted alkyl" refers to an alkyl group substituted with one or more substituents, preferably 1 to 4 substituents, at any available point of attachment. Exemplary substituents include but are not limited to one or more of the following groups: hydrogen, halogen (*e.g.,* a single halogen substituent or multiple halo substitutents forming, in the latter case, groups such as CF₃ or an alkyl group bearing Cl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORe, NR_{b}R_{c}, NR_{b}S(=O)₂Re, NR_{b}P(=O)₂Re, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. In the aforementioned exemplary substitutents, groups such as alkyl, cycloalkyl, alkenyl, alkynyl, cycloalkenyl, heterocycle and aryl can themselves be optionally substituted.

The term "alkenyl" refers to a straight or branched chain hydrocarbon radical containing from 2 to 12 carbon atoms and at least one carbon-carbon double bond. Exemplaries of such groups include ethenyl or allyl. The term "C₂-C₆ alkenyl" refers to a straight or branched chain hydrocarbon radical containing from 2 to 6 carbon atoms and at least one carbon-carbon double bond, such as ethylenyl, propenyl, 2-propenyl, (*E*)-but-2-enyl, (*Z*)-but-2-enyl, 2-methy(*E*)-but-2-enyl, 2-methy(*Z*)-but-2-enyl, 2,3-dimethy-but-2-enyl, (*Z*)-pent-2-enyl, (*E*)-pent-1-enyl, (*Z*)-hex-1-enyl, (*E*)-pent-2-enyl, (*Z*)-hex-2-enyl, (*E*)-hex-2-enyl, (*Z*)-hex-1-enyl, (*E*)-hex-1-enyl,, (*Z*)-hex-3-enyl, (*E*)-hex-3-enyl, and (*E*)-hex-1,3-dienyl. "Substituted alkenyl" refers to an alkenyl group substituted with one or more substituents, preferably 1 to 4 substituents, at any available point of attachment. Exemplary substituents include but are not limited to one or more of the following groups: hydrogen, halogen *(e.g.,* a single halogen substituent or multiple halo substitutents forming, in the latter case, groups such as CF₃ or an alkyl group bearing Cl₃), cyano, nitro, oxo (*i.e.,* =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substitutents can themselves be optionally substituted.

The term "alkynyl" refers to a straight or branched chain hydrocarbon radical containing from 2 to 12 carbon atoms and at least one carbon to carbon triple bond. An exemplary of such groups includes ethynyl. The term "C₂-C₆ alkynyl" refers to a straight or branched chain hydrocarbon radical containing from 2 to 6 carbon atoms and at least one carbon-carbon triple bond, such as ethynyl, prop-1-ynyl, prop-2-ynyl, but-1-ynyl, but-2-ynyl, pent-1-ynyl, pent-2-ynyl, hex-1-ynyl, hex-2-ynyl, hex-3-ynyl. "Substituted alkynyl" refers to an alkynyl group substituted with one or more substituents, preferably 1 to 4 substituents, at any available point of attachment. Exemplary substituents include but are not limited to one or more of the following groups: hydrogen, halogen (*e.g.,* a single halogen substituent or multiple halo substitutents forming, in the latter case, groups such as CF₃ or an alkyl group bearing Cl₃), cyano, nitro, oxo (*i.e.,* =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substitutents can themselves be optionally substituted.

The term "cycloalkyl" refers to a fully saturated cyclic hydrocarbon group containing from 1 to 4 rings and 3 to 8 carbons per ring. "C₃-C₇ cycloalkyl" refers to cyclopropyl, cyclobutyl, cyclopentyl, cyclohexyl, or cycloheptyl. "Substituted cycloalkyl" refers to a cycloalkyl group substituted with one or more substituents, preferably 1 to 4 substituents, at any available point of attachment. Exemplary substituents include but are not limited to one or more of the following groups: hydrogen, halogen (*e.g.,* a single halogen substituent or multiple halo substitutents forming, in the latter case, groups such as CF₃ or an alkyl group bearing Cl₃), cyano, nitro, oxo (*i.e.,* =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substitutents can themselves be optionally substituted. Exemplary substituents also include spiro-attached or fused cylic substituents, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substitutents can themselves be optionally substituted.

The term "cycloalkenyl" refers to a partially unsaturated cyclic hydrocarbon group containing 1 to 4 rings and 3 to 8 carbons per ring. Exemplaries of such groups include cyclobutenyl, cyclopentenyl, cyclohexenyl, *etc.* "Substituted cycloalkenyl" refers to a cycloalkenyl group substituted with one more substituents, preferably 1 to 4 substituents, at any available point of attachment. Exemplary substituents include but are not limited to one or more of the following groups: hydrogen, halogen (*e.g.,* a single halogen substituent or multiple halo substitutents forming, in the latter case, groups such as CF₃ or an alkyl group bearing Cl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substitutents can themselves be optionally substituted. Exemplary substituents also include spiro-attached or fused cylic substituents, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "aryl" refers to cyclic, aromatic hydrocarbon groups that have 1 to 5 aromatic rings, especially monocyclic or bicyclic groups such as phenyl, biphenyl or naphthyl. Where containing two or more aromatic rings (bicyclic, *etc.*)*,* the aromatic rings of the aryl group may be joined at a single point (*e.g.,* biphenyl), or fused (*e.g.,* naphthyl, phenanthrenyl and the like). "Substituted aryl" refers to an aryl group substituted by one or more substituents, preferably 1 to 3 substituents, at any available point of attachment. Exemplary substituents include but are not limited to one or more of the following groups: hydrogen, halogen (*e.g.,* a single halogen substituent or multiple halo substitutents forming, in the latter case, groups such as CF₃ or an alkyl group bearing Cl₃), cyano, nitro, oxo (*i.e.,* =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substitutents can themselves be optionally substituted. Exemplary substituents also include fused cylic groups, especially fused cycloalkyl, fused cycloalkenyl, fused heterocycle, or fused aryl, where the aforementioned cycloalkyl, cycloalkenyl, heterocycle and aryl substituents can themselves be optionally substituted.

The terms "heterocycle" and "heterocyclic" refer to fully saturated, or partially or fully unsaturated, including aromatic (*i.e.,* "heteroaryl") cyclic groups (for example, 4 to 7 membered monocyclic, 7 to 11 membered bicyclic, or 8 to 16 membered tricyclic ring systems) which have at least one heteroatom in at least one carbon atom-containing ring. Each ring of the heterocyclic group containing a heteroatom may have 1, 2, 3, or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. (The term "heteroarylium" refers to a heteroaryl group bearing a quaternary nitrogen atom and thus a positive charge.) The heterocyclic group may be attached to the remainder of the molecule at any heteroatom or carbon atom of the ring or ring system. Exemplary monocyclic heterocyclic groups include azetidinyl, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, hexahydrodiazepinyl, 4-piperidonyl, pyridyl, pyrazinyl, pyrimidinyl, pyridazinyl, triazinyl, triazolyl, tetrazolyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, and the like. Exemplary bicyclic heterocyclic groups include indolyl, isoindolyl, benzothiazolyl, benzoxazolyl, benzoxadiazolyl, benzothienyl, benzo[d][1,3]dioxolyl, 2,3-dihydrobenzo[b][1,4]dioxinyl, quinuclidinyl, quinolinyl, tetrahydroisoquinolinyl, isoquinolinyl, benzimidazolyl, benzopyranyl, indolizinyl, benzofuryl, benzofurazanyl, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]pyridinyl] or furo[2,3-b]pyridinyl), dihydroisoindolyl, dihydroquinazolinyl (such as 3,4-dihydro-4-oxo-quinazolinyl), triazinylazepinyl, tetrahydroquinolinyl and the like. Exemplary tricyclic heterocyclic groups include carbazolyl, benzidolyl, phenanthrolinyl, acridinyl, phenanthridinyl, xanthenyl and the like.

"Substituted heterocycle" and "substituted heterocyclic" (such as "substituted heteroaryl") refer to heterocycle or heterocyclic groups substituted with one or more substituents, preferably 1 to 4 substituents, at any available point of attachment. Exemplary substituents include but are not limited to one or more of the following groups: hydrogen, halogen (*e.g.,* a single halogen substituent or multiple halo substitutents forming, in the latter case, groups such as CF₃ or an alkyl group bearing Cl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substitutents can themselves be optionally substituted. Exemplary substituents also include spiro-attached or fused cylic substituents at any available point or points of attachment, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "alkylamino" refers to a group having the structure -NHR', wherein R' is hydrogen, alkyl or substituted alkyl, cycloalkyl or substituted cyclolakyl, as defined herein. Examples of alkylamino groups include, but are not limited to, methylamino, ethylamino, n-propylamino, iso-propylamino, cyclopropylamino, n-butylamino, tert-butylamino, neopentylamino, n-pentylamino, hexylamino, cyclohexylamino, and the like.

The term "dialkylamino" refers to a group having the structure -NRR', wherein R and R' are each independently alkyl or substituted alkyl, cycloalkyl or substituted cycloalkyl, cycloalkenyl or substituted cyclolalkenyl, aryl or substituted aryl, heterocylyl or substituted heterocyclyl, as defined herein. R and R' may be the same or different in an dialkyamino moiety. Examples of dialkylamino groups include, but are not limited to, dimethylamino, methyl ethylamino, diethylamino, methylpropylamino, di(n-propyl)amino, di(isopropyl)amino, di(cyclopropyl)amino, di(n-butyl)amino, di(tert-butyl)amino, di(neopentyl)amino, di(n-pentyl)amino, di(hexyl)amino, di(cyclohexyl)amino, and the like. In certain embodiments, R and R' are linked to form a cyclic structure. The resulting cyclic structure may be aromatic or non-aromatic. Examples of cyclic diaminoalkyl groups include, but are not limited to, aziridinyl, pyrrolidinyl, piperidinyl, morpholinyl, pyrrolyl, imidazolyl, 1,3,4-trianolyl, and tetrazolyl.

The terms "halogen" or "halo" refer to chlorine, bromine, fluorine or iodine.

Unless otherwise indicated, any heteroatom with unsatisfied valences is assumed to have hydrogen atoms sufficient to satisfy the valences.

The compounds of the present invention may form salts which are also within the scope of this invention. Reference to a compound of the present invention is understood to include reference to salts thereof, unless otherwise indicated. The term "salt(s)", as employed herein, denotes acidic and/or basic salts formed with inorganic and/or organic acids and bases. In addition, when a compound of the present invention contains both a basic moiety, such as but not limited to a pyridine or imidazole, and an acidic moiety such as but not limited to a carboxylic acid, zwitterions ("inner salts") may be formed and are included within the term "salt(s)" as used herein. Pharmaceutically acceptable (*i.e.,* non-toxic, physiologically acceptable) salts are preferred, although other salts are also useful, *e.g.,* in isolation or purification steps which may be employed during preparation. Salts of a compound of the present invention may be formed, for example, by reacting a compound I with an amount of acid or base, such as an equivalent amount, in a medium such as one in which the salt precipitates or in an aqueous medium followed by lyophilization.

The compounds of the present invention which contain a basic moiety, such as but not limited to an amine or a pyridine or imidazole ring, may form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, hydroxyethanesulfonates (*e.g.,* 2-hydroxyethanesulfonates), lactates, maleates, methanesulfonates, naphthalenesulfonates (*e.g.,* 2-naphthalenesulfonates), nicotinates, nitrates, oxalates, pectinates, persulfates, phenylpropionates (*e.g.,* 3-phenylpropionates), phosphates, picrates, pivalates, propionates, salicylates, succinates, sulfates (such as those formed with sulfuric acid), sulfonates, tartrates, thiocyanates, toluenesulfonates such as tosylates, undecanoates, and the like.

Compounds of the present invention which contain an acidic moiety, such but not limited to a carboxylic acid, may form salts with a variety of organic and inorganic bases. Exemplary basic salts include ammonium salts, alkali metal salts such as sodium, lithium and potassium salts, alkaline earth metal salts such as calcium and magnesium salts, salts with organic bases (for example, organic amines) such as benzathines, dicyclohexylamines, hydrabamines (formed with N,N-bis(dehydroabietyl) ethylenediamine), N-methyl-D-glucamines, N-methyl-D-glycamides, t-butyl amines, and salts with amino acids such as arginine, lysine and the like. Basic nitrogen-containing groups may be quaternized with agents such as lower alkyl halides (*e.g.,* methyl, ethyl, propyl, and butyl chlorides, bromides and iodides), dialkyl sulfates (*e.g.,* dimethyl, diethyl, dibutyl, and diamyl sulfates), long chain halides (*e.g.,* decyl, lauryl, myristyl and stearyl chlorides, bromides and iodides), aralkyl halides (*e.g.,* benzyl and phenethyl bromides), and others.

Solvates of the compounds of the present invention include, for example, hydrates.

Compounds of the present invention, and salts or solvates thereof, may exist in their tautomeric form (for example, as an amide or imino ether). All such tautomeric forms are contemplated herein as part of the present invention.

All stereoisomers of the present compounds (for example, those which may exist due to asymmetric carbons on various substituents), including enantiomeric forms and diastereomeric forms, are contemplated within the scope of this invention. Individual stereoisomers of the compounds of the invention may, for example, be substantially free of other isomers (*e.g.,* as a pure or substantially pure optical isomer having a specified activity), or may be admixed, for example, as racemates or with all other, or other selected, stereoisomers. The chiral centers of the present invention may have the S or R configuration as defined by the International Union of Pure and Applied Chemistry (IUPAC) 1974 Recommendations. The racemic forms can be resolved by physical methods, such as, for example, fractional crystallization, separation or crystallization of diastereomeric derivatives or separation by chiral column chromatography. The individual optical isomers can be obtained from the racemates by any suitable method, including without limitation, conventional methods, such as, for example, salt formation with an optically active acid followed by crystallization.

Compounds of the present invention are, subsequent to their preparation, preferably isolated and purified to obtain a composition containing an amount by weight equal to or greater than 90%, for example, equal to greater than 95%, equal to or greater than 99% pure ("substantially pure" compound I), which is then used or formulated as described herein. Such "substantially pure" compounds of the present invention are also contemplated herein as part of the present invention.

All configurational isomers of the compounds of the present invention are contemplated, either in admixture or in pure or substantially pure form. The definition of compounds of the present invention embraces both *cis* (*Z*) and *trans* (*E*) alkene isomers, as well as *cis* and *trans* isomers of cyclic hydrocarbon or heterocyclic rings.

Throughout the specifications, groups and substituents thereof may be chosen to provide stable moieties and compounds.

Definitions of specific functional groups and chemical terms are described in more detail below. For purposes of this invention, the chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in "Organic Chemistry", Thomas Sorrell, University Science Books, Sausalito: 1999.

Certain compounds of the present invention may exist in particular geometric or stereoisomeric forms. The present invention contemplates all such compounds, including *cis-*and *trans*-isomers, *R-* and *S*-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

Isomeric mixtures containing any of a variety of isomer ratios may be utilized in accordance with the present invention. For example, where only two isomers are combined, mixtures containing 50:50, 60:40, 70:30, 80:20, 90:10, 95:5, 96:4, 97:3, 98:2, 99:1, or 100:0 isomer ratios are all contemplated by the present invention. Those of ordinary skill in the art will readily appreciate that analogous ratios are contemplated for more complex isomer mixtures.

The present invention also includes isotopically labeled compounds, which are identical to the compounds disclosed herein, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention, or an enantiomer, diastereomer, tautomer, or pharmaceutically acceptable salt or solvate thereof, which contain the aforementioned isotopes and/or other isotopes of other atoms are within the scope of this invention. Certain isotopically labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labeled compounds can generally be prepared by carrying out the procedures disclosed in the Schemes and/or in the Examples below, by substituting a readily available isotopically labeled reagent for a non-isotopically labeled reagent.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

It will be appreciated that the compounds, as described herein, may be substituted with any number of substituents or functional moieties. In general, the term "substituted" whether preceded by the term "optionally" or not, and substituents contained in formulas of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. For purposes of this invention, heteroatoms such as nitrogen may have hydrogen substituents and/or any permissible substituents of organic compounds described herein which satisfy the valencies of the heteroatoms. Furthermore, this invention is not intended to be limited in any manner by the permissible substituents of organic compounds. Combinations of substituents and variables envisioned by this invention are preferably those that result in the formation of stable compounds useful in the treatment, for example, of infectious diseases or proliferative disorders. The term "stable", as used herein, preferably refers to compounds which possess stability sufficient to allow manufacture and which maintain the integrity of the compound for a sufficient period of time to be detected and preferably for a sufficient period of time to be useful for the purposes detailed herein.

### Compounds

The novel cyclosporin derivatives of the present invention are potent inhibitors of viruses such as HCV, HBV, and HIV.

In one aspect, the present invention provides a compound Formula (I): 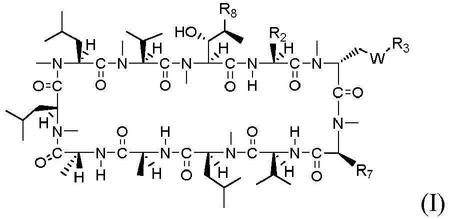 or pharmaceutically acceptable salt thereof, wherein:
R₈ is n-butyl, (*E*)-but-2-enyl, or 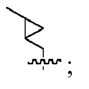
R₂ is ethyl, 1-hydroxyethyl, isopropyl or n-propyl;
W is O or S;
R₃ is:
   (C₁-C₆)alkyl, optionally substituted by one or more groups R₄ which may be the same or different;
   (C₂-C₆)alkenyl, optionally substituted by one or more groups which may be the same or different selected from halogen, hydroxy, amino, monoalkylamino and dialkylamino;
   (C₂-C₆)alkynyl, optionally substituted by one or one or more groups which may be the same or different selected from halogen, hydroxy, amino, monoalkylamino and dialkylamino;
   (C₃-C₇)cycloalkyl, optionally substituted by one or more groups which may be the same or different selected from halogen, hydroxy, amino, monoalkylamino and dialkylamino;
   phenyl or CH₂-phenyl, optionally substituted by one or more groups which may be the same or different selected from halogen, hydroxy, (C₁-C₆)alkyl;
R₇ is 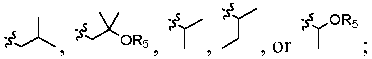
R₅ is:
   H;
   (C₁-C₆)alkyl, optionally substituted by one or more groups R₆ which may be the same or different;
   (C₂-C₆)alkenyl, optionally substituted by one or more groups which may be the same or different selected from hydroxy, (C₁-C₆)alkyl, aryl (e.g., phenyl), (CH₂)ₚOR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)ₚC(=O)OR_{A};
   (C₂-C₆)alkynyl, optionally substituted by one or one or more groups which may be the same or different selected from halogen, hydroxy, amino, monoalkylamino and dialkylamino;
   (C₃-C₇)cycloalkyl, optionally substituted by one or more groups which may be the same or different selected from halogen, hydroxy, amino, monoalkylamino and dialkylamino;
   phenyl or CH₂-phenyl, optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₄ is independently halogen, hydroxy, aryl (e.g., phenyl), O(CH₂)ₘOR_{A}, O(CH₂)ₘO(CH₂)ₘOR_{A}, C(=O)(C₁-C₆)alkyl, C(=O)OR_{A}, C(=O)NR_{A}R_{B}, -NR_{A}R_{B},-NR_{C}CH₂(CH₂)ₚNR_{A}R_{B}, NR_{C}[CH₂(CH₂)ₚNR_{A}]ₘCH₂(CH₂)ₙNR_{A}R_{B}, O[CH₂(CH₂)ₚO]ₘCH₂(CH₂)ₙOR_{A}, OCH₂(CH₂)ₚNR_{A}R_{B}, or O[CH₂(CH₂)ₚO]ₘCH₂(CH₂)ₙNR_{A}R_{B};
each occurrence of R₆ is independently halogen, hydroxy, aryl (e.g., phenyl), S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₙNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently: hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl optionally substituted with from one to five groups which may be the same or different selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
p is an integer of 0, 1, 2, 3, 4, or 5; and
m is an integer of 1, 2, 3, 4 or 5.

In certain embodiments, R₈ is n-butyl. In certain other embodiments, R₈ is (*E*)-but-2-enyl. In certain other embodiments, R₈ is 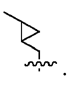 In certain embodiments, wherein R₂ is ethyl.

In certain embodiments, the compound of Formula I has the structure of Formulae (II) through (V): 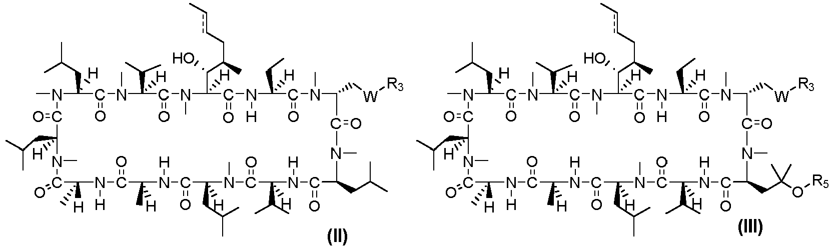 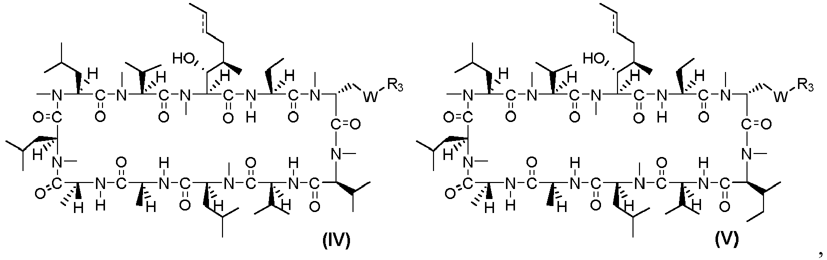 or pharmaceutically acceptable salt thereof, wherein: 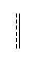
represents a single bond or double bond;
each W is independently O or S;
each R₃ is independently:
   (C₁-C₆)alkyl, optionally substituted by one or more groups R₄ which may be the same or different;
   (C₂-C₆)alkenyl, optionally substituted by one or more groups which may be the same or different selected from halogen, hydroxy, (C₁-C₆)alkyl, aryl (e.g., phenyl), (CH₂)ₚOR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{c}(CH₂)ₘNR_{c}(CH₂)ₘNR_{A}R_{B}, (CH₂)ₚC(=O)NR_{A}R_{B}, (CH₂)ₚC(=O)OR_{A};
   (C₂-C₆)alkynyl, optionally substituted by one or one or more groups which may be the same or different selected from halogen, hydroxy, amino, monoalkylamino and dialkylamino;
   (C₃-C₇)cycloalkyl, optionally substituted by one or more groups which may be the same or different selected from halogen, hydroxy, amino, monoalkylamino and dialkylamino;
   phenyl or CH₂-phenyl, optionally substituted by one or more groups which may be the same or different selected from halogen, hydroxy, (C₁-C₆)alkyl;
each R₅ is independently:
   H;
   (C₁-C₆)alkyl, optionally substituted by one or more groups R₆ which may be the same or different;
   (C₂-C₆)alkenyl, optionally substituted by one or more groups which may be the same or different selected from halogen, hydroxy, (C₁-C₆)alkyl, aryl (e.g., phenyl), (CH₂)ₚOR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{c}(CH₂)ₘNR_{c}(CH₂)ₘNR_{A}R_{B}, (CH₂)ₚC(=O)NR_{A}R_{B}, (CH₂)ₚC(=O)OR_{A};
   (C₂-C₆)alkynyl, optionally substituted by one or one or more groups which may be the same or different selected from halogen, hydroxy, amino, monoalkylamino and dialkylamino;
   (C₃-C₇)cycloalkyl, optionally substituted by one or more groups which may be the same or different selected from halogen, hydroxy, amino, monoalkylamino and dialkylamino;
   phenyl or CH₂-phenyl, optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₄ is independently halogen, hydroxy, aryl (e.g., phenyl), O(CH₂)ₘOR_{A}, O(CH₂)ₘO(CH₂)ₘOR_{A}, C(=O)(C₁-C₆)alkyl, C(=O)OR_{A}, C(=O)NR_{A}R_{B}, -NR_{A}R_{B},-NR_{C}CH₂(CH₂)ₚNR_{A}R_{B}, NR_{C}[CH₂(CH₂)ₚNR_{A}]ₘCH₂(CH₂)ₙNR_{A}R_{B}, O[CH₂(CH₂)ₚO]ₘCH₂(CH₂)ₙOR_{A}, OCH₂(CH₂)ₚNR_{A}R_{B}, or O[CH₂(CH₂)ₚO]ₘCH₂(CH₂)ₙNR_{A}R_{B};
each occurrence of R₆ is independently halogen, hydroxy, aryl (e.g., phenyl), S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl optionally substituted with from one to five groups which may be the same or different selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
   or R_{A} and R_{B}, together with the nitrogen atom to which they are attached, form -N=CH-NR_{F}R_{F'}, -N=CMe-NR_{F}R_{F'}, or -NR_{F}C(=NH)NR_{F}R_{F'};
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl;
each occurrence of R_{F} and R_{F'} is independently hydrogen, (C₁-C₆)alkyl, phenyl, benzyl, or R_{F} and R_{F'}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
p is an integer of 0, 1, 2, 3, 4, 5, or 6;
m is an integer of 1, 2, 3, 4, 5, or 6; and
n is an integer of 1, 2, 3, 4, 5 or 6.

In certain embodiments, W is O. In certain other embodiments, W is S.

In certain embodiments, m is 1. In certain other embodiments, m is 2. In yet other embodiments, m is 3. In yet other embodiments, m is 4 or 5.

In certain embodiments, p is 0. In certain other embodiments, p is 1. In yet other embodiments, m is 2. In yet other embodiments, m is 3, 4 or 5.

In certain embodiments, R₃ is H, methyl, ethyl, n-propyl, i- propyl, n-butyl, i-butyl, t-butyl, CH₂CMe₃, phenyl, CH₂-phenyl,

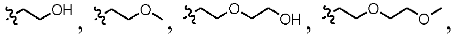

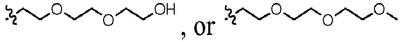

In certain embodiments, R₃ is -(CH₂)ₙNR_{A}R_{B}, wherein n is an integer of 2, 3, 4, 5 or 6; and wherein each occurrence of R_{A} and R_{B} is independently hydrogen; (C₁-C₄)alkyl, optionally substituted by one or more groups R_{D} which may be the same or different, in which each occurrence of R_{D} is independently halogen, hydroxy, O(C₁-C₄)alkyl, C(=O)(C₁-C₄)alkyl, C(=O)O(C₁-C₄)alkyl; or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from (C₁-C₄)alkyl, phenyl and benzyl.

In certain embodiments, R₃ is -(CH₂)ₙNR_{A}R_{B}, wherein n is an integer of 2, 3, 4, 5 or 6; and wherein R_{A} and R_{B}, together with the nitrogen atom to which they are attached, form a saturated or unsaturated heterocyclic ring containing from three to seven ring atoms, which ring may optionally contain another heteroatom selected from nitrogen, oxygen and sulfur and may be optionally substituted by from one to four groups which may be the same or different selected from (C₁-C₄)alkyl, phenyl and benzyl.

In certain embodiments, n is 2. In certain other embodiments, n is 3. In yet other embodiments, n is 4, 5, or 6.

In certain embodiments, R₃ is 2-aminoethyl, 2-aminopropyl, 3-aminopropyl, 2-monoalkylaminoethyl, 2-monoalkylaminopropyl, 3-monoalkylaminopropyl, 2-dialkylaminoethyl, 2-dialkylaminopropyl, or 3-dialkylaminopropyl, wherein said alkyl is (C₁-C₄)alkyl.

In certain embodiments, R₃ is 2-aminoethyl, 2-aminopropyl, 3-aminopropyl, 2-monoalkylaminoethyl, 2-monoalkylaminopropyl, 3-monoalkylaminopropyl, 2-dialkylaminoethyl, 2-dialkylaminopropyl, or 3-dialkylaminopropyl, wherein said alkyl is (C₁-C₄)alkyl, wherein R₃ is dimethylamino ethyl, diethylaminoethyl, methylethylaminoethyl, methyl-iso-butylaminoethyl, ethyl-iso-butylaminoethyl, methyl-tert-butylaminoethyl, or ethyl-tert-butylaminoethyl.

In certain embodiments, R₃ is 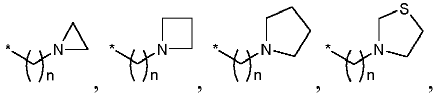 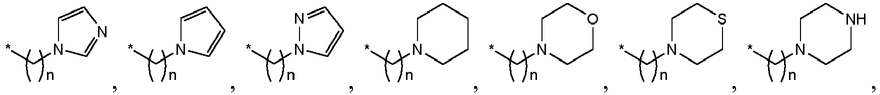 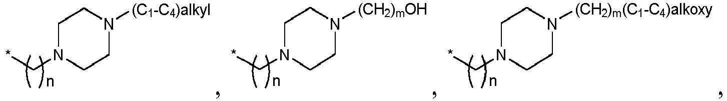 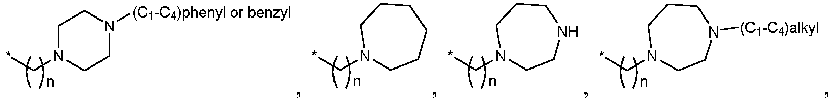 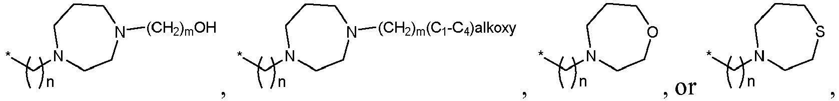 in which n is an integer of 2, 3, 4, 5, or 6, and m is an integer of 2, 3, or 4. In certain embodiments, n is 2. In certain other embodiments, n is 3. In yet other embodiments, n is 4, or 5, or 6. In certain embodiments, m is 2. In certain other embodiments, m is 3. In certain other embodiments, m is 4.

In certain embodiments, R₅ is H, (C₁-C₆)alkyl, (C₂-C₆)alkenyl, phenyl, benzyl, CH₂-S-(C₁-C₆)alky, CH₂-O-(C₁-C₆)alkyl, (C₂-C₆)OR_{A}, (C₁-C₆)-monoalkyl amine, (C₁-C₆)-dialkyl amine, or (C₁-C₆)-cyclic amine, in which said phenyl or benzyl is optionally substituted by one to three substitutents selected from (C₁-C₄)alkyl, (C₁-C₄)alkoxy, and halogen; and R_{A} is H, (C₁-C₆)alkyl, phenyl, CH₂-phenyl, (C₁-C₆)alkylOH, (CH₂)ₚO(CH₂)ₘOH, (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl; p is an integer of 0, 1, 2, 3, 4, or 5; and m is an integer of 1, 2, 3, 4 or 5.

In certain embodiments, R₅ is H. In certain other embodiments, R₅ is methyl. In yet other embodiments, R₅ is CH₂-S-(C₁-C₆)alky, e.g., CH₂-S-CH₃. In yet other embodiments, R₅ is CH₂-O-(C₁-C₆)alkyl, e.g., CH₂-O-CH₂-CH₃. In yet other embodiments, R₅ is (C₂-C₆)alkenyl, e.g., CH₂-CH=CH₂. In yet other embodiments, R₅ is benzyl. In yet other embodiments, R₅ is (C₂-C₆)OH. In yet other embodiments, R₅ is (C₁-C₆)-monoalkyl amine, e.g., CH₂-NH-Me. In yet other embodiments, R₅ is (C₁-C₆)-dialkyl amine, e.g., CH₂-CH₂-N(Et)₂. In yet other embodiments, R₅ is (C₁-C₆)-cyclic amine, e.g., CH₂-CH₂-morpholine.

In certain embodiments, each occurrence R_{A} and R_{B} is independently H, (C₁-C₆)alkyl, phenyl, CH₂-phenyl, (C₁-C₆)alkylOH, (CH₂)ₚO(CH₂)ₘOH, or (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl. In certain other embodiments, R_{A} and R_{B}, together with the nitrogen atom to which they are attached, form a heterocycle selected from 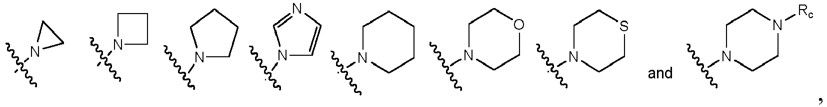 in which R_{C} is H, Me, Et, n-Pr, i-Pr, n-Bu, i-Bu, t-Bu, CH₂CMe₃, Ph, CH₂Ph, CH₂CH₂OH, or CH₂CH₂O(C₁-C₄)alkyl.

In certain embodiments, wherein 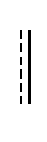 represents a single bond. In certain other embodiments, wherein 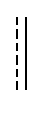 represents a double bond.

In another aspect, the present invention provides a compound of Formulae (IIa)-(Va): 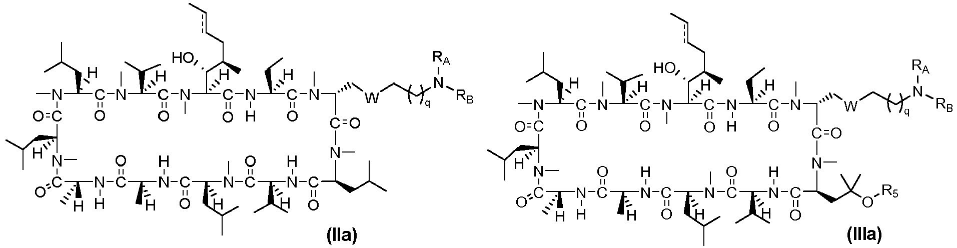 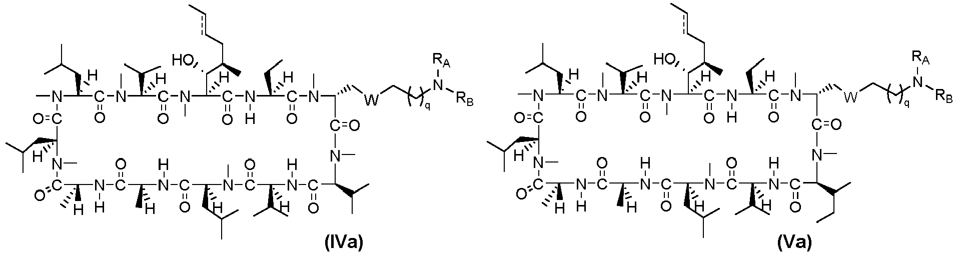 or a pharmaceutically acceptable salt thereof, wherein: 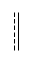
represents a single bond or double bond;
each W is independently O or S;
each R₅ is independently:
   H;
   (C₁-C₆)alkyl, optionally substituted by one or more groups R₆ which may be the same or different;
   (C₂-C₆)alkenyl, optionally substituted by one or more groups which may be the same or different selected from halogen, hydroxy, (C₁-C₆)alkyl, aryl (e.g., phenyl), (CH₂)ₚOR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B},
   (CH₂)ₚNR_{A}R_{B}, (CH₂)ₚNR_{C}(CH₂)ₘNR_{A}R_{B}, (CH₂)ₚNR_{C}(CH₂)ₘNR_{c}(CH₂)ₘNR_{A}R_{B}, (CH₂)ₚC(=O)NR_{A}R_{B}, (CH₂)ₚC(=O)OR_{A};
   (C₂-C₆)alkynyl, optionally substituted by one or one or more groups which may be the same or different selected from halogen, hydroxy, amino, monoalkylamino and dialkylamino;
   (C₃-C₇)cycloalkyl, optionally substituted by one or more groups which may be the same or different selected from halogen, hydroxy, amino, monoalkylamino and dialkylamino;
   phenyl or CH₂-phenyl, optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₆ is independently halogen, hydroxy, aryl (e.g., phenyl), S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl optionally substituted with from one to five groups which may be the same or different selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl;
each p is independently an integer of 0, 1, 2, 3, 4, or 5; and
each of m, n and q is independently an integer of 1, 2, 3, 4 or 5.

In certain embodiments, the compound of Formula I has the structure of Formulae (II) through (V): 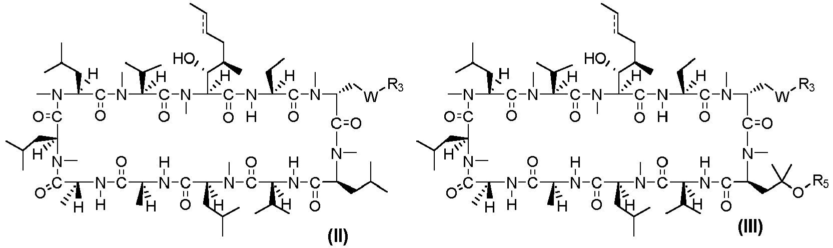 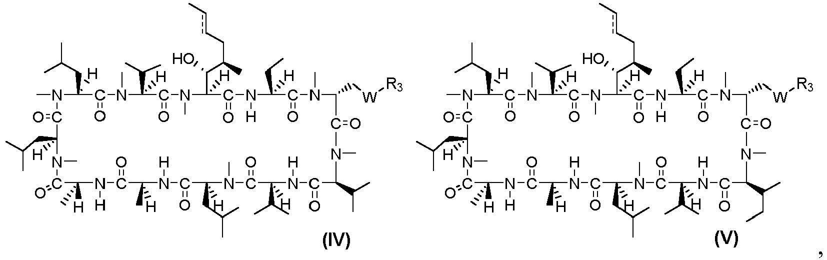 or a pharmaceutically acceptable salt thereof, wherein: 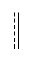
represents a single bond or double bond;
each W is independently O or S;
each R₃ is independently 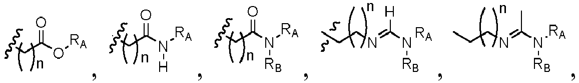 or 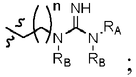
each R₅ is independently:
   H;
   (C₁-C₆)alkyl, optionally substituted by one or more groups R₆ which may be the same or different;
   (C₂-C₆)alkenyl, optionally substituted by one or more groups which may be the same or different selected from halogen, hydroxy, (C₁-C₆)alkyl, aryl (e.g., phenyl), (CH₂)ₚOR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{c}(CH₂)ₘNR_{A}R_{B}, (CH₂)ₚC(=O)NR_{A}R_{B}, (CH₂)ₚC(=O)OR_{A};
   (C₂-C₆)alkynyl, optionally substituted by one or one or more groups which may be the same or different selected from halogen, hydroxy, amino, monoalkylamino and dialkylamino;
   (C₃-C₇)cycloalkyl, optionally substituted by one or more groups which may be the same or different selected from halogen, hydroxy, amino, monoalkylamino and dialkylamino;
   phenyl or CH₂-phenyl, optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₆ is independently halogen, hydroxy, aryl (e.g., phenyl), S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
   hydrogen;
   (C₁-C₆)alkyl, optionally substituted by one or more groups R_{D} which may be the same or different;
   (C₂-C₆)alkenyl or (C₂-C₆)alkynyl;
   (C₃-C₇)cycloalkyl optionally substituted with (C₁-C₆)alkyl;
   phenyl optionally substituted with from one to five groups which may be the same or different selected from halogen, -O(C₁-C₆)alkyl, -C(=O)O(C₁-C₆)alkyl, amino, alkylamino and dialkylamino;
   or a heterocyclic ring which may be saturated or unsaturated containing five or six ring atoms and from one to three heteroatoms which may be the same or different selected from nitrogen, sulfur and oxygen;
   or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl;
each p is independently an integer of 0, 1, 2, 3, 4, or 5; and
each of m, n and q is independently an integer of 1, 2, 3, 4 or 5.

In certain embodiments, q is 1. In certain other embodiments, q is 2.

In certain embodiments, W is S. In certain other embodiments, W is O.

In certain embodiments, R₃ is (C₁-C₆)alkyl. In certain other embodiments, R₃ is NR_{C}CH₂(CH₂)ₚNR_{A}R_{B}.

In certain embodiments, R₅ is H, (C₁-C₆)alkyl, (C₂-C₆)alkenyl, phenyl, benzyl, CH₂-S-(C₁-C₆)alkyl, CH₂-O-(C₁-C₆)alkyl, (C₂-C₆)OR_{A}, (C₁-C₆)-monoalkyl amine, (C₁-C₆)-dialkyl amine, or (C₁-C₆)-cyclic amine, in which said phenyl or benzyl is optionally substituted by one to three substitutents selected from (C₁-C₄)alkyl, (C₁-C₄)alkoxy, and halogen; and R_{A} is H, (C₁-C₆)alkyl, phenyl, CH₂-phenyl, (C₁-C₆)alkylOH, (CH₂)ₚO(CH₂)ₘOH, (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl; p is an integer of 0, 1, 2, 3, 4, or 5; and m is an integer of 1, 2, 3, 4 or 5.

In certain other embodiments, R₅ is H, (C₁-C₄)alkyl, (C₂-C₄)alkenyl, phenyl, benzyl, CH₂-S-(C₁-C₄)alkyl, CH₂-O-(C₁-C₄)alkyl, (CH₂)₂OH, or (CH₂)₂O(C₁-C₄)alkyl. In certain embodiments, R₅ is H. In certain other embodiments, R₅ is methyl.

In certain embodiments, each occurrence R_{A} and R_{B} is independently H, (C₁-C₆)alkyl, phenyl, CH₂-phenyl, (C₁-C₆)alkylOH, (CH₂)ₚO(CH₂)ₘOH, or (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl. In certain other embodiments, each occurrence R_{A} and R_{B} is independently H or (C₁-C₆)alkyl. In yet other embodiments, R_{A} and R_{B}, together with the nitrogen atom to which they are attached, form a heterocycle selected from 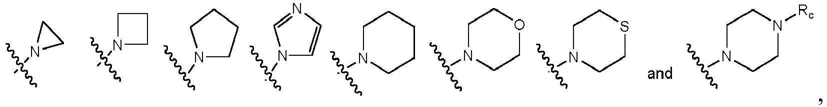 in which R_{C} is H, Me, Et, n-Pr, i-Pr, n-Bu, i-Bu, t-Bu, CH₂CMe₃, Ph, CH₂Ph, or CH₂CH₂OH and CH₂CH₂OR_{d}.

In one aspect, the present disclosure (compounds additional to those claimed may be disclosed herein) provides a compound selected from the following: 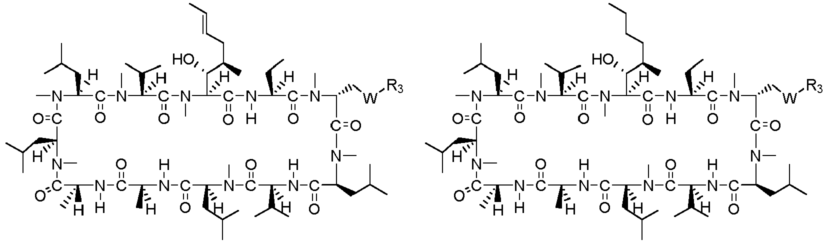
W=O, S, N-H, and N-Rₐ
R₃ = H, Me, Et, n-Pr, i-Pr, n-Bu, i-Bu, t-Bu, CH₂CMe₃, Ph, CH₂Ph 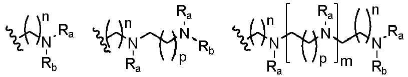 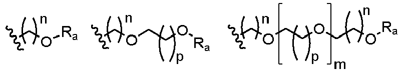 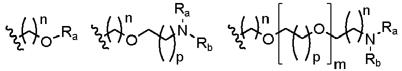 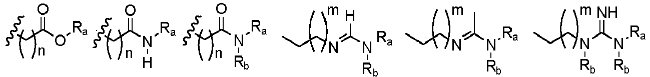
n = 2, 3, 4, 5, 6
m = 1, 2, 3, 4, 5, 6
p = 1, 2, 3, 4, 5, 6
Rₐ = H, Me, Et, n-Pr, i-Pr, n-Bu, i-Bu, t-Bu, CH₂CMe₃, Ph, CH₂Ph 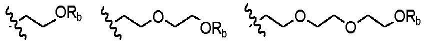 R_{b} = H, Me, Et, n-Pr, i-Pr, n-Bu, i-Bu, t-Bu, CH₂CMe₃, Ph, CH₂Ph 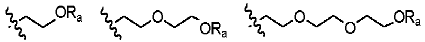 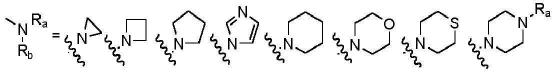 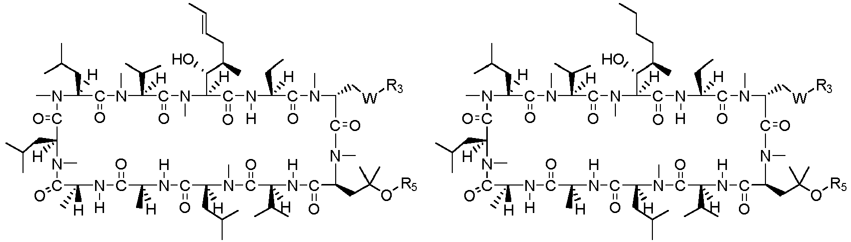
W=O, S, N-H, or N-Rₐ
R₃ = H, Me, Et, n-Pr, i-Pr, n-Bu, i-Bu, t-Bu, CH₂CMe₃, Ph, CH₂Ph 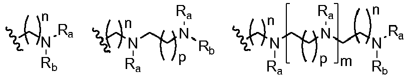 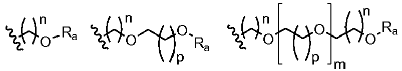 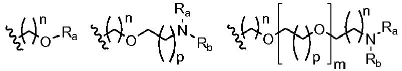 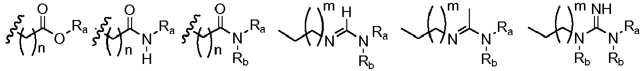
n = 2, 3, 4, 5, 6
m = 1, 2, 3, 4, 5, 6
p = 1, 2, 3, 4, 5, 6
Rₐ = H, Me, Et, n-Pr, i-Pr, n-Bu, i-Bu, t-Bu, CH₂CMe₃, Ph, CH₂Ph 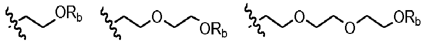 R_{b} = H, Me, Et, n-Pr, i-Pr, n-Bu, i-Bu, t-Bu, CH₂CMe₃, Ph, CH₂Ph 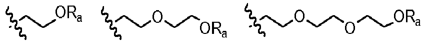 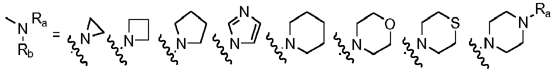 R₅ = H, Me, Et, n-Pr, i-Pr, n-Bu, i-Bu, t-Bu, CH₂CMe₃, Ph, CH₂Ph 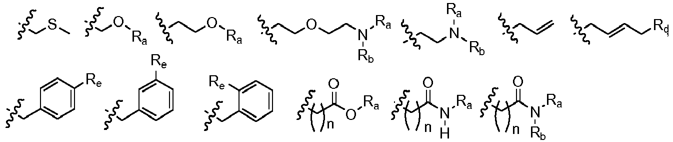
R_{d} = Rₐ, ORₐ, R₃
Rₑ = H, Me, Et, ORₐ, R₃, CH₂ORₐ, CH₂CH₂ORₐ,

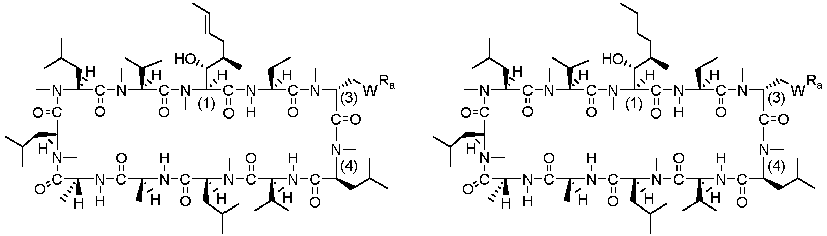

W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 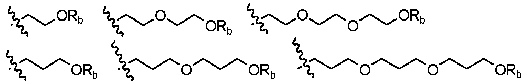 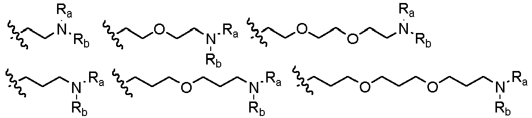 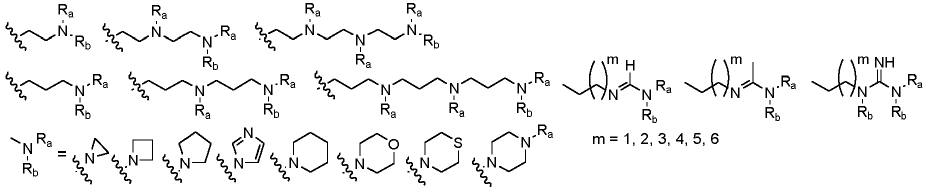 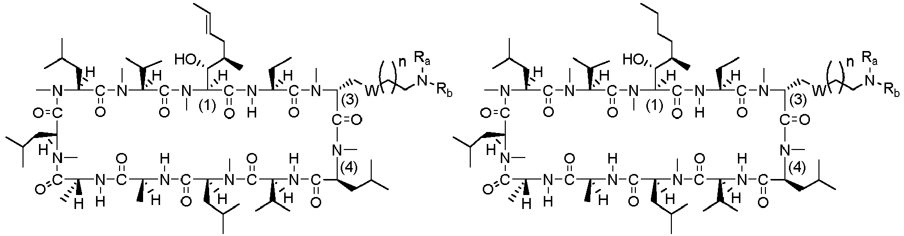
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 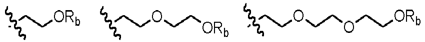
R_{b} = H, Me, Et, n-Pr, i-Pr, n-Bu, i-Bu, t-Bu, CH₂CMe₃, Ph, CH₂Ph 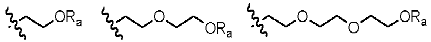 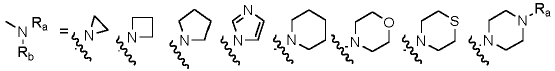 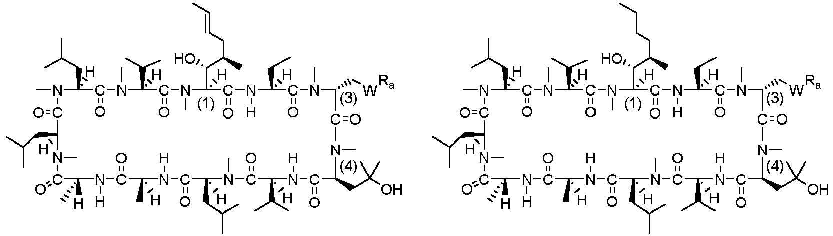
W=O, S, N-H, or N-Rₐ
Rₐ = H, Me, Et, n-Pr, i-Pr, n-Bu, i-Bu, t-Bu, CH₂CMe₃, Ph, CH₂Ph R_{b} = H, Me, Et, n-Pr, i-Pr, n-Bu, i-Bu, t-Bu, CH₂CMe₃, Ph, CH₂Ph 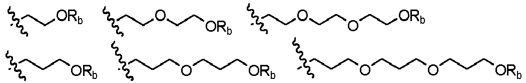 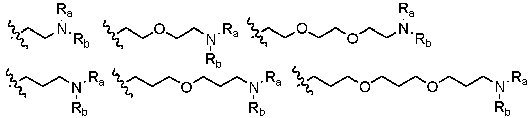 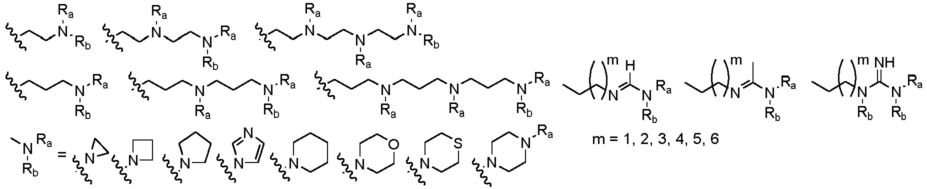 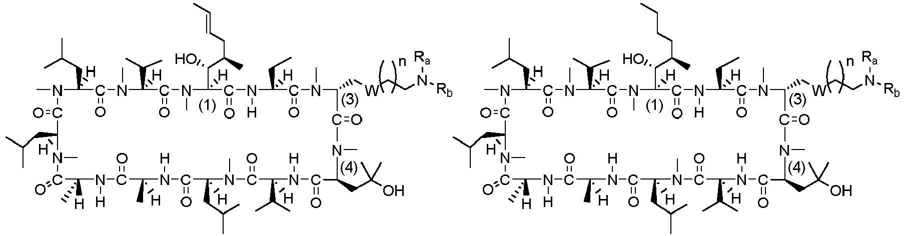
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 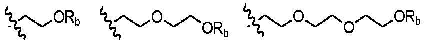
R_{b} = H, Me, Et, n-Pr, i-Pr, n-Bu, i-Bu, t-Bu, CH₂CMe₃, Ph, CH₂Ph 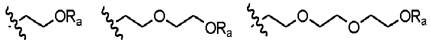 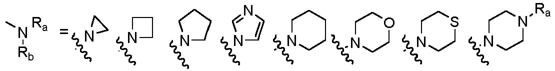 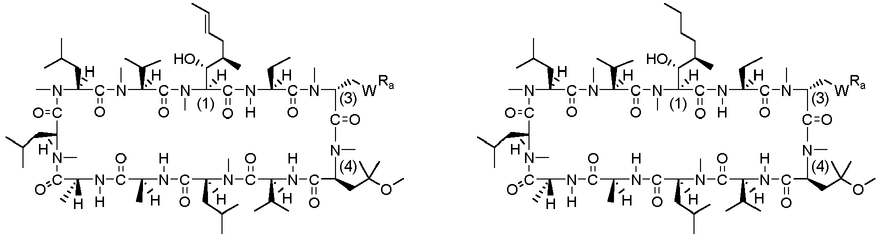
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 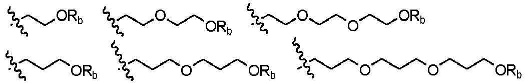 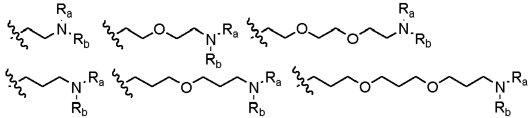 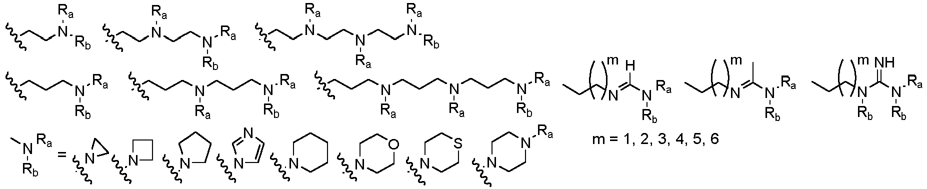 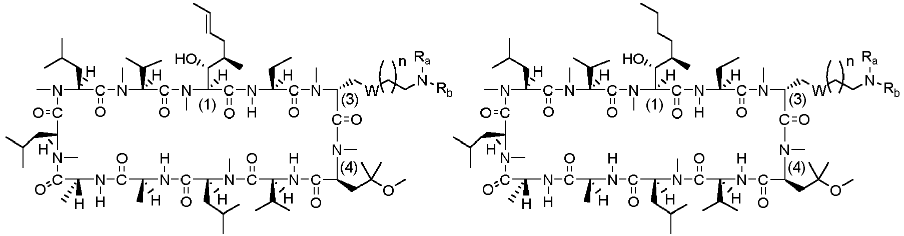
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 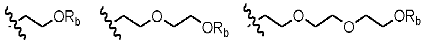
R_{b} = H, Me, Et, n-Pr, i-Pr, n-Bu, i-Bu, t-Bu, CH₂CMe₃, Ph, CH₂Ph 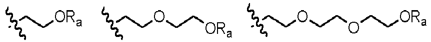 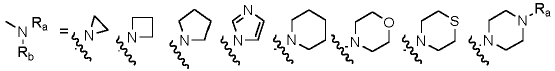 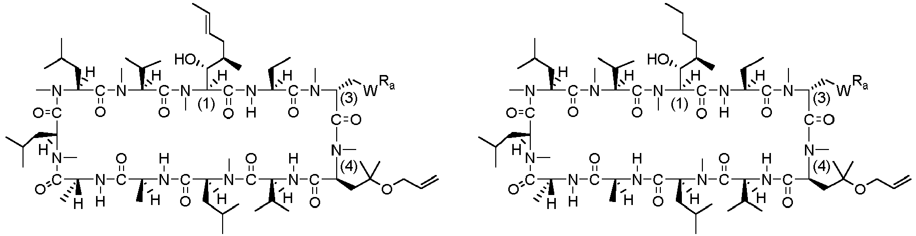
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 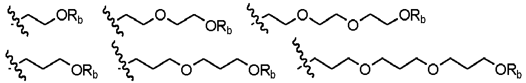 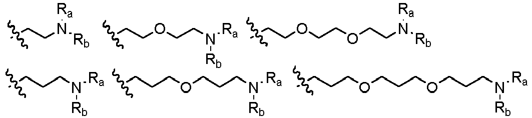 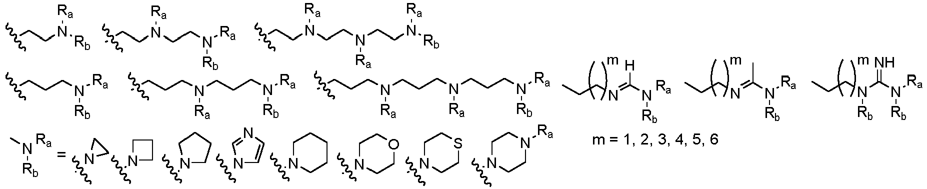 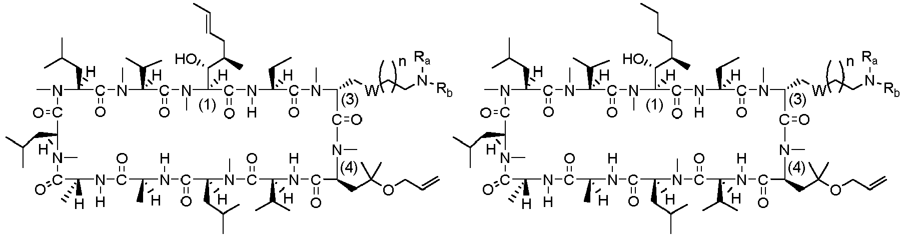
W=O, S, N-H, or N-Rₐ
n = 1, 2, 3, 4, 5, 6
Rₐ = H, Me, Et, n-Pr, i-Pr, n-Bu, i-Bu, t-Bu, CH₂CMe₃, Ph, CH₂Ph 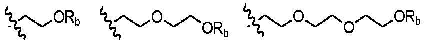
R_{b} = H, Me, Et, n-Pr, i-Pr, n-Bu, i-Bu, t-Bu, CH₂CMe₃, Ph, CH₂Ph 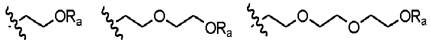 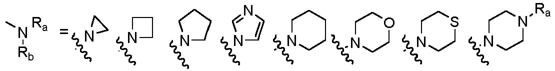 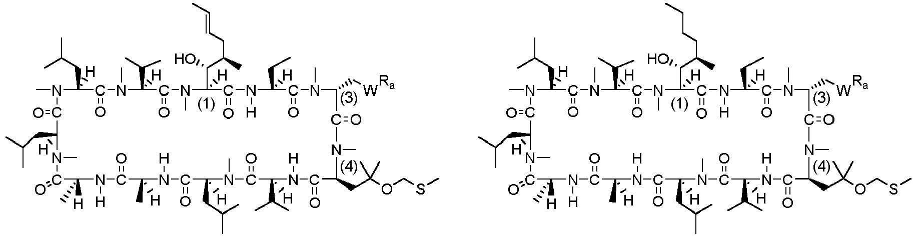
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 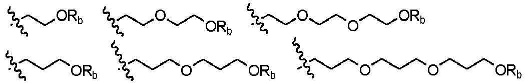 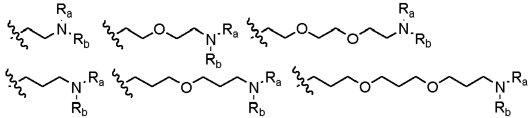 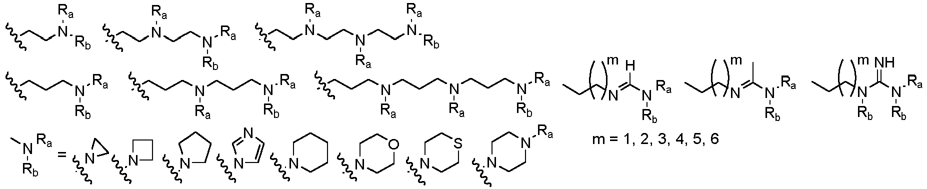 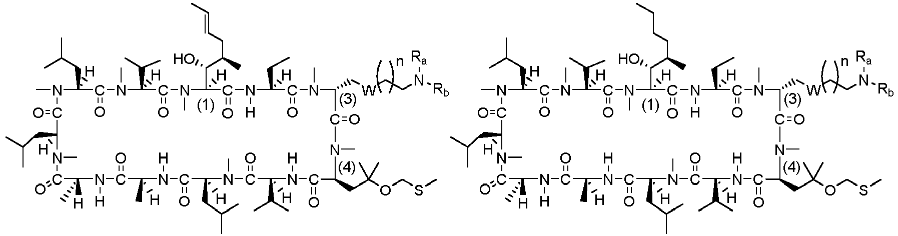
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 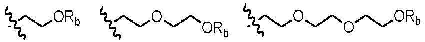
R_{b} = H, Me, Et, n-Pr, i-Pr, n-Bu, i-Bu, t-Bu, CH₂CMe₃, Ph, CH₂Ph 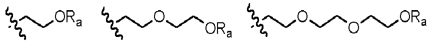 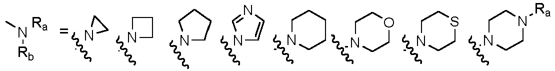 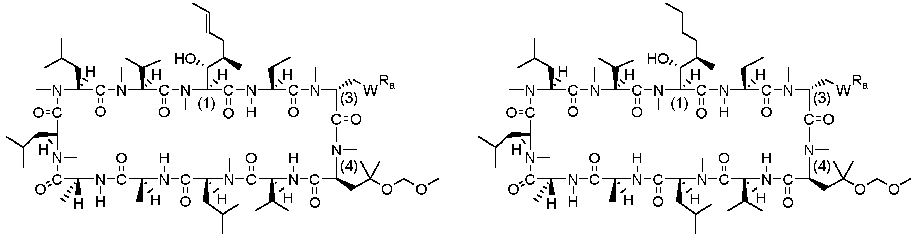
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 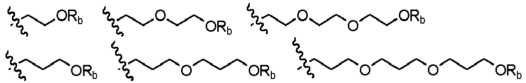 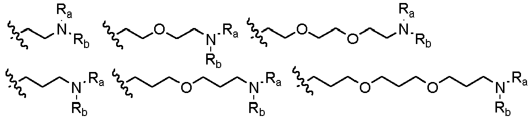 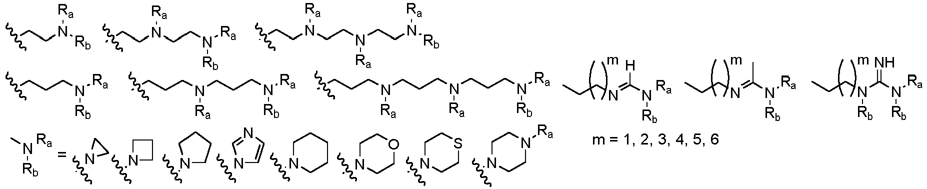 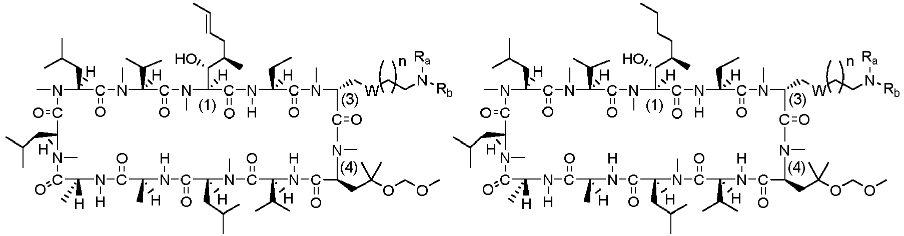
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 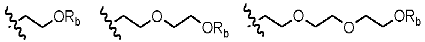
R_{b} = H, Me, Et, n-Pr, i-Pr, n-Bu, i-Bu, t-Bu, CH₂CMe₃, Ph, CH₂Ph 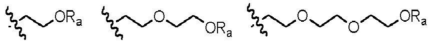 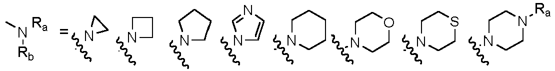 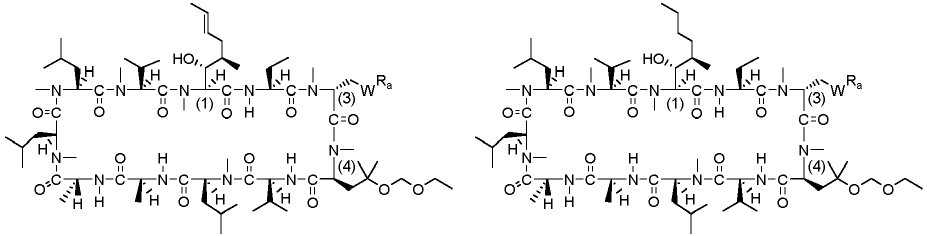
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 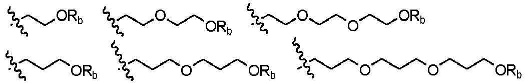 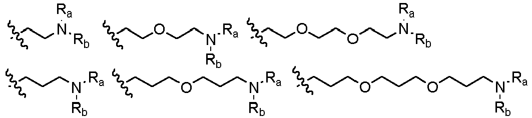 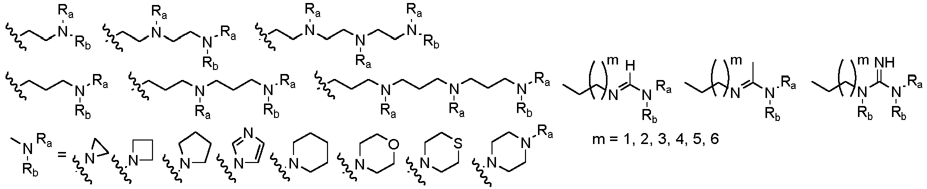 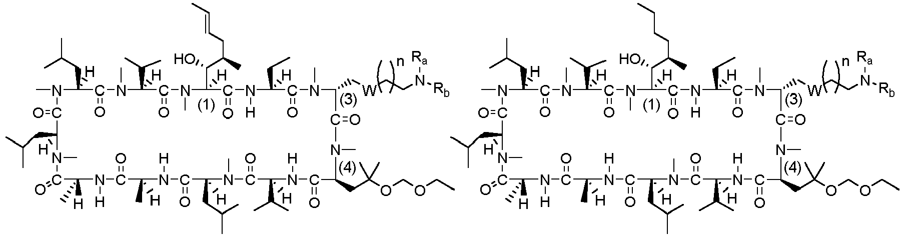
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 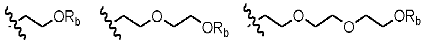
R_{b} = H, Me, Et, n-Pr, i-Pr, n-Bu, i-Bu, t-Bu, CH₂CMe₃, Ph, CH₂Ph 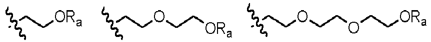 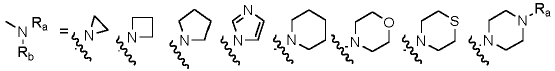 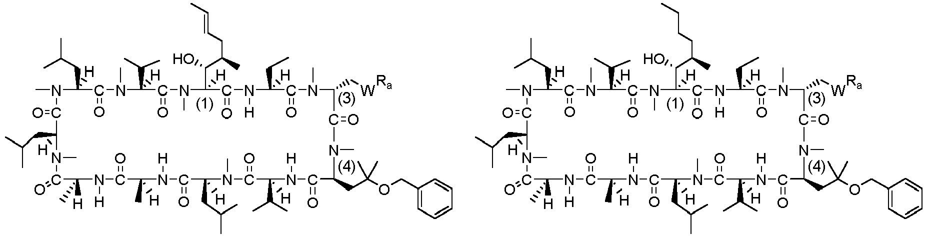
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 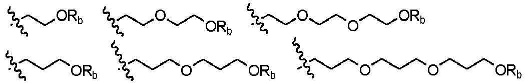 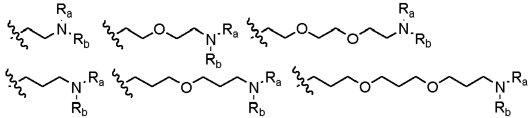 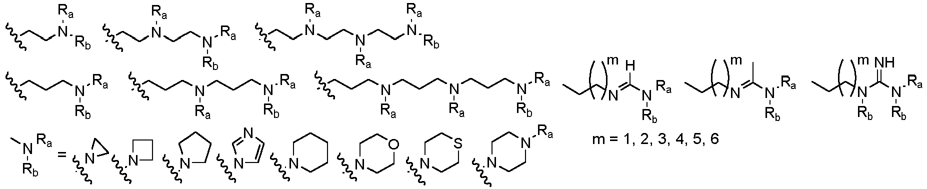 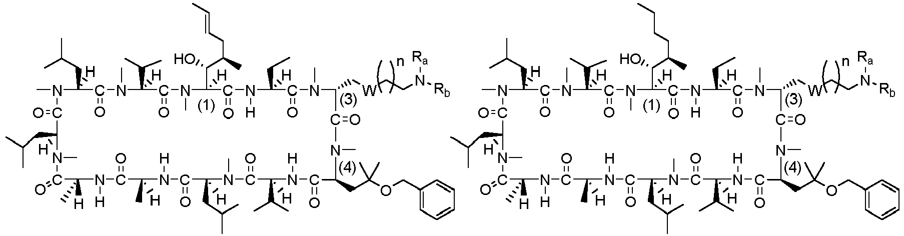
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 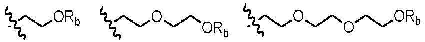
R_{b} = H, Me, Et, n-Pr, i-Pr, n-Bu, i-Bu, t-Bu, CH₂CMe₃, Ph, CH₂Ph 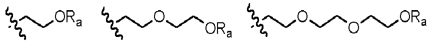 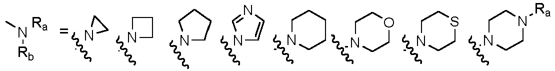 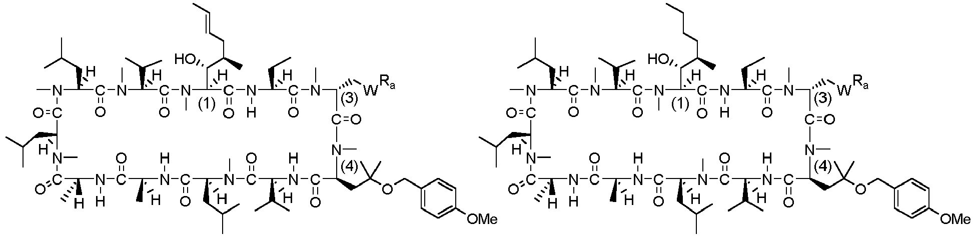
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 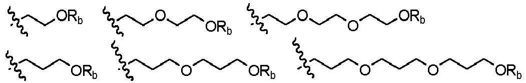 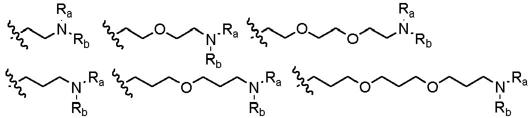 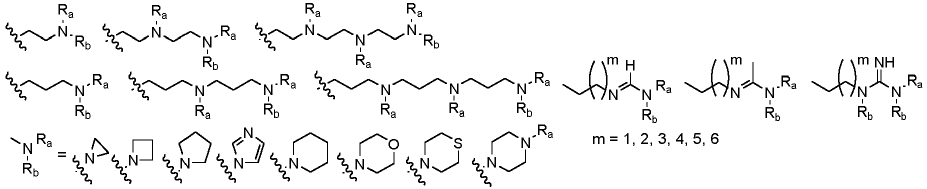 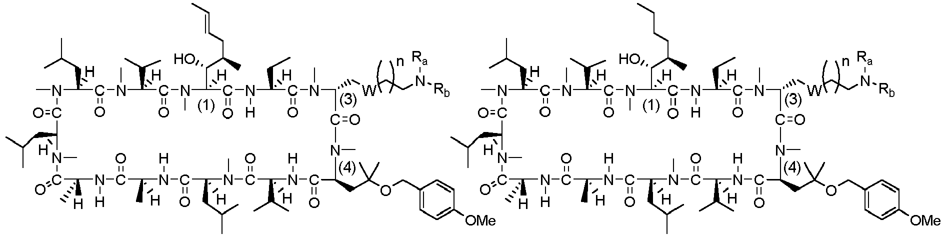
W=O, S, N-H, or N-Rₐ
n = 1, 2, 3, 4, 5, 6
Rₐ = H, Me, Et, n-Pr, i-Pr, n-Bu, i-Bu, t-Bu, CH₂CMe₃, Ph, CH₂Ph 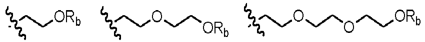
R_{b} = H, Me, Et, n-Pr, i-Pr, n-Bu, i-Bu, t-Bu, CH₂CMe₃, Ph, CH₂Ph 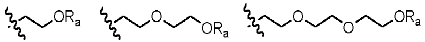 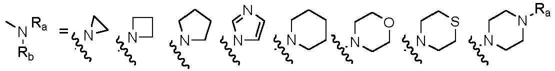 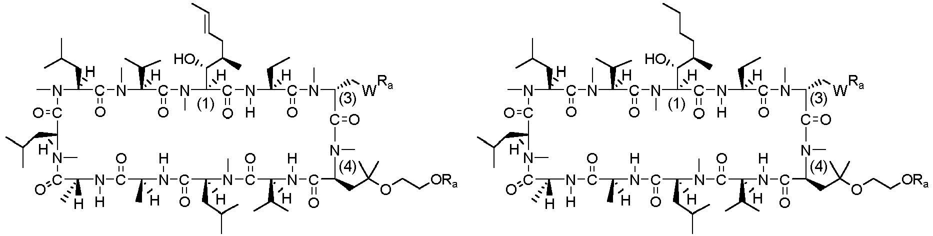
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 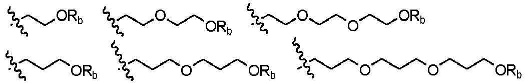 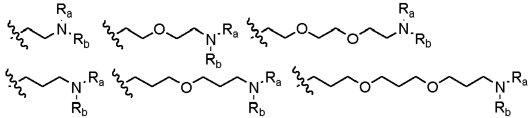 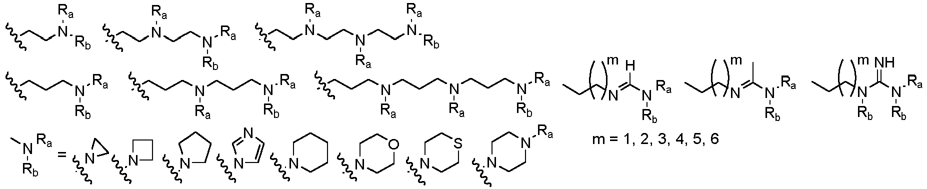 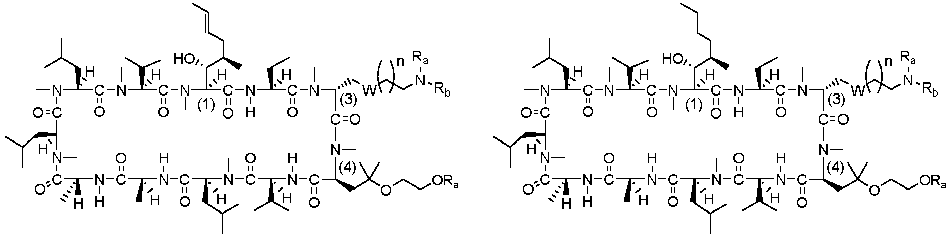
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 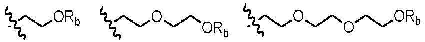
R_{b} = H, Me, Et, n-Pr, i-Pr, n-Bu, i-Bu, t-Bu, CH₂CMe₃, Ph, CH₂Ph 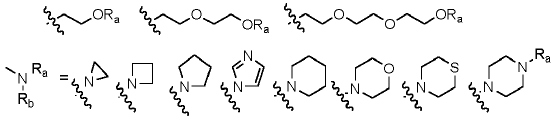 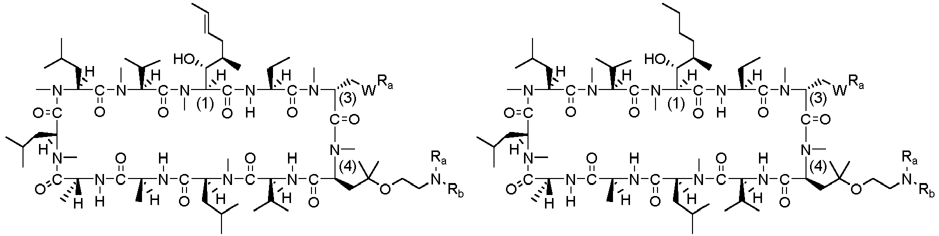
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 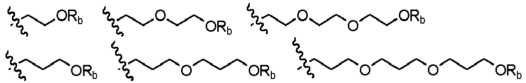 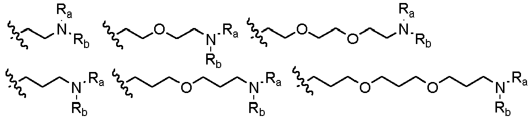 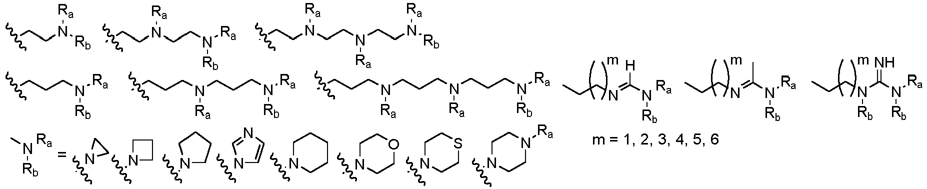 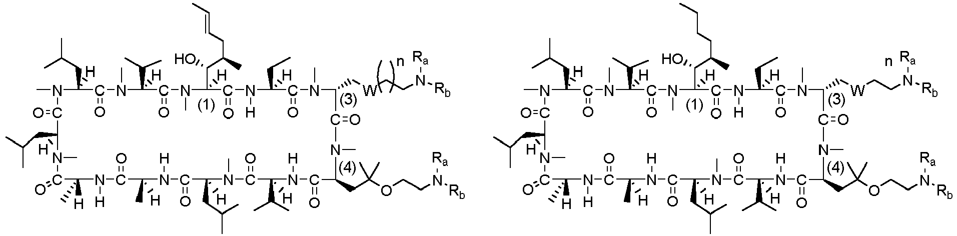
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 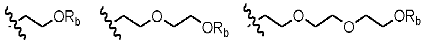
R_{b} = H, Me, Et, n-Pr, i-Pr, n-Bu, i-Bu, t-Bu, CH₂CMe₃, Ph, CH₂Ph 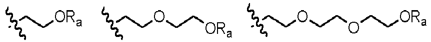 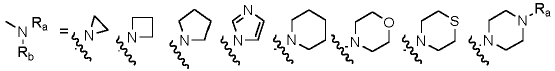 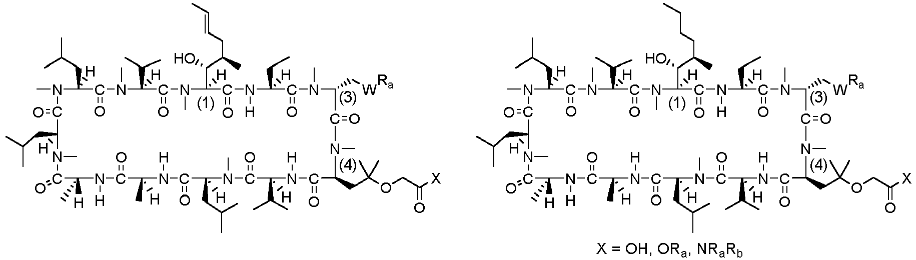
W=O, S, N-H, or N-Rₐ
Rₐ = H, Me, Et, n-Pr, i-Pr, n-Bu, i-Bu, t-Bu, CH₂CMe₃, Ph, CH₂Ph
R_{b} = H, Me, Et, n-Pr, i-Pr, n-Bu, i-Bu, t-Bu, CH₂CMe₃, Ph, CH₂Ph 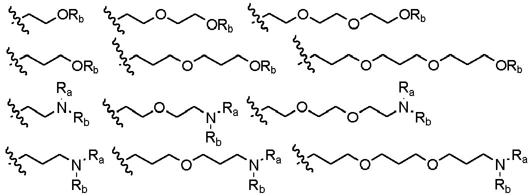 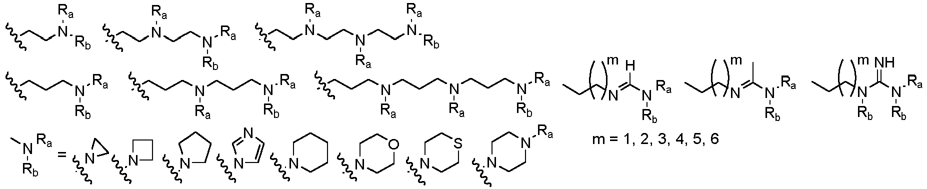 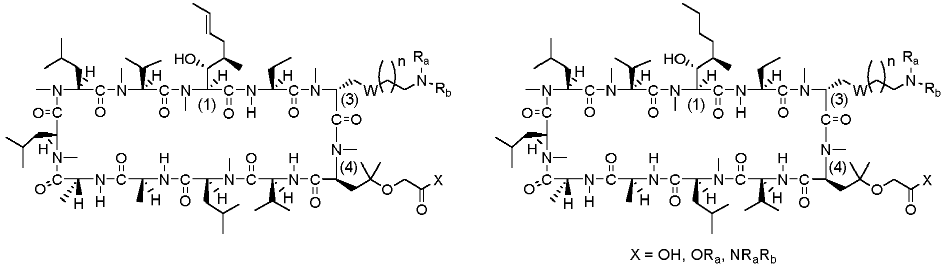
W=O, S, N-H, or N-Rₐ
n=1,2,3,4,5,6
Rₐ = H, Me, Et, n-Pr, i-Pr, n-Bu, i-Bu, t-Bu, CH₂CMe₃, Ph, CH₂Ph 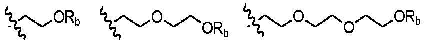
R_{b} = H, Me, Et, n-Pr, i-Pr, n-Bu, i-Bu, t-Bu, CH₂CMe₃, Ph, CH₂Ph 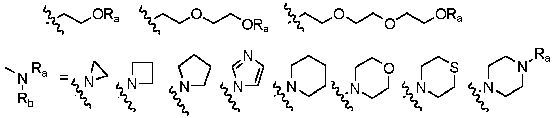
or a pharmaceutically acceptable salt thereof.

In a further aspect, the present invention provides a compound as described in the Examples.

In certain embodiments, the compound is selected from:
[(S)-(2-(N,N-Diethylamino)ethylthio)methyl-Sar]-3-[(γ-hydroxy)-N-MeLeu]-4-cyclosporin,
[(S)-(2-(N-Ethyl-N-isopropylamino)ethylthio)methyl-Sar]-3-[(γ-hydroxy)-N-MeLeu]-4-cyclosporin,
[(S)-(2-(N-Isobutylamino)ethylthio)methyl-Sar]-3-[(γ-hydroxy)-N-MeLeu]-4-cyclosporin,
[(S)-(2-(N-Ethyl-N-Isobutylamino)ethylthio)methyl-Sar]-3-[(γ-hydroxy)-N-MeLeu]-4-cyclosporin,
[(S)-(2-(N-Isobutyl-N-methylamino)ethylthio)methyl-Sar]-3-[(γ-hydroxy)-N-MeLeu]-4-cyclosporin,
[(S)-(2-(N-Neopentylamino)ethylthio)methyl-Sar]-3-[(γ-hydroxy)-N-MeLeu]-4-cyclosporin,
[(S)-(2-(N-Methyl-N-Neopentylamino)ethylthio)methyl-Sar]-3-[(γ-hydroxy)-N-MeLeu]-4-cyclosporin,
[(S)-(2-(N-Ethyl-N-neopentylamino)ethylthio)methyl-Sar]-3-[(γ-hydroxy)-N-MeLeu]-4-cyclosporin,
[(S)-(2-(N-Piperidinyl)ethylthio)methyl-Sar]-3-[(γ-hydroxy)-N-MeLeu]-4-cyclosporin,
[(S)-(2-(N-Morpholino)ethylthio)methyl-Sar]-3-[(γ-hydroxy)-N-MeLeu]-4-cyclosporin,
[(S)-(2-(N-Thiomorpholino)ethylthio)methyl-Sar]-3-[(γ-hydroxy)-N-MeLeu]-4-cyclosporin,
[(S)-(2-(4-Methyl-N-piperazinyl)ethylthio)methyl-Sar]-3-[(γ-hydroxy)-N-MeLeu]-4-cyclosporin,
[(S)-(3-(N,N-Diethylamino)propylthio)methyl-Sar]-3-[(γ-hydroxy)-N-MeLeu]-4-cyclosporin,
[(S)-(3-(N-Ethyl-N-isopropylamino)propylthio)methyl-Sar]-3-[(γ-hydroxy)-N-MeLeu]-4-cyclosporin,
[(S)-(3-(N-Neopentylamino)propylthio)methyl-Sar]-3-[(γ-hydroxy)-N-MeLeu]-4-cyclosporin,
[(S)-(3-(N-Pyrrolidinyl)propylthio)methyl-Sar]-3-[(γ-hydroxy)-N-MeLeu]-4-cyclosporin,
[(S)-(3-(N-Piperidinyl)propylthio)methyl-Sar]-3-[(γ-hydroxy)-N-MeLeu]-4-cyclosporin,
[(S)-(3-(N-Morpholino)propylthio)methyl-Sar]-3-[(γ-hydroxy)-N-MeLeu]-4-cyclosporin,
[(S)-(3-(N-Thiomorpholino)propylthio)methyl-Sar]-3-[(γ-hydroxy)-N-MeLeu]-4-cyclosporin,
[(S)-(3-(4-Methyl-N-piperazinyl)propylthio)methyl-Sar]-3-[(γ-hydroxy)-N-MeLeu]-4-cyclosporin,
[(S)-(4-(N,N-Diethylamino)butylthio)methyl-Sar]-3-[(γ-hydroxy)-N-MeLeu]-4-cyclosporin,
[(S)-(4-(N-Ethyl-N-isopropylamino)butylthio)methyl-Sar]-3-[(γ-hydroxy)-N-MeLeu]-4-cyclosporin,
[(S)-(4-(N-Isobutylamino)butylthio)methyl-Sar]-3-[(γ-hydroxy)-N-MeLeu]-4-cyclosporin,
[(S)-(4-(N-Isobutyl-N-methylamino)butylthio)methyl-Sar]-3-[(γ-hydroxy)-N-MeLeu]-4-cyclosporin,
[(S)-(4-(N-Ethyl-N-isobutylamino)butylthio)methyl-Sar]-3-[(γ-hydroxy)-N-MeLeu]-4-cyclosporin,
[(S)-(4-(N-Neopentylamino)butylthio)methyl-Sar]-3-[(γ-hydroxy)-N-MeLeu]-4-cyclosporin,
[(S)-(4-(N-Methyl-N-neopentylamino)butylthio)methyl-Sar]-3-[(γhydroxy)-N-MeLeu]-4-cyclosporin,
[(S)-(4-(N-Piperidinyl)butylthio)methyl-Sar]-3-[(γ-hydroxy)-N-MeLeu]-4-cyclosporin,
[(S)-(4-(N-Morpholino)butylthio)methyl-Sar]-3-[(γ-hydroxy)-N-MeLeu]-4-cyclosporin,
[(S)-(4-(N-Thiomorpholino)butylthio)methyl-Sar]-3-[(γ-hydroxy)-N-MeLeu]-4-cyclosporin,
[(S)-(4-(4-Methyl-N-piperazinyl)butylthio)methyl-Sar]-3-[(γ-hydroxy)-N-MeLeu]-4-cyclosporin,
[(R)-(3-(N-Morphlino)propoxy)methyl-Sar]-3-[(γ-hydroxy)-N-MeLeu]-4-cyclosporin,
[(S)-(2-(N,N-Diethylamino)ethylthio)methyl-Sar]-3-[(γ-methoxy)-N-MeLeu]-4-cyclosporin,
[(S)-(2-(N-Pyrrolidinyl)ethylthio)methyl-Sar]-3-[(γ-methoxy)-N-MeLeu]-4-cyclosporin,
[(S)-(3-(N,N-Dimethylamino)propylthio)methyl-Sar]-3-[(γ-methoxy)-N-MeLeu]-4-cyclosporin,
[(S)-(3-(N,N-Diethylamino)propylthio)methyl-Sar]-3-[(γ-methoxy)-N-MeLeu]-4-cyclosporin,
[(S)-(3-(N-Piperidinyl)propylthio)methyl-Sar]-3-[(γ-methoxy)-N-MeLeu]-4-cyclosporin,
[(S)-(4-(N-Piperidinyl)butylthio)methyl-Sar]-3-[(γ-methoxy)-N-MeLeu]-4-cyclosporin,
[(R)-(2-(N,N-Diethylamino)ethoxy)methyl-Sar]-3-[(γ-methoxy)-N-MeLeu]-4-cyclosporin, and
[(R)-(3-(N,N-Diethylamino)propoxy)methyl-Sar]-3-[(γ-methoxy)-N-MeLeu]-4-cyclo sporin.

In another aspect, the present invention provides a pharmaceutical composition comprising at least one compound described herein and a pharmaceutically-acceptable carrier or diluent.

In a further aspect, the present invention provides a compound for use in treating or preventing a viral infection, hepatitis C virus infection or HIV infection in a mammalian species.

### Methods of Preparation

In certain embodiments, the compound of formulae (I) and (II) can be prepared by treating cyclosporin A or an analog thereof with a base (*e.g.,* LDA) to form a sarcosine enolate at 3-position, and then CO₂ gas is introduced to yield carboxylic acid-3-cyclosporin. After formation of its corresponding methyl ester and reduction of the methyl ester side chain to alcohol, its mesylate or chloride can be produced by treatment with MsCl in dichloromethane solution. Both of mesylate and chloride can be converted to methylene on the sarcosine by treatment with a base (*e.g.*, NaH). When sulfur nuclectrophile is used for 1,4-addition reaction on the methylene group, the methylene sulfur ether chain with S-conformation can be formed on the sarcosine of position 3 as novel anti-viral cyclosporine derivatives. For example:

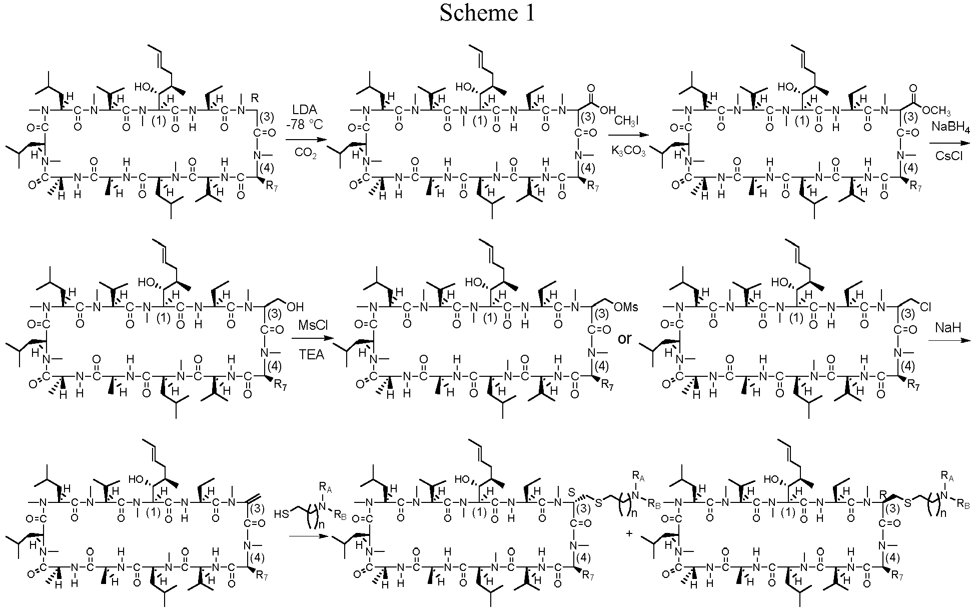

In certain embodiments, the above resulting alcohol can be converted to its methylene oxygen ether side chain to form new anti-viral cyclosporine derivatives. For example:

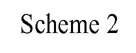

In Schemes 1-2 above, the symbols have the same meaning as defined in the claims and throughout the specification, unless otherwise noted.

In certain other embodiments, the compound of formula (IIa) or (IIIa) can be obtained according to the procedures described herein.

### Pharmaceutical Compositions

This invention also provides a pharmaceutical composition comprising at least one of the compounds as described herein or a pharmaceutically-acceptable salt or solvate thereof, and a pharmaceutically-acceptable carrier.

The phrase "pharmaceutically-acceptable carrier" as used herein means a pharmaceutically-acceptable material, composition or vehicle, such as a liquid or solid filler, diluent, excipient, solvent or encapsulating material, involved in carrying or transporting the subject pharmaceutical agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as butylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

As set out above, certain embodiments of the present pharmaceutical agents may be provided in the form of pharmaceutically-acceptable salts. The term "pharmaceutically-acceptable salt", in this respect, refers to the relatively non-toxic, inorganic and organic acid addition salts of compounds of the present invention. These salts can be prepared *in situ* during the final isolation and purification of the compounds of the invention, or by separately reacting a purified compound of the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, for example, Berge et al., (1977) "Pharmaceutical Salts", J. Pharm. Sci. 66:1-19).

The pharmaceutically acceptable salts of the subject compounds include the conventional nontoxic salts or quaternary ammonium salts of the compounds, e.g., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, butionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In other cases, the compounds of the present invention may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge *et al.,* supra)

Wetting agents, emulsifiers and lubricants, such as sodium lauryl sulfate, magnesium stearate, and polyethylene oxide-polybutylene oxide copolymer as well as coloring agents, release agents, coating agents, sweetening, flavoring and perfuming agents, preservatives and antioxidants can also be present in the compositions.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated and the particular mode of administration. The amount of active ingredient, which can be combined with a carrier material to produce a single dosage form will generally be that amount of the compound which produces a therapeutic effect. Generally, out of 100%, this amount will range from about 1% to about 99% of active ingredient, preferably from about 5% to about 70%, most preferably from about 10% to about 30%.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically-acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, sodium carbonate, and sodium starch glycolate; solution retarding agents, such as paraffin; absorption accelerators, such as quaternary ammonium compounds; wetting agents, such as, for example, cetyl alcohol, glycerol monostearate, and polyethylene oxide-polybutylene oxide copolymer; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxybutylmethyl cellulose), lubricant, inert diluent, preservative, disintegrant (for example, sodium starch glycolate or cross-linked sodium carboxymethyl cellulose), surface-active or dispersing agent. Molded tablets, may be, made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxybutylmethyl cellulose in varying butortions to provide the desired release profile, other polymer matrices, liposomes and/or microspheres. They may be sterilized by, for example, filtration through a bacteria-retaining filter, or by incorporating sterilizing agents in the form of sterile solid compositions, which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples are embedding compositions, which can be used include polymeric substances and waxes. The active ingredient can also be in micro-encapsulated form, if apbutriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isobutyl alcohol, ethyl carbonate, ethyl acetate, benzyl alcohol, benzyl benzoate, butylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. Additionally, cyclodextrins, e.g., hydroxybutyl-.beta.-cyclodextrin, may be used to solubilize compounds.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active pharmaceutical agents of the invention.

Formulations of the present invention which are suitable for vaginal administration also include pessaries, tampons, creams, gels, pastes, foams or spray formulations containing such carriers as are known in the art to be apbutriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically-acceptable carrier, and with any preservatives, buffers, or butellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary butellants, such as chlorofluorohydrocarbons and volatile unsubstituted hydrocarbons, such as butane and butane.

Transdermal patches have the added advantage of providing controlled delivery of a compound of the present invention to the body. Such dosage forms can be made by dissolving, or dispersing the pharmaceutical agents in the buter medium. Absorption enhancers can also be used to increase the flux of the pharmaceutical agents of the invention across the skin. The rate of such flux can be controlled, by either providing a rate controlling membrane or dispersing the compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally-administered drug form is accomplished by dissolving or suspending the drug in an oil vehicle. One strategy for depot injections includes the use of polyethylene oxide-polybutylene oxide copolymers wherein the vehicle is fluid at room temperature and solidifies at body temperature.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly (orthoesters) and poly (anhydrides). Depot injectable formulations are also prepared by entrapping the drug in liposomes or microemulsions, which are compatible with body tissue.

When the compounds of the present invention are administered as pharmaceuticals, to humans and animals, they can be given per se or as a pharmaceutical composition containing, for example, 0.1% to 99.5% (more preferably, 0.5% to 90%) of active ingredient in combination with a pharmaceutically acceptable carrier.

The compounds and pharmaceutical compositions of the present invention can be employed in combination therapies, that is, the compounds and pharmaceutical compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, the compound of the present invention may be administered concurrently with another anti-HCV agent), or they may achieve different effects (e.g., control of any adverse effects).

The compounds of the invention may be administered intravenously, intramuscularly, intraperitoneally, subcutaneously, topically, orally, or by other acceptable means. The compounds may be used to treat arthritic conditions in mammals (i.e., humans, livestock, and domestic animals), birds, lizards, and any other organism, which can tolerate the compounds.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The representative examples which follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. The following examples contain important additional information, exemplification and guidance which can be adapted to the practice of this invention in its various embodiments thereof.

### Examples

### Example 1 - (this example relates to the synthesis of intermediate) [α-Methoxycarbonyl-Sar]-3-cyclosporin

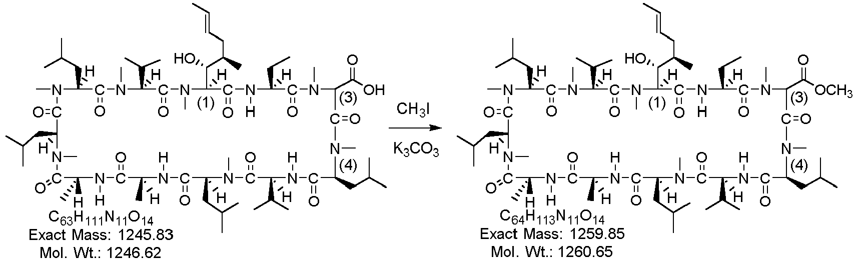

[α-Carboxy-sar]-3-cyclosporin (5.00 g, 4.01 mmol) was dissolved in 30 ml N,N-dimethylformamide. Iodomethane (2.85 g, 20.10 mmol) and potassium carbonate (1.38 g, 10.00 mmol) were added. The mixture was stirred at room temperature for 2 hours. Then 60 ml of ethyl acetate and 60 ml of water were added and the mixture was separated. The ethyl acetate layer was washed with brine, dried over magnesium sulfate and evaporated under reduced pressure to give 5.32 g of crude product, which was directly used for the next step without purification (yield: ~ 100%) [Molecular Formula: C₆₄H₁₁₃N₁₁O₁₄; Exact Mass: 1259.85; MS (m/z): 1260.7 (M+1)⁺, 1282.7 (M+Na)⁺; TLC R_{f}: 0.55 (dichloromethane/methanol = 9/1)].

### Example 2 - (this example relates to the synthesis of intermediate) [(R)-α-Hydroxymethyl-Sar]-3-cyclosporin

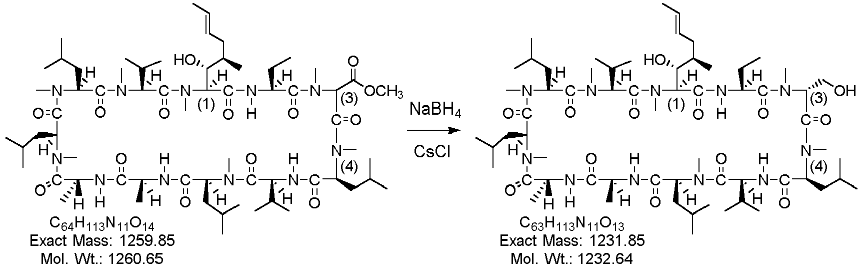

[α-Methoxycarbonyl-Sar]-3-cyclosporin (2.00 g, 1.59 mmol) was dissolved in tetrahydrofuran (30 ml). Cesium chloride (1.33 g) and sodium borohydride (0.60 g, 15.89 mmol) were added in portions. The 30 ml of methanol was added dropwise to the mixture over 2 hours. After addition, the mixture was stirred at room temperature overnight. Most solvent was then evaporated under reduced pressure. Ethyl acetate (50 ml) and water (50 ml) were added. The ethyl acetate layer was separated and washed with brine, dried over magnesium sulfate and evaporated under reduced pressure to give 1.99 g of crude product, which was purified by on silica gel column with dichloromethane/methanol (from 100:0 to 95:5) to give the 1.50 g of pure product (yield: 76%) [Molecular Formula: C₆₃H₁₁₃N₁₁O₁₃; Exact Mass: 1231.85; MS (m/z): 1232.7 (M+1)⁺, 1254.7 (M+Na)⁺].

### Example 3 - (this example relates to the synthesis of intermediate)

### [α-Methylmethanesulfonate-Sar]-3-cyclosporin

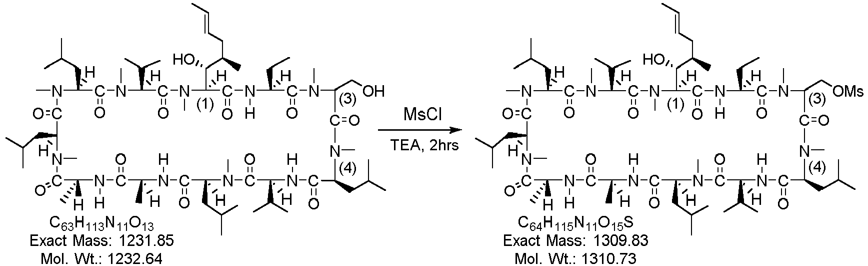

To a solution of [α-hydroxymethyl-Sar]-3-cyclosporin (30 mg, 0.024 mmol) in methylene chloride (2 ml) at 0 °C were added triethylamine (52.8 µl, 0.38 mmol), and methanesulfonyl chloride (23 mg, 0.20 mmol). The mixture was stirred at room temperature for two hours. Then reaction mixture was washed with brine, dried over magnesium sulfate and evaporated under reduced pressure to give 33 mg of crude product, which was directly used in next step reaction without further purification [Molecular Formula: C₆₄H₁₁₅N₁₁O₁₅S; Exact Mass: 1309.83; MS (m/z): 1310.7 (M+1)⁺].

### Example 4 - (this example relates to the synthesis of intermediate)

### [α-Chloromethyl-Sar]-3-cyclosporin

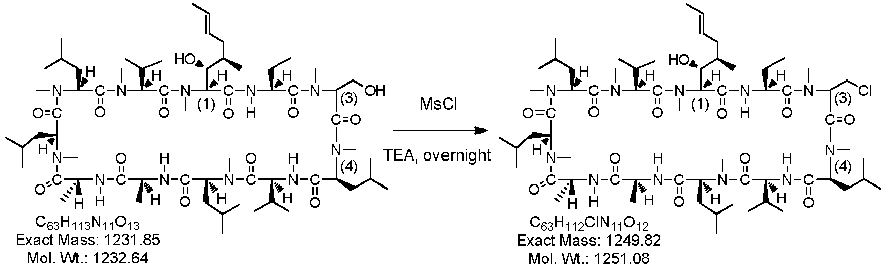

To a solution of [α-hydroxymethyl-Sar]-3-cyclosporin (30 mg, 0.024 mmol) in methylene chloride (2 ml) at 0 °C were added triethylamine (52.8 µL, 0.384 mmol, 16 equivalents) and methanesulfonyl chloride (23 mg, 0.20 mmol). The mixture was stirred at room temperature overnight. Then the reaction mixture was washed with brine, dried over magnesium sulfate and evaporated under reduced pressure to give 30 mg of crude product, which was directly used in next step reaction without further purification [Molecular Formula: C₆₃H₁₁₂ClN₁₁O₁₂; Exact Mass: 1249.82; MS (m/z): 1250.7 (M+1)⁺, 1272.9 (M+Na)⁺].

### Example 5 - (this example relates to the synthesis of intermediate)

### [α-Methylene-Sar]-3-cyclosporin

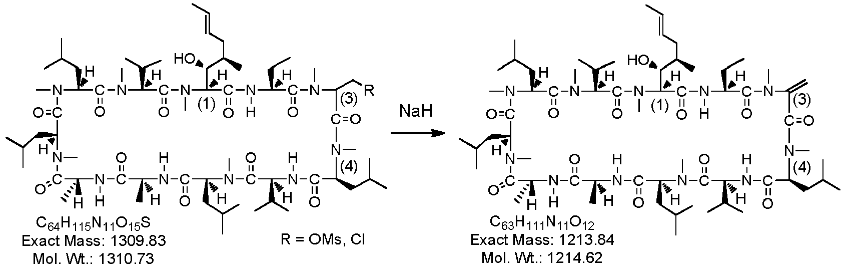

To a solution of either [α-methanesulfonatemethyl-Sar]-3-cyclosporin (33 mg, 0.025 mmol) or [α-chloromethyl-Sar]-3-cyclosporin (30 mg, 0.025 mmol) in tetrahydrofuran (3 ml) was added sodium hydride (15.3 mg, 60% in oil, 0.38 mmol, 10 equivalents) at 0 °C. The mixture was stirred at 0 °C for one hour and then warmed up to room temperature for 30 minutes. After removal of solvent, the residue was dissolved in 20 ml of dichloromethane. The dichloromethane layer was washed with 1 N hydrochloric acid, saturated sodium bicarbonate solution and brine, dried over magnesium sulfate and evaporated under reduced pressure. The residue was purified by chromatography on silica gel using dichloromethylene/methanol (20/1) to give 16 mg of product (yield: 54%) [Molecular Formula: C₆₃H₁₁₁N₁₁O₁₂; Exact Mass: 1213.84; MS (m/z): 1214.7 (M+1)⁺, 1236.7 (M+Na)⁺; TLC R_{f}: 0.55 (ethyl acetate/methanol = 20/1); HPLC RT: 7.0 min. (C8 reverse phase column: 150 mm; acetonitrile/water (0.05% trifluoroacetic acid); operation temperature: 64 °C; detector: 210 nm)].

### Example 6

### [(S)-(2-(N,N-Dimethylamino)ethylthio)methyl-Sar]-3-cyclosporin (Isomer B) and [(R)-(2-(N,N-Dimethylamino)ethylthio)methyl-Sar]-3-cyclosporin (Isomer A)

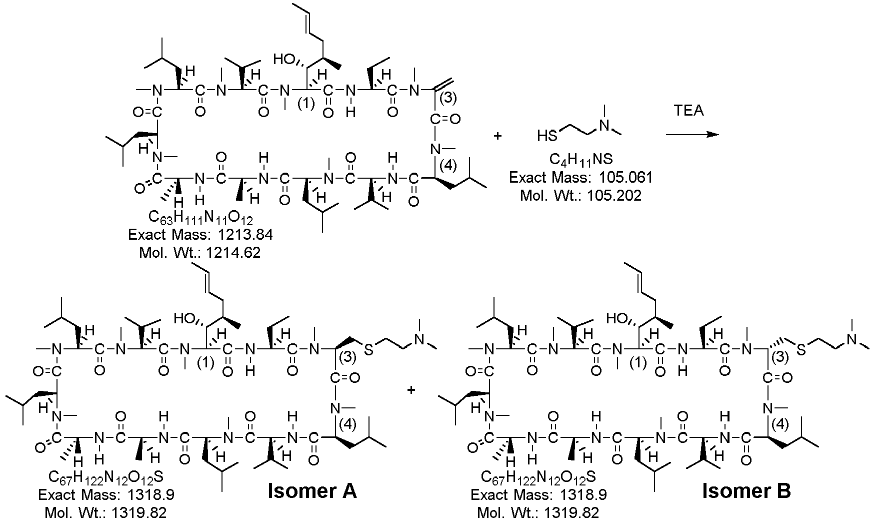

To a solution of [α-methylene-Sar]-3-cyclosporine (0.60 g, 0.50 mmol) and 2-(dimethylamino)ethanethiol (0.63 g, 6.00 mmol) in methanol (20 ml) was added triethylamine (0.82 ml, 6.0 mmol). The reaction mixture was stirred overnight at room temperature. After removal of solvent, the residue was subjected to chromatography using methylene/methanol as eluent to give 0.35 g of (R)-2-(N,N-dimethylamino)ethylthiomethyl-Sar]-3-cyclosporin (isomer A) and 0.20 g of [(S)-2-(N,N-dimethylamino)ethylthiomethyl-Sar]-3-cyclosporin (isomer B) [Molecular Formula: C₆₇H₁₂₂N₁₂O₁₂S; Exact Mass: 1218.9; MS (m/z): 1319.80 (M+1)⁺; TLC R_{f}: 0.20 (ethyl acetate/methanol = 5/1); HPLC RT: 12.55 min. (isomer A) and 13.22 min. (isomer B) (C8 reverse phase column: 250 mm; acetonitrile/water (0.05% trifluoroacetic acid); operation temperature: 64 °C; detector: 210 nm)].

### Example 7

### [(S)-(2-(N,N-Diethylamino)ethylthio)methyl-Sar]-3-cyclosporin (Isomer B) and [(R)-(2-(N,N-Diethylamino)ethylthio)methyl-Sar]-3-cyclosporin (Isomer A)

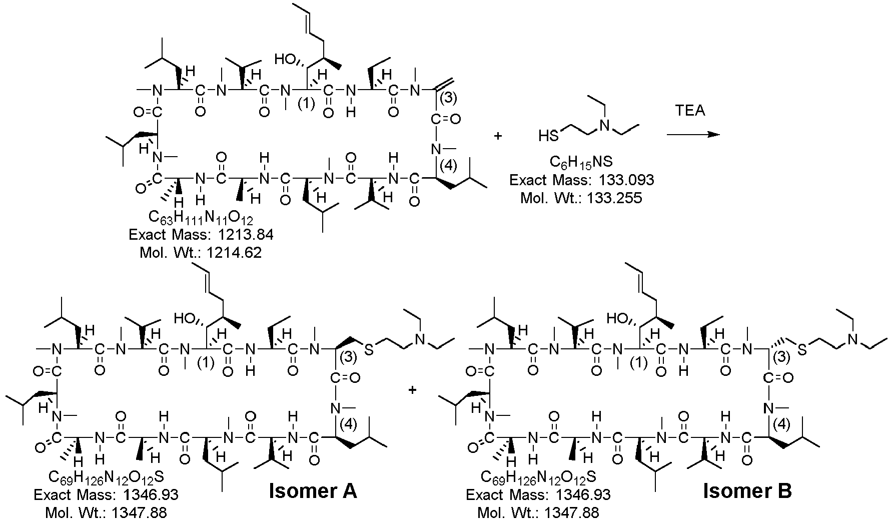

To a solution of [α-methylene-Sar]-3-cyclosporin (0.31 g, 0.25 mmol) and 2-diethylaminoethanethiol (0.40 g, 3.00 mmol) in methanol (10 ml) was added triethylamine (0.41 ml, 3.00 mmol, 12 equivalents). The reaction mixture was stirred overnight at room temperature. After removal of solvent, the residue was subjected to chromatography using methylene/methanol as eluent to yield 0.20 g of [(R)-2-(N,N-Diethylamino)ethylthiomethyl-Sar]-3-cyclosporin (isomer A) and 0.08 g of [(S)-2-(N,N-Diethylamino)ethylthiomethyl-Sar]-3-cyclosporin (isomer B) [Molecular Formula: C₆₉H₁₂₆N₁₂O₁₂S; Exact Mass: 1346.93; MS (m/z): 1347.80 (M+1)⁺; TLC R_{f}: 0.23 (ethyl acetate/methanol = 5/1); HPLC RT: 13.37 min. (isomer A) and 13.91 min. (isomer B) (C8 reverse phase column: 250 mm; acetonitrile/water (0.05% trifluoroacetic acid); operation temperature: 64 °C; detector: 210 nm)].

### Example 8

### [(R)-(2-(N,N-Diethylamino)ethoxy)methyl-Sar]-3-cyclosporin

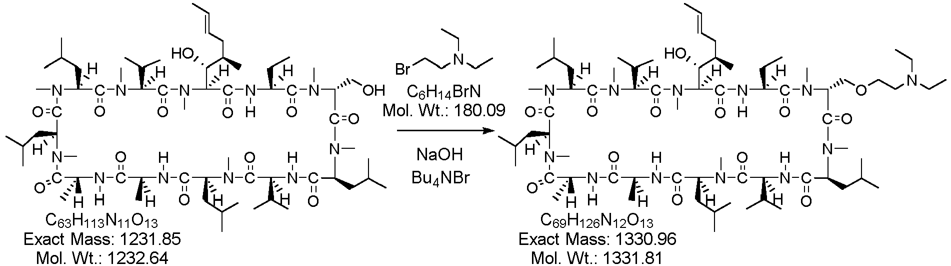

To a solution of [(R)-α-hydroxymethyl-Sar]-3-cyclosporin (0.36 g, 0.29 mmol) in benzene (30 ml) were added a solution of sodium hydroxide (1.20 g, 30 mmol) in water (2 ml), 2-bromo-N,N-diethylethylamine hydrobromide (3.80 g, 14.56 mmol) and tetra-n-butylammonium bromide(0.20 g, 0.62 mmol). The reaction mixture was stirred at 30 °C for 20 hours. After diluted with ice water, the mixture was separated. The aqueous layer was extracted with dichloromethane (30 ml). The combined organic layers were washed with brine, dried over magnesium sulfate and evaporated under reduced pressure. The residue was purified by chromatography (dichloromethane/methanol = 96/4) to give 210 mg of product [Molecular Formula: C₆₉H₁₂₆N₁₂O₁₃; Exact Mass: 1330.96; MS (m/z): 1331.71(M+1)⁺; TLC R_{f}: 0.38 (dichloromethane/methanol = 95/5); HPLC RT: 14.12 min. (C8 reverse phase column: 250 mm; acetonitrile/0.077% ammonium acetate in water; operation temperature: 64 °C; detector: 210 nm)].

### Example 9

### [(R)-α-(tert-Butoxycarbonylmethoxy)methyl-Sar]-3-cyclosporin

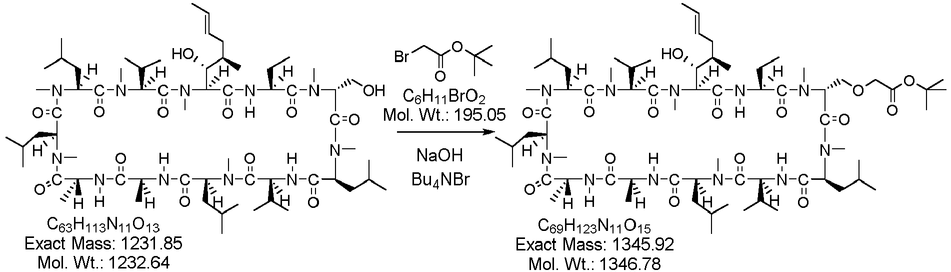

To a solution of [(R)-α-hydroxymethyl-Sar]-3-cyclosporin (0.50 g, 0.41 mmol) in benzene (30 ml) were added a solution of sodium hydroxide (1.00 g, 25.00 mmol) in water (1 ml), t-butyl bromoacetate (3.20 g, 16.41 mmol) and tetra-n-butylammonium bromide (0.40 g, 1.24 mmol). The mixture was stirred at room temperature for 10 hours. After diluted with ice water, the mixture was separated. The aqueous layer was extracted with dichloromethane (30 ml). The combined organic layers were washed with brine, dried over magnesium sulfate and evaporated under reduced pressure. The residue was purified by chromatography (hexane/acetone = 2/1) to give 0.41 g product [Molecular Formula: C₆₉H₁₂₃N₁₁O₁₅; Exact Mass: 1345.92; MS (m/z): 1346.61 (M+1)⁺; TLC R_{f}: 0.60 (dichloromethane/methanol = 95/5); HPLC RT: 18.29 min. (C8 reverse phase column: 250 mm; acetonitrile/0.077% ammonium acetate in water; operation temperature: 64 °C; detector: 210 nm)].

### Example 10

### [(R)-α-(Ethoxycarbonylmethoxy)methyl-Sar]-3-cyclosporin

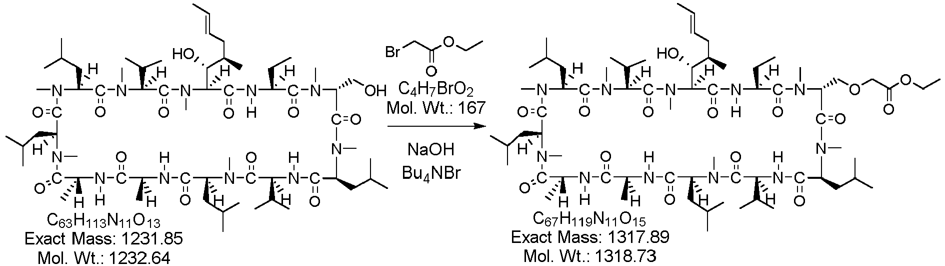

To a solution of [(R)-α-hydroxymethyl-Sar]-3-cyclosporin (0.35 g, 0.28 mmol) in benzene (15 ml) were added a solution of sodium hydroxide (0.60 g, 15.00 mmol) in water (1 ml), ethyl bromoacetate (1.60 g, 9.58 mmol) and tetra-n-butylammonium bromide (0.20 g, 0.62 mmol). The mixture was stirred at room temperature for 10 hours. After diluted with ice water, the mixture was separated. The aqueous layer was extracted with dichloromethane (15 ml). The combined organic layers were washed with brine, dried over magnesium sulfate and evaporated under reduced pressure. The residue was purified by chromatography (hexane/acetone = 2/1) to give 0.31 g of product [Molecular Formula: C₆₇H₁₁₉N₁₁O₁₅; Exact Mass: 1317.89; MS (m/z): 1318.46 (M+1)⁺; TLC R_{f}: 0.55 (dichloromethane/methanol = 95/5); HPLC RT: 17.40 min. (C8 reverse phase column: 250 mm; acetonitrile/0.077% ammonium acetate in water; operation temperature: 64 °C; detector: 210 nm)].

### Example 11

### [(R)-α-(Carboxymethoxy)methyl-Sar]-3-cyclosporin

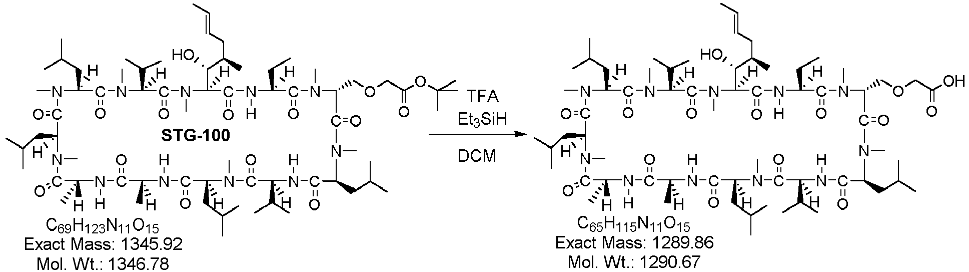

To a solution of [(R)-α-((tert-butoxycarbonyl)methoxy)methyl-Sar]-3-cyclosporin (0.18 g, 0.13 mmol) in dichloromethane 5 ml were added trifuloroacetic acid (1 ml) and Et₃SiH (10 drops). The mixture was stirred at room temperature for 3 hours and concentrated under reduced pressure. Then dichloromethane (10 ml) and water (10 ml) were added and the mixture was separated. The dichloromethane layer was dried over magnesium sulfate and evaporated under reduced pressure. The residue was purified by C-18 chromatography (acetonitrile/water) to give 75 mg of product [Molecular Formula: C₆₅H₁₁₅N₁₁O₁₅; Exact Mass: 1289.86; MS (m/z): 1290.56 (M+1)⁺; HPLC RT: 11.03 min. (C8 reverse phase column: 250 mm; acetonitrile/water (0.05% trifluoroacetic acid); operation temperature: 64 °C; detector: 210 nm)].

### Example 12

### [(R)-α-(Carboxymethoxy)methyl-Sar]-3-cyclosporin-sodium salt

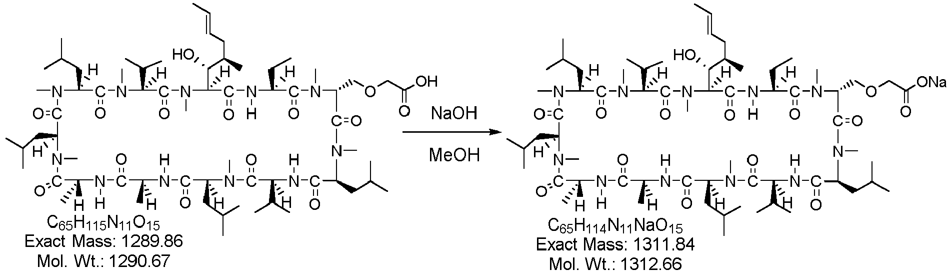

To a solution of [(R)-α-(carboxymethoxy)methyl-Sar]-3-cyclosporin (30 mg, 0.02 mmol) in methanol (1 ml) was added a solution of sodium hydroxide (1.00 mg, 0.02 mmol) in water (0.5 ml). The mixture was stirred at room temperature 1 hour and dried in high vacuum to give 28 mg of product [Molecular Formula: C₆₅H₁₁₄N₁₁NaO₁₅; Exact Mass: 1311.84; MS (m/z): 1290.56 (M+1-Na)⁺; HPLC RT: 10.98 min. (C8 reverse phase column: 250 mm; acetonitrile/water (0.05% trifluoroacetic acid); operation temperature: 64 °C; detector: 210 nm)].

### Example 13

### [(R)-(2-(N,N-Dimethylamino)ethoxy)methyl-Sar]-3-cyclosporin

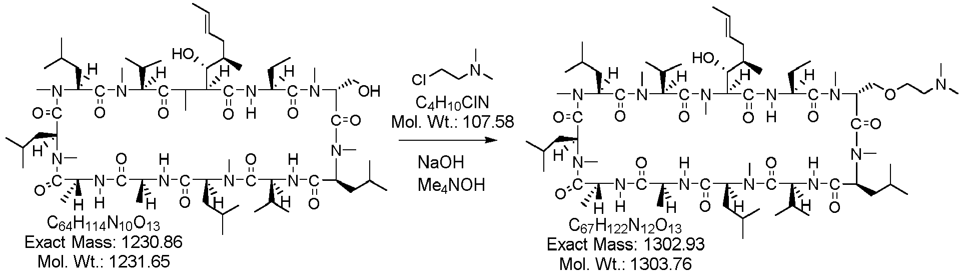

To a solution of [(R)-α-hydroxymethyl-Sar]-3-cyclosporin (1.03 g, 0.84 mmol) in benzene (50 ml) were added a solution of sodium hydroxide (1.34 g, 33.47 mmol) in water (1.34 ml), tetramethylammonium hydroxide pentahydrate (3.04 g, 16.73 mmol) and 2-dimethylaminoethyl chloride hydrochloride (2.41 g, 16.73 mmol). The mixture was stirred at room temperature for 5 days. Sodium bicarbonate saturated solution (100 ml) was added and the mixture was separated. Then the aqueous layer was extracted with ethyl acetate (50 ml x 2). The combined organic layers were dried over magnesium sulfate and evaporated under reduced pressure. After purified on silica gel, 303 mg product was obtained [Molecular Formula: C₆₇H₁₂₂N₁₂O₁₃; Exact Mass: 1302.93; MS (m/z): 1303.70 (M+1)⁺, 1325.85 (M+Na)⁺; TLC R_{f}: 0.36 (dichloromethane/methanol = 9/1); HPLC RT: 18.19 min. (C8 reverse phase column: 250 mm; acetonitrile/0.077% ammonium acetate in water; operation temperature: 64 °C; detector: 210 nm)].

### Example 14

### [(R)-(2-(N-Morpholino)ethoxy)methyl-Sar]-3-cyclosporin

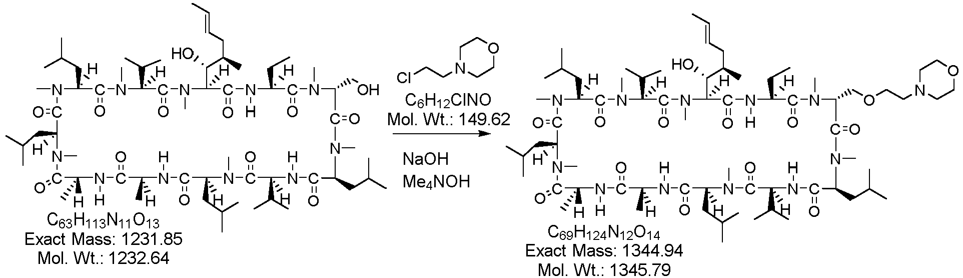

To a solution of [(R)-α-hydroxymethyl-Sar]-3-cyclosporin (0.27 g, 0.22 mmol) in benzene (20 ml) were added a solution of sodium hydroxide (0.70 g, 17.55 mmol) in water (0.70 ml), tetramethylammonium hydroxide pentahydrate (0.80 g, 4.39 mmol) and 2-(4-morpholinyl)ethyl chloride hydrochloride (0.82 g, 4.39 mmol). The mixture was stirred at 30 to 40°C for a week. Sodium bicarbonate saturated solution (30 ml) was added and then the mixture was separated. The aqueous layer was extracted with ethyl acetate (25 ml x 2). The combined organic layers were dried over magnesium sulfate and evaporated under reduced pressure. After purified on silica gel, 56 mg of product was obtained [Molecular Formula: C₆₉H₁₂₄N₁₂O₁₄; Exact Mass: 1344.94; MS (m/z): LCMS: 1345.72 (M+1)⁺, 1367.83 (M+Na)⁺; TLC R_{f}: 0.50 (dichloromethane/methanol = 9/1); HPLC RT: 16.64 min. (C8 reverse phase column: 250 mm; acetonitrile/0.077% ammonium acetate in water; operation temperature: 64 °C; detector: 210 nm)].

### Example 15

### [(R)-(2-(N-Pyrrolidinyl)ethoxy)methyl-Sar]-3-cyclosporin

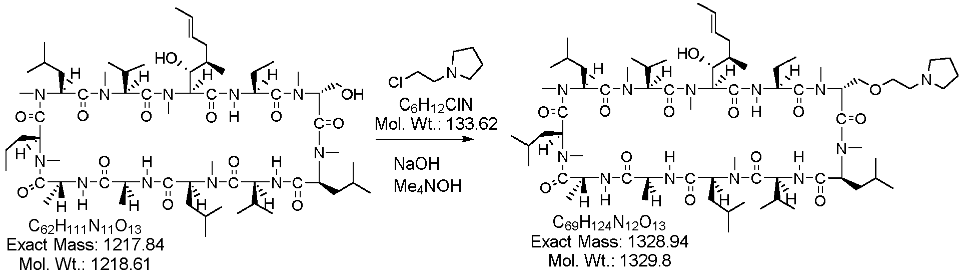

To a solution of [(R)-α-hydroxymethyl-Sar]-3-cyclosporin (0.320 g, 0.26 mmol) in benzene (20 ml) were added a solution of sodium hydroxide (0.83 g, 20.80 mmol) in water (0.85 ml), tetramethylammonium hydroxide pentahydrate (0.95 g, 5.20 mmol) and 1-(2-chloroethyl)pyrrolidine hydrochloride (0.88 g, 5.20 mmol). The mixture was stirred at room temperature for a weekend. Sodium bicarbonate saturated solution (30 ml) was added and the mixture was separated. The aqueous layer was extracted with ethyl acetate (25 ml x 2). The combined organic layers were dried over magnesium sulfate and evaporated under reduced pressure. After purified on silica gel, 103 mg of product was obtained [Molecular Formula: C₆₉H₁₂₄N₁₂O₁₃; Exact Mass: 1328.94; MS (m/z): 1329.75 (M+1)⁺, 1351.82 (M+Na)⁺; TLC R_{f}: 0.37 (dichloromethane/methanol = 9/1); HPLC RT: 18.94 min. (C8 reverse phase column: 250 mm; acetonitrile/0.077% ammonium acetate in water; operation temperature: 64 °C; detector: 210 nm)].

### Example 16

### [(R)-(2-(N-Piperidinyl)ethoxy)methyl-Sar]-3-cyclosporin

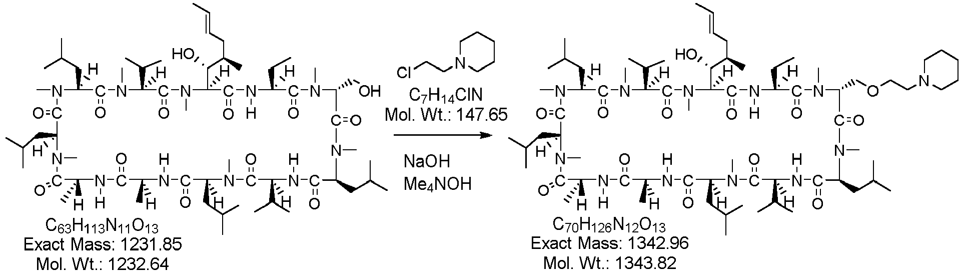

To a solution of [(R)-α-hydroxymethyl-Sar]-3-cyclosporin (0.28 g, 0.22 mmol) in benzene (20 ml) were added a solution of sodium hydroxide (0.36 g, 9.07 mmol) in water (0.36 ml), tetramethylammonium hydroxide pentahydrate (0.82 mg, 4.53 mmol) and 1-(2-chloroethyl)piperdine hydrochloride (0.83 g, 4.53 mmol). The mixture was stirred at 30 to 40°C for 20 hours. Sodium bicarbonate saturated solution (30 ml) was added and the mixture was separated. Then the aqueous layer was extracted with ethyl acetate (25 ml x 2). The combined organic layers were dried over magnesium sulfate and evaporated under reduced pressure. After purified on silica gel, 121 mg of product was obtained [Molecular Formula: C₇₀H₁₂₆N₁₂O₁₃; Exact Mass: 1342.96; MS (m/z): 1343.76 (M+1)⁺, 1365.83 (M+Na)⁺; TLC R_{f}: 0.44 (dichloromethane/methanol = 9/1); HPLC RT: 19.26 min. (C8 reverse phase column: 250 mm; acetonitrile/0.077% ammonium acetate in water; operation temperature: 64 °C; detector: 210 nm)].

### Example 17

### [(R)-α-(3,3-Dimethoxypropoxy)methyl-Sar]-3-cyclosporin

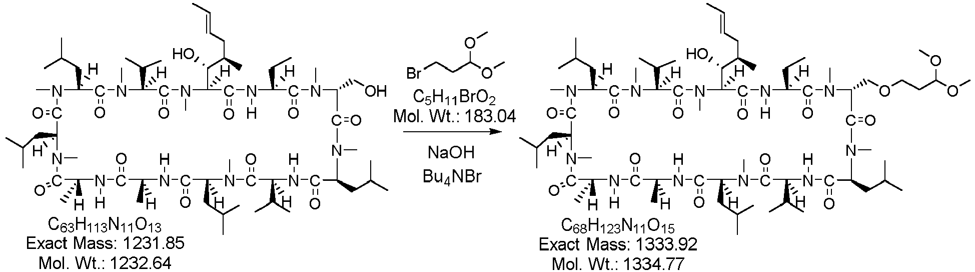

To a solution of [(R)-α-hydroxymethyl-Sar]-3-cyclosporin (0.50 g, 0.41 mmol) in benzene (30 ml) were added a solution of sodium hydroxide (1.00 g, 25.00 mmol) in water (1 ml), 3-bromopropionaldehyde dimethyl acetal (1.80 g, 10.00 mmol) and tetra-n-butylammonium bromide (0.20 g, 0.62 mmol). After stirred at room temperature for 10 hours, the mixture was diluted with ice water and the mixture was separated. The aqueous layer was extracted with dichloromethane (20 ml). The combined organic layers were washed with brine, dried over magnesium sulfate and evaporated under reduced pressure to give 0.48 g crude product, which was used for next step [Molecular Formula: C₆₈H₁₂₃N₁₁O₁₅; Exact Mass: 1333.92; MS (m/z): 1334.50 (M+1)⁺].

### Example 18

### [(R)-α-(2-Formylethoxy)methyl-Sar]-3-cyclosporin

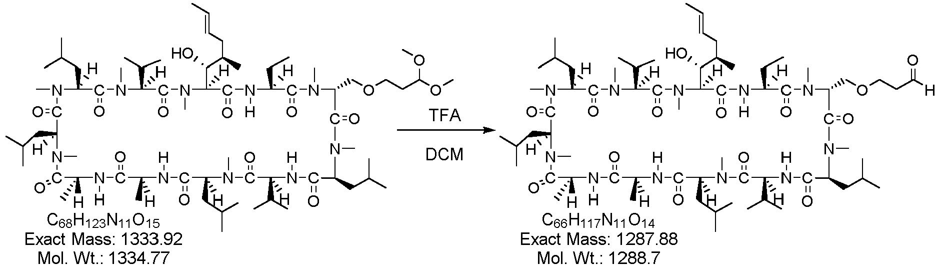

To a solution of crude [(R)-(3,3-dimethoxypropoxy)methyl-Sar]-3-cyclosporin (0.48 g, 0.36 mmol) in dichloromethane (30 ml) were added trifuloroacetic acid (5 ml) and water (4 ml) at 0 °C. Then the mixture was allowed to warm to room temperature and stirred for 3 hours. After the mixture was separated, the dichloromethane layer was washed with saturated sodium bicarbonate solution (20 ml), dried over magnesium sulfate and evaporated under reduced pressure. The residue was purified by chromatography (hexane/acetone = 2/1) to give 0.31 g of product [Molecular Formula: C₆₆H₁₁₇N₁₁O₁₄; Exact Mass: 1287.88; MS (m/z): 1288.63 (M+1)⁺].

### Example 19

### [(R)-(3-(N,N-Dimethylamino)propoxy)methyl-Sar]-3-cyclosporin

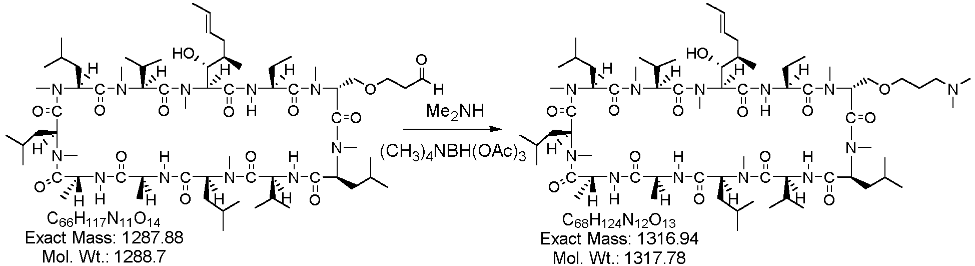

To a solution of [(R)-(2-formylethoxy)methyl-Sar]-3-cyclosporin (0.13 g, 0.10 mmol) in chloroform (5 ml) were added dimethylamine hydrochloride (0.10 g, 1.22 mmol) and acetic acid (5 drops). After the mixture was stirred at room temperature for 5 minutes, tetramethylammonium triacetoxyborohydride (65 mg, 0.25 mmol) was added in portions and stirring was continued for 1 hour. Then dichloromethane (10 ml) and saturated sodium bicarbonate solution (10 ml) were added and separated. The organic layer was washed with brine, dried over magnesium sulfate and evaporated under reduced pressure. The residue was purified by chromatography (dichloromethane/methanol = 96/4) to give 89 mg of product [Molecular Formula: C₆₈H₁₂₄N₁₂O₁₃; Exact Mass: 1316.94; MS (m/z): 1317.64 (M+1)⁺; TLC R_{f}: 0.39 (dichloromethane/methanol = 95/5); HPLC RT: 13.92 min. (C8 reverse phase column: 250 mm; acetonitrile/0.077% ammonium acetate in water; operation temperature: 64 °C; detector: 210 nm)].

### Example 20

### [(R)-(3-(N,N-Diethylamino)propoxy)methyl-Sar]-3-cyclosporin

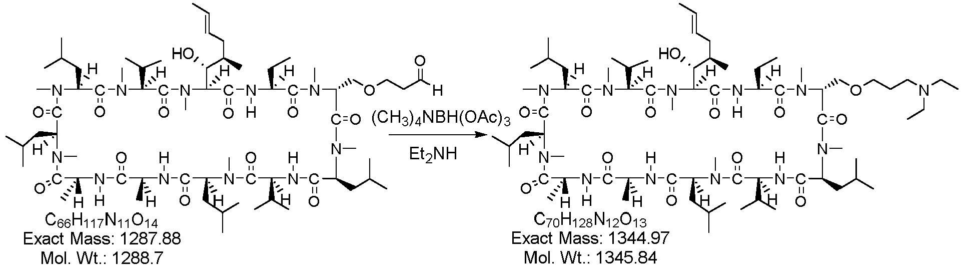

To a solution of [(R)-(2-formylethoxy)methyl-Sar]-3-cyclosporin (100 mg, 0.08 mmol) in chloroform (4 ml) were added diethylamine (100 mg, 1.37 mmol) and acetic acid (4 drops). After the mixture was stirred at room temperature for 5 minutes, tetramethylammonium triacetoxyborohydride (50 mg, 0.19 mmol) was added in portions and stirring was continued for 1 hour. Then dichloromethane (10 ml) and saturated sodium bicarbonate solution (10 ml) were added and the mixture was separated. The organic layer was washed with brine, dried over magnesium sulfate and evaporated under reduced pressure. The residue was purified by chromatography (dichloromethane/methanol = 96/4) to give 56 mg of product [Molecular Formula: C₇₀H₁₂₈N₁₂O₁₃; Exact Mass: 1344.97; MS (m/z): 1345.71 (M+1)⁺; TLC R_{f}: 0.40 (dichloromethane/methanol = 95/5); HPLC RT: 14.59 min. (C8 reverse phase column: 250 mm; acetonitrile/0.077% ammonium acetate in water; operation temperature: 64 °C; detector: 210 nm)].

### Example 21

### [(R)-(3-(N-Morphlino)propoxy)methyl-Sar]-3-cyclosporin

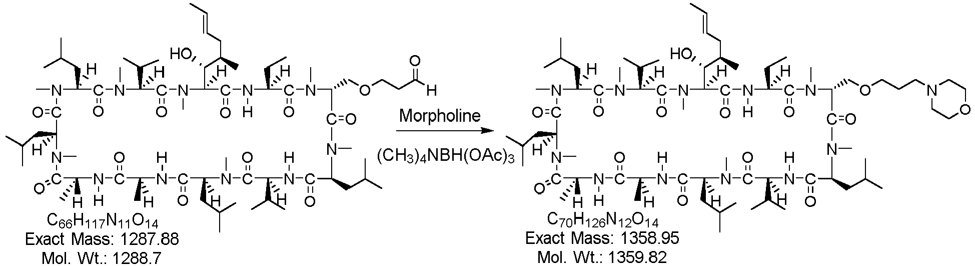

To a solution of [(R)-(2-formylethoxy)methyl-Sar]-3-cyclosporin (300 mg, 0.23 mmol) in dichloromethane (15 ml) were added morpholine (101 mg, 1.16 mmol) and tetramethylammonium triacetoxyborohydride (306 mg, 1.16 mmol). The reaction mixture was stirred at room temperature for two hours. Then sodium bicarbonate saturated solution (30 ml) and dichloromethane (15 ml) were added and the mixture was separated. The dichloromethane layer was dried over magnesium sulfate and evaporated under reduced pressure. After purified on silica gel, 124 mg product was obtained [Molecular Formula: C₇₀H₁₂₆N₁₂O₁₄; Exact Mass: 1358.95; MS (m/z): 1359.71(M+1)⁺, 1381.79 (M+Na)⁺; TLC R_{f}: 0.40 (dichloromethane/methanol = 9/1); HPLC RT: 14.2 min. (C8 reverse phase column: 250 mm; acetonitrile/0.077% ammonium acetate in water; operation temperature: 64 °C; detector: 210 nm)].

### Example 22

### [(R)-(3-(N-Pyrrolidinyl)propoxy)methyl-Sar]-3-cyclosporin

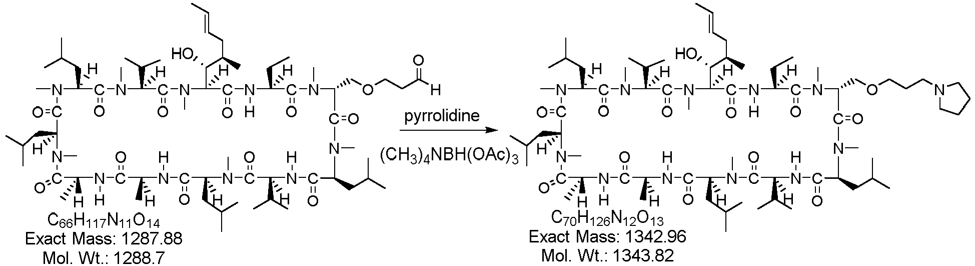

To a solution of [(R)-α-hydroxymethyl-Sar]-3-cyclosporin (315 mg, 0.24 mmol) in dichloromethane (15 ml) were added pyrrolidine (87 mg, 1.22 mmol) and tetramethylammonium triacetoxyborohydride (322 mg, 1.22 mmol). The reaction mixture was stirred at room temperature for 2 hours. Then sodium bicarbonate saturated solution (30 ml) and dichloromethane (15 ml) were added and the mixture was separated. The dichloromethane layer was dried over magnesium sulfate and evaporated under reduced pressure. After purified on silica gel, 22 mg product was obtained [Molecular Formula: C₇₀H₁₂₆N₁₂O₁₃; Exact Mass: 1342.96; MS (m/z): 1343.75 (M+1)⁺, 1365.82 (M+Na)⁺; TLC R_{f}: 0.33 (dichloromethane/methanol = 9/1); HPLC RT: 14.3 min. (C8 reverse phase column: 250 mm; acetonitrile/0.077% ammonium acetate in water; operation temperature: 64 °C; detector: 210 nm)].

### Example 23

### [(R)-(3-(N-Piperidinyl)propoxy)methyl-Sar]-3-cyclosporin

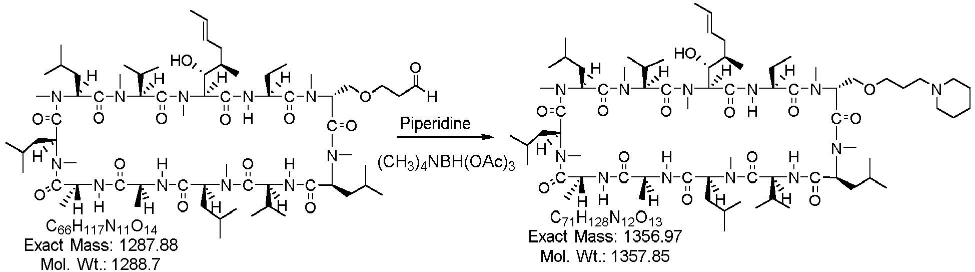

To a solution of [(R)-(2-formylethoxy)methyl-Sar]-3-cyclosporine (350 mg, 0.27 mmol) in dichloromethane (20 ml) were added piperidine (115 mg, 1.34 mmol) and tetramethylammonium triacetoxyborohydride (352 mg, 1.34 mmol). The reaction mixture was stirred overnight at room temperature. Then sodium bicarbonate saturated solution (30 ml) and dichloromethane (15 ml) were added and the mixture was separated. The dichloromethane layer was dried over magnesium sulfate and evaporated under reduced pressure. After purified on silica gel, 35 mg product was obtained [Molecular Formula: C₇₁H₁₂₈N₁₂O₁₃; Exact Mass: 1356.97; MS (m/z): 1357.76 (M+1)⁺, 1379.83 (M+Na)⁺; TLC R_{f}: 0.36 (dichloromethane/methanol = 9/1); HPLC RT: 14.4 min. (C8 reverse phase column: 250 mm; acetonitrile/0.077% ammonium acetate in water; operation temperature: 64 °C; detector: 210 nm)].

### Example 24 - (this example relates to the synthesis of intermediate)

### [α-Carboxy-Sar]-3-[(γ-hydroxy)-N-MeLeu]-4-cyclosporin

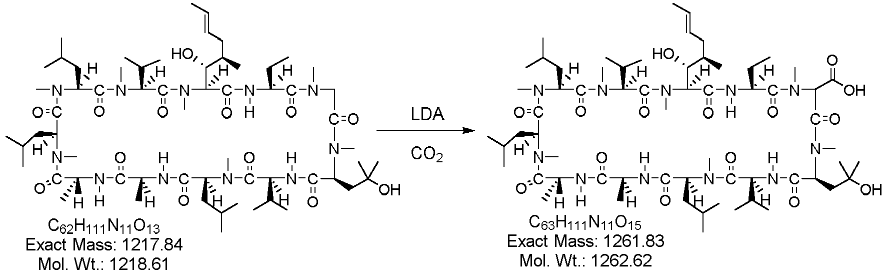

To a solution of LDA (2.0 M in tetrahydrofuran, 23 ml, 46 mmol) in tetrahydrofuran (80 ml) at -78°C under nitrogen, [(γ-hydroxy)-N-MeLeu]-4-cyclosporin (4.40 g, 3.61 mmol) in tetrahydrofuran (15 ml) was added over 3 min. After the mixture was stirred at -78°C for 3 hours, carbon dioxide gas was bubbled into the reaction mixture for 1 hour. Then the mixture was allowed to warm to room temperature slowly and kept stirring for 3 hours. Most of tetrahydrofuran was evaporated. Dichloromethane (100 ml) and water (50 ml) were added. The PH of the mixture was adjusted to around 5 by adding aqueous citric acid solution. The mixture was separated and the organic layer was washed with brine, dried over magnesium sulfate and evaporated under reduced pressure to give 3.20 g of crude product, which was used for next step without purification [Molecular Formula: C₆₃H₁₁₁N₁₁O₁₅; Exact Mass: 1261.83; MS (m/z): 1262.49 (M+1)⁺].

[(γ-Hydroxy)-N-MeLeu]-4-cyclosporin was prepared by *Sebekia benihana* biotransformation according to a method described by Kuhnt M. et al., 1996, Microbial Biotransformation Products of Cyclosporin A, J. Antibiotics, 49 (8), 781.

### Example 25 - (this example relates to the synthesis of intermediate)

### [α-Methoxycarbonyl-Sar]-3-[(γ-hydroxy)-N-MeLeu]-4-cyclosporin

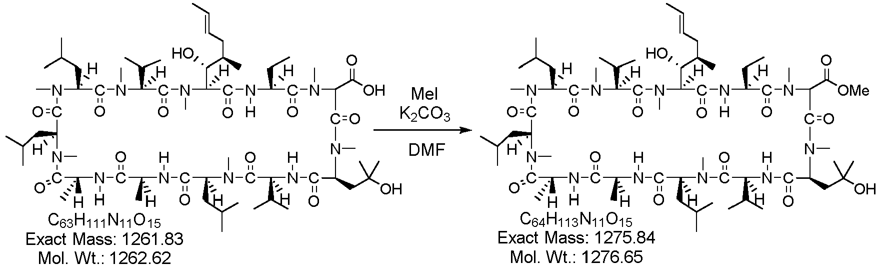

To a mixture of [α-carboxy-Sar]-3-[(γ-hydroxy)-N-MeLeu]-4-cyclosporin (3.20 g 2.53 mmol) and potassium carbonate (1.30 g, 9.40 mmol) in N,N-dimethylformamide (20 ml) was added iodomethane (1.80 g, 12.70 mmol). The mixture was stirred overnight at room temperature. Dichloromethane (80 ml) and water (50 ml) were added and the mixture was separated. The dichloromethane layer was washed with water (25 ml) and brine (25 ml), dried over magnesium sulfate and evaporated under reduced pressure to give crude 3.00 g of product [Molecular Formula: C₆₄H₁₁₃N₁₁O₁₅; Exact Mass: 1275.84; MS (m/z): 1276.75 (M+1)⁺].

### Example 26 - (this example relates to the synthesis of intermediate)

### [(R)-α-Hydroxymethyl-Sar]-3-[(γ-hydroxy)-N-MeLeu]-4-cyclosporin

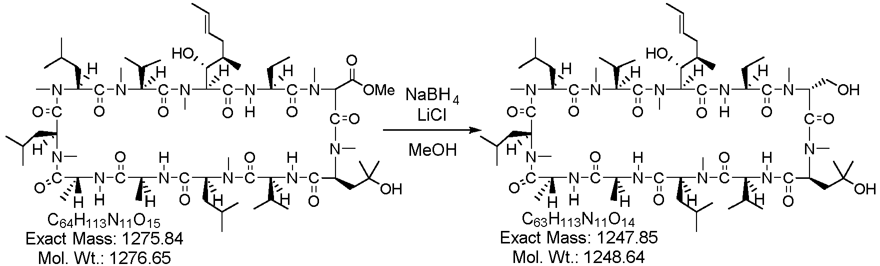

To a suspension of [α-methoxycarbonyl-Sar]-3-[(γ-hydroxy)-N-MeLeu]-4-cyclosporin (3.00 g, 2.35 mmol) and lithium chloride (1.50 g, 35.30 mmol) in methanol (100 ml) was added sodium borohydride (2.50 g, 66.10 mmol) in portions. The mixture was stirred overnight at room temperature. Most of solvent was evaporated under reduced pressure. Dichloromethane (80 ml) and water (50 ml) were added and the mixture was separated. The dichloromethane layer was washed with brine, dried over magnesium sulfate and evaporated under reduced pressure. The residue was purified by chromatography (dichloromethane/methanol = 96/4) to give 1.30 g of product [Molecular Formula: C₆₃H₁₁₃N₁₁O₁₄; Exact Mass: 1247.85; MS (m/z): 1248.48 (M+1)⁺; ¹H NMR spectrum (600 MHz, CDCl₃, δ in ppm): 0.68 (d, J = 5.4Hz, 3H), 0.80-1.00 (m, 30H), 1.07 (d, J = 6.0Hz, 3H), 1.16 -1.29 (m, 10H), 1.32 (d, J = 7.2Hz, 3H), 1.39-1.46 (m, 2H), 1.59-1.63 (m, 6H), 1.68-1.83 (m, 7H), 2.02-2.11 (m, 4H), 2.31-2.33 (m, 1H), 2.37-2.42 (m, 2H), 2.67 (s, 6H), 3.09 (s, 3H), 3.19 (s, 3H), 3.20 (s, 3H), 3.22 (s, 3H), 3.47 (s, 3H), 3.72-3.75 (m, 1H), 3.82 (br, 1H), 3.97-3.99 (m, 1H), 4.07-4.10 (m, 1H), 4.50-4.52 (m, 1H), 4.65-4.67 (t, J = 8.4 Hz, 1H), 4.79-4.81 (m, 1H), 4.90-4.95 (m, 2H), 5.00 -5.05 (m, 2H), 5.09 (d, J = 10.8Hz, 1H), 5.30-5.35 (m, 2H), 5.46 (d, J = 6.0Hz, 1H), 5.52-5.53 (m, 1H), 5.66-5.68 (m, 1H), 7.12 (d, J = 7.8Hz, 1H), 7.47 (d, J = 8.4Hz, 1H), 7.60 (d, J = 7.2Hz, 1H), 7.87-7.89 (d, J = 9.6Hz, 1H)].

### Example 27 - (this example relates to the synthesis of intermediate)

### [α-Methylene-Sar]-3-[(γ-hydroxy)-N-MeLeu]-4-cyclosporin

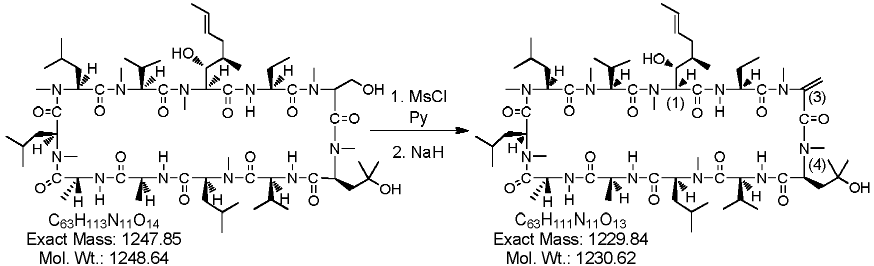

[α-Methylene-Sar]-3-[(γ-hydroxy)-N-MeLeu]-4-cyclosporin was prepared according to the method described in Example 4 and 5 [Molecular Formula: C₆₃H₁₁₁N₁₁O₁₃; Exact Mass: 1229.84; MS (m/z): 1230.6 (M+1)⁺, 1252.82 (M+Na)⁺; TLC R_{f}: 0.50 (ethyl acetate/methanol = 10/1); HPLC RT: 15.38 min. (C8 reverse phase column: 250 mm; acetonitrile/water (0.05% trifluoroacetic acid); operation temperature: 64 °C; detector: 210 nm); ¹H NMR spectrum (600 MHz, CDCl₃, δ in ppm): 0.72 (d, J = 5.4Hz, 3H), 0.84-1.00 (m, 30H), 1.17-1.26 (m, 15H), 1.34 (d, J = 6.0 Hz, 3H), 1.44 -1.47 (m, 2H), 1.59-1.62 (m, 6H), 1.69-1.76 (m, 4H), 1.94-1.99 (m, 1H), 2.09-2.13 (m, 3H), 2.34-2.37 (m, 3H), 2.65(s, 3H), 2.67 (s, 3H), 3.09 (s, 3H)), 3.10 (s, 3H), 3.19 (s, 3H), 3.44 (s, 3H), 3.46 (s, 3H), 3.80 (m, 1H), 3.91 (m, 1H), 4.47-4.50 (m, 1H), 4.68-4.71(t, J = 9.0Hz, 1H), 4.78-4.81 (m, 1H), 4.98-5.02 (m, 2H), 5.06-5.11 (m, 3H), 5.24 (s, 1H), 5.32 (m, 2H), 5.41-5.43 (m, 2H), 5.64-5.66 (m, 1H), 7.11 (d, J = 7.2Hz, 1H), 7.49 (d, J = 7.2Hz, 1H), 7.7 4 (d, J = 8.4Hz, 1H), 7.84 (d, J = 9.6Hz, 1H)].

### Example 28

### [(S)-(2-(N,N-Dimethylamino)ethylthio)methyl-Sar]-3-[(γ-hydroxy)-N-MeLeu]-4-cyclosporin (Isomer B) and [(R)-(2-(N,N-Dimethylamino)ethylthio)methyl-Sar]-3-[(γ-hydroxy)-N-MeLeu]-4-cyclosporin (Isomer A)

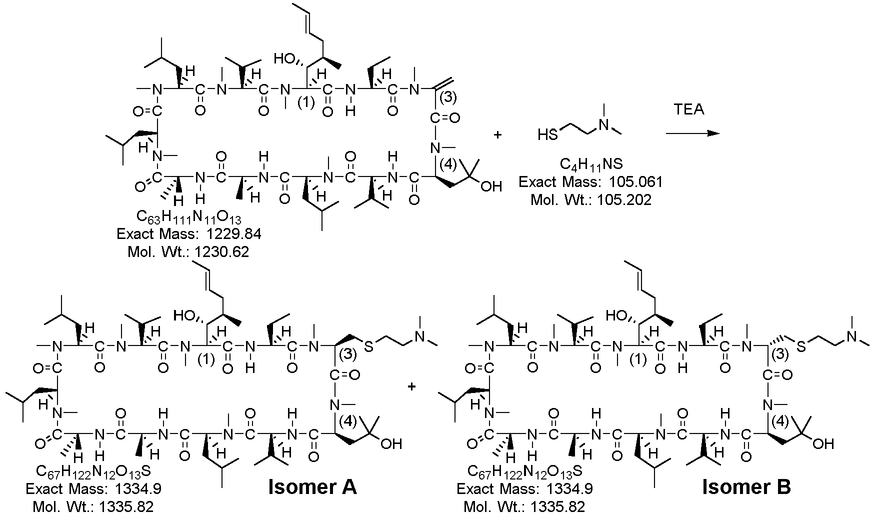

[α-Methylene-Sar]-3-[(γ-hydroxy)-N-MeLeu]-4-cyclosporin (0.62 g, 0.50 mmol) and 2-(dimethylamino)ethanethiol (0.49 g, 6.00 mmol) were dissolved in methanol (30 ml) followed by adding triethylamine (0.82 ml, 6.00 mmol). The mixture was stirred overnight at room temperature. After removal of solvent, the residue was subjected to chromatography using dichloromethane/methanol as eluent to yield 0.41 g of [(R)-(2-(N,N-dimethylamino)ethylthio)methyl-Sar]-3-[(γ-hydroxy)-N-MeLeu]-4-cyclosporin (isomer A) and 0.18 g of [(S)-(2-(N,N-dimethylamino)ethylthio)methyl-Sar]-3-[(y-hydroxy)-N-MeLeu]-4-cyclosporin (isomer B) [Molecular Formula: C₆₇H₁₂₂N₁₂O₁₃S; Exact Mass: 1334.9; MS (m/z): 1335.7(M+1)⁺; TLC R_{f}: 0.05 (ethyl acetate/methanol= 5/1); HPLC RT: 10.88 min.(isomer A) and 11.30 min. (isomer B) (C8 reverse phase column: 250 mm; acetonitrile/water (0.05% trifluoroacetic acid); operation temperature: 64 °C; detector: 210 nm)].

### Example 29

### [(S)-(2-(N,N-Diethylamino)ethylthio)methyl-Sar]-3-[(γ-hydroxy)-N-MeLeu]-4-cyclosporin (Isomer B) and [(R)-(2-(N,N-Diethylamino)ethylthio)methyl-Sar]-3-[(γ-hydroxy)-N-MeLeu]-4-cyclosporin (Isomer A)

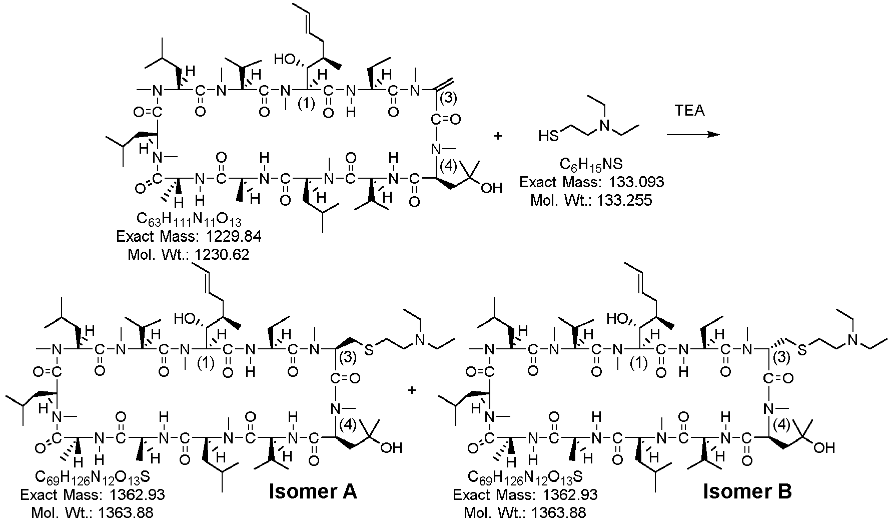

[α-Methylene-Sar]-3-[(γ-hydroxy)-N-MeLeu]-4-cyclosporin (0.31 g, 0.25 mmol) and 2-diethylaminoethanethiol (0.40 g, 3.00 mmol) were dissolved in methanol (30 ml), followed by adding triethylamine (0.41 ml, 3.00 mmol). The mixture was stirred overnight at room temperature. After removal of solvent, the residue was subjected to chromatography using dichloromethane/methanol as eluent to yield 0.15 g of [(R)-(2-(N,N-diethylamino)ethylthio)methyl-Sar]-3-[(γ-hydroxy-N-MeLeu]-4-cyclosporin (isomer A) and 0.10 g of [(S)-(2-(N,N-diethylamino)ethylthio)methyl-Sar]-3-[(γ-hydroxy-N-MeLeu]-4-cyclosporin (isomer B) [Molecular Formula: C₆₉H₁₂₆N₁₂O₁₃S; Exact Mass: 1362.93; MS (m/z): 1363.75 (M+1)⁺; TLC R_{f}: 0.1 (ethyl acetate/methanol = 5/1); HPLC RT: 11.64 min.(isomer A) and 11.85 min. (isomer B) (C8 reverse phase column: 250 mm; acetonitrile/water (0.05% trifluoroacetic acid); operation temperature: 64 °C; detector: 210 nm)].

### Example 30

### [(S)-(2-(N-Morpholino)ethylthio)methyl-Sar]-3-[(γ-hydroxy)-N-MeLeu]-4-cyclosporin

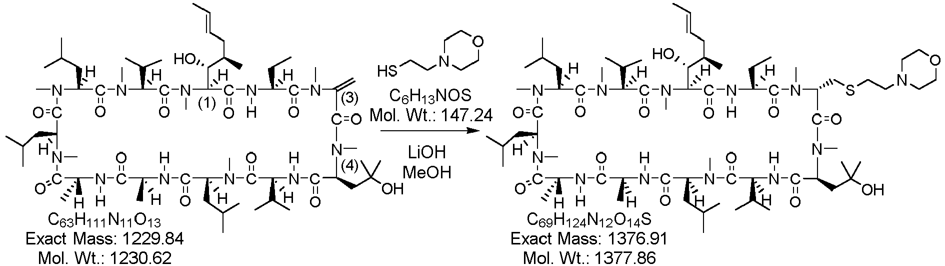

To a solution of [α-methylene-Sar]-3-[(γ-hydroxy)-N-MeLeu]-4-cyclosporin (260 mg, 0.21 mmol) and 2-morpholinoethanethiol (300 mg, 2.04 mmol) in methanol (30 ml) was added lithium hydroxide (140 mg, 5.83 mmol). The reaction mixture was stirred at room temperature overnight. Most of solvent was evaporated under reduced pressure. Dichloromethane (30 ml) and water (30 ml) were added and the mixture was separated. The organic layer was washed with water and brine, dried over magnesium sulfate and evaporated under reduced pressure. The residue was purified by chromatography (dichloromethane/methanol = 97/3) to give 102 mg of product [Molecular Formula: C₆₉H₁₂₄N₁₂O₁₄S; Exact Mass: 1376.91; MS (m/z): 1399.85 (M+Na)⁺; TLC Rf: 0.30 (dichloromethane/methanol = 9/1); HPLC RT: 11.03 min. (C8 reverse phase column: 250 mm; acetonitrile/0.077% ammonium acetate in water; operation temperature: 64 °C; detector: 210 nm); ¹H NMR spectrum (600 MHz, CDCl₃, δ in ppm): 0.68 (d, J=6.6Hz, 3 H), 0.79 (d, J = 6.6Hz, 3H), 0.82 (m, 6H,), 0.85 (d, J = 6.6Hz, 3H), 0.88 (d, J = 7.2Hz, 3H), 0.90 (d, J = 6.6Hz, 3H), 0.93 (d, J = 6.6 Hz, 3H), 0.97-1.00 (m, 9 H), 1.08 (d, J=6.6Hz, 3H), 1.21-1.25 (m, 11H), 1.31 (d, J=7.2Hz, 3H), 1.39-1.47 (m, 2 H), 1.54-1.61 (m, 8H), 1.66-1.70 (m, 2 H), 1.75 (m, 1H), 2.01-2.11 (m, 4 H), 2.36-2.43 (m, 7H), 2.55-2.59 (m, 2 H), 2.67 (m, 8 H), 2.93-3.04 (m, 2H), 3.10 (s, 3 H), 3.24 (s, 6H), 3.26 (s, 3H), 3.48 (s, 3H), 3.52 (br, 1H), 3.67 (m, 6H), 4.51 (m, 1 H), 4.59 (t, J =8.4Hz, 1H), 4.81 (m, 1 H), 4.94-5.00 (m, 2H), 5.04 (t, J=6.6Hz, 1H), 5.08 (d, J=10.8Hz,1H), 5.27-5.31 (m, 1H), 5.33-5.37 (m, 1H),5.48 (m, 2H), 5.67 (m,1H), 7.14 (d, J=7.8Hz, 1H), 7.49 (d, J=7.8Hz, 1H), 7.64 (d, J=8.4Hz, 1H), 8.11 (d, J=9.6Hz, 1H)].

### Example 31

### [(S)-(2-(N-Piperidinyl)ethylthio)methyl-Sar]-3-[(γ-hydroxy)-N-MeLeu]-4-cyclosporin

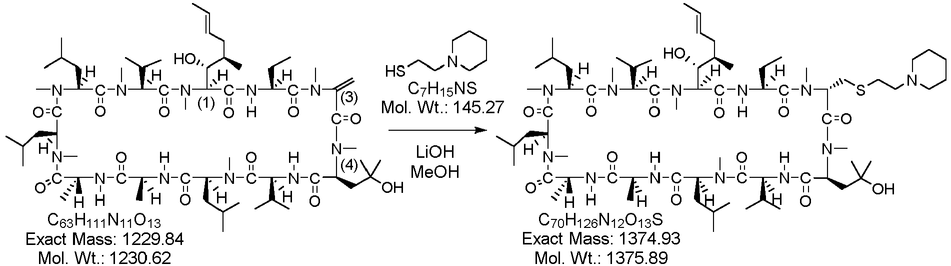

[α-Methylene-Sar]-3-[(γ-hydroxy)-N-MeLeu]-4-cyclosporin (0.37 g, 0.30 mmol) and 2-(N-piperidino)ethylthiol (0.44 g, 3.00 mmol) were dissolved in methanol (30 ml), followed by adding 10 equivalents of lithium hydroxide. The mixture was stirred overnight at room temperature. After removal of solvent, the residue was dissolved in dichloromethane (30 ml). The dichloromethane solution was washed with brine, dried over magnesium sulfate and evaporated under reduced pressure. The residue was purified by flash chromatography using dichloromethane/methanol as eluent to give 0.20 g of product [Molecular Formula: C₇₀H₁₂₆N₁₂O₁₃S; Exact Mass: 1374.93; MS (m/z): 1375.65 (M+1)⁺, 1397.80 (M+Na)⁺; TLC R_{f}: 0.18 (ethyl acetate/methanol = 5/1); HPLC RT: 12.09 min. (C8 reverse phase column: 250 mm; acetonitrile/water (0.05% trifluoroacetic acid); operation temperature: 64 °C; detector: 210 nm)].

### Example 32

### [(S)-(2-(4-Methyl-N-piperazinyl)ethylthio)methyl-Sar]-3-[(γ-hydroxy)-N-MeLeu]-4-cyclosporin

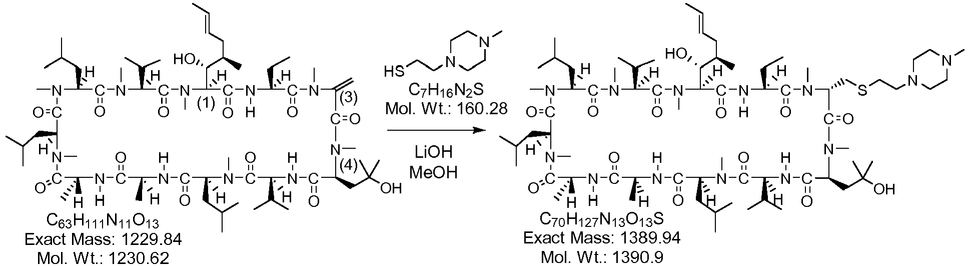

[α-Methylene-Sar]-3-[(γ-hydroxy)-N-MeLeu]-4-cyclosporin (0.30 g, 0.24 mmol) and 2-(4-methylpiperazino)ethylthiol (0.42 g, 2.62 mmol) were dissolved in methanol (30 ml), followed by adding 10 equivalents of lithium hydroxide. The mixture was stirred overnight at room temperature. After removal of solvents, the residue was dissolved in methylene chloride (30 ml). The dichloromethane solution was washed with brine, dried over magnesium sulfate and evaporated under reduced pressure. The residue was purified by flash chromatography using dichloromethane/methanol as eluent to give 0.22 g of product [Molecular Formula: C₇₀H₁₂₇N₁₃O₁₃S; Exact Mass: 1389.94; MS (m/z): 1390.9 (M+1)⁺; TLC R_{f}: 0.08 (ethyl acetate/methanol = 5/1); HPLC RT: 10.07 min. (C8 reverse phase column: 250 mm; acetonitrile/water (0.05% trifluoroacetic acid); operation temperature: 64 °C; detector: 210 nm)].

### Example 33

### [(S)-(2-(N-Pyrrolidinyl)ethylthio)methyl-Sar]-3-[(γ-hydroxy)-N-MeLeu]-4-cyclosporin

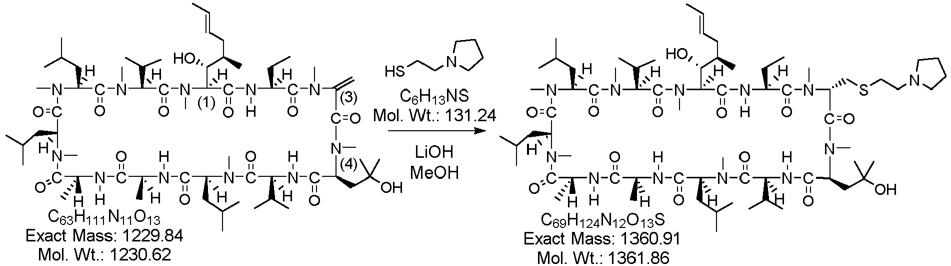

To a solution of [α-methylene-Sar]-3-[(**γ**-hydroxy)-N-MeLeu]-4-cyclosporin (280 mg, 0.23 mmol) and 2-(N-pyrrolidinyl)ethanethiol (280 mg, 2.14 mmol) in methanol (30 ml) was added lithium hydroxide (114 mg, 4.75 mmol). The reaction mixture was stirred overnight at room temperature. Most of solvent was evaporated under reduced pressure. Dichloromethane (30 ml) and water (30 ml) were added and the mixture was separated. The organic layer was washed with water and brine, dried over magnesium sulfate and evaporated under reduced pressure. The residue was purified by chromatography (dichloromethane/methanol = 96/4) to give 126 mg of product [Molecular Formula: C₆₉H₁₂₄N₁₂O₁₃S; Exact Mass: 1360.91; MS (m/z): 1361.80 (M+1)⁺; TLC R_{f}: 0.23 (dichloromethane/methanol = 95/5); HPLC RT: 11.59 min. (C8 reverse phase column: 250 mm; acetonitrile/0.077% ammonium acetate in water; operation temperature: 64 °C; detector: 210 nm)].

### Example 34

### [(S)-α-(2-Aminoethylthio)methyl-Sar]-3-[(γ-hydroxy)-N-MeLeu]-4-cyclosporin

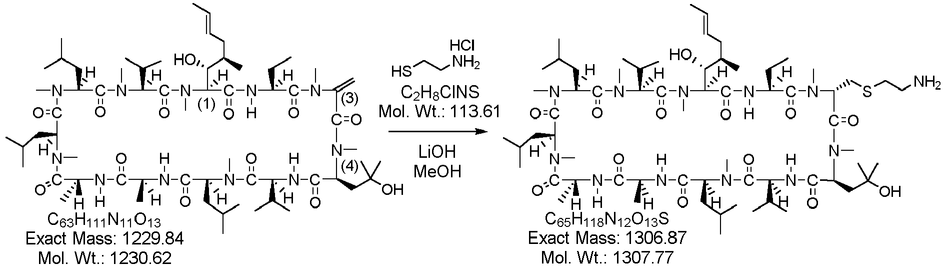

[α-Methylene-Sar]-3-[(**γ**-hydroxy)-N-MeLeu]-4-cyclosporin (0.86 g, 0.70 mmol) and 2-aminoethanethiol hydrochloride (0.80 g, 7.00 mmol) were dissolved in methanol (80 ml), followed by adding 20 equivalents of lithium hydroxide. The mixture was stirred overnight at room temperature. After removal of solvent, the residue was subjected to the flash chromatography using dichloromethane/methanol as eluent to give 0.60 g of product [Molecular Formula: C₆₅H₁₁₈N₁₂O₁₃S; Exact Mass: 1306.87; MS (m/z): 1307.56(M+1)⁺, 1329.73 (M+Na)⁺, TLC R_{f}: 0.025 (dichloromethane/methanol = 5/1); HPLC RT: 10.97 min. (C8 reverse phase column: 250 mm; acetonitrile/water (0.05% trifluoroacetic acid); operation temperature: 64 °C; detector: 210 nm)].

### Example 35

### [(S)-α-(2-(N-Isopropylamino)ethylthio)methyl-Sar]-3-[(γ-hydroxy)-N-MeLeu]-4-cyclosporin

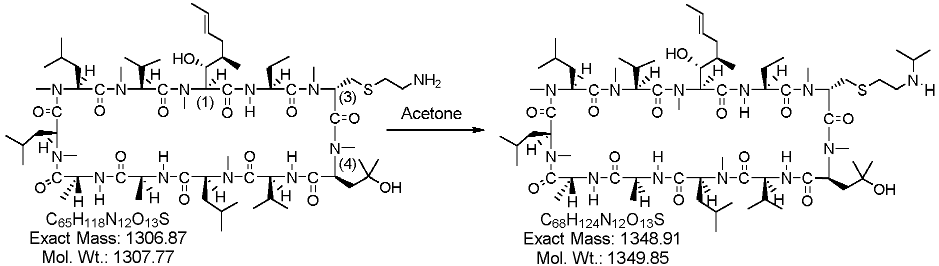

[(S)-α-(2-(Amino)ethylthio)methyl-Sar]-3-[(**γ**-hydroxy)-N-MeLeu]-4-cyclosporin (0.31 g, 0.25 mmol) and acetone (0.40 ml) were dissolved in chloroform (30 ml), followed by adding 2.5 equivalents of tetramethylammonium triacetoxyborohydride in portions and a few drops of acetic acid. The mixture was stirred at room temperature for two hours. Then the reaction mixture was washed with saturated sodium bicarbonate solution and brine, dried over magnesium sulfate and evaporated under reduced pressure. The residue was purified by flash chromatography on silica gel using methylene/methanol as eluent to give 0.25 g of pure product [Molecular Formula: C₆₈H₁₂₄N₁₂O₁₃S; Exact Mass: 1348.91; MS (m/z): 1349.59 (M+1)⁺; TLC R_{f}: 0.1 (ethyl acetate/methanol = 5/1); HPLC RT: 11.97 min.(C8 reverse phase column: 250 mm; acetonitrile/water (0.05% trifluoroacetic acid); operation temperature: 64 °C; detector: 210 nm)].

### Example 36

### [(S)-(2-(N-Ethyl-N-isopropylamino)ethylthio)methyl-Sar]-3-[(γ-hydroxy)-N-MeLeu]-4-cyclosporin

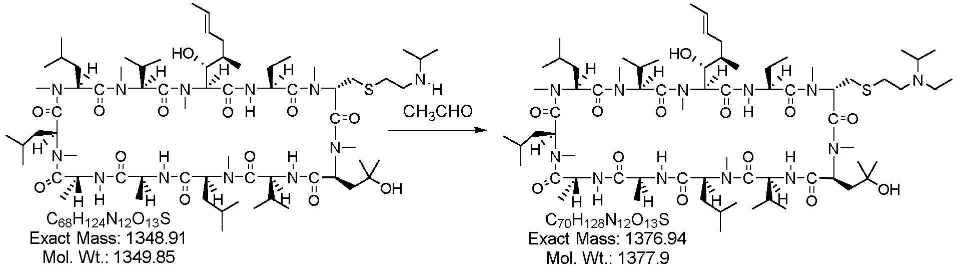

[(S)-(2-(N-Isopropylamino)ethylthio)methyl-Sar]-3-[(**γ**-hydroxy)-N-MeLeu]-4-cyclosporin (49 mg, 0.034 mmol) and acetaldehyde (100 µl, 37% in water) were mixed with chloroform (10 ml), followed by adding 2.5 equivalents of tetramethylammonium triacetoxyborohydride. The mixture was stirred at room temperature for two hours. Then the reaction mixture was washed with saturated sodium bicarbonate solution and brine, dried over magnesium sulfate and evaporated under reduced pressure. The residue was purified by flash chromatography on silica gel using dichloromethane/methanol as eluent to give 37 mg of pure product [Molecular Formula: C₇₀H₁₂₈N₁₂O₁₃S; Exact Mass: 1376.94; MS (m/z): 1377.84 (M+1)⁺; TLC R_{f}: 0.15 (ethyl acetate/methanol = 5/1); HPLC RT: 12.36 min. (C8 reverse phase column: 250 mm; acetonitrile/water (0.05% trifluoroacetic acid); operation temperature: 64 °C; detector: 210 nm)].

### Example 37

### [(S)-(2-(N-Isopropyl-N-methylamino)ethylthio)methyl-Sar]-3-[(γ-hydroxy)-N-MeLeu]-4-cyclosporin

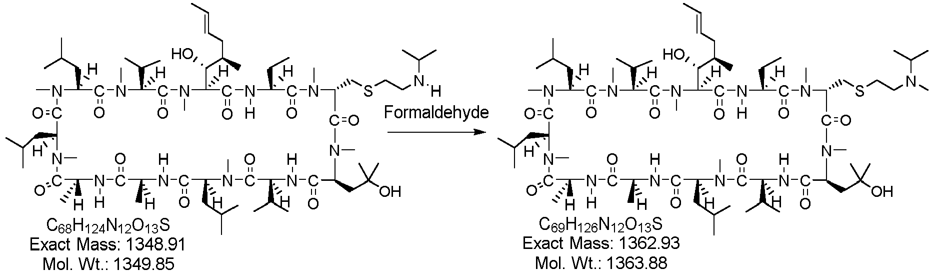

[(S)-(2-(N-Isopropylamino)ethylthio)methyl-Sar]-3-[(**γ**-hydroxy)-N-MeLeu]-4-cyclosporin (49 mg, 0.034 mmol) and formaldehyde (100 µl, 37% in water) were mixed with chloroform (10 ml), followed by adding 2.5 equivalents of tetramethylammonium triacetoxyborohydride. The mixture was stirred at room temperature for two hours. Then the reaction mixture was washed with saturated sodium bicarbonate solution and brine, dried over magnesium sulfate and evaporated under reduced pressure. The residue was purified by flash chromatography on silica gel using dichloromethane/methanol as eluent to give 30 mg of pure product [Molecular Formula: C₆₉H₁₂₆N₁₂O₁₃S; Exact Mass: 1362.93; MS (m/z): 1363.72(M+1)⁺, 1385.81(M+Na)⁺; TLC R_{f}: 0.15 (ethyl acetate/methanol= 5:1); HPLC RT: 12.26 min. (C8 reverse phase column: 250 mm; acetonitrile/water (0.05% trifluoroacetic acid); operation temperature: 64 °C; detector: 210 nm)].

### Example 38

### [(S)-(2-(N,N-Diisobutylamino)ethylthio)methyl-Sar]-3-[(γ-hydroxy)-N-MeLeu]-4-cyclosporin

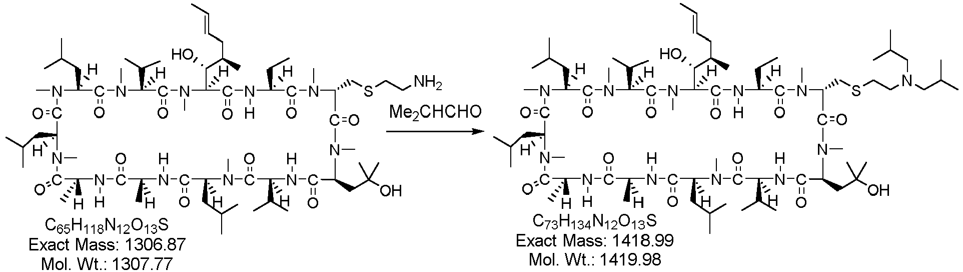

[(S)-α-(2-Aminoethylthio)methyl-Sar]-3-[(γ-hydroxy)-N-MeLeu]-4-cyclosporin (42 mg, 0.032 mmol) and isobutyraldehyde (15 µl) was dissolved in chloroform (10 ml), followed by adding 2.5 equivalents of tetramethylammonium triacetoxyborohydride in portions. The mixture was stirred at room temperature for two hours. Then the reaction mixture was washed with saturated sodium bicarbonate solution and brine, dried over magnesium sulfate and evaporated under reduced pressure. The residue was purified by flash chromatography on silica gel using dichloromethane/methanol as eluent to give 23 mg of pure product [Molecular Formula: C₇₃H₁₃₄N₁₂O₁₃S; Exact Mass: 1418.99; MS (m/z): 1419.73(M+1)⁺, 1441.87(M+Na)⁺; TLC R_{f}: 0.36 (ethyl acetate/methanol = 5:1); HPLC RT: 14.46 min. (C8 reverse phase column: 250 mm; acetonitrile/water (0.05% trifluoroacetic acid); operation temperature: 64 °C; detector: 210 nm)].

### Example 39

### [(S)-(2-(N-Isobutylamino-N-isopropyl)ethylthio)methyl-Sar]-3-[(y-hydroxy)-N-MeLeu]-4-cyclosporin

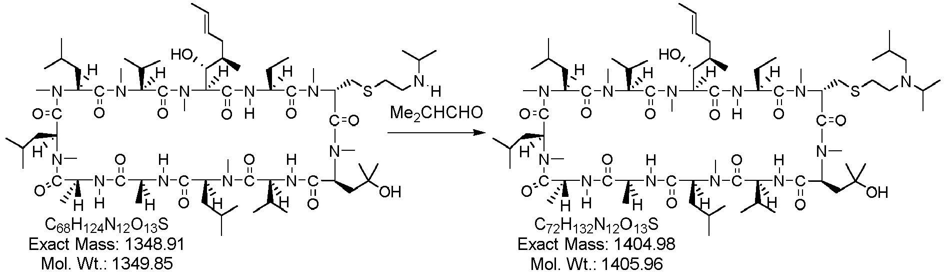

[(S)-(2-(N-isopropylamino)ethylthio)methyl-Sar]-3-[(γ-hydroxy)-N-MeLeu]-4-cyclosporin (0.25 g, 0.20 mmol) and isobutyraldehyde (91 µl, 10 mmol) were dissolved in chloroform (30 ml), followed by adding 2.5 equivalents of tetramethylammonium triacetoxyborohydride in portion. The mixture was stirred at room temperature for two hours. Then the reaction mixture was washed with saturated sodium bicarbonate solution and brine, dried over magnesium sulfate and evaporated under reduced pressure. The residue was purified by flash chromatography on silica gel using dichloromethane/methanol as eluent to give 19 mg of pure product [Molecular Formula: C₇₂H₁₃₂N₁₂O₁₃S; Exact Mass: 1404.98; MS (m/z): 1405.89 (M+1)⁺, 1427.94 (M+Na)⁺; TLC R_{f}: 0.25 (ethyl acetate/methanol = 5/1); HPLC RT: 14.46 min. (C8 reverse phase column: 250 mm; acetonitrile/water (0.05% trifluoroacetic acid); operation temperature: 64 °C; detector: 210 nm)].

### Example 40

### [(S)-(2-(N-Neopentylamino)ethylthio)methyl-Sar]-3-[(γ-hydroxy)-N-MeLeu]-4-cyclosporin

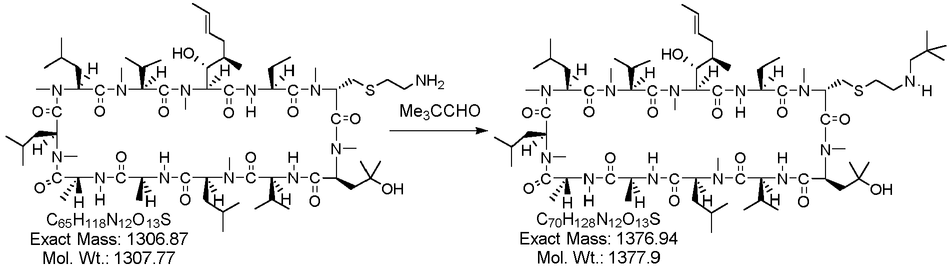

[(S)-(2-(Amino)ethylthio)methyl-Sar]-3-[(γ-hydroxy)-N-MeLeu]-4-cyclosporin (0.45 g, 0.34 mmol) and pivalaldehyde (100 µl, 37% in water) were mixed with chloroform (50 ml), followed by adding 2.5 equivalents of tetramethylammonium triacetoxyborohydride. The mixture was stirred at room temperature for two hours. Then the reaction mixture was washed with saturated sodium bicarbonate solution and brine, dried over magnesium sulfate and evaporated under reduced pressure. The residue was purified by flash chromatography on silica gel using dichloromethane/methanol as eluent to give 11 mg of pure product [Molecular Formula: C₇₀H₁₂₈N₁₂O₁₃S; Exact Mass: 1376.94; MS (m/z): 1377.72 (M+1)⁺, 1399.82 (M+Na)⁺; TLC R_{f}: 0.15 (ethyl acetate/methanol = 5/1); HPLC RT: 12.36 min. (C8 reverse phase column: 250 mm; acetonitrile/water (0.05% trifluoroacetic acid); operation temperature: 64 °C; detector: 210 nm)].

### Example 41

### [(S)-(2-(N-Methyl-N-neopentylamino)ethylthio)methyl-Sar]-3-[(γ-hydroxy)-N-MeLeu]-4-cyclosporin

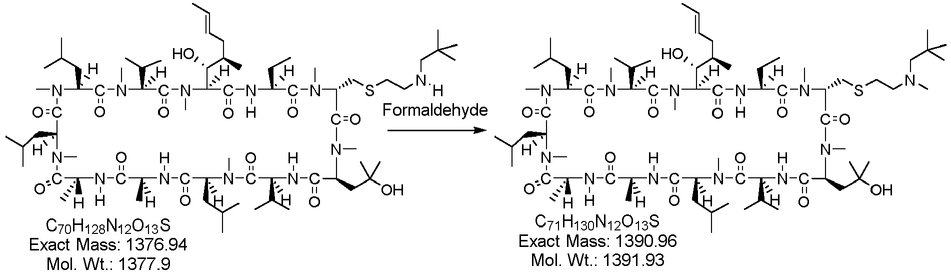

[(S)-(2-(N-Neopentylamino)ethylthio)methyl-Sar]-3-[(γ-hydroxy)-N-MeLeu]-4-cyclosporin (49 mg, 0.034 mmol) and formaldehyde (100 µl, 37% in water) were mixed with chloroform (10 ml), followed by adding 2.5 equivalents of tetramethylammonium triacetoxyborohydride. The mixture was stirred at room temperature for two hours. Then the reaction mixture was washed with saturated sodium bicarbonate solution and brine, dried over magnesium sulfate and evaporated under reduced pressure. The residue was purified by flash chromatography on silica gel using dichloromethane/methanol as eluent to give 31 mg of pure product [Molecular Formula: C₇₁H₁₃₀N₁₂O₁₃S; Exact Mass: 1390.96; MS (m/z): 1391.71 (M+1)⁺, 1413.86 (M+Na)⁺; TLC R_{f}: 0.25 (ethyl acetate/methanol = 5/1); HPLC RT: 13.28 min.(C8 reverse phase column: 250 mm; acetonitrile/water (0.05% trifluoroacetic acid); operation temperature: 64 °C; detector: 210 nm)].

### Example 42

### [(S)-(2-(N-Ethyl-N-neopentylamino)ethylthio)methyl-Sar]-3-[(γ-hydroxy)-N-MeLeu]-4-cyclosporin

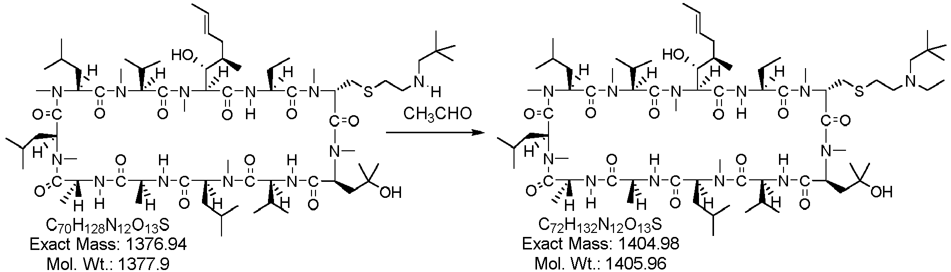

[(S)-(2-(N-Neopentylamino)ethylthio)methyl-Sar]-3-[(γ-hydroxy)-N-MeLeu]-4-cyclosporin (46 mg, 0.034 mmol) and acetaldehyde (10 µl, 0.17 mmol) were dissolved in chloroform/methanol, followed by adding 2.5 equivalents of tetramethylammonium triacetoxyborohydride in portions. The mixture was stirred at room temperature for two hours. Then the reaction mixture was washed with saturated sodium bicarbonate solution and brine, dried over magnesium sulfate and evaporated under reduced pressure. The residue was purified by flash chromatography on silica gel using dichloromethane/methanol as eluent to give 28 mg of product [Molecular Formula: C₇₂H₁₃₂N₁₂O₁₃S; Exact Mass: 1404.98; MS (m/z): 1405.75 (M+1)⁺, 1427.95 (M+Na)⁺; TLC R_{f}: 0.25 (ethyl acetate/methanol = 5/1); HPLC RT: 13.65 min. (C8 reverse phase column: 250 mm; acetonitrile/water (0.05% trifluoroacetic acid); operation temperature: 64 °C; detector: 210 nm)].

### Example 43

### [(S)-(3-(N-Piperidinyl)propylthio)methyl-Sar]-3-[(γ-hydroxy)-N-MeLeu]-4-cyclosporin

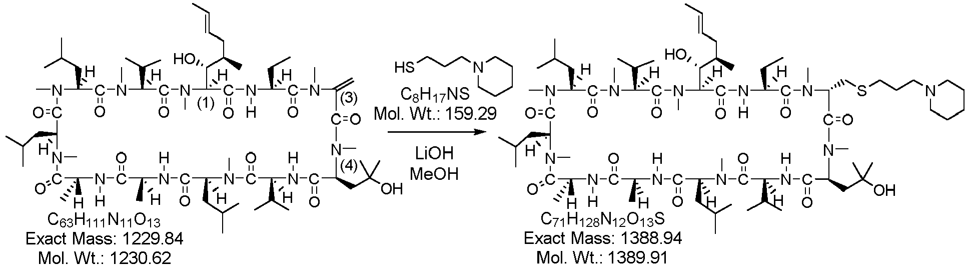

To a solution of [α-methylene-Sar]-3-[(γ-hydroxy)-N-MeLeu]-4-cyclosporin (250 mg, 0.20 mmol) and 3-(N-piperidinyl)propanethiol (318 mg, 2.00 mmol) in methanol (30 ml) was added lithium hydroxide (96 mg, 4.00 mmol). The reaction mixture was stirred at room temperature overnight. Then most of solvent was evaporated under reduced pressure. Dichloromethane (30 ml) and water (30 ml) were added and the mixture was separated. The organic layer was washed with water and brine, dried over magnesium sulfate and evaporated under reduced pressure. The residue was purified by chromatography (dichloromethane/methanol = 96/4) to give 135 mg of product [Molecular Formula: C₇₁H₁₂₈N₁₂O₁₃S; Exact Mass: 1388.94; MS (m/z): 1389.84 (M+1)⁺; TLC Rf: 0.30 (dichloromethane/methanol = 95/5); HPLC RT: 12.19 min. (C8 reverse phase column: 250 mm; acetonitrile/0.077% ammonium acetate in water; operation temperature: 64 °C; detector: 210 nm); ¹H NMR spectrum (600 MHz, CDCl₃, δ in ppm): 0.68 (d, J=6.0Hz, 3H), 0.79 (d, J=6.0Hz, 3H), 0.82-0.86 (m, 9H), 0.88 (d, J=6.6Hz, 3H), 0.91 (d, J=6.6Hz, 3H), 0.93 (d, J=6.6Hz, 3H), 0.97-1.00 (m, 9H), 1.09 (d, J=6.6Hz, 3H), 1.21-1.24 (m, 11H), 1.31-1.46 (m, 8H), 1.53 (m, 5H), 1.61 (m, 11H), 1.67-1.70 (m, 2H), 1.74-1.76 (m, 2H), 1.99-2.11 (m, 4H), 2.31-2.35 (m, 4H), 2.37-2.41 (m, 2H), 2.53-2.60 (m, 2H), 2.67 (s, 6H), 2.91-2.98 (m. 2H), 3.09 (s, 3H), 3.24 (s, 6H), 3.26 (s, 3H), 3.48 (s, 3H), 3.56 (m, 1H), 3.65 (m, 1H), 4.51 (m, 1H), 4.58 (t, J=8.4Hz, 1H), 4.81 (m, 1H), 4.94-5.02 (m, 2H), 5.04 (t, J=6.6Hz, 1H), 5.08 (d, J=10.8Hz, 1H), 5.28-5.32 (m, 1H), 5.33-5.37 (m, 1H), 5.49 (m, 2H), 5.67 (dd, J=10.8Hz and 3.6Hz, 1H), 7.14 (d, J=8.4Hz, 1H), 7.49 (d, J=8.4Hz, 1H), 7.64 (d, J=8.4Hz, 1H), 8.09 (d, J=10.2Hz, 1H)].

### Example 44

### [(S)-(3-(N-Pyrrolidinyl)propylthio)methyl-Sar]-3-[(γ-hydroxy)-N-MeLeu]-4-cyclosporin

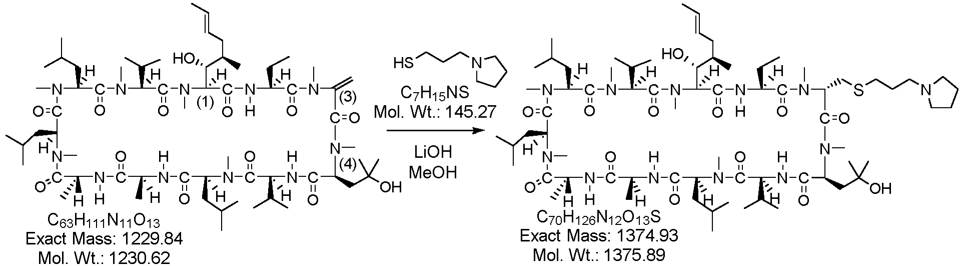

To a solution of [α-methylene-Sar]-3-[(γ-hydroxy)-N-MeLeu]-4-cyclosporin (213 mg, 0.17 mmol) and 3-(N-pyrrolidinyl)propanethiol (280 mg, 1.93 mmol) in methanol (25 ml) was added lithium hydroxide (94mg, 3.92 mmol). The reaction mixture was stirred at room temperature overnight. Then most of solvent was evaporated under reduced pressure. Dichloromethane (30 ml) and water (30 ml) were added and the mixture was separated. The organic layer was washed with water and brine, dried over magnesium sulfate and evaporated under reduced pressure. The residue was purified by chromatography (dichloromethane/methanol = 95/5) to give 57 mg of product [Molecular Formula: C₇₀H₁₂₆N₁₂O₁₃S; Exact Mass: 1374.93; MS (m/z): 1375.75 (M+1)⁺; TLC Rf: 0.23 (dichloromethane/methanol = 95/5); HPLC RT: 11.83 min. (C8 reverse phase column: 250 mm; acetonitrile/0.077% ammonium acetate in water; operation temperature: 64 °C; detector: 210 nm)].

### Example 45

### [(S)-(3-(N-Morpholino)propylthio)methyl-Sar]-3-[(γ-hydroxy)-N-MeLeu]-4-cyclosporin

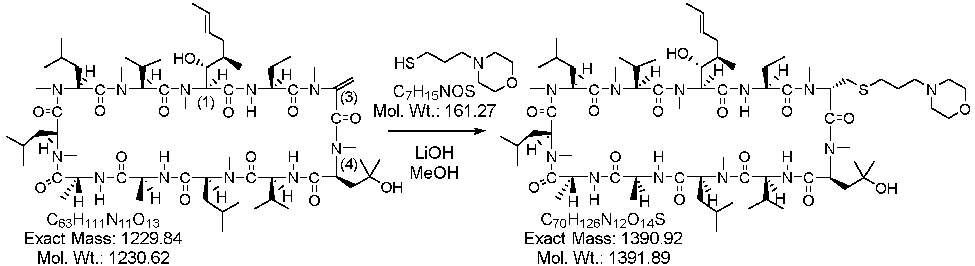

To the solution of [α-methylene-Sar]-3-[(γ-hydroxy)-N-MeLeu]-4-cyclosporin (210 mg, 0.17 mmol) and 3-morpholinopropanethiol (300 mg, 1.86 mmol) in methanol 25 ml was added lithium hydroxide (140 mg, 5.83 mmol). The reaction mixture was stirred at room temperature overnight. Then most of solvent was evaporated under reduced pressure. Dichloromethane (30 ml) and water (30 ml) were added and the mixture was separated. The organic layer was washed with water and brine, dried over magnesium sulfate and evaporated under reduced pressure. The residue was purified by chromatography (dichloromethane/methanol = 97/3) to give 78 mg of product [Molecular Formula: C₇₀H₁₂₆N₁₂O₁₄S; Exact Mass: 1390.92; MS (m/z): 1413.77 (M+Na)⁺; TLC Rf: 0.33 (dichloromethane/methanol = 9/1); HPLC RT: 11.35 min. (C8 reverse phase column: 250 mm; acetonitrile/0.077% ammonium acetate in water; operation temperature: 64 °C; detector: 210 nm); ¹H NMR spectrum (600 MHz, CDCl₃, δ in ppm): 0.68 (d, J=6.0Hz, 3H), 0.79 (d, J=5.4Hz, 3H), 0.82-0.86 (m, 9H), 0.88 (d, J=6.6Hz, 3H), 0.91 (d, J=6.6Hz, 3H), 0.93 (d, J=6.6Hz, 3H), 0.97-1.00 (m, 9H), 1.09 (d, J=6.6Hz, 3H), 1.21-1.24 (m, 11H), 1.31 (d, J=7.2Hz, 3H), 1.38-1.46 (m, 2H), 1.61 (m, 11H), 1.67-1.70 (m, 2H), 1.74-1.76 (m, 2H), 2.03-2.11 (m, 4H), 2.35-2.43 (m, 8H), 2.55-2.63 (m, 2H), 2.67 (s, 6H), 2.91-2.98 (m. 2H), 3.10 (s, 3H), 3.24 (3, 6H), 3.26 (s, 3H), 3.49 (s, 3H), 3.52 (m, 1H), 3.65-3.67 (m, 5H), 4.51 (m, 1H), 4.59 (t, J=8.4Hz, 1H), 4.81 (m, 1H), 4.94-5.01 (m, 2H), 5.04 (t, J=6.6Hz, 1H), 5.08 (d, J=12Hz, 1H), 5.28-5.30 (m, 1H), 5.33-5.37 (m, 1H), 5.49 (m, 2H), 5.67 (m, 1H), 7.14 (d, J=7.8Hz, 1H), 7.49 (d, J=8.4Hz, 1H), 7.65 (d, J=7.2Hz, 1H), 8.12 (d, J=9.6Hz, 1H)].

### Example 46

### [(S)-(3-(4-Methyl-N-piperazinyl)propylthio)methyl-Sar]-3-[(y-hydroxy)-N-MeLeu]-4-cyclosporin

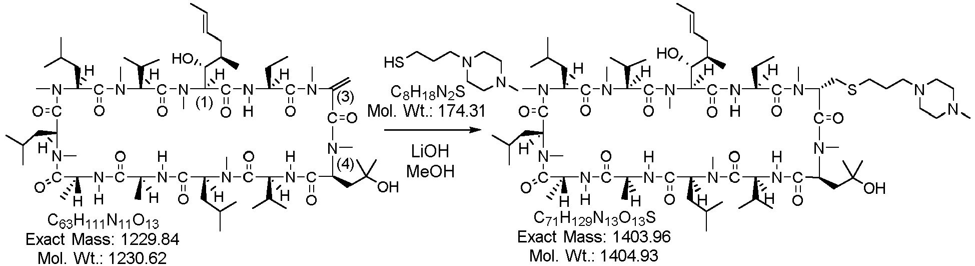

[α-Methylene-Sar]-3-[(γ-hydroxy)-N-MeLeu]-4-cyclosporin (0.30 g, 0.24 mmol) and 3-(4-methylpiperazino)propylthiol (0.42 g, 2.44 mmol) were dissolved in methanol (25 ml), followed by adding 10 equivalents of lithium hydroxide. The mixture was stirred overnight at room temperature. After removal of solvent, the residue was purified by flash chromatography using dichloromethane/methanol as eluent to give 0.20 g of product [Molecular Formula: C₇₁H₁₂₉N₁₃O₁₃S; Exact Mass: 1403.96; MS (m/z): 1404.9 (M+1)⁺, 1426.9 (M+Na)⁺; TLC R_{f}: 0.10 (ethyl acetate/methanol = 5/1); HPLC RT: 10.07 min. (C8 reverse phase column: 250 mm; acetonitrile/water (0.05% trifluoroacetic acid); operation temperature: 64 °C; detector: 210 nm)].

### Example 47

### [(S)-(3-(N,N-Dimethylamino)propylthio)methyl-Sar]-3-[(γ-hydroxy)-N-MeLeu]-4-cyclosporin

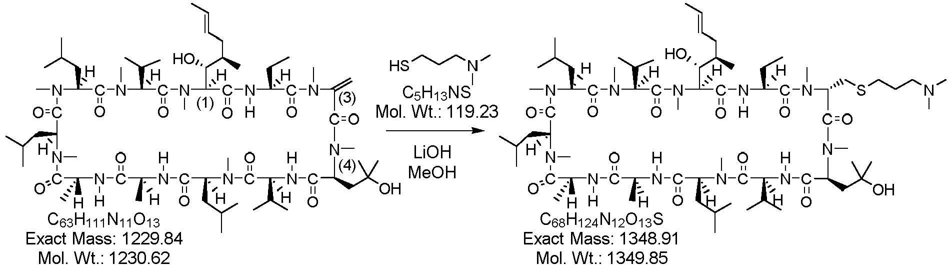

[α-Methylene-Sar]-3-[(γ-hydroxy)-N-MeLeu]-4-cyclosporin (0.30 g, 0.24 mmol) and 3-(N,N-dimethyl)propylthiol (0.36 g, 2.40 mmol) were dissolved in methanol (25 ml), followed by adding lithium hydroxide (59 mg, 2.44 mmol). The mixture was stirred at room temperature overnight. After removal of solvent, the residue was purified by flash chromatography using dichloromethane/methanol as eluent to give 0.18 g of pure product [Molecular Formula: C₆₈H₁₂₄N₁₂O₁₃S; Exact Mass: 1348.91; MS (m/z): 1349.70 (M+1)⁺, 1371.83 (M+Na); TLC R_{f}: 0.15 (ethyl acetate/methanol = 5/1); HPLC RT: 11.53 min. (C8 reverse phase column: 250 mm; acetonitrile/water (0.05% trifluoroacetic acid); operation temperature: 64 °C; detector: 210 nm)].

### Example 48

### [(S)-(3-(N,N-Diethylamino)propylthio)methyl-Sar]-3-[(γ-hydroxy)-N-MeLeu]-4-cyclosporin

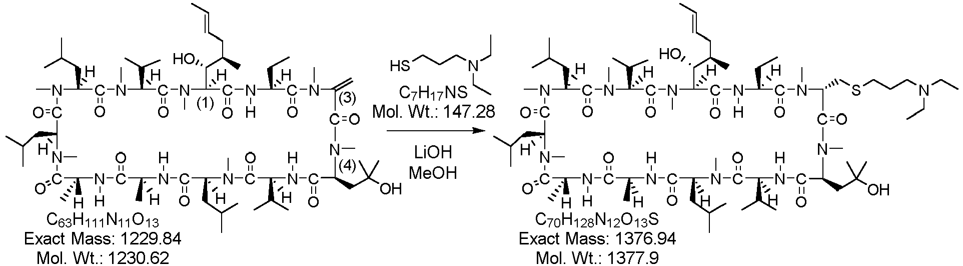

[α-Methylene-Sar]-3-[(γ-hydroxy)-N-MeLeu]-4-cyclosporin (0.30 g, 0.24 mmol) and 3-(N,N-dimethyl)propylthiol (0.36 g, 2.44 mmol) were dissolved in methanol (25 ml), followed by adding lithium chloride (59 mg, 2.4 mmol). The mixture was stirred at room temperature overnight. After removal of solvent, the residue was purified by flash chromatography using dichloromethane/methanol as eluent to give 0.30 g of product [Molecular Formula: C₇₀H₁₂₈N₁₂O₁₃S; Exact Mass: 1376.94; MS (m/z): 1377.90 (M+1)⁺, 1399.76 (M+Na)⁺; TLC R_{f}: 0.17 (ethyl acetate/methanol = 5/1); HPLC RT: 12.06 min. (C8 reverse phase column: 250 mm; acetonitrile/water (0.05% trifluoroacetic acid); operation temperature: 64 °C; detector: 210 nm)].

### Example 49

### [(S)-(3-(N-Ethyl-N-isopropylamino)propylthio)methyl-Sar]-3-[(γ-hydroxy)-N-MeLeu]-4-cyclosporin

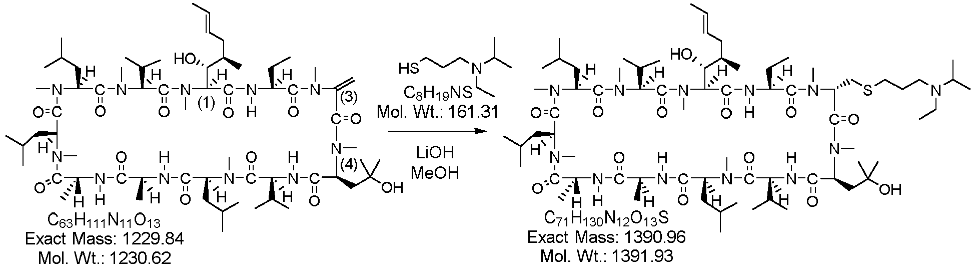

To a solution of [α-methylene-Sar]-3-[(γ-hydroxy)-N-MeLeu]-4-cyclosporin (200 mg, 0.16 mmol) and 3-(N-ethyl-N-isopropylamino)propylthiol (200 mg, 1.25 mmol) in methanol (25 ml) was added lithium hydroxide (89 mg, 3.71 mmol). The reaction mixture was stirred at room temperature overnight. Then most of solvent was evaporated under reduced pressure. Dichloromethane (30 ml) and water (30 ml) were added and the mixture was separated. The organic layer was washed with water and brine, dried over magnesium sulfate and evaporated under reduced pressure. The residue was purified by chromatography (dichloromethane/methanol = 97/3) to give 88 mg of product [Molecular Formula: C₇₁H₁₃₀N₁₂O₁₃S; Exact Mass: 1390.96; MS (m/z): 1413.81 (M+Na)⁺; TLC Rf: 0.40 (dichloromethane/methanol = 9/1); HPLC RT: 12.49 min. (C8 reverse phase column: 250 mm; acetonitrile/0.077% ammonium acetate in water; operation temperature: 64 °C; detector: 210 nm)].

### Example 50 [(R)-(2-(N,N-Diethylamino)ethoxy)methyl-Sar]-3-[(γ-hydroxy)-N-MeLeu]-4-cyclosporin

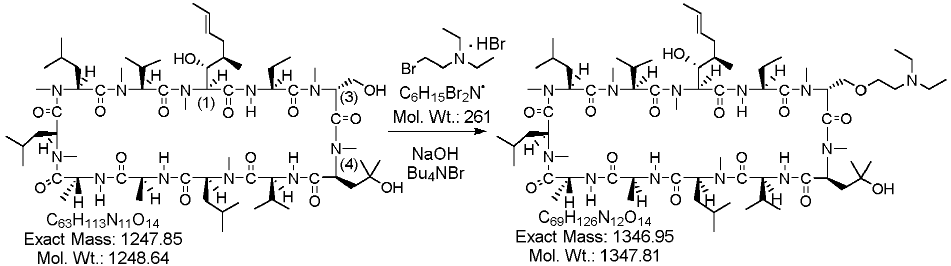

To a solution of [(R)-α-hydroxymethyl-Sar]-3-[(γ-hydroxy)-N-MeLeu]-4-cyclosporin (0.25 g, 0.20 mmol) in benzene (30 ml) were added a solution of sodium hydroxide (1.00 g, 25 mmol) in water (2 ml), 2-bromo-N,N-diethylethylamine hydrobromide (2.80 g, 10.72 mmol) and tetra-n-butylammonium bromide (0.2 g, 0.62 mmol). The mixture was stirred at 30 °C for 20 hours. Then ice water (30 ml) was added and the mixture was separated. The aqueous layer was extracted with dichloromethane (25 ml). The combined organic layers were washed with brine, dried over magnesium sulfate and concentrated under reduced pressure. The residue was purified by chromatography (dichloromethane/methanol = 96/4) to give 240 mg of product [Molecular Formula: C₆₉H₁₂₆N₁₂O₁₄; Exact Mass: 1346.95; MS (m/z): 1347.59 (M+1)⁺; TLC Rf: 0.41 (dichloromethane/methanol = 9/1); HPLC RT: 12.20 min. (C8 reverse phase column: 250 mm; acetonitrile/0.077% ammonium acetate in water; operation temperature: 64 °C; detector: 210 nm)].

### Example 51

### [(R)-(2-(N-Piperidinyl)ethoxy)methyl-Sar]-3-[(γ-hydroxy)-N-MeLeu]-4-cyclosporin

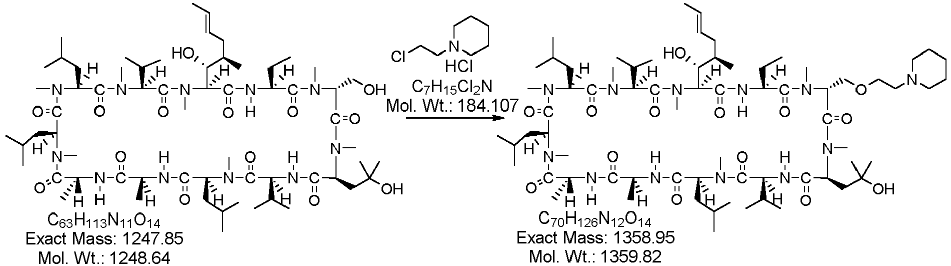

To a solution of [(R)-α-hydroxymethyl-Sar]-3-[(γ-hydroxy)-N-MeLeu]-4-cyclosporin (300 mg, 0.24 mmol) in benzene (15 ml) were added sodium hydroxide (0.38 g. 9.60 mmol), tetramethylammonium hydroxide pentahydrate (0.44 g, 2.40 mmol) and 1-(2-chloroethyl)piperidine hydrochloride (0.44 g, 2.40 mmol). The mixture was stirred at 30 °C for 36 hours. Then ice water (20 ml) was added and the mixture was separated. The aqueous layer was extracted with ethyl acetate (20 ml). The combined organic layers were washed with brine, dried over magnesium sulfate and evaporated under reduced pressure. The residue was purified by chromatography on silica gel with dichloromethane/methanol (95/5) as eluent to give 100 mg of pure product [Molecular Formula: C₇₀H₁₂₆N₁₂O₁₄; Exact Mass: 1358.95; MS (m/z): 1359.69 (M+1)⁺, 1381.75 (M+Na)⁺; TLC R_{f}: 0.05 (dichloromethane/methanol = 20/1); HPLC RT: 12.43 min. (C8 reverse phase column: 150 mm; acetonitrile/water (0.05% trifluoroacetic acid); operation temperature: 64 °C; detector: 210 nm)].

### Example 52

### [(R)-(2-(N-Morpholino)ethoxy)methyl-Sar]-3-[(γ-hydroxy)-N-MeLeu]-4-cyclosporin

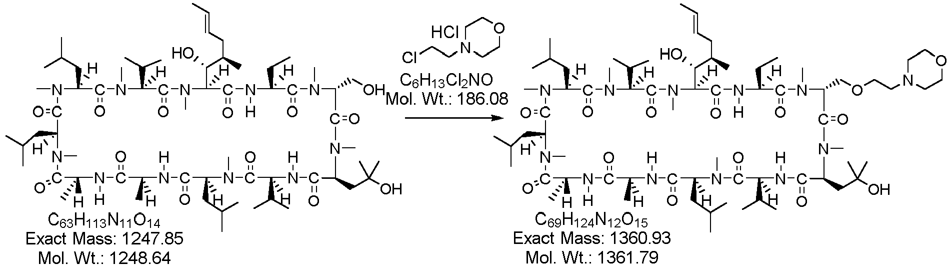

To a solution of [(R)-α-hydroxymethyl-Sar]-3-[(γ-hydroxy)-N-MeLeu]-4-cyclosporin (0.50 g, 0.40 mmol) in benzene (20 ml) were added sodium hydroxide (0.64 g, 16.00 mmol), tetramethylammonium hydroxide pentahydrate (0.72 g, 4.00 mmol) and 4-(2-chloroethyl)morphorline hydrochloride (0.74 g, 4.00 mmol). The mixture was stirred at 30 °C for a week. Then ice water (20 ml) was added and the mixture was separated. The aqueous layer was extracted with ethyl acetate (20 ml). The combined organic layers were washed with brine, dried over magnesium sulfate and evaporated under reduced pressure. The residue was purified by chromatography on silica gel with dichloromethane/methanol (95/5) as eluent to give 60 mg of product [Molecular Formula: C₆₉H₁₂₄N₁₂O₁₅; Exact Mass: 1360.93; MS (m/z): 1361.63 (M+1)⁺, 1383.75 (M+Na)⁺; TLC R_{f}: 0.10 (dichloromethane/methanol = 5:1); HPLC RT: 11.49 min. (C8 reverse phase column: 150 mm; acetonitrile/water (0.05% trifluoroacetic acid); operation temperature: 64 °C; detector: 210 nm)].

### Example 53

### [(R)-(2-(N,N-Dimethylamino)ethoxy)methyl-Sar]-3-[(γ-hydroxy)-N-MeLeu]-4-cyclosporin

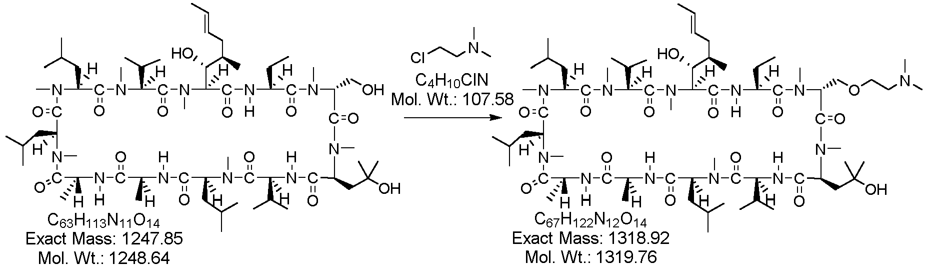

To a solution of [(R)-α-hydroxymethyl-Sar]-3-[(γ-hydroxy)-N-MeLeu]-4-cyclosporin (0.37 g, 0.30 mmol) in benzene (15 ml) were added sodium hydroxide (0.48 g. 12.00 mmol), tetramethylammonium hydroxide pentahydrate (0.54 g, 3.00 mmol) and 3-dimethylaminoethyl chloride hydrochloride (0.43 g, 3.00 mmol). The mixture was stirred at 30 °C for 36 hours. Then ice water (20 ml) was added and the mixture was separated. The aqueous layer was extracted with ethyl acetate (20 ml). The combined organic layers were washed with brine, dried over magnesium sulfate and evaporated under reduced pressure. The residue was purified by chromatography on silica gel with dichloromethane/methanol (95/5) as eluent to give 90 mg of pure product [Molecular Formula: C₆₇H₁₂₂N₁₂O₁₄; Exact Mass: 1318.92; MS (m/z):1319.70 (M+1)⁺, 1341.80 (M+Na)⁺); TLC R_{f}: 0.05 (dichloromethane/methanol = 5:1); HPLC RT: 11.43 min. (C8 reverse phase column: 150 mm; acetonitrile/water (0.05% trifluoroacetic acid); operation temperature: 64 °C; detector: 210 nm)].

### Example 54

### [(R)-(2-(N-Pyrrolidinyl)ethoxy)methyl-Sar]-3-[(γ-hydroxy)-N-MeLeu]-4-cyclosporin

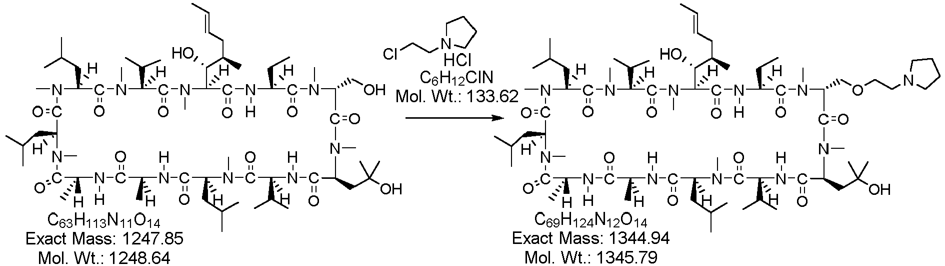

To a solution of [(R)-α-hydroxymethyl-Sar]-3-[(γ-hydroxy)-N-MeLeu]-4-cyclosporin (0.38 g, 0.30 mmol) in benzene (15 ml) were added sodium hydroxide (0.48 g, 12.00 mmol), tetramethylammonium hydroxide pentahydrate (0.54 g, 3.00 mmol) and 1-(2-chloroethyl)pyrrolidine hydrochloride (0.44 g, 3.00 mmol). The mixture was stirred at 30 °C for 36 hours. Then ice water (20 ml) was added and the mixture was separated. The aqueous layer was extracted with ethyl acetate (20 ml). The combined organic layers were washed with brine, dried over magnesium sulfate and evaporated under reduced pressure. The residue was purified by chromatography on silica gel with dichloromethane/methanol (95/5) as eluent to give to give 120 mg of the expected isomer [Molecular Formula: C₆₉H₁₂₄N₁₂O₁₄; Exact Mass:1344.94; MS (m/z): 1345.62 (M+1)⁺, 1367.76 (M+Na)⁺; TLC R_{f}: 0.05 (dichloromethane/methanol = 10/1); HPLC RT: 12.09 min. (C8 reverse phase column: 150 mm; acetonitrile/water (0.05% trifluoroacetic acid); operation temperature: 64 °C; detector: 210 nm)].

### Example 55

### [(R)-α-(2-(1,3-Dioxan-2-yl)ethoxy)methyl-Sar]-3-[(γ-hydroxy)-N-MeLeu]-4-cyclosporin

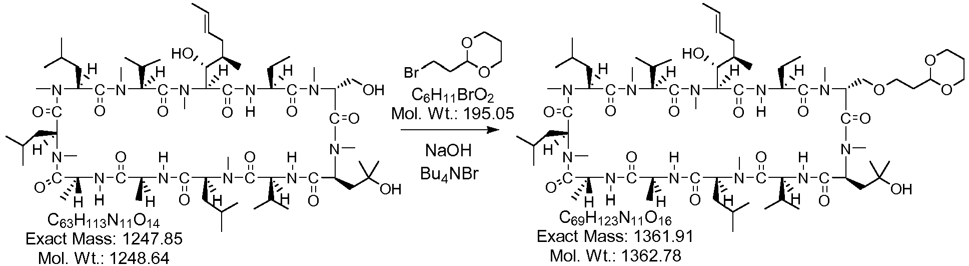

[(R)-α-Hydroxymethyl-Sar]-3-[(γ-hydroxy)-N-MeLeu]-4-cyclosporin (5.00 g, 4.01 mmol) was dissolved in benzene (100 ml). 2-(2-Bromoethyl)-1,3-dioxane (7.82g, 40.10 mmol), tetra-n-butylammonium bromide (0.99 g, 3.09 mmol), sodium hydroxide (3.21 g, 8.02 mmol) and water (3.3 ml) were added. The reaction mixture was stirred at 35 °C for nine hours. And the stirring was continued overnight at room temperature. Then 50 ml of brine was added and the mixture was separated. The aqueous layer was extracted with ethyl acetate (25 ml × 2). The combined organic layers were dried over magnesium sulfate and evaporated under reduced pressure. After purified on silica gel with hexane/acetone as eluent, 1.50 g of product obtained [Molecular Formula: C₆₉H₁₂₃N₁₁O₁₆; Exact Mass: 1361.91; (m/z): 1362.64 (M+1)⁺, 1384.85 (M+Na)⁺].

### Example 56

### [(R)-α-(2-Formylethoxy)methyl-Sar]-3-[(γ-hydroxy)-N-MeLeu]-4-cyclosporin

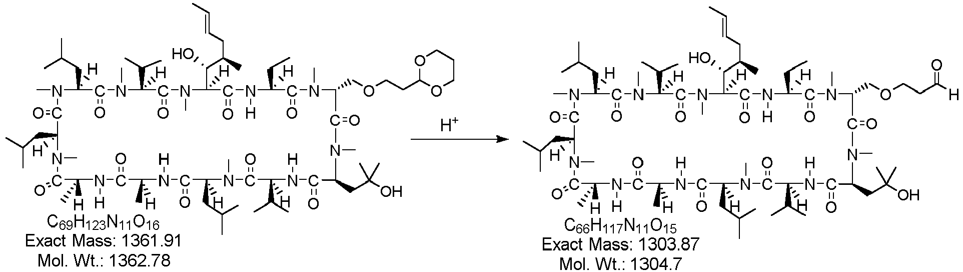

[(R)-α-(2-(1,3-Dioxan-2-yl)ethoxy)methyl-Sar]-3-[(γ-hydroxy)-N-MeLeu]-4-cyclosporin (1.29 g, 0.95 mmol) was dissolved in dioxane (25 ml), followed by adding hydrochloric acid solution (1 N, 25 ml). The reaction mixture was stirred overnight at room temperature. Most of dioxane was evaporated under reduced pressure. Then the aqueous layer was extracted with ethyl acetate (25 ml × 2). The combined ethyl acetate layers were dried over magnesium sulfate and evaporated under reduced pressure. The residue was purified on silica gel with hexane/acetone as eluent to give 600 mg of product [Molecular Formula: C₆₆H₁₁₇N₁₁O₁₅; Exact Mass: 1303.87; MS (m/z): 1304.59 (M+1)⁺, 1326.78 (M+Na)⁺; HPLC RT: 14.2 min. (C8 reverse phase column: 250 mm; acetonitrile/water (0.05% trifluoroacetic acid); operation temperature: 64 °C; detector: 210 nm)].

### Example 57

### [(R)-(3-(N-Morpholino)propoxy)methyl-Sar]-3-[(γ-hydroxy)-N-MeLeu]-4-cyclosporin

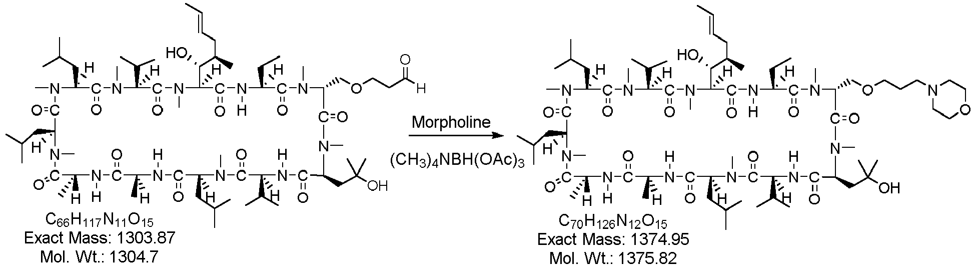

[(R)-(2-Formylethoxy)methyl-Sar]-3-[(y-hydroxy)-N-MeLeu]-4-cyclosporin (300 mg, 0.23 mmol) was dissolved in dichloromethane (15 ml). Morpholine (100 mg, 1.15 mmol) and tetramethylammonium triacetoxyborohydride (302 mg, 1.15 mmol) were added. The reaction mixture was stirred overnight at room temperature. Then sodium bicarbonate saturated solution (30 ml) and dichloromethane (15ml) were added and the mixture was separated. The dichloromethane layer was dried over magnesium sulfate and evaporated under reduced pressure. After purified on silica gel, 105 mg of pure product was obtained [Molecular Formula: C₇₀H₁₂₆N₁₂O₁₅; Exact Mass: 1374.95; MS (m/z): 1375.70 (M+1)⁺, 1397.80 (M+Na)⁺; TLC R_{f}: 0.37 (dichloromethane/methanol = 9/1); HPLC RT: 12.2 min. (C8 reverse phase column: 250 mm; acetonitrile/0.077% ammonium acetate in water; operation temperature: 64 °C; detector: 210 nm)].

### Example 58

### [(R)-(3-(N-Pyrrolidinyl)propoxy)methyl-Sar]-3-[(γ-hydroxy)-N-MeLeu]-4-cyclosporin

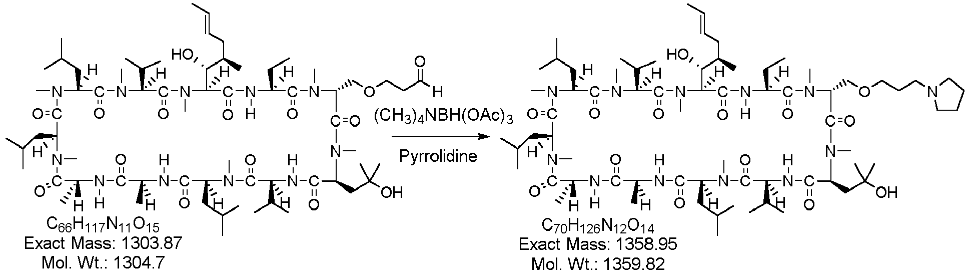

[(R)-α-(2-Formylethoxy)methyl-Sar]-3-[(γ-hydroxy)-N-MeLeu]-4-cyclosporin (200 mg, 0.15 mmol) was dissolved in dichloromethane (15 ml). Pyrrolidine (95 mg, 1.34 mmol) and tetramethylammonium triacetoxyborohydride (353 mg, 1.34 mmol) were added. The reaction mixture was stirred overnight at room temperature. Then sodium bicarbonate saturated solution (30 ml) and dichloromethane (15 ml) were added and the mixture was separated. The dichloromethane layer was dried over magnesium sulfate and evaporated under reduced pressure. After purified on silica gel, 50 mg of pure product was obtained [Molecular Formula: C₇₀H₁₂₆N₁₂O₁₄; Exact Mass: 1358.95; MS (m/z): 1359.74 (M+1)⁺, 1381.79 (M+Na)⁺; TLC R_{f}: 0.40 (dichloromethane/methanol = 9/1); HPLC RT: 12.7 min. (C8 reverse phase column: 250 mm; acetonitrile/0.077% ammonium acetate in water; operation temperature: 64 °C; detector: 210 nm)].

### Example 59

### [(R)-(3-(N-Piperidinyl)propoxy)methyl-Sar]-3-[(γ-hydroxy)-N-MeLeu]-4-cyclosporin

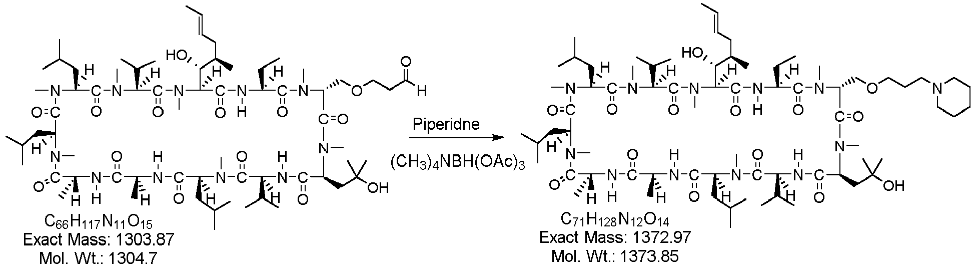

[(R)-α-(2-Formylethoxy)methyl-Sar]-3-[(γ-hydroxy)-N-MeLeu]-4-cyclosporin (200 mg, 0.15 mmol) was dissolved in dichloromethane (15 ml). Piperidine (114 mg, 1.34 mmol) and tetramethylammonium triacetoxyborohydride (353 mg, 1.34 mmol) were added. The reaction mixture was stirred overnight at room temperature. Then sodium bicarbonate saturated solution (30 ml) and dichloromethane (15 ml) were added and the mixture was separated. The dichloromethane layer was dried over magnesium sulfate and evaporated under reduced pressure. After purified on silica gel, 43 mg of product was obtained [Molecular Formula: C₇₁H₁₂₈N₁₂O₁₄; Exact Mass: 1372.97; (m/z): MS (m/z): 1373.79 (M+1)⁺, 1395.86 (M+Na)⁺; TLC R_{f}: 0.27 (dichloromethane/methanol = 9/1); HPLC RT: 17.8 min. (C8 reverse phase column: 250 mm; acetonitrile/0.077% ammonium acetate in water; operation temperature: 64 °C; detector: 210 nm)].

### Example 60

### [(R)-(3-(N,N-Dimethylamino)propoxy)methyl-Sar]-3-[(γ-hydroxy)-N-MeLeu]-4-cyclosporin

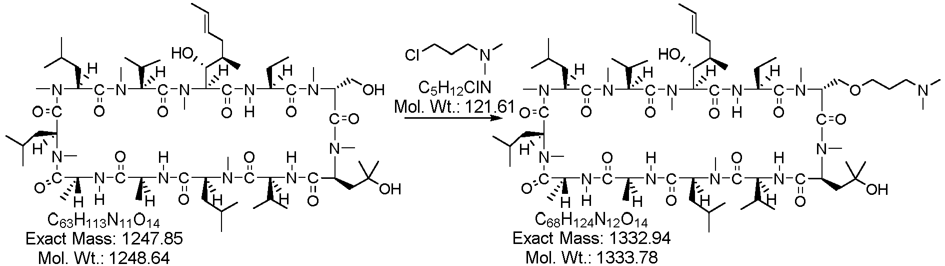

To a solution of [(R)-α-hydroxymethyl-Sar]-3-[(γ-hydroxy)-N-MeLeu]-4-cyclosporin (0.38 g, 0.30 mmol) in benzene (15 ml) were added sodium hydroxide (0.48 g, 12.00 mmol), tetramethylammonia hydroxide (0.54 g, 3.0 mmol) and 3-dimethylaminoethyl chloride hydrochloride (0.43 g, 3.00 mmol). The mixture was stirred at 30 °C for 36 hours. Then ice water (20 ml) was added and the mixture was separated. The aqueous layer was extracted with ethyl acetate (20 ml). The combined organic layers were washed with brine, dried over magnesium sulfate and evaporated under reduced pressure. The residue was purified by chromatography on silica gel with dichloromethane/methanol (95/5) as eluent to give to give 70 mg of pure product [Molecular Formula: C₆₈H₁₂₄N₁₂O₁₄; Exact Mass: 1332.94. MS (m/z): 1333.64 (M+1)⁺, 1355.73 (M+Na)⁺; TLC R_{f}: 0.04 (dichloromethane/methanol = 5/1); HPLC RT: 11.78 min. (C8 reverse phase column: 150 mm; acetonitrile/water (0.05% trifluoroacetic acid); operation temperature: 64 °C; detector: 210 nm)].

### Example 61

### [(R)-(3-(N,N-Diethylamino)propoxy)methyl-Sar]-3-[(γ-hydroxy)-N-MeLeu]-4-cyclosporin

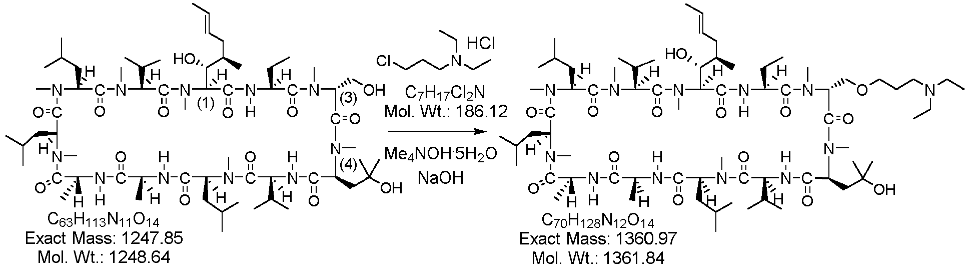

To a solution of [(R)-α-hydroxymethyl-Sar]-3-[(γ-hydroxy)-N-MeLeu]-4-cyclosporin (250 mg, 0.20 mmol) in benzene (15 ml) were added a solution of sodium hydroxide (400 mg, 10.00 mmol) in water (0.5 ml), 3-diethylaminepropyl chloride hydrochloride (500 mg, 2.69 mmol) and tetramethylammonium hydroxide pentahydrate (430 mg, 2.41 mmol). The mixture was stirred at 32 °C for 4 days. Then ice water (30 ml) was added and the mixture was separated. The aqueous layer was extracted with dichloromethane (50 ml). The combined organic layers were washed with brine, dried over magnesium sulfate and concentrated under reduced pressure. The residue was purified by chromatography (dichloromethane/methanol = 95/5) to give 120 mg of product [Molecular Formula: C₇₀H₁₂₈N₁₂O₁₄; Exact Mass: 1360.97; MS (m/z): 1361.72 (M+1)⁺; TLC Rf: 0.38 (dichloromethane/methanol = 9/1); HPLC RT: 16.71 min. (C8 reverse phase column: 250 mm; acetonitrile/0.077% ammonium acetate in water; operation temperature: 64 °C; detector: 210 nm)].

### Example 62 - (this example relates to the synthesis of intermediate)

### [(γ-(Methylthio)methoxy)-N-MeLeu]-4-cyclosporin

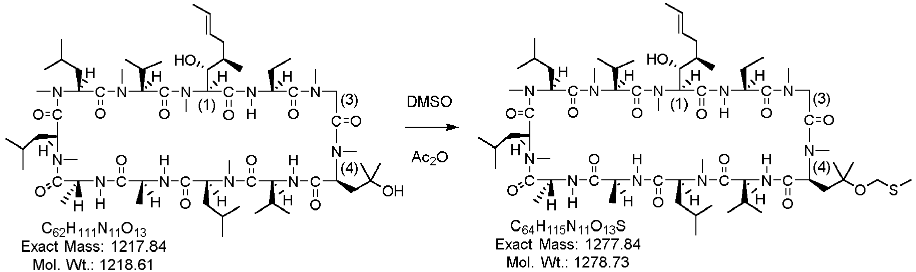

To a solution of [(γ-hydroxy)-N-MeLeu]-4-cyclosporin (4.50 g, 3.70 mmol) in anhydrous dimethyl sulfoxide (25 ml) was added acetic anhydride (15 ml). The reaction mixture was stirred at room temperature for 17 hours. After diluted with ethyl acetate (75 ml), the mixture was washed with saturated sodium bicarbonate water solution and brine, dried over magnesium sulfate and evaporated under reduced pressure. The residue was purified on silica gel chromatography with dichloromethane/methanol (98/2) as eluant to give 2.35 g of [(γ-methylthio)methoxy-N-MeLeu]-4-cyclosporin [Molecular Formula: C₆₄H₁₁₅N₁₁O₁₃S; Exact Mass: 1277.84; MS (m/z): 1300.70 (M+Na)⁺; TLC R_{f}: 0.30 (dichloromethane/methanol = 95/5); HPLC RT: 19.57 min. (C8 reverse phase column: 250 mm; acetonitrile/0.077% ammonium acetate in water; operation temperature: 64 °C; detector: 210 nm)].

### Example 63 - (this example relates to the synthesis of intermediate)

### [γ-(Methoxy)-N-MeLeu]-4-cyclosporin

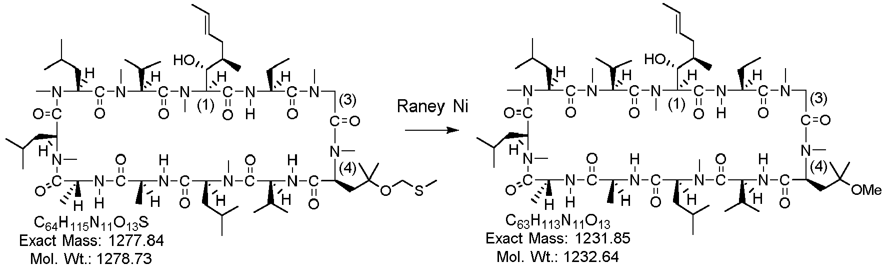

To a solution of [γ-(Methylthio)methoxy-N-MeLeu]-4-cyclosporin (1.20 g, 0.94 mmol) in anhydrous tetrahydrofuran (40 ml) was added Raney Ni (∼ 2 g). The resulting suspension was stirred and heated to 60 °C for 30 minutes and the reaction was monitored by LC-MS. The reaction mixture was filtered and the filter cake was washed with tetrahydrofuran. The filtrate was collected and evaporated under reduced pressure. The residue was purified by chromatography using eluant of ethyl acetate/methanol (97.5/2.5) to give 0.60 g of product [Molecular Formula: C₆₃H₁₁₃N₁₁O₁₃; Exact Mass: 1231.85; MS (m/z): 1232.70 (M+1)⁺; TLC R_{f}: 0.46 (dichloromethane/methanol = 95/5); HPLC RT: 20.63 min. (C8 reverse phase column: 250 mm; acetonitrile/0.077% ammonium acetate in water; operation temperature: 64 °C; detector: 210 nm)].

### Example 64 - (this example relates to the synthesis of intermediate)

### [α-Carboxy-Sar]-3-[(γ-methoxy)-N-MeLeu]-4-cyclosporin

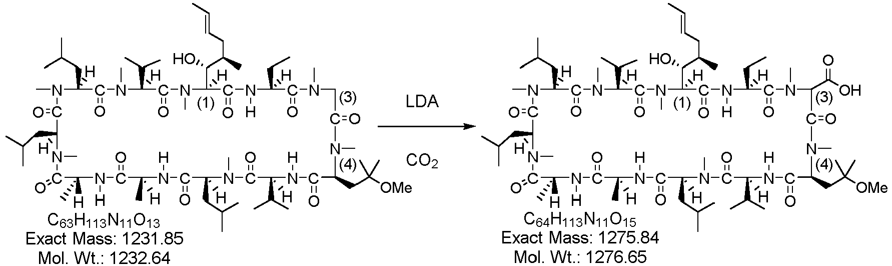

n-BuLi (2.8 M in tetrahydrofuran/hexane, 5.00 ml, 14.00 mmol) was added to a solution of diisopropylamine (1.44 g, 14.30 mmol) in tetrahydrofuran (30 ml) at -78 °C under nitrogen atmosphere. After the mixture was stirred for one and half hour, a solution of [y-(methoxy)-N-MeLeu]-4-cyclosporin (1.20 g, 0.97 mmol) in tetrahydrofuran (6 ml) was added slowly. The stirring was continued at -78 °C for 2 hours. Then carbon dioxide gas was bubbled into the reaction mixture for one hour. The mixture was allowed to warm to room temperature slowly and stirred for another 3 hours. After most of solvent was evaporated under reduced pressure, dichloromethane (30 ml) and water (30 ml) were added. The PH of the mixture was adjusted to around 5 by adding aqueous citric acid. The mixture was separated, and the dichloromethane layer was washed with brine, dried over magnesium sulfate and concentrated under reduced pressure to give 1.20 g of crude product used for next step [Molecular Formula: C₆₄H₁₁₃N₁₁O₁₅; Exact Mass: 1275.84; MS (m/z): 1298.53(M+Na)⁺].

### Example 65 - (this example relates to the synthesis of intermediate)

### [α-Methoxycarbonyl-Sar]-3-[(γ-methoxy)-N-MeLeu]-4-cyclosporin

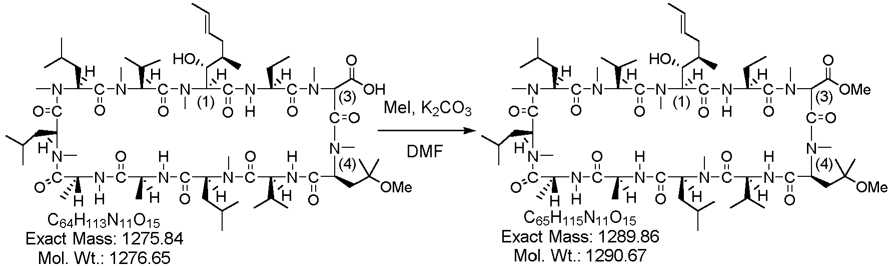

To a mixture of [α-carboxy]-3-[(γ-methoxy)-N-MeLeu]-4-cyclosporin (1.20 g. 0.94 mmol) and potassium carbonate (0.80 g, 5.79 mmol) in N,N-dimethylformamide (25 ml) was added iodomethane (0.80 g, 5.63 mmol). The mixture was stirred at room temperature overnight. Dichloromethane (75 ml) and water (30 ml) were added and the mixture was separated. The dichloromethane layer was washed with water (25 ml) and brine (25 ml), dried magnesium sulfate and concentrated under reduced pressure to give 1.10 g of crude product [Molecular Formula: C₆₅H₁₁₅N₁₁O₁₅; Exact Mass: 1289.86; MS (m/z): 1312.72(M+Na)⁺].

### Example 66 - (this example relates to the synthesis of intermediate)

### [(R)-α-Hydroxymethyl-Sar]-3-[(γ-methoxy)-N-MeLeu]-4-cyclosporin

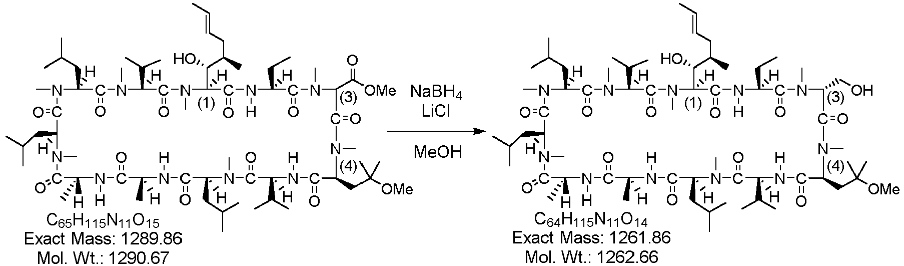

To a suspension of [α-methoxycarbonyl-Sar]-3-[(γ-methoxy)-N-MeLeu]-4-cyclosporin (1.10 g, 0.85 mmol) and lithium chloride (1.00 g, 23.53 mmol) in methanol (80 ml) was added sodium borohydride (2.00 g, 52.91 mmol) in portions. The mixture was stirred at room temperature overnight and concentrated under reduced pressure. Dichloromethane (50 ml) and water (30 ml) were added and the mixture was separated. The dichloromethane layer was washed with brine, dried over magnesium sulfate and evaporated under reduced pressure. The residue was purified by chromatography (dichloromethane/methanol = 97/3) to give 310 mg of product [Molecular Formula: C₆₄H₁₁₅N₁₁O₁₄; Exact Mass: 1261.86; MS (m/z): 1262.68 (M+1)⁺].

### Example 67 - (this example relates to the synthesis of intermediate)

### [α-Methylene-Sar]-3-[(γ-methoxy)-N-MeLeu]-4-cyclosporin

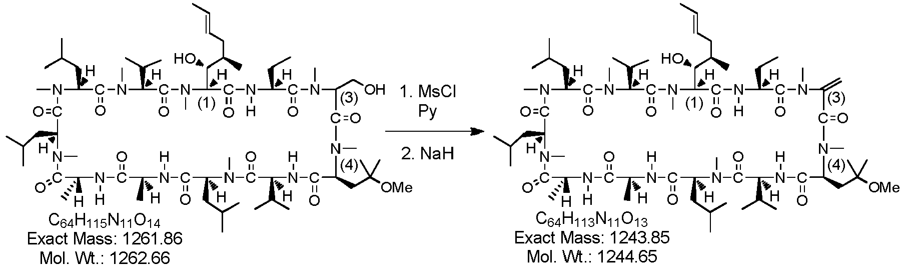

[α-Methylene-Sar]-3-[(γ-methoxy)-N-MeLeu]-4-cyclosporin was prepared according to the method described in Example 4 and 5 [Molecular Formula: C₆₄H₁₁₃N₁₁O₁₃; Exact Mass: 1243.85; MS (m/z): 1244.57 (M+1)⁺; TLC R_{f}: 0.34 (hexane/acetone = 6/1); HPLC RT: 17.10 min. (C8 reverse phase column: 250 mm; acetonitrile/water (0.05% trifuloroacetic acid), operation temperature: 64 °C; detector: 210 nm].

### Example 68

### [(S)-(2-(N,N-Dimethylamino)ethylthio)methyl-Sar]-3-[(y-methoxy)-N-MeLeu]-4-cyclosporin

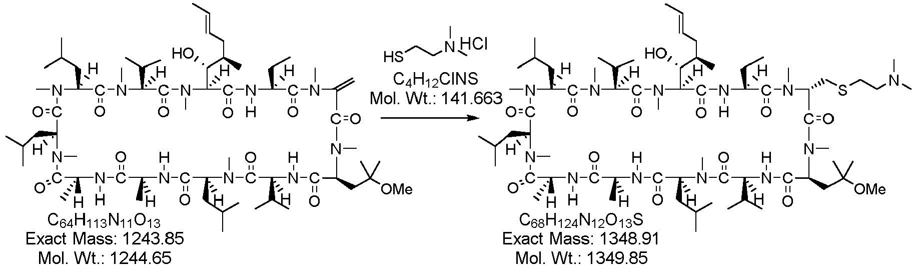

[α-Methylene-Sar]-3-[(γ-methoxy)-N-MeLeu]-4-cyclosporin (0.24 g, 0.19 mmol) and 2-(N,N-dimethylamino)ethylthiol hydrochloride (0.27 g, 1.91 mmol) was dissolved in methanol (30 ml), followed by adding 20 equivalents of lithium hydroxide. The mixture was stirred overnight at room temperature. After removal of solvent, the residue was purified by flash chromatography using methylene chloride/methanol (96/4) as eluent to give 0.11 g of pure product [Molecular Formula: C₆₈H₁₂₄N₁₂O₁₃S; Exact Mass: 1348.93; MS (m/e): 1349.85 (M+1)⁺, 1371.81 (M+Na)⁺; TLC R_{f}: 0.20 (ethyl acetate/methanol (5:1); HPLC RT: 12.42 min. (C8 reverse phase column: 250 mm; acetonitrile/water (0.05% trifluoroacetic acid); operation temperature: 64 °C; detector: 210 nm)].

### Example 69

### [(S)-(2-(N,N-Diethylamino)ethylthio)methyl-Sar]-3-[(γ-methoxy)-N-MeLeu]-4-cyclosporin

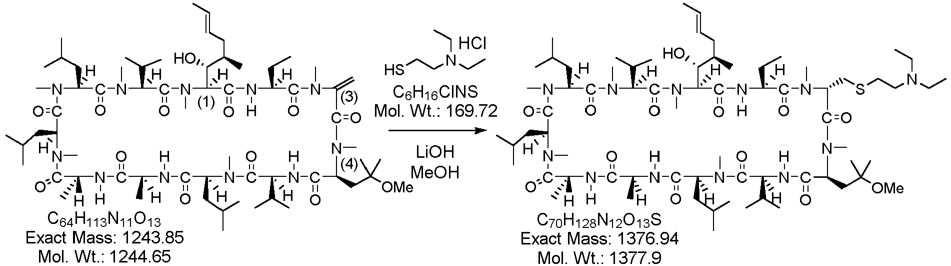

To a solution of [α-methylene-Sar]-3-[(γ-methoxy)-N-MeLeu]-4-cyclosporin (280 mg, 0.23 mmol) and 2-diethylaminoethanethiol hydrochloride (570 mg, 3.37 mmol) in methanol (15 ml) was added lithium hydroxide (142 mg, 5.92 mmol). The reaction mixture was stirred overnight at room temperature. Most of solvent was evaporated under reduced pressure. Dichloromethane (80 ml) and water (30 ml) were added and the mixture was separated. The organic layer was washed with water and brine, dried over magnesium sulfate and evaporated under reduced pressure. The residue was purified by chromatography (dichloromethane/methanol = 97/3) to give 110 mg of product [Molecular Formula: C₇₀H₁₂₈N₁₂O₁₃S; Exact Mass: 1376.94; MS (m/z): 1377.67 (M+1)⁺; TLC R_{f}: 0.35 (dichloromethane/methanol = 95/5); HPLC RT: 13.17 min. (C8 reverse phase column: 250 mm; acetonitrile/water (0.05% trifluoroacetic acid); operation temperature: 64 °C; detector: 210 nm)].

### Example 70

### [(S)-(2-(N-Pyrrolidinyl)ethylthio)methyl-Sar]-3-[(γ-methoxy)-N-MeLeu]-4-cyclosporin

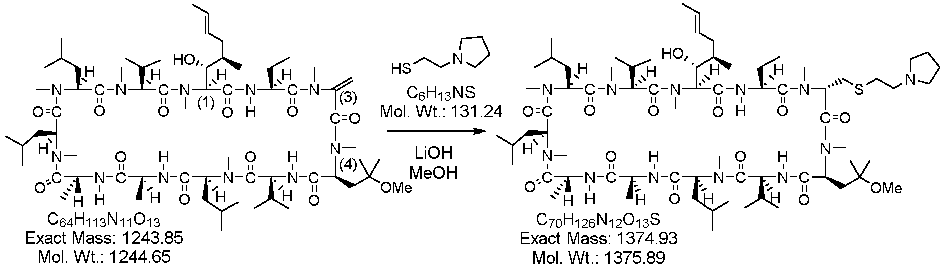

To a solution of [α-methylene-Sar]-3-[(γ-methoxy)-N-MeLeu]-4-cyclosporin (230 mg, 0.18 mmol) and 2-(N-pyrrolidinyl)ethanethiol (340 mg, 2.59 mmol) in methanol (15 ml) was added lithium hydroxide (120 mg, 5.00 mmol). The reaction mixture was stirred overnight at room temperature. Most of solvent was evaporated under reduced pressure. Dichloromethane (30 ml) and water (30 ml) were added and the mixture was separated. The organic layer was washed with water and brine, dried over magnesium sulfate and evaporated under reduced pressure. The residue was purified by chromatography (dichloromethane/methanol = 96/4) to give 55 mg of product [Molecular Formula: C₇₀H₁₂₆N₁₂O₁₃S; Exact Mass: 1374.93; MS (m/z): 1375.57 (M+1)⁺; TLC R_{f}: 0.29 (dichloromethane/methanol = 95/5); HPLC RT: 12.90 min. (C8 reverse phase column: 250 mm; acetonitrile/water (0.05% trifluoroacetic acid); operation temperature: 64 °C; detector: 210 nm)].

### Example 71

### [(S)-(2-(N-Morpholino)ethylthio)methyl-Sar]-3-[(γ-methoxy)-N-MeLeu]-4-cyclosporin

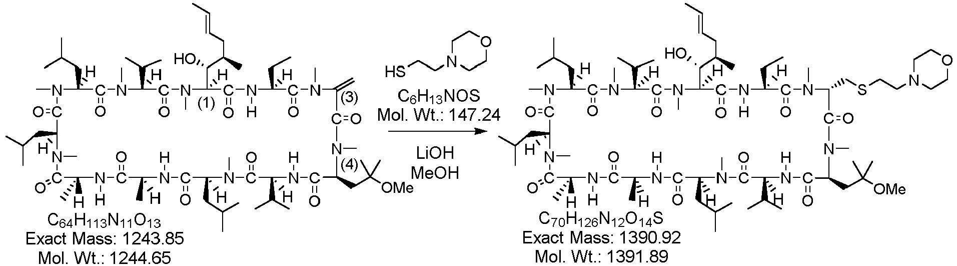

To a solution of [α-methylene-Sar]-3-[(γ-methoxy)-N-MeLeu]-4-cyclosporin (250 mg, 0.20 mmol) and 2-morpholinoethanethiol (260 mg, 1.76 mmol) in methanol (15 ml) was added lithium hydroxide (120 mg, 5.00 mmol). The reaction mixture was stirred at room temperature overnight. Most of solvent was evaporated under reduced pressure. Dichloromethane (60 ml) and water (30 ml) were added and the mixture was separated. The organic layer was washed with water and brine, dried over magnesium sulfate and evaporated under reduced pressure. The residue was purified by chromatography (dichloromethane/methanol = 97/3) to give 70 mg of product [Molecular Formula: C₇₀H₁₂₆N₁₂O₁₄S; Exact Mass: 1390.92; MS (m/z): 1391.58 (M+1)⁺; TLC Rf: 0.38 (dichloromethane/methanol = 9/1); HPLC RT: 12.48 min. (C8 reverse phase column: 250 mm; acetonitrile/water (0.05% trifluoroacetic acid); operation temperature: 64 °C; detector: 210 nm)].

### Example 72

### [(S)-(3-(N,N-Dimethylamino)propylthio)methyl-Sar]-3-[(y-methoxy)-N-MeLeu]-4-cyclosporin

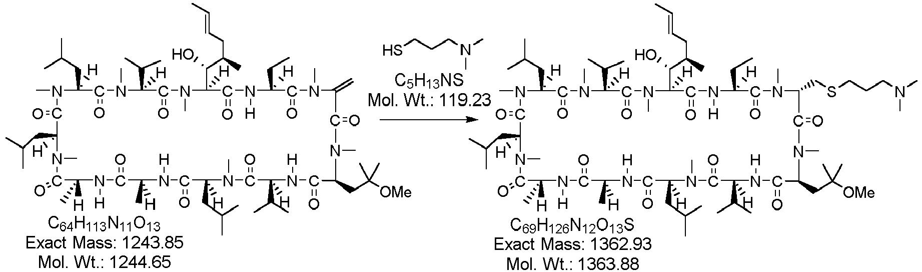

[α-Methylene-Sar]-3-[(γ-methoxy)-N-MeLeu]-4-cyclosporin (0.25 g, 0.20 mmol) and 3-(N,N-dimethylamino)propylthiol (0.24 g, 2.00 mmol) were dissolved in methanol (20 ml), followed by adding 10 equivalents of lithium hydroxide. The mixture was stirred overnight at room temperature. After removal of solvent, the residue was purified by flash chromatography using methylene chloride/methanol as eluent to give 80 mg of pure product [Molecular Formula: C₆₉H₁₂₆N₁₂O₁₃S; Exact Mass: 1362.93; MS (m/e): 1363.70 (M+1)⁺. TLC R_{f}: 0.20 (ethyl acetate/methanol = 10/1); HPLC RT: 12.82 min. (C8 reverse phase column: 250 mm; acetonitrile/water (0.05% trifluoroacetic acid); operation temperature: 64 °C; detector: 210 nm)].

### Example 73

### [(S)-(3-(N,N-Diethylamino)propylthio)methyl-Sar]-3-[(y-methoxy)-N-MeLeu]-4-cyclosporin

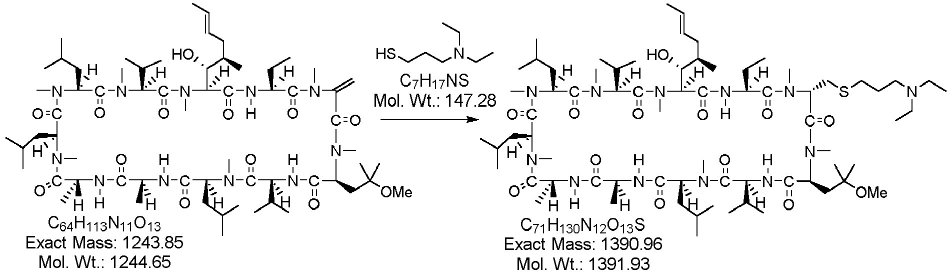

[α-Methylene-Sar]-3-[(γ-methoxy)-N-MeLeu]-4-cyclosporin (0.25 g, 0.20 mmol) and 3-(N,N-diethylamino)propylthiol (0.30 g, 2.00 mmol) were dissolved in methanol (20 ml), followed by adding 10 equivalents of lithium hydroxide. The mixture was stirred overnight at room temperature. After removal of solvent, the residue was purified by flash chromatography using methylene chloride/methanol as eluent to give 120 mg of pure product [Molecular Formula: C₇₁H₁₃₀N₁₂S; Exact Mass: 1390.96; MS (m/e): 1391.64 (M+1)⁺, 1413.79 (M+Na)⁺; TLC R_{f}: 0.25 (ethyl acetate/methanol =10/1); HPLC RT: 13.57 min. (C8 reverse phase column: 250 mm; acetonitrile/water (0.05% trifluoroacetic acid); operation temperature: 64 °C; detector: 210 nm)].

### Example 74

### [(S)-(3-(N-Piperidinyl)propylthio)methyl-Sar]-3-[(γ-methoxy)-N-MeLeu]-4-cyclosporin

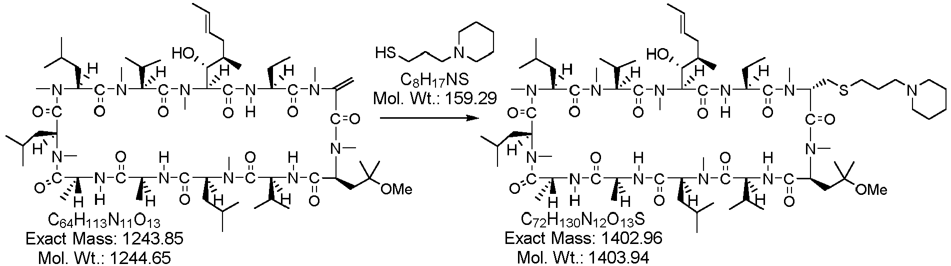

[α-Methylene-Sar]-3-[(γ-methoxy)-N-MeLeu]-4-cyclosporin (0.37 g, 0.30 mmol) and 3-(N-piperidino)propylthiol (0.48 g, 3.00 mmol) were dissolved in methanol (30 ml), followed by adding 10 equivalents of lithium hydroxide. The mixture was stirred overnight at room temperature. After removal of solvent, the residue was purified by flash chromatography using methylene chloride/methanol as eluent to give 60 mg of pure product [Molecular Formula: C₇₂H₁₃₀N₁₂O₁₃S; Exact Mass: 1402.96; MS (m/e): 1403.69 (M+1)⁺, 1425 (M+Na)⁺; TLC: R_{f}: 0.3 (ethyl acetate/methanol = 10/1); HPLC RT: 13.59 min. (C8 reverse phase column: 250 mm; acetonitrile/water (0.05% trifluoroacetic acid); operation temperature: 64 °C; detector: 210 nm)].

### Example 75

### [(S)-(3-(N-Pyrrolidinyl)propylthio)methyl-Sar]-3-[(γ-methoxy)-N-MeLeu]-4-cyclosporin

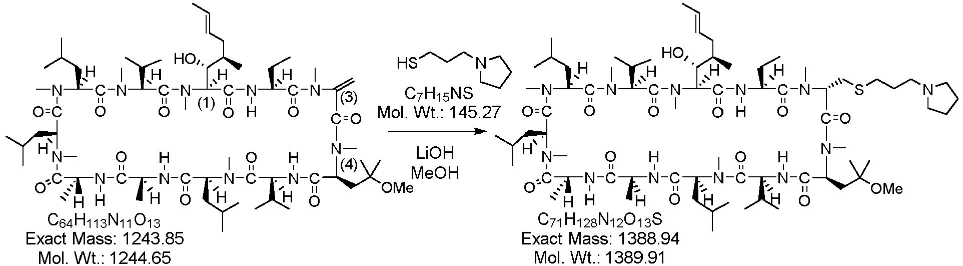

To a solution of [α-methylene-Sar]-3-[(γ-methoxy)-N-MeLeu]-4-cyclosporin (280 mg, 0.23 mmol) and 3-(N-pyrrolidinyl)propanethiol (350 mg, 2.41 mmol) in methanol (15 ml) was added lithium hydroxide (120 mg, 5.00 mmol). The reaction mixture was stirred at room temperature overnight. Then most of solvent was evaporated under reduced pressure. Dichloromethane (80 ml) and water (25 ml) were added and the mixture was separated. The organic layer was washed with water and brine, dried over magnesium sulfate and evaporated under reduced pressure. The residue was purified by chromatography (dichloromethane/methanol = 94/6) to give 47 mg of product [Molecular Formula: C₇₁H₁₂₈N₁₂O₁₃S; Exact Mass: 1388.94; MS (m/z): 1389.68 (M+1)⁺; TLC Rf: 0.30 (dichloromethane/methanol = 95/5); HPLC RT: 13.25 min. (C8 reverse phase column: 250 mm; acetonitrile/water (0.05% trifluoroacetic acid); operation temperature: 64 °C; detector: 210 nm)].

### Example 76

### [(S)-(3-(N-Morphlino)propylthio)methyl-Sar]-3-[(y-methoxy)-N-MeLeu]-4-cyclosporin

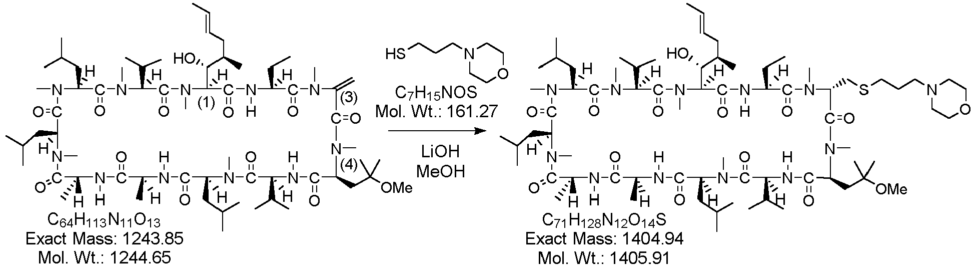

To a solution of [α-Methylene-Sar]-3-[(γ-methoxy)-N-MeLeu]-4-cyclosporin (320 mg, 0.26 mmol) and 3-morpholinopropanethiol (600 mg, 3.73 mmol) in methanol (25 ml) was added lithium hydroxide (140 mg, 5.83 mmol). The reaction mixture was stirred at room temperature overnight. Then most of solvent was evaporated under reduced pressure. Dichloromethane (60 ml) and water (25 ml) were added and the mixture was separated. The organic layer was washed with water and brine, dried over magnesium sulfate and evaporated under reduced pressure. The residue was purified by chromatography (dichloromethane/methanol = 97/3) to give 58 mg of product [Molecular Formula: C₇₁H₁₂₈N₁₂O₁₄S; Exact Mass: 1404.94; MS (m/z): 1405.52 (M+1)⁺; TLC Rf: 0.39 (dichloromethane/methanol = 9/1); HPLC RT: 15.96 min. (C8 reverse phase column: 250 mm; acetonitrile/0.077% ammonium acetate in water; operation temperature: 64 °C; detector: 210 nm)].

### Example 77

### [(R)-(2-(N,N-Diethylamino)ethoxy)methyl-Sar]-3-[(γ-methoxy)-N-MeLeu]-4-cyclosporin

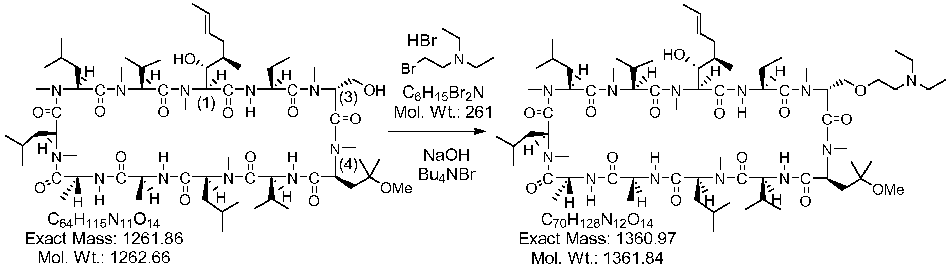

To a solution of [(R)-α-hydroxymethyl-Sar]-3-[(γ-methoxy)-N-MeLeu]-4-cyclosporin (0.20 g, 0.16 mmol) in benzene (10 ml) were added a solution of sodium hydroxide (0.48 g, 12.00 mmol) in water (1 ml), 2-bromo-N,N-diethylethylamine hydrobromide (1.10 g, 4.21 mmol) and tetra-n-butylammonium bromide (0.10 g, 0.31 mmol). The mixture was stirred at 35 °C for 40 hours. Ice water (10 ml) was added and the mixture was separated. The aqueous layer was extracted with dichloromethane (20 ml). The combined organic layers was washed with brine, dried over magnesium sulfate and concentrated under reduced pressure. The residue was purified by chromatography (dichloromethane/methanol = 96/4) to give 36 mg of product [Molecular Formula: C₇₀H₁₂₈N₁₂O₁₄; Exact Mass: 1360.97; MS (m/z): 1383.74 (M+Na)⁺; TLC R_{f}: 0.32 (dichloromethane/methanol = 95/5); HPLC RT: 13.66 min. (C8 reverse phase column: 250 mm; acetonitrile/0.077% ammonium acetate in water; operation temperature: 64 °C; detector: 210 nm); ¹H NMR spectrum (600 MHz, CDCl₃, δ in ppm): 0.65(d, J = 4.8Hz, 3H), 0.82 (m, 9H), 0.87 (m, 6H), 0.91 (d, J = 6. Hz, 3H), 0.93 (d, J = 6.0Hz, 3H), 0.98-1.01 (m, 15 H), 1.07 (d, J=6.6Hz, 3H), 1.11(s, 6H), 1.23 (m, 6 H), 1.33 (d, J=7.2 Hz, 3H), 1.39-1.47 (m, 2H), 1.53-1.58 (m, 4H), 1.6 (m, 3H), 1.67-1.75 (m, 3H), 1.98-2.12 (m, 4H), 2.43-2.48 (m, 3H), 2.50-2.54 (m, 4H), 2.60 (t, J=6.0Hz, 2H), 2.67 (s, 3H), 2.68 (s, 3H), 3.07 (s, 3H), 3.10 (s, 3H), 3.12 (s, 3H), 3.24 (s, 3H), 3.26 (s, 3H), 3.48 (m, 4H), 3.52-3.56 (m, 1H), 3.60-3.62 (m, 1H), 3.67-3.70 (m, 1H), 3.80 (m, 1H), 4.06 (t, J=9.6 Hz, 1H), 4.52 (m, 1H), 4.57 (m, 1H), 4.80 (m, 1H), 4.91 (t, J=7.8Hz, 1H), 5.04 (m, 3H), 5.11 (d, J=11.4Hz, 1H), 5.28-5.34 (m, 2 H), 5.50 (d, J=7.2Hz, 1 H), 5.67 (m, 1H), 7.10 (d, J=7.8Hz 1H), 7.48 (d, J=7.80Hz,1H), 7.58 (d, J=7.2Hz,1H), 7.91 (d, J=10.2Hz,1H)].

### Example 78

### [(R)-(2-(N,N-Dimethylamino)ethoxy)methyl-Sar]-3-[(γ-methoxy)-N-MeLeu]-4-cyclosporin

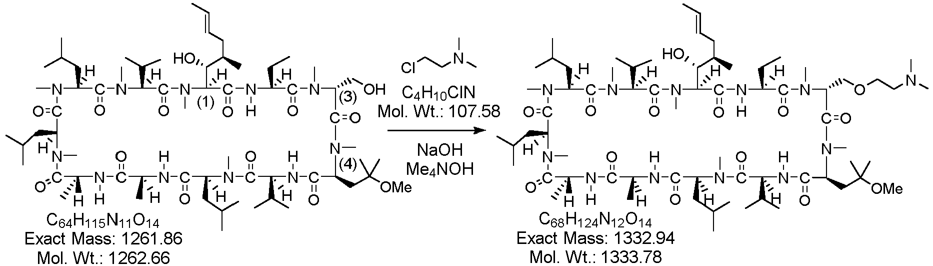

To a solution of [(R)-α-hydroxymethyl-Sar]-3-[(γ-methoxy)-N-MeLeu]-4-cyclosporin (250 mg, 0.20 mmol) in benzene (20 ml) were added a solution of sodium hydroxide (633 mg, 15.85 mmol) in water (0.70 ml), tetramethylammonium hydroxide pentahydrate (720 mg, 3.96 mmol) and 2-dimethylaminoethyl chloride hydrochloride (570 mg, 3.96 mmol). The mixture was stirred at 40 to 50 °C for two days. Sodium hydroxide (633 mg, 15.85 mmol) in water (0.70 ml), tetramethylammonium hydroxide pentahydrate (720 mg, 3.96 mmol) and 2-dimethylaminoethyl chloride hydrochloride (570 mg, 3.96 mmol) were added and the mixture was kept stirring at 40 to 50 °C for another two days. Another portion of 2-dimethylaminoethyl chloride hydrochloride (1.14 g, 7.91 mmol) was added and the stirring was continued at 40 to 50 °C for one more day. Sodium bicarbonate saturated solution (30 ml) was added and the mixture was separated. Then the aqueous layer was extracted with ethyl acetate (25 ml × 2). The combined organic layers were dried over magnesium sulfate and evaporated under reduced pressure. The residue was dissolved in ethyl acetate (25 ml). The resulting ethyl acetate phase was washed with acetic acid solution (5 ml in 10 ml water) and sodium bicarbonate saturated solution (30 ml), dried over magnesium sulfate and evaporated under reduced pressure. After purified on silica gel, 21 mg product was obtained [Molecular Formula: C₆₈H₁₂₄N₁₂O₁₄; Exact Mass: 1332.94; MS (m/z): 1333.75 (M+1)⁺, 1355.87 (M+Na)⁺; TLC R_{f}: 0.22 (dichloromethane/methanol = 9/1); HPLC RT: 17.3 min. (C8 reverse phase column: 250 mm; acetonitrile/0.077% ammonium acetate in water; operation temperature: 64 °C; detector: 210 nm)]

### Example 79

### [(R)-(2-(N-Morphlino)ethoxy)methyl-Sar]-3-[(γ-methoxy)-N-MeLeu]-4-cyclosporin

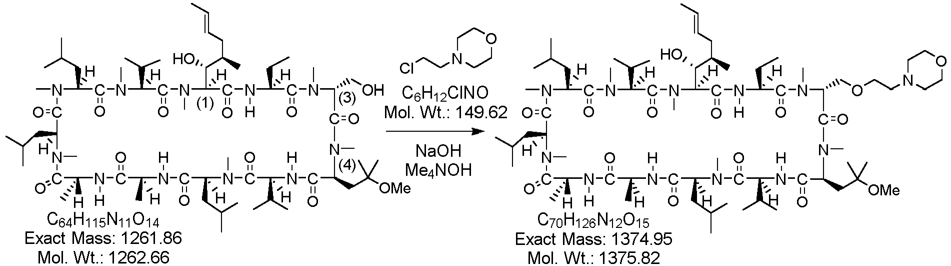

To a solution of [(R)-α-hydroxymethyl-Sar]-3-[(γ-methoxy)-N-MeLeu]-4-cyclosporin (250 mg, 0.20 mmol) in benzene (20 ml) was added a solution of sodium hydroxide (633 mg, 15.85 mmol) in water (0.70 ml), followed by tetramethylammonium hydroxide pentahydrate (720 mg, 3.96 mmol) and 2-(4-morpholinyl)ethyl chloride hydrochloride (737 mg, 3.96 mmol). The mixture was stirred at 40 to 50 °C for two days. Sodium hydroxide (633 mg, 15.85 mmol) in water (0.70 ml), tetramethylammonium hydroxide pentahydrate (720 mg, 3.96 mmol) and 2-(4-morpholinyl)ethyl chloride hydrochloride (737 mg, 3.96 mmol) were added and the mixture was kept stirring at 40 to 50 °C for another two days. Another portion of 2-(4-morpholinyl)ethyl chloride hydrochloride (1.47 g, 7.91 mmol) was added and the stirring was continued at 40 to 50 °C for two more days. Sodium bicarbonate saturated solution (30 ml) was added and the mixture was separated. Then the aqueous layer was extracted with ethyl acetate (25 ml × 2). The combined organic layers were dried over magnesium sulfate and evaporated under reduced pressure. The residue was dissolved in ethyl acetate (25 ml). And the resulting ethyl acetate phase was washed with acetic acid solution (5 ml in 10 ml water) and sodium bicarbonate saturated solution (30 ml), dried over magnesium sulfate and evaporated under reduced pressure. After purified on silica gel, 45 mg product was obtained [Molecular Formula: C₇₀H₁₂₆N₁₂O₁₅; Exact Mass: 1374.95; MS (m/z): 1375.63 (M+1)⁺, 1397.79 (M+Na)⁺; TLC R_{f}: 0.42 (dichloromethane/methanol = 9/1); HPLC RT: 12.9 min. (C8 reverse phase column: 250 mm; acetonitrile/0.077% ammonium acetate in water; operation temperature: 64 °C; detector: 210 nm)]

### Example 80

### [(R)-(3-(N,N-Dimethylamino)propoxy)methyl-Sar]-3-[(γ-methoxy)-N-MeLeu]-4-cyclosporine

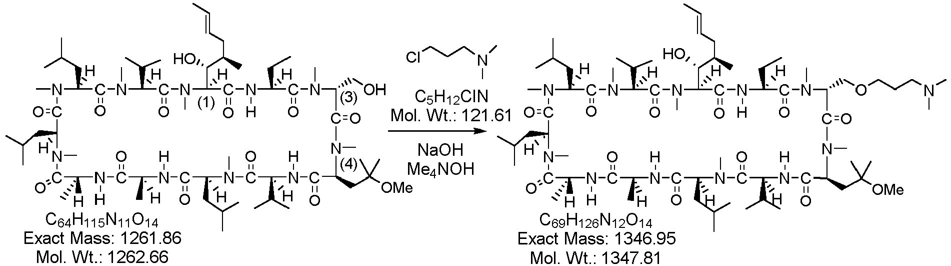

To a solution of [(R)-α-hydroxymethyl-Sar]-3-[(γ-methoxy)-N-MeLeu]-4-cyclosporin (250 mg, 0.20 mmol) in benzene (20 ml) were added a solution of sodium hydroxide (633 mg, 15.85 mmol) in water (0.70 ml), tetramethylammonium hydroxide pentahydrate (720 mg, 3.96 mmol) and 3-dimethylaminopropyl chloride hydrochloride (626 mg, 3.96 mmol). The mixture was stirred at 40 to 50 °C for two days. Sodium hydroxide (633 mg, 15.85 mmol) in water (0.70 ml), tetramethylammonium hydroxide pentahydrate (720 mg, 3.96 mmol) and 3-dimethylaminopropyl chloride hydrochloride (626 mg, 3.96 mmol) were added and the mixture was kept stirring at 40 to 50 °C for another two days. Another portion of 3-dimethylaminopropyl chloride hydrochloride (1.25 g, 7.91 mmol) was added and the stirring was continued at 40 to 50 °C for one more day. Sodium bicarbonate saturated solution (30 ml) was added and the mixture was separated. Then the aqueous layer was extracted with ethyl acetate (25 ml × 2). The combined organic layers were dried over magnesium sulfate and evaporated under reduced pressure. The residue was dissolved in ethyl acetate (25 ml). And the resulting ethyl acetate phase was washed with acetic acid solution (5 ml in 10 ml water) and sodium bicarbonate saturated solution (30 ml), dried over magnesium sulfate and evaporated under reduced pressure. After purified on silica gel, 36 mg product was obtained [Molecular Formula: C₆₉H₁₂₆N₁₂O₁₄; Exact Mass: 1346.95; MS (m/z): 1347.65 (M+1)⁺, 1369.74 (M+Na)⁺; TLC R_{f}: 0.21 (dichloromethane/methanol = 9/1). HPLC RT: 18.8 min. (C8 reverse phase column: 250 mm; acetonitrile/0.077% ammonium acetate in water; operation temperature: 64 °C; detector: 210 nm)].

### Example 81

### [(R)-(3-(N,N-Diethylamino)propoxy)methyl-Sar]-3-[(γ-methoxy)-N-MeLeu]-4-cyclosporine

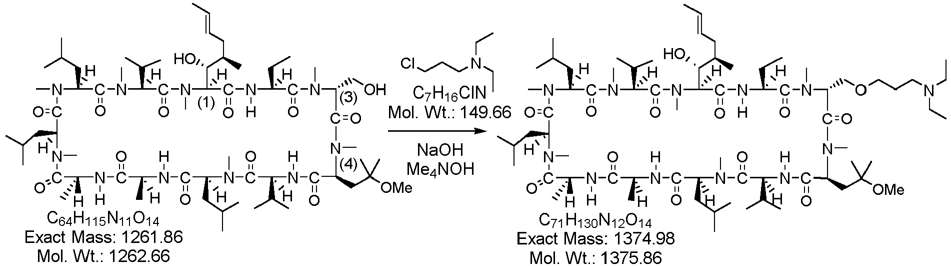

To a solution of [(R)-α-hydroxymethyl-Sar]-3-[(γ-methoxy)-N-MeLeu]-4-cyclosporin (250 mg, 0.20 mmol) in benzene 20 ml was added a solution of sodium hydroxide (633 mg, 15.85 mmol) in water (0.70 ml), followed by adding tetramethylammonium hydroxide pentahydrate (720 mg, 3.96 mmol) and 3-diethylaminopropyl chloride hydrochloride (737 mg, 3.96 mmol). The mixture was stirred at 40 to 50 °C for two days. Sodium hydroxide (633 mg, 15.85 mmol) in water (0.70 ml), tetramethylammonium hydroxide pentahydrate (720 mg, 3.96 mmol) and 3-diethylaminopropyl chloride hydrochloride (737 mg, 3.96 mmol) were added and the mixture was kept stirring at 40 to 50 °C for another two days. Another portion of 3-diethylaminopropyl chloride hydrochloride (1.47 g, 7.91 mmol) was added and the stirring was continued at 40 to 50 °C for two more days. Sodium bicarbonate saturated solution (30 ml) was added and the mixture was separated. The aqueous layer was extracted with ethyl acetate (25 ml × 2). Then the combined organic layers were dried over magnesium sulfate and evaporated under reduced pressure. The residue was dissolved in ethyl acetate (25 ml). And the resulting ethyl acetate phase was washed with acetic acid solution (5 ml in 10 ml water) and sodium bicarbonate saturated solution (30 ml), dried over magnesium sulfate and evaporated under reduced pressure. After purified on silica gel, 38 mg product was obtained [Molecular Formula: C₇₁H₁₃₀N₁₂O₁₄; Exact Mass: 1374.98; MS (m/z): 1375.70 (M+1)⁺, 1397.80 (M+Na)⁺; TLC R_{f}: 0.24 (dichloromethane/methanol = 9/1); HPLC RT: 19.6 min. (C8 reverse phase column: 250 mm; acetonitrile/0.077% ammonium acetate in water; operation temperature: 64 °C; detector: 210 nm)]

### Example 82 - (this example relates to the synthesis of intermediate)

### [α-Methylene-Sar]-3-[(γ-methylthiomethoxy)-N-MeLeu]-4-cyclosporin

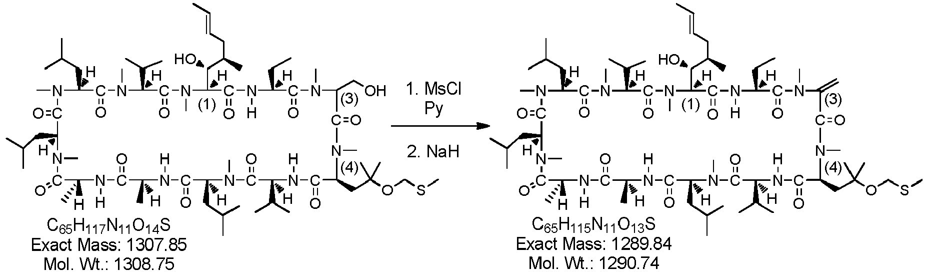

[α-Methylene-Sar]-3-[(γ-methylthiomethoxy)-N-MeLeu]-4-cyclosporin was prepared according to the method described in Example 4 and 5. The product was purified by chromatography on silica gel (ethyl acetate/methanol) [Molecular Formula: C₆₅H₁₁₅N₁₁O₁₃S; Exact Mass: 1289.84; MS (m/z): 1290.70 (M+1)⁺, 1312.67 (M+Na)⁺].

### Example 83

### [(S)-(2-(N,N-Dimethylamino)ethylthio)methyl-Sar]-3-[(γ-methylthio)methoxy-N-MeLeu]-4-cyclosporin

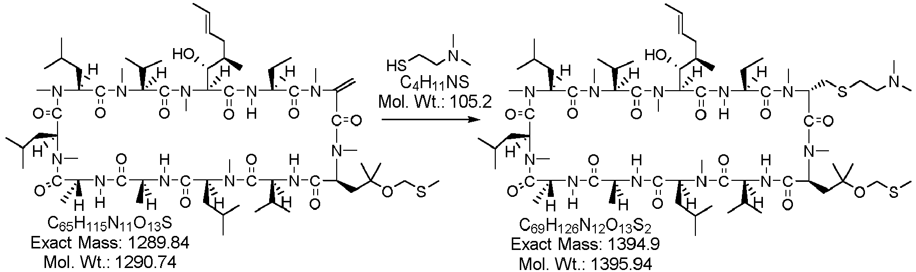

[α-Methylene-Sar]-3-[(γ-methylthiomethoxy)-N-MeLeu]-4-cyclosporin (0.32 g, 0.25 mmol) and 2-(N,N-dimethyl)ethanethiol (0.26 g, 2.50 mmol) were dissolved in methanol (20 ml), followed by adding 24 equivalents of triethylamine. The mixture was stirred overnight. After removal of solvent, the residue was subject to chromatography using dichloromethane/methanol as eluent to give 0.14 g of pure product [Molecular Formula, C₆₉H₁₂₆N₁₂O₁₃S₂; Exact Mass: 1394.90; MS (m/z): 1395.70 (M+1)⁺, 1417.68 (M+Na)⁺; TLC R_{f}: 0.10 (ethyl acetate/methanol = 10:1); HPLC RT: 13.30 min. (C8 reverse phase column: 250 mm; acetonitrile/water (0.05% trifluoroacetic acid); operation temperature: 64 °C; detector: 210 nm)].

### Example 84

### [(S)-(2-(N,N-Diethylamino)ethylthio)methyl-Sar]-3-[(γ-methylthio)methoxy-N-MeLeu]-4-cyclosporin

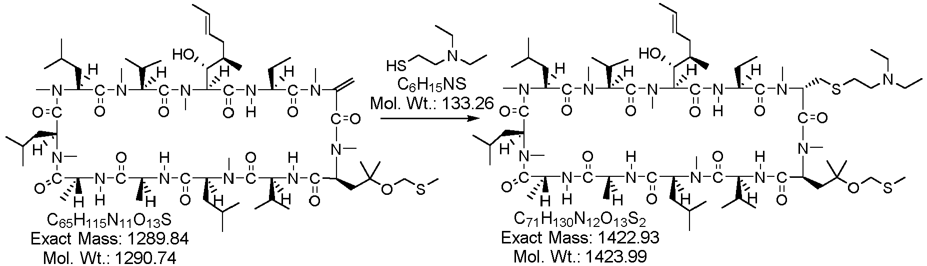

[α-Methylene-Sar]-3-[(γ-methylthiomethoxy)-N-MeLeu]-4-cyclosporin (0.27 g, 0.21 mmol) and 2-(N,N-diethyl)ethanethiol (0.28 g, 2.10 mmol) were dissolved in methanol (20 ml), followed by adding 24 equivalents of triethylamine. The mixture was stirred overnight at room temperature. After removal of solvent, the residue was purified by chromatography on silica gel using dichloromethane/methanol as eluent to give 0.17 g of pure product [Molecular Formula, C₇₁H₁₃₀N₁₂O₁₃S₂; Exact Mass: 1422.93; MS(m/z): 1423.70 (M+1)⁺, 1445.67 (M+Na)⁺; TLC R_{f}: 0.35 (ethyl acetate/methanol = 10:1); HPLC RT: 13.95 min. (C8 reverse phase column: 250 mm; acetonitrile/water (0.05% trifluoroacetic acid); operation temperature: 64 °C; detector: 210 nm)].

### Example 85 - (this example relates to the synthesis of intermediate)

### [γ-Ethoxymethoxy-N-MeLeu]-4-cyclosporin

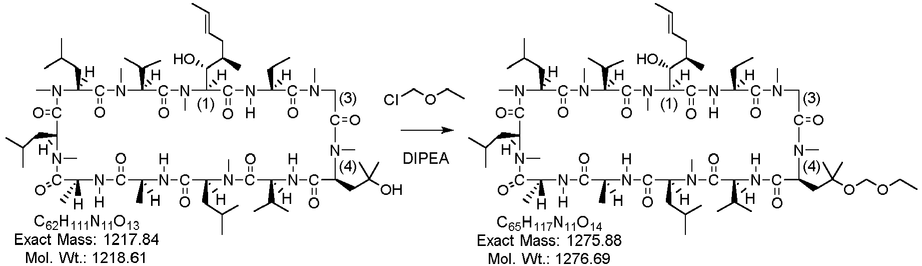

To a solution of [(γ-hydroxy)-N-MeLeu]-4-cyclosporin (1.20 g, 0.99 mmol) in dichloromethane (80 ml) was added diisopropylethylamine (FW 129.25, d 0.742, 1.32 ml, 0.98 g, 7.60 mmol), followed by adding chloromethyl ethyl ether (FW 94.54, d 1.02, 2.22 ml, 2.27 g, 24 mmol) dropwise. The mixture was stirred overnight at room temperature and TLC was used to monitor the completion of the reaction. The reaction mixture was washed with 1 N hydrochloric acid, saturated sodium bicarbonate water solution and brine. After dried over magnesium sulfate, the mixture was evaporated under reduced pressure to give a yellowish oil, which was further purified by flash chromatography using dichloromethane/methanol as eluent to give 0.95 g of the product [Molecular Formula: C₆₅H₁₁₇N₁₁O₁₄; Exact Mass: 1275.88; MS (m/z): 1276.70 (M+H)⁺, 1298.70 (M+Na)⁺; TLC R_{f}: 0.37 (ethyl acetate)].

### Example 86 - (this example relates to the synthesis of intermediate)

### [α-Methylene-Sar]-3-[(γ-ethoxymethoxy)-N-MeLeu]-4-cyclosporin

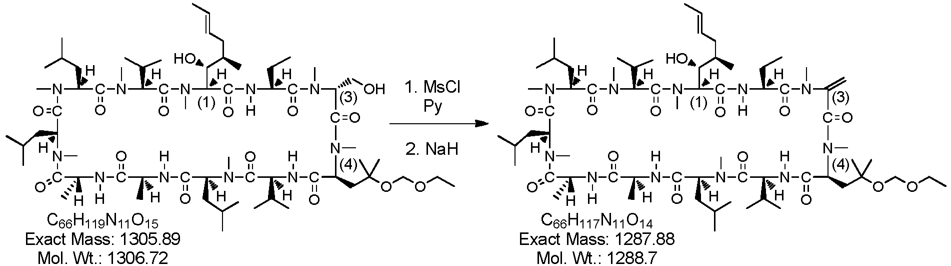

[α-Methylen-Sar]-3-[(γ-ethoxymethoxy)-N-MeLeu]-4-cyclosporin was prepared according to the method described in Example 4 and 5. The product was purified by chromatography on silica gel with ethyl acetate/methanol as eluent [Molecular Formula: C₆₆H₁₁₇N₁₁O₁₄; Exact Mass: 1287.68; MS (m/z): 1288.72 (M+1)⁺, 1310.70 (M+Na)⁺].

### Example 87

### [(S)-(2-(N,N-Diethylamino)ethylthio)methyl-Sar]-3-[(γ-ethoxy)methoxy-N-MeLeu]-4-cyclosporin

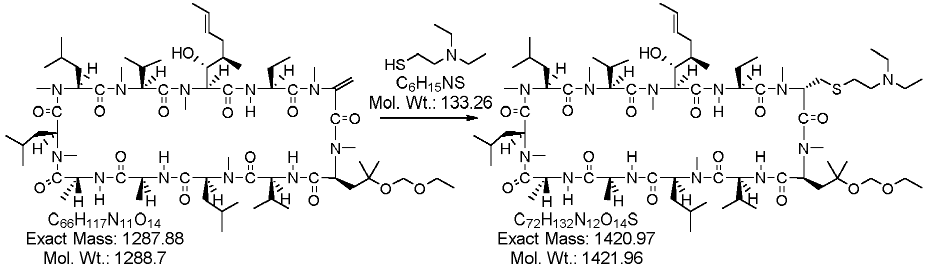

[α-Methylene-Sar]-3-[(γ-ethoxymethoxy)-N-MeLeu]-4-cyclosporin (0.27 g, 0.21 mmol) and 2-(N,N-diethyl)ethanethiol (0.28 g, 2.1 mmol) were dissolved in methanol (20 ml), followed by adding 12 equivalents of triethylamine. The mixture was stirred overnight at room temperature. After removal of solvent, the residue was purified by chromatography on silica gel using dichloromethane/methanol as eluent to give 90 mg of pure product [Molecular Formula: C₇₂H₁₃₂N₁₂O₁₄S; Exact Mass: 1420.97; MS (m/z): 1421.75 (M+1)⁺, 1443.72 (M + Na)⁺; TLC: R_{f}: 0.40 (ethyl acetate/methanol = 10/1); HPLC RT: 13.58 min. (C8 reverse phase column: 250 mm; acetonitrile/water (0.05% trifluoroacetic acid); operation temperature: 64 °C; detector: 210 nm)].

### Example 88 - (this example relates to the synthesis of intermediate)

### [α-Carboxy-Sar]-3-[N-MeIle]-4-cyclosporin

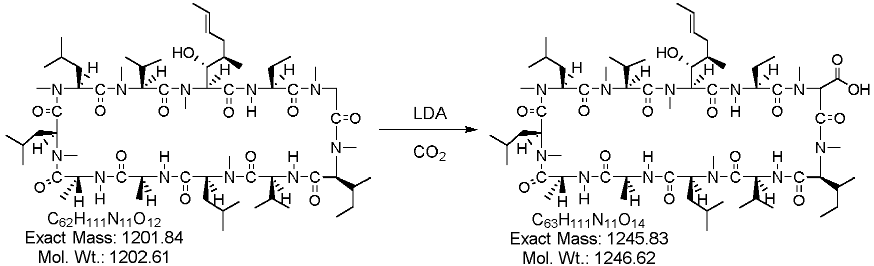

To a solution of LDA (2.0 M in tetrahydrofuran, 5 ml, 10 mmol) in tetrahydrofuran (15 ml) at -78 °C under nitrogen atmosphere was added [N-MeIle]-4-cyclosporin (1.20 g, 1.00 mmol) in tetrahydrofuran (15 ml) over 3 min. After the mixture was stirred at -78 °C for 3 hours, carbon dioxide gas was bubbled into the reaction mixture for 1 hour. Then the mixture was allowed to warm to room temperature slowly and kept stirring for another 3 hours. Most of tetrahydrofuran was evaporated under reduced pressure. Dichloromethane (100 ml) and water (50 ml) were added. The PH of the mixture was adjusted to around 5 by adding aqueous citric acid solution. The mixture was then separated and the organic layer was washed with brine, dried over magnesium sulfate and evaporated under reduced pressure to give 1.10 g of crude product, which was used for next step without purification [Molecular Formula: C₆₃H₁₁₁N₁₁O₁₄; Exact Mass: 1245.83; MS (m/z): 1246.68 (M+1)⁺].

### Example 89 - (this example relates to the synthesis of intermediate)

### [α-Methoxycarbonyl-Sar]-3-[N-MeIle]-4-cyclosporin

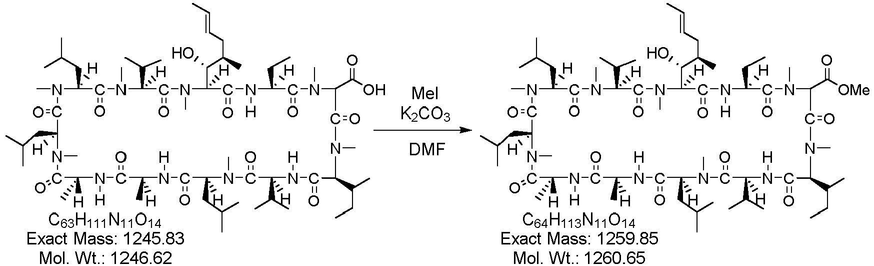

To a mixture of [α-carboxy-Sar]-3-[N-MeIle]-4-cyclosporin (1.00 g, 0.80 mmol) and potassium carbonate (0.70 g, 5.07 mmol) in N,N-dimethylformamide (10 ml) was added iodomethane (1.50 g, 10.56 mmol). The mixture was stirred overnight at room temperature. Dichloromethane (80 ml) and water (50 ml) were added and the mixture was separated. The dichloromethane layer was washed with water (25 ml) and brine (25 ml), dried over magnesium sulfate and evaporated under reduced pressure to give crude 1.00 g of product [Molecular Formula: C₆₄H₁₁₃N₁₁O₁₄; Exact Mass: 1259.85; MS (m/z): 1260.51(M+1)⁺].

### Example 90 - (this example relates to the synthesis of intermediate)

### [(R)-α-Hydroxymethyl-Sar]-3-[N-MeIle]-4-cyclosporin

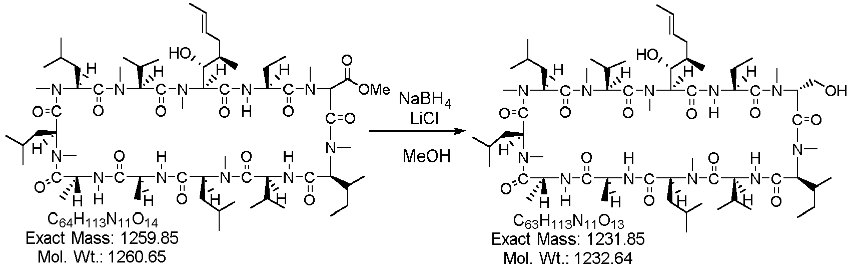

To a suspension of [α-methoxycarbonyl-Sar]-3-[N-MeIle]-4-cyclosporin (1.00 g, 0.79 mmol) and lithium chloride (0.60 g, 14.11 mmol) in methanol (80 ml) was added sodium borohydride (3.00 g, 79.26 mmol) in portions. The mixture was stirred overnight at room temperature. Most of solvent was evaporated under reduced pressure. Dichloromethane (100 ml) and water (50 ml) were added and the mixture was separated. The dichloromethane layer was washed with brine, dried over magnesium sulfate and evaporated under reduced pressure. The residue was purified by chromatography (dichloromethane/methanol = 97/3) to give 420 mg of product [Molecular Formula: C₆₃H₁₁₃N₁₁O₁₃; Exact Mass: 1231.85; MS (m/z): 1232.59 (M+1)⁺; TLC Rf: 0.32 (dichloromethane/methanol = 95/5); HPLC RT: 14.32 min. (C8 reverse phase column: 250 mm; acetonitrile/water (0.05% trifluoroacetic acid); operation temperature: 64 °C; detector: 210 nm)].

### Example 91- (this example relates to the synthesis of intermediate)

### [α-Methylene-Sar]-3-[N-MeIle]-4-cyclosporin

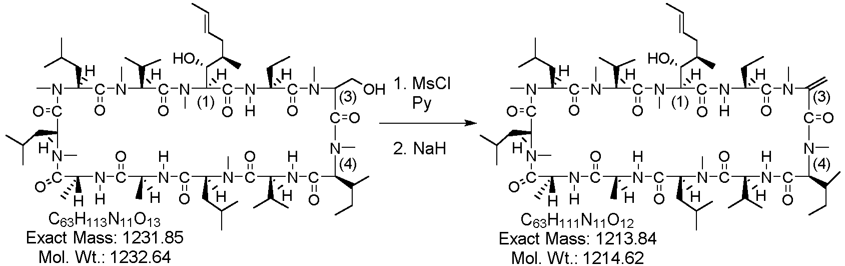

[α-Methylene-Sar]-3-[N-MeIle]-4-cyclosporin was prepared according to the method described in Example 4 and 5 [Molecular Formula: C₆₃H₁₁₁N₁₁O₁₂; Exact Mass: 1213.84; MS (m/z): 1214.59 (M+1)⁺; TLC R_{f}: 0.34 (hexane/acetone= 6/1); HPLC RT: 17.47 min. (C8 reverse phase column: 250mm; acetonitrile/water (0.05% trifuloroacetic acid), operation temperature: 64 °C; detector: 210 nm].

### Example 92

### [(S)-(2-(N,N-Diethylamino)ethylthio)methyl-Sar]-3-[N-MeIle]-4-cyclosporin

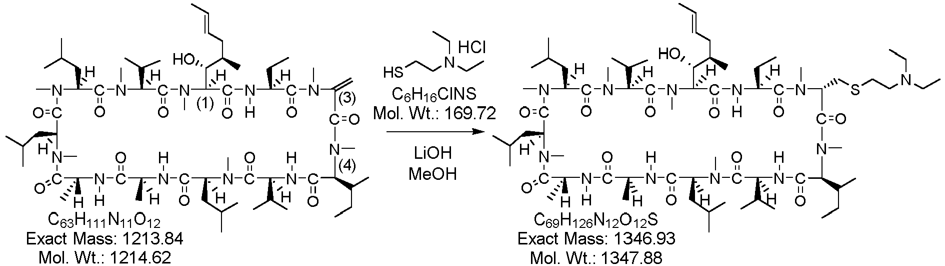

To a solution of [α-Methylene-Sar]-3-[N-MeIle]-4-cyclosporin (300 mg, 0.25 mmol) and 2-diethylaminoethanethiol hydrochloride (408 mg, 2.41 mmol) in methanol (20 ml) was added lithium hydroxide (116 mg, 4.83 mmol). The reaction mixture was stirred overnight at room temperature. Most of solvent was evaporated under reduced pressure. Dichloromethane (80 ml) and water (30 ml) were added and the mixture was separated. The organic layer was washed with water and brine, dried over magnesium sulfate and evaporated under reduced pressure. The residue was purified by chromatography (dichloromethane/methanol = 96/4) to give 170 mg of product [Molecular Formula: C₆₉H₁₂₆N₁₂O₁₂S; Exact Mass: 1346.93; MS (m/z): 1347.68 (M+1)⁺; TLC R_{f}: 0.32 (dichloromethane/methanol = 95/5); HPLC RT: 13.54 min. (C8 reverse phase column: 250 mm; acetonitrile/water (0.05% trifluoroacetic acid); operation temperature: 64 °C; detector: 210 nm)].

### Example 93

### [(R)-(2-(N,N-Diethylamino)ethoxy)methyl-Sar]-3-[N-MeIle]-4-cyclosporin

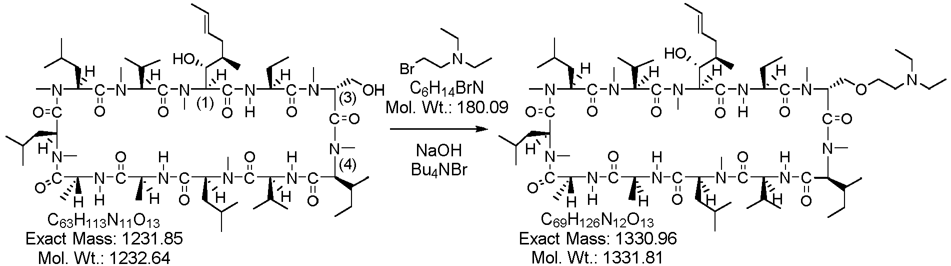

To a solution of [(R)-α-hydroxymethyl-Sar]-3-[N-MeIle]-4-cyclosporin (0.39 g, 0.32 mmol) in benzene (20 ml) were added a solution of sodium hydroxide (0.80 g, 20 mmol) in water (1 ml), 2-bromo-N,N-diethylethylamine hydrobromide (2.40 g, 9.20 mmol) and tetra-n-butylammonium bromide (0.10 g, 3.10 mmol). The mixture was stirred at 30 °C for 40 hours. Ice water (10 ml) was added and the mixture was separated. The aqueous layer was extracted with dichloromethane (50 ml). The combined organic layers was washed with brine, dried over magnesium sulfate and concentrated under reduced pressure. The residue was purified by chromatography (dichloromethane/methanol = 96/4) to give 270 mg of product [Molecular Formula: C₆₉H₁₂₆N₁₂O₁₃; Exact Mass: 1330.96; MS (m/z): 1331.73(M+1)⁺; TLC R_{f}: 0.34 (dichloromethane/methanol = 95/5); HPLC RT: 13.42 min. (C8 reverse phase column: 250 mm; acetonitrile/water (0.05% trifluoroacetic acid); operation temperature: 64 °C; detector: 210 nm)].

### Example 94 - (this example relates to the synthesis of intermediate)

### [α-Methylene-Sar]-3-[N-MeVal]-4-cyclosporin

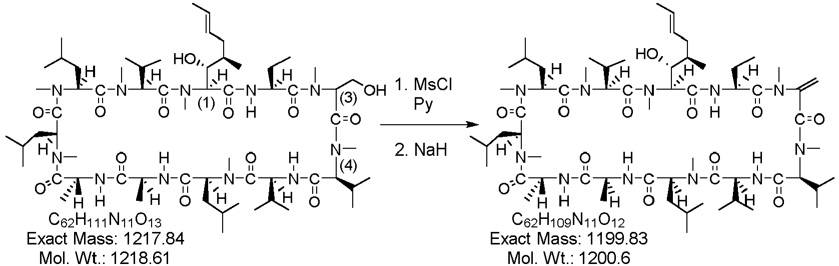

[α-Methylene]-3-[N-MeVal]-4-cyclosporin was prepared according to the method described in Example 4 and 5. The product was purified by chromatography on silica gel (ethyl acetate/methanol). [Molecular Formula: C₆₂H₁₀₉N₁₁O₁₂; Exact Mass: 1199.83; MS (m/z): 1200.56 (M+1)⁺, 1222.72 (M+Na⁾⁺].

### Example 95

### [(S)-(2-(N,N-dimethylamino)ethylthio)methyl-Sar]-3-[N-MeVal]-4-cyclosporin

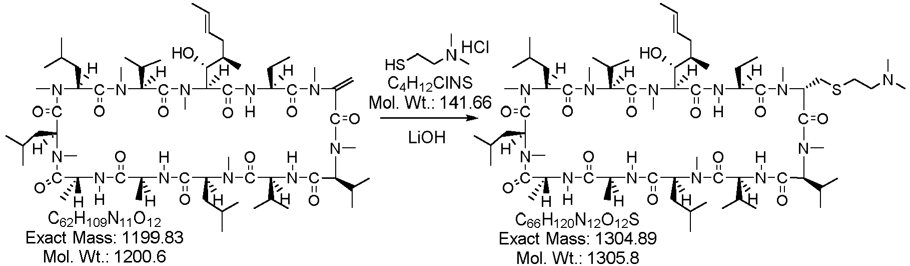

[α-Methylene-Sar]-3-[N-MeVal]-4-cyclosporin (88 mg, 0.07 mmol) and 2-(N,N-dimethyl)ethanethiol hydrochloride (0.10 g, 7.30 mmol) were dissolved in methanol (20 ml), followed by adding 20 equivalents of lithium hydroxide. The mixture was stirred overnight at room temperature. After removal of solvent, the residue was purified by flash chromatography using dichloromethane/methanol as eluent to give 30 mg of pure product [Molecular Formula: C₆₆H₁₂₀N₁₂O₁₂S; Exact Mass: 1304.89; MS (m/z): 1305.68 (M+1)⁺, 1327.83 (M+Na)⁺; TLC R_{f}: 0.05 (ethyl acetate/methanol = 5/1); HPLC RT: 12.23 min. (C8 reverse phase column: 250 mm; acetonitrile/water (0.05% trifluoroacetic acid); operation temperature: 64 °C; detector: 210 nm)].

### Example 96

### [(S)-(2-(N,N-Diethylamino)ethylthio)methyl-Sar]-3-[N-MeVal]-4-cyclosporin

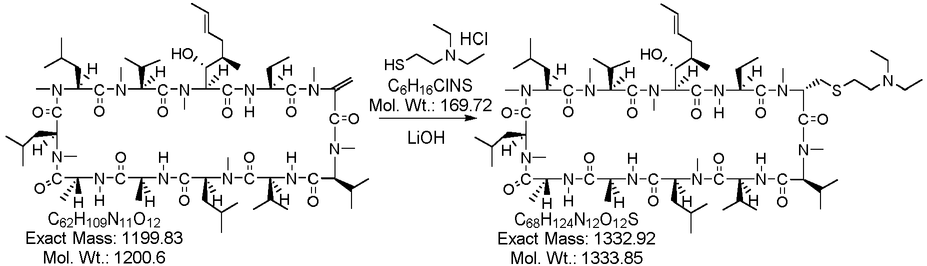

[α-Methylene-Sar]-3-[N-MeVal]-4-cyclosporin (0.20 g, 0.16 mmol) and 2-(N,N-diethyl)ethanethiol hydrochloride (0.28 g, 1.70 mmol) were dissolved in methanol, followed by adding 20 equivalents of lithium hydroxide. The mixture was stirred overnight. After removal of solvent, the residue was purified by chromatography on silica gel using dichloromethane/methanol as eluent to give 100 mg of pure product [Molecular Formula: C₆₈H₁₂₄N₁₂O₁₂S; Exact Mass: 1332.92; MS (m/e): 1333.58 (M+1)⁺, 1355.79 (M+Na)⁺; TLC R_{f}: 0.08 (ethyl acetate/methanol = 5/1); HPLC RT: 12.77 min. (C8 reverse phase column: 250 mm; acetonitrile/water (0.05% trifluoroacetic acid); operation temperature: 64 °C; detector: 210 nm)].

### Example 97 - (this example relates to the synthesis of intermediate)

### [(R)-α-Hydroxymethyl-Sar]-3-[N-MeVal]-4-cyclosporin

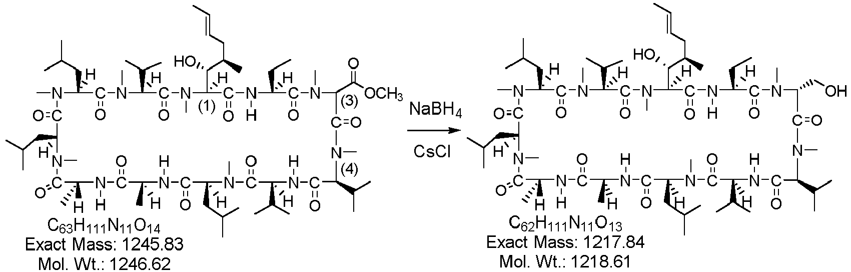

[(R)-α-Hydroxymethyl-Sar]-3-[N-MeVal]-4-cyclosporin was prepared according to the method described in Example 2. The product was purified by chromatography on silica gel (ethyl acetate/methanol) [Molecular Formula: C₆₂H₁₁₁N₁₁O₁₃; Exact Mass: 1217.84; MS (m/z): 1218.56 (M+1)⁺, 1240.75 (M+Na)⁺].

### Example 98

### [(R)-(2-(N,N-dimethylamino)ethoxy)methyl-Sar]-3-[N-MeVal]-4-cyclosporin

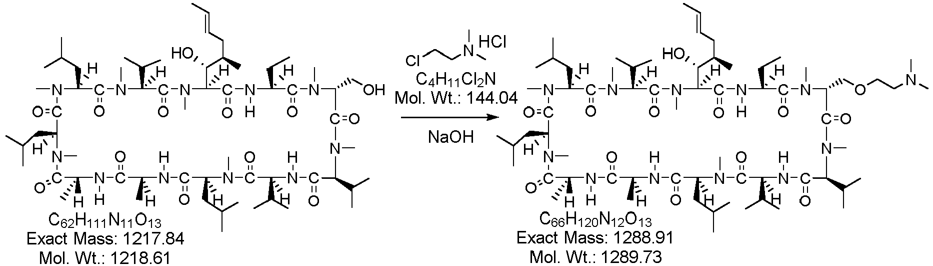

To a solution of [(R)-α-hydroxymethyl-Sar]-3-[N-MeVal]-4-cyclosporin (0.12 g, 0.10 mmol) in benzene (15 ml) were added sodium hydroxide (0.20 g. 5.00 mmol), tetramethylammonium hydroxide pentahydrate (0.18 g, 1.00 mmol) and 3-dimethylaminoethyl chloride hydrochloride (0.14 g, 1.00 mmol). The mixture was stirred at 30 °C overnight. Ice water (20 ml) was added and the mixture was separated. The aqueous layer was extracted with ethyl acetate (20 ml). The combined organic layers were washed with brine, dried over magnesium sulfate and evaporated under reduced pressure. The residue was subjected to chromatography on silica gel (dichloromethane/methanol = 95/5) to give the 30 mg of pure product [Molecular Formula: C₆₆H₁₂₀N₁₂O₁₃; Exact Mass: 1288.91; MS (m/z): 1289.73 (M+1)⁺, 1311.71 (M+Na)⁺; TLC R_{f} : 0.14 (dichloromethane/methanol = 10/1); HPLC RT: 12.00 min. (C8 reverse phase column: 250 mm; acetonitrile/water (0.05% trifluoroacetic acid); operation temperature: 64 °C; detector: 210 nm)].

### Example 99 - (this example does not fall within the scope of the claims)

### [(R)-α-(N-Piperidinyl)methyl-Sar]-3-[(γ-hydroxy)-NMeLeu]-4-cyclosporin

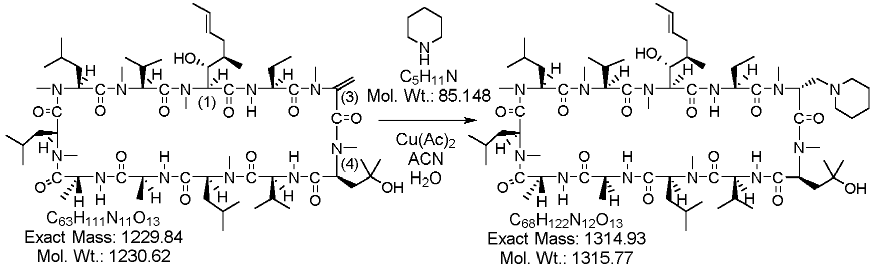

[α-Methylene-Sar]-3-[(y-hydroxy)-NMeLeu]-4-cyclosporin (0.37 g, 0.30 mmol) and piperidine (0.26 g, 3.00 mmol) were dissolved in acetonitrile/water (20 ml) in the presence of the catalytic amount of copper (II) acetate. The mixture was stirred overnight at room temperature. After removal of solvent, the residue was dissolved in dichloromethane (30 ml). The dichloromethane phase was washed with brine, dried over magnesium sulfate and evaporated under reduced pressure. The residue was further purified by chromatography on silica gel (dichloromethane/methanol, 96/4) to give 0.17 g of product [Molecular Formula: C₆₈H₁₂₂N₁₂O₁₃; Exact Mass: 1314.93; MS (m/z): 1315.74 (M+1)⁺, 1337.86 (M+Na)⁺; TLC R_{f}: 0.10 (ethyl acetate/methanol = 5/1); HPLC RT: 11.70 min. (C8 reverse phase column: 150 mm; acetonitrile/water (0.05% TFA); operation temperature: 64 °C; detector: 210 nm)].

### Reference Example 1

### [α-Carboxy-Sar]-3-cyclosporin

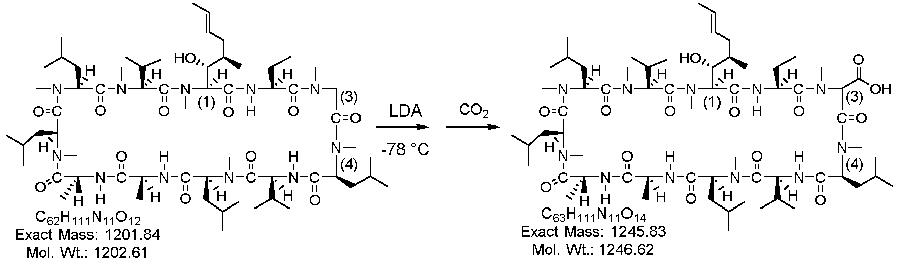

n-Butyllithium (2.87 M, 27 mmol, 9.4 ml, 10 eq) was added to a solution of diisopropylamine (3.8 ml, 27 mmol, 10 eq) in tetrahydrofuran (80 ml) at -78 °C under nitrogen. After the reaction mixture was stirred for an hour, a solution of cyclosporine A (3.2 g, 2.66 mmol) in tetrahydrofuran (15 ml) was added over 10 min. The stirring was continued at -78 °C for 2 hours. Carbon dioxide gas was bubbled through the reaction mixture for 20-25 minutes and stirred at -78 °C for another hour. Then the cooling bath was removed and the reaction mixture was allowed to warm up to 0°C slowly. Most of tetrahydrofuran was removed under vacuum at room temperature. The residue was quenched by the addition of saturated citric acid solution and the pH of the mixture was adjusted to around 7-8. The unreacted cyclosporin was extracted with ether (40 ml × 2). The PH of the aqueous layer was adjusted to 3∼4 with 1 N hydrochloric acid and the precipitated oil was extracted with ethyl acetate (100 ml). The aqueous layer was extracted with ethyl acetate (100 ml × 3). The combined ethyl acetate layers were washed with brine, dried over magnesium sulfate and evaporated under reduced pressure to give semi-solid product (2.61 g, yield: 78%) [Molecular Formula: C₆₃H₁₁₁N₁₁O₁₄; Exact Mass: 1245.83; MS (m/z): 1246.7 (M+1)⁺, 1268.7 (M+Na)⁺].

[α-Carboxy-Sar]-3-cyclosporin was synthesized according to a procedure described by Seebach D, et al., 1993, Helv Chim Acta, 76, 1564-1590.

### Reference Example 2

### Synthesis of [N-MeVal]-4-cyclosporin (SDZ-220-384)

[N-MeVal]-4-Cyclosporin (SDZ 220-384) was prepared according to procedures described by Papageorgiou C, et al., 1994, Bioorg & Med Chem Lett, 4, 267-272 and its key cylosporine ring-opening between position 3 and 4 cited as reference 14: Su Z and Wenger R, Unpublished results; Papageorgiou C, et al., 1994, J. Med. Chem., 37, 3674-3676 and its key cylosporine ring-opening between position 3 and 4 cited as reference 11: Su Z and Wenger R, Unpublished results.

### Cyclosporin A-acetate

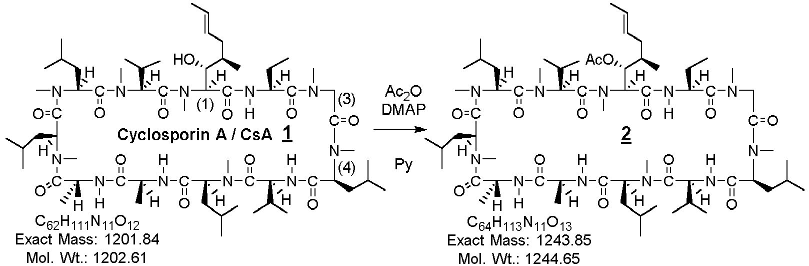

To a solution of cyclosporin A (**1**) (12.00 g, 19.98 mmol) in acetic anhydride (MW: 102.09, d 1.082, 40 ml) were added pyridine (MW: 79.01, d 0.978, 40 ml) and 4-N,N-dimethylaminopyridine (MW: 122.17, 0.40 g). This mixture was stirred for overnight at room temperature, and then the mixture was diluted with 600 ml of ethyl acetate. The mixture was washed with brine, saturated ammonium chloride solution and 15% of sodium bicarbonate solution. The organic phase was dried over sodium sulphate, filtered and evaporated under the reduced pressure. Then all of pyridine was azeotropically evaporated out under the reduced pressure by adding toluene to the mixture to give a pale yellow solid residue, which was purified by flash chromatography on a silica gel column (100-200 mesh) with eluent of ethyl acetate/hexane (1/3) to give the 11.80 g (9.48 mmol, 95%) of cyclosporin A-acetate (**2**).

### MeLeuValMeLeuAlaDAlaMeLeuMeLeuMeValMeBmt(OAc)AbuSar-Ome

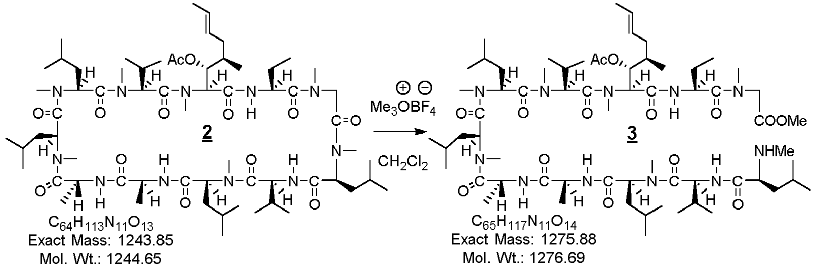

To a suspension of trimethyloxonium-fluoroborate (MW: 147.91, 2.96 g, 20 mmol, 2.50 equiv.) in dichloromethane (80 ml) was added cyclosporine A-acetate (**2**) (10.00 g, 8.00 mmol). The suspension was stirred for 18 hours at room temperature, and then a solution of sodium methoxide (9.90 mmol) in methanol (40 ml) was added. After the mixture was stirred for another half hour, 2 N solution of sulfuric acid in methanol (40 ml) was added. The mixture was stirred for 15-30 minutes at room temperature and neutralized with 15% potassium bicarbonate solution. Then the mixture was extracted twice with 700 ml of ethyl acetate. The combined organic layers were washed with brine, dried over sodium sulphate and evaporated under reduced pressure. The residue was purified by flash chromatography on a silica column (100-200 mesh) with eluent of methanol/methyl t-butyl ether to give the 7.15 g (5.60 mmol, 70%) of linear undecapeptide peptide (**3**).

### Phenylthiourea-MeLeuValMeLeuAlaDAlaMeLeuMeLeuMeValMeBmt(OAc)AbuSar-Ome

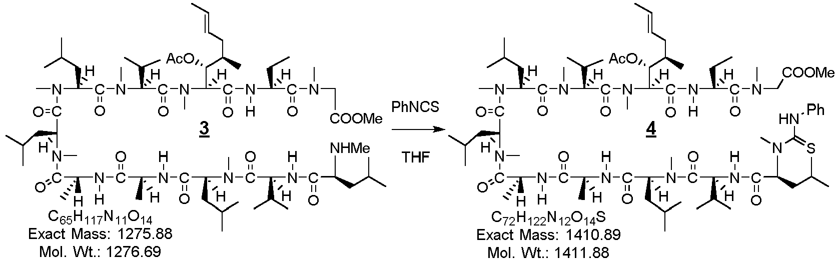

To a solution of linear undecapeptide peptide (**3**) (7.00 g, 5.50 mmol) in tetrahydrofuran (80 ml) was added phenyl isothiocyanate (MW: 135.19, d 1.130, 0.86 ml, 7.15 mmol, 1.30 equiv.). The mixture was stirred for 3 hours at room temperature and evaporated under reduced pressure. The residue was purified by flash chromatography on a silica gel column (100-200 mesh) with eluent of acetone in hexane (1/5) to give the 6.99 g (4.95 mmol, 90%) of linear phenylthiourea undecapeptide (**4**) [Exact Mass: 1410.89; MS m/z: 1433.88 (M+Na)⁺].

### ValMeLeuAlaDAlaMeLeuMeLeuMeValMeBmt(OAc)AbuSar-Ome

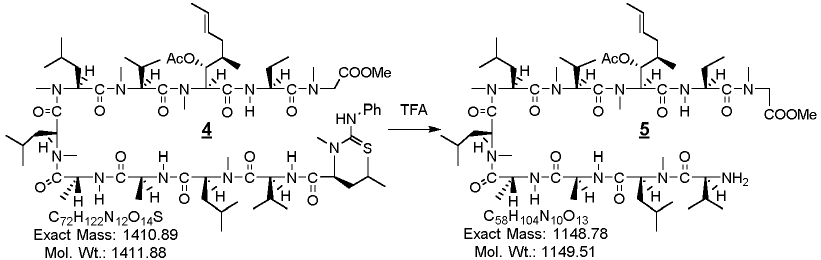

To a solution of linear phenylthiourea undecapeptide (**4**) (6.80 g, 4.82 mmol) in toluene (300 ml) was added trifluoroacetic acid (MW: 114.02, d 1.480, 8.00 ml) at room temperature. The mixture was stirred for 1.5 to 2 hours and quenched by a slurry of sodium bicarbonate in water. Then the mixture was separated and the water phase was extracted with toluene (100 ml) and ethyl acetate (100 ml) subsequently. The combined organic layers were dried over sodium sulphate and evaporated under reduced pressure. The residue was purified by flash chromatography on a silica column (100-200 mesh) with eluent of acetone/hexane (3/1) to give the 3.88 g (3.37 mmol, 70%) of linear decapeptide peptide (**5**) [Exact Mass: 1148.78; MS m/z: 1149.78 (M+1)⁺].

This Edman degradation was carried according to the similar method described by Edman P, et al, 1967, Eur. J. Biochem., 1, 80.

### BocMeValValMeLeuAlaDAlaMeLeuMeLeuMeValMeBmt(OAc)AbuSar-Ome

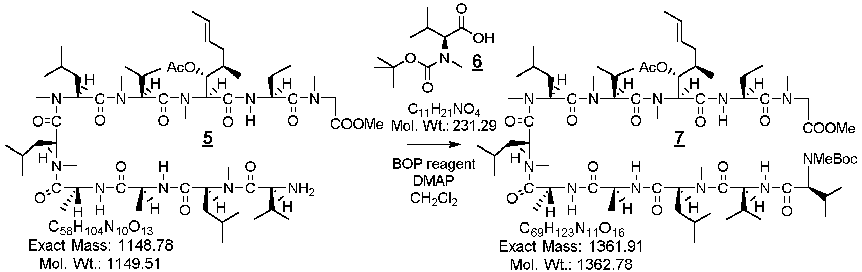

To a solution of linear decapeptide peptide (**5**) (3.80 g, 3.30 mmol) in dichloromethane (150 ml) were added Boc-MeVal (**6**) (MW: 231.29, 0.92 g, 3.96 mmol, 1.2 equiv.), 1-propanephosphonic acid cyclic anhydride (MW: 318.18, 2.10 ml, 50 wt.% solution in ethyl acetate) and triethylamine (MW: 101.19, d 0.726, 0.46 ml, 3.30 mmol) at 0 °C. The resulting mixture was stirred at room temperature for 5 hours. Then the mixture was washed with brine. The aqueous layer was extracted with ethyl acetate (100 ml). The combined organic layers were dried over sodium sulphate. Removal of the solvent under reduced pressure gave the residue, which was purified by flash chromatography on a silica column (100-200 mesh) with eluent of acetone/hexane (1/2.5) to give the 4.05 g (2.97 mmol, 90%) of linear Boc-N-MeVal-decapeptide peptide (**7**) [Exact Mass: 1361.91; MS m/z: 1384.91 (M+Na)⁺].

### BocMeValValMeLeuAlaDAlaMeLeuMeLeuMeValMeBmt(OAc)AbuSar-OH

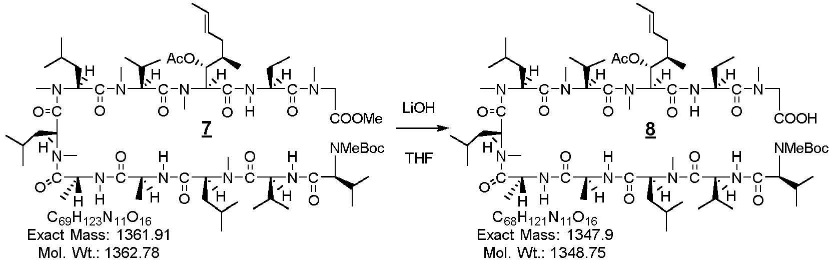

To a solution of linear [Boc-N-MeVal]-4-decapeptide peptide (**7**) (4.00 g, 2.94 mmol) in ethyl alcohol (150 ml) at 0 °C was added 0.5 N sodium hydroxide solution (7.1 ml, 1.20 equiv.). The mixture was stirred and kept at 0 °C for 16 hours. Then the PH of the mixture was adjusted to around 3 by adding 0.5 N hydrochloric acid. Most of solvent was evaporated under the reduced pressure and the residue was dissolved in 200ml of ethyl acetate. The mixture was washed with a pH 3 buffer, dried over sodium sulphate, filtered and evaporated under the reduced pressure. The residue was purified by flash chromatography on a silica column (100-200 mesh) with eluent of methanol/ethyl acetate (1/8) to yield 2.55 g (1.89 mmol, 64.3%) of the free acid (**8**).

### MeValValMeLeuAlaDAlaMeLeuMeLeuMeValMeBmt(OAc)AbuSar-OH

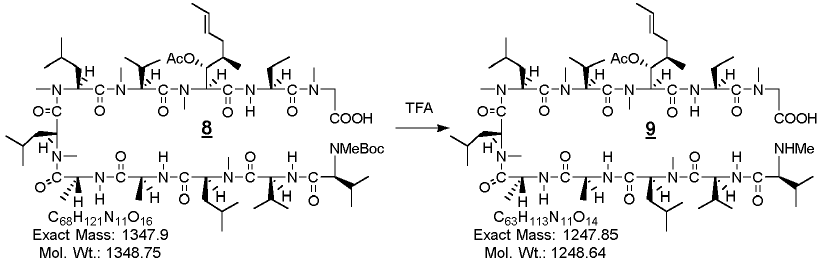

To a solution of free acid (**8**) (2.55g, 1.89 mmol) in dichloromethane (25 ml) was slowly added 5 ml of trifluoroacetic acid (MW: 114.02, d 1.480) at 0 °C. The solution was stirred at room for 2 hours. Then ethyl acetate (300 ml) was added and the solvent was removed under reduced pressure. Another ethyl acetate (300 ml) was added and the solvent was removed under reduced pressure again. The residue was purified by flash chromatography on a silica gel column (100-200 mesh) with eluent of methanol/acetone (1/3) to give the 2.01g (1.61 mmol, 85%) of linear [N-MeVal]-4-decapeptide peptide free acid (**9**) [Exact Mass: 1247.85; MS m/z: 1248.85 (M+1)⁺].

### [N-MeVal]-4-Cyclosporin acetate

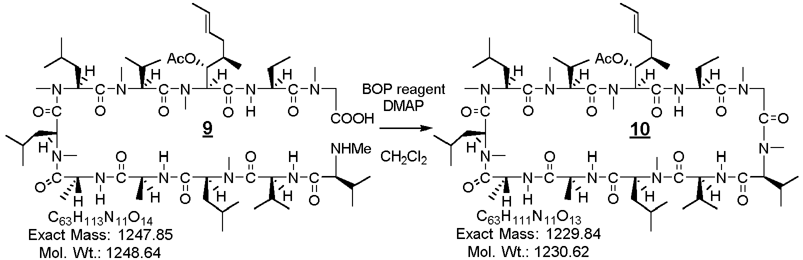

To a solution of linear [N-MeVal]-4-decapeptide peptide free acid (**9**) (1.03 g, 0.83 mmol) in dichloromethane (250 ml) were added 1-propanephosphonic acid cyclic anhydride (MW: 318.18, 0.53 ml, 50wt.% solution in ethyl acetate), 2,4,6-collidine (MW: 121.18, d 0.917, 0.11 ml, 0.83 mmol)) at 0 °C. The mixture was stirred at room temperature for 24 hours. Then the mixture was passed through a thin layer of silica gel and washed twice by 40 ml of ethyl acetate. The collected organic solution was evaporated under the reduced pressure. The residue was purified by flash chromatography on a silica gel column (230-400 mesh) with eluent of methanol/acetone (1/6) to give the 611 mg (0.50 mmol, 60%) of (N-Methyl-Val)-4-Cyclosporin acetate (**10**) [Exact Mass: 1229.84; MS m/z: 1252.82 (M+Na)⁺].

### [N-MeVal]-4-Cyclosporin

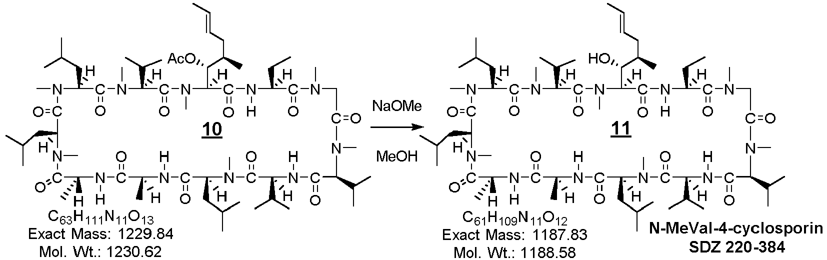

To a solution of [N-MeVal]-4-Cyclosporin acetate (**10**) (0.60 g, 0.49 mmol) in methanol (40 ml) was added a solution of sodium methoxide in methanol (0.5 M, 1.9 ml, 2.0 equiv.). The mixture was stirred for 0.5 hour at 0 °C and 24 hours at room temperature. The PH of the mixture was adjusted to around 6 by adding 0.5 N hydrochloric acid. After the solvent was evaporated under reduced pressure, the residue was dissolved in 200 ml of ethyl acetate. The organic solution was washed by aqueous sodium bicarbonate and brine, dried over sodium sulphate and filtered. After removal of solvent, the residue was purified by flash chromatography on a silica gel column (230-400 mesh) with eluent of acetone/hexane (1/2) to give the 406 mg (0.34 mmol, 70%) of [N-MeVal]-4-Cyclosporin (**11**) [Exact Mass: 1187.83;84; MS m/z: 1210.81 (M+Na].

### Reference Example 3

[N-MeIle]-4-Cyclosporin (NIM-811) was prepared according to the procedure used for the synthesis of (N-MeVal)-4-cyclosporin (SDZ 220-384).

### Reference Example 4

The side chain intermediates were synthesized according to procedures described by Urquhart GG, 1994, Org Synth, Coll. Vol III, 363

### 2-Morpholinoethanethiol

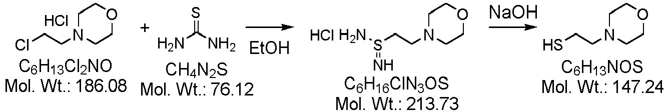

A mixture of 4-(2-chloroethyl)morpholine (7.00 g, 37 mmol) and thiourea (2.90 g, 38 mmol) in 95% ethanol (55 ml) was heated to reflux for 24 hours. A solution of sodium hydroxide (3.40 g, 85 mmol) in water (20 ml) was added, and the mixture was continued to reflux for another 3 hours. After cooled to room temperature, the mixture was evaporated under reduced pressure. The residue was mixed with benzene. The benzene layer was washed with brine, dried over magnesium sulfate and evaporated to provide 3.80 g of crude product, which was used for the addition reaction.

### 2-(N-Piperidinyl)ethanethiol

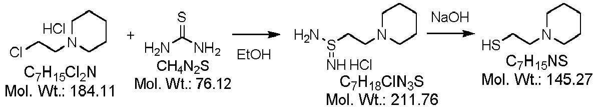

The mixture of 1-(2-chloroethyl)piperidine hydrochloride (7.00 g, 38 mmol) and thiourea (4.60 g, 61 mmol) in 95% ethanol (30 ml) was heated to reflux for 24 hours. A solution of sodium hydroxide (2.40 g) in water (20 ml) was added. The mixture was continued to reflux for another 3 hours. After cooled to room temperature, the mixture was evaporated under reduced pressure. The residue was mixed with ether. The organic layer was washed with brine, dried over magnesium sulfate and evaporated under reduced pressure to give 3.20 g of crude product, which was used for the addition reaction without purification.

### 2-(N-Pyrrolidinyl)ethanethiol

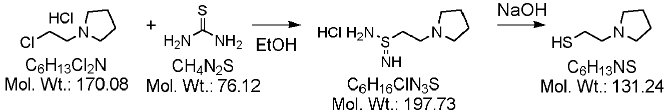

The mixture of 1-(2-chloroethyl)piperidine hydrochloride (7.0 g, 41 mmol) and thiourea (3.20 g, 40 mmol) in 95% ethanol (30 ml) was heated to reflux for 24 hours. A solution of sodium hydroxide (3.40 g, 85 mmol) in water (20 ml) was added. The mixture was continued to reflux for another 3 hours. After cooled to room temperature, the mixture was evaporated under reduced pressure. The residue was mixed with benzene. The organic layer was washed with brine, dried over magnesium sulfate and evaporated under reduced pressure to give 3.80 g of crude product, which was used for the addition reaction without purification.

### 3-(N-Pyrrolidinyl)propanethiol

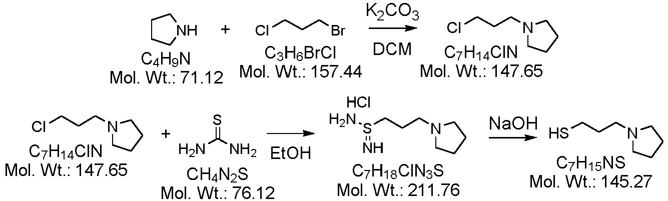

To a suspension of 1-bromoro-3-chloropropane (30.00 g, 191 mmol) and potassium carbonate (17.00 g, 123 mmol) in dichloromethane (160 ml) was added pyrrolidine (3.50 g, 49 mmol) portions. The mixture was stirred at room temperature overnight. Then the mixture was filtered and evaporated under reduced pressure. The residue was purified by chromatography (ethyl acetate/methanol = 95/5) to give 6.00 g of product.

A mixture of 1-(3-chloropropyl)pyrrolidine (3.4 g, 23 mmol) and thiourea (1.8 g, 23 mmol) in 95% ethanol (55 ml) was heated to reflux for 24 hours. A solution of sodium hydroxide (1.20 g, 30 mmol) in water (10 ml) was added, and the mixture was continued to reflux for another 3 hours. After cooled to room temperature, the mixture was evaporated under reduced pressure. The residue was mixed with benzene. The organic layer was washed with brine, dried over magnesium sulfate and evaporated under reduced pressure to give 1.80 g of crude product, which was used for the addition reaction.

### 3-(N-Piperidinyl)propanethiol

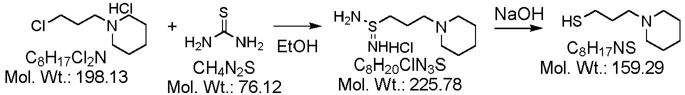

The mixture of 1-(3-chloropropyl)piperidine hydrochloride (7.50 g, 38 mmol) and thiourea (4.60 g, 61 mmol) in 95% ethanol (30 ml) was heated to reflux for 24 hours. A solution of sodium hydroxide (2.40 g) in water (20 ml) was added. The mixture was continued to reflux for another 3 hours. After cooled to room temperature, the mixture was evaporated under reduced pressure. The residue was mixed with ether. The organic layer was washed with brine, dried over magnesium sulfate and evaporated under reduced pressure to give 3.50 g of crude product, which was used for the addition reaction without purification.

### 3-Morpholinopropanethiol

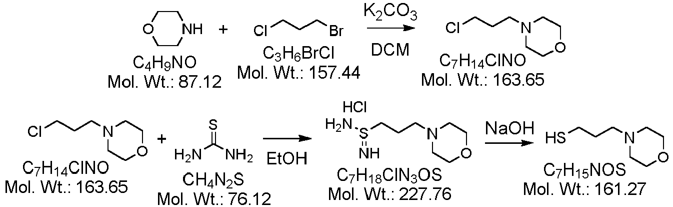

To a suspension of 1-bromoro-3-chloropropane (30.00 g, 191 mmol) and potassium carbonate (14.00 g, 101 mmol) in dichloromethane (160 ml) was added morpholine (4.00 g, 46 mmol) in portions. Then the mixture was stirred at room temperature overnight. The mixture was filtered and evaporated under reduced pressure. The residue was purified by chromatography (ethyl acetate) to give 5.60 g of product.

A mixture of 1-(3-chloropropyl)morpholine (4.20 g, 25.76 mmol) and thiourea (2.00 g, 26.27 mmol) in 95% ethanol (55 ml) was heated to reflux for 24 hours. A solution of sodium hydroxide (1.3 g, 32.50 mmol) in water (10 ml) was added, and the mixture was continued to reflux for another 3 hours. After cooled to room temperature, the mixture was evaporated under reduced pressure. The residue was mixed with benzene. The benzene layer was washed with brine, dried over magnesium sulfate and evaporated under reduced pressure to give 2.20 g of crude product, which was used for the addition reaction.

### 2-(4-Methyl-N-piperazinyl)ethanethiol

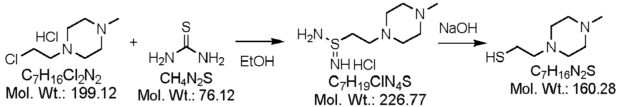

A mixture of 2-(4-methylpiperazino)ethyl chloride (8.00 g, 40 mmol) and thiourea (4.87 g, 64 mmol) in 95% ethanol (55 ml) was heated to reflux for 24 hours. A solution of sodium hydroxide (2.60 g) in water (20 ml) was added, and the mixture was continued to reflux for another 3 hours. After cooled to room temperature, the mixture was evaporated under reduced pressure. The residue was mixed with benzene. The benzene layer was washed with brine, dried magnesium sulfate and evaporated to provide 3.0 g of crude product, which was used for the addition reaction.

### 3-(4-Methyl-N-piperazinyl)propanethiol

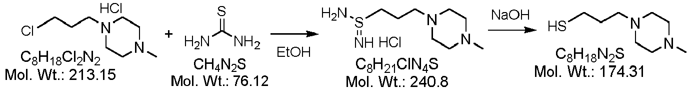

A mixture of 3-(4-methylpiperazino)propyl chloride (8.5 g, 40 mmol) and thiourea (4.87 g, 64 mmol) in 95% ethanol (70 ml) was heated to reflux for 24 hours. A solution of sodium hydroxide (2.6 g) in water (20 ml) was added, and the mixture was continued to reflux for another 3 hours. After cooled to room temperature, the mixture was evaporated under reduced pressure. The residue was mixed with ether. The organic layer was washed with brine, dried over magnesium sulfate and evaporated to provide 2.5 g of crude product, which was used for the addition reaction.

### 3-(N-Ethyl-N-isopropylamino)propanethiol

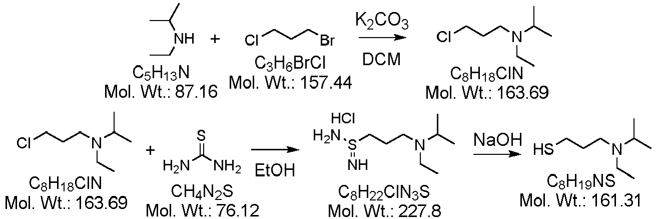

To a suspension of 1-bromoro-3-chloropropane (11.00 g, 70 mmol) and potassium carbonate (13.00 g, 94 mmol) in dichloromethane (100 ml) was added ethylisopropylamine (4.10 g, 47 mmol) in portions. The mixture was stirred at room temperature overnight. The mixture was filtered and concentrated under reduced pressure. The residue was purified by chromatography (ethyl acetate/methanol = 95/5) to give 6.10 g of product.

A mixture of 3-chloropropylethylisopropylamiune (4.20 g, 25.66 mmol) and thiourea (2.00 g, 26.32 mmol) in 95% ethanol (55 ml) was heated to reflux for 24 hours. A solution of sodium hydroxide (1.30 g, 32.50 mmol) in water (10 ml) was added, and the mixture was continued to reflux for another 3 hours. After cooled to room temperature, the mixture was evaporated under reduced pressure. The residue was mixed with benzene. The benzene layer was washed with brine, dried over magnesium sulfate and evaporated under reduced pressure to give 1.20 g of crude product, which was used for the addition reaction.

### Example 100

### Stability Testing of [(R)-3-(N,N-Dimethylamino)ethylthio-Sar]-3-[(γ-Hydroxy)-NMeLeu]-4-cyclosporin (SCY-635) and Cyclosporin Derivatives

Stability of Cyclosporin derivatives was evaluated in methanol at 65 °C and 50 °C, and HPLC was used to monitor possible isomerization of these compounds. It was found that SCY-635 is not stable and can easily convert to its corresponding epimer, which is expected to have low or no anti-viral activity.

### Epimerization of SCY-635* in MeOH at 65 °C

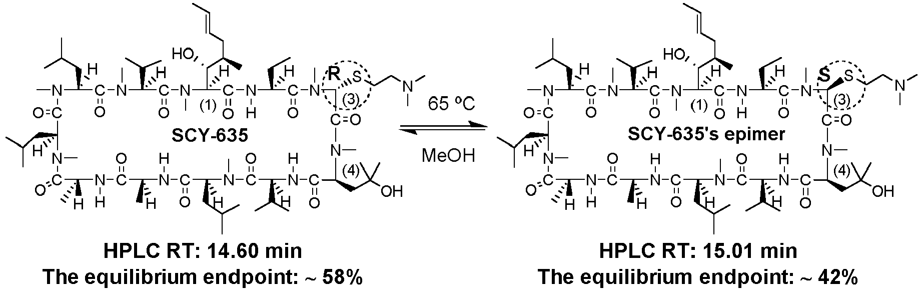

| Epimerization | SCY-635's epimer % | | | | |
|---|---|---|---|---|---|
| | 2 hours | 4 hours | 6 hours | 8 hours | 10 hours |
| **SCY-635** 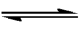 **SCY-635's epimer** | 24% | 35% | 39% | 41% | 43% |

| | | | | | |
|---|---|---|---|---|---|
| *SCY-635 was prepared according to a method described by: Evans M, et al., 2003, Bioorg. Med. Chem. Lett., 4, 4415-4419; Carry J, et al., 2004, Synlett. 2, 316-320; or U.S. Pat. No. 5,994,299. | | | | | |

**Epimerization of SCY-635's epimer* in MeOH at 65 °C**

| Epimerization | SCY-635 % | | |
|---|---|---|---|
| | 3 hours | 6 hours | 10 hours |
| **SCY-635's epimer** 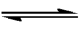 **SCY-635** | 51% | 58% | 58% |

*During the stability study, it was found that SCY-635 transformed into its epimer, which was separated as a pure compound. HPLC RT: 14.60 minutes (SCY-635) and: 15.01 minutes (its epimer) (C8 reverse phase column, 250 mm, acetonitrile/0.077% NH₄OAc in water, operation temperature: 64 °C; Detector: 210 nm).

When the epimer was treated with MeOH at 65 °C, it also was found that it partially transformed to SCY-635. At the endpoint of the equilibrium, this solution contained about 58% of SCY-635 and about 42% of epimer.

**Table 1, Epimerization of [(R)-2-(N, N-dimethylamino)ethylthio-Sar]-3-cyclosporin in MeOH at 65 °C**

| Compound | Epimerization% | | |
|---|---|---|---|
| | 2 hours | 4 hours | 6 hours |
| 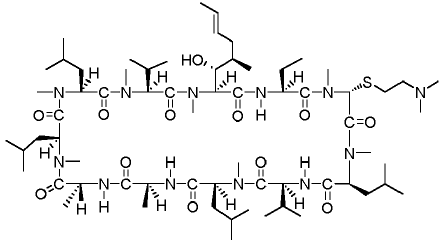 | ∼12% | ∼19% | ∼23% |
| [(R)-2-(N, N-Dimethylamino)ethylthio-Sar]-3-cyclosporin | | | |

**Table 2, Epimerization of [(R)-3-(N-Morpholino)propylthio-Sar]-3-[(γ-hydroxy)-NMeLeu]-4-cyclosporin in MeOH at 65 °C**

| Compound | Epimerization% in MeOH at 65 °C | | | | |
|---|---|---|---|---|---|
| | 2 hours | 4 hours | 6 hours | 8 hours | 10 hours |
| 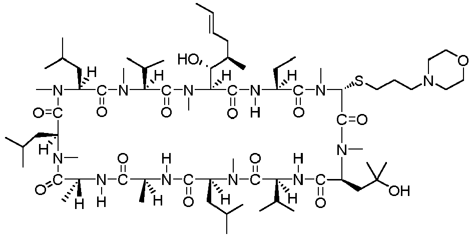 | 0% | 0% | 0% | Less than 1% | ∼10% |
| [(R)-3-(Morpholino)propylthio-Sar]-3-[(γ-hydroxy)-NMeLeu]-4-cyclosporin | | | | | |

**Table 3, Epimerization of [(R)-3-(N-Morpholino)propylthio-Sar]-3-[(γ-Methoxy)-NMeLeu]-4-cyclosporin in MeOH at 65 °C**

| Compound | Epimerization% in MeOH at 65 °C | | | | | |
|---|---|---|---|---|---|---|
| | 2 hours | 4 hours | 10 hours | 22 hours | 30 hours | 38 hours |
| 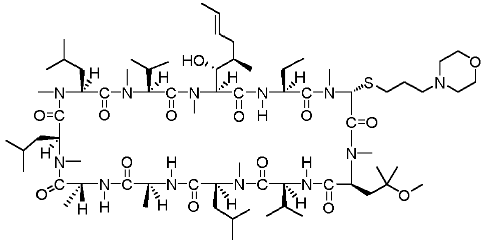 | 0% | 0% | 0% | 0% | 0% | 0% |
| [(R)-3-(N-Morpholino)propylthio-Sar]-3-[(γ-methoxy)-NMeLeu]-4-cyclosporin | | | | | | |

**Table 4, Epimerization of Cyclosporin Derivatives in MeOH at 50 °C**

| Compounds | Epimerization% | | |
|---|---|---|---|
| | 29 hours | 77 hours | 125 hours |
| 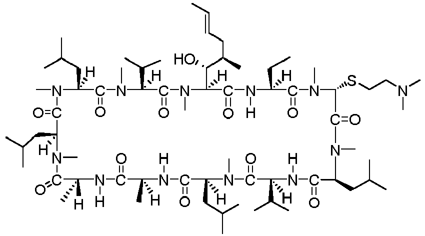 | ∼26% | ∼32% | ∼35% |
| [(R)-2-(N, N-Dimethylamino)ethylthio-Sar]-3-cyclosporin | | | |
| 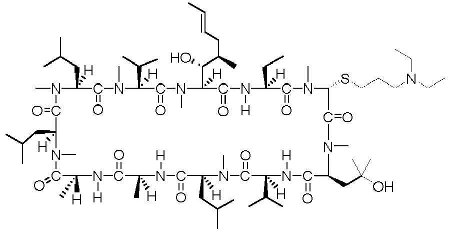 | 0% | Less than 1% | ∼12% |
| [(R)-3-(N, N Diethylamino)propylthio-Sar]-3-[(γ-hydroxy)-NMeLeu]-4-cyclosporin | | | |
| 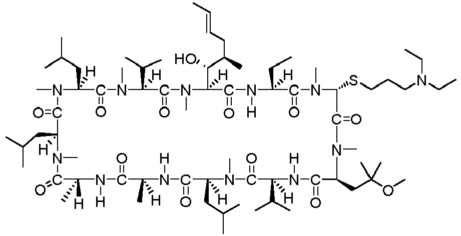 | 0% | 0% | 0% |
| [(R)-3-(N, N-Diethylamino)propylthio-Sar]-3-[(γ-methoxy)-NMeLeu]-4-cyclosporin | | | |
| 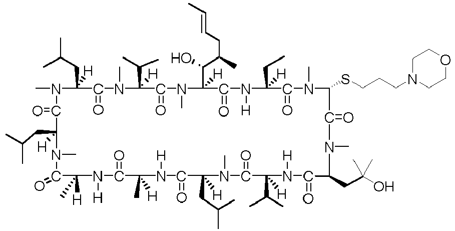 | 0% | 0% | 0% |
| [(R)-3-(N-Morpholino)propylthio-Sar]-3-[(γ-hydroxy)-NMeLeu]-4-cyclosporin | | | |
| 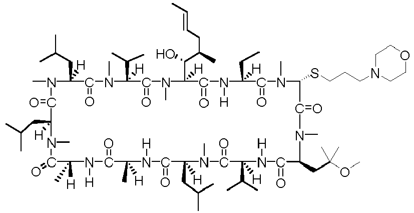 | 0% | 0% | 0% |
| [(R)-3-(N-Morpholino)propylthio-Sar]-3-[(γ-methoxy)-NMeLeu]-4-cyclosporin | | | |
| 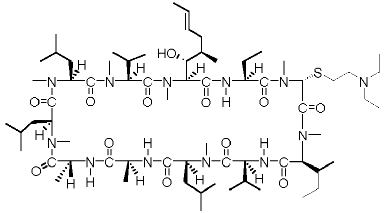 | ∼4% | ∼10% | ∼16% |
| [(R)-2-(N,N-Diethylamino)ethylthio-Sar]-3-[NMeIle]-4-cyclosporin | | | |
| 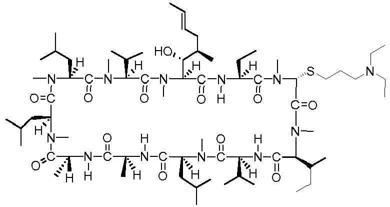 | 0% | 0% | 0% |
| [(R)-3-(N,N-Diethylamino)propylthio-Sar]-3-[NMeIle]-4-cyclosporin | | | |
| 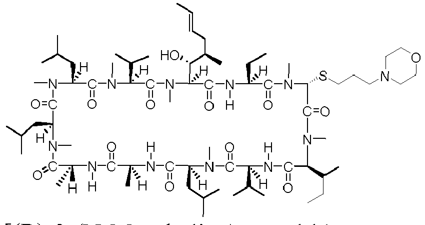 | 0% | 0% | 0% |
| [(R)-3-(N-Morpholino)propylthio-Sar]-3-[NMeIle]-4-cyclosporin | | | |
| 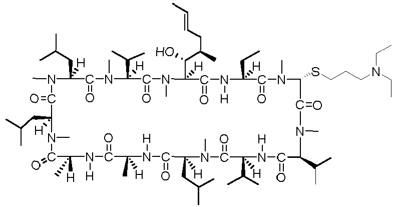 | 0% | 0% | 0% |
| [(R)-3-(N,N-Diethylamino)propylthio-Sar]-3-[N-MeVal]-4-cyclosporin | | | |
| 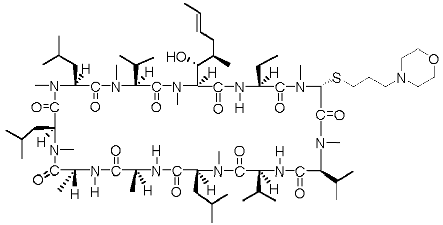 | 0% | 0% | 0% |
| [(R)-3-(N-Morpholino)propylthio-Sar]-3-[NMeVal]-4-cyclosporin | | | |

**Table 5, Epimerization of Cyclosporin Derivatives in MeOH at 50 °C - 58 °C**

| Compound | Epimerization% after 168 hours |
|---|---|
| 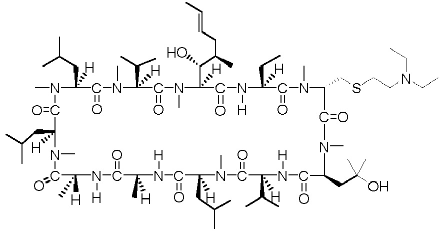 | 0% |
| [(S)-(2-(N,N-Diethylamino)ethylthio)methyl-Sar]-3-[(γ-hydroxy)-NMeLeu]-4-cyclosporin (Example 29, isomer B) | |
| 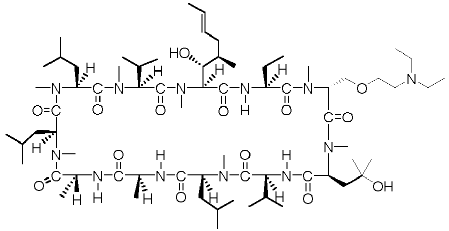 | 0% |
| [(R)-(2-(N,N-Diethylamino)ethoxy)methyl-Sar]-3-[(γ-hydroxy)-NMeLeu]-4-cyclosporin (Example 50) | |
| 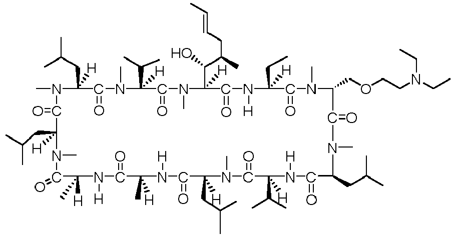 | 0% |
| [(R)-(2-(N,N-Diethylamino)ethoxy)methyl-Sar]-3-cyclosporin (Example 8) | |
| 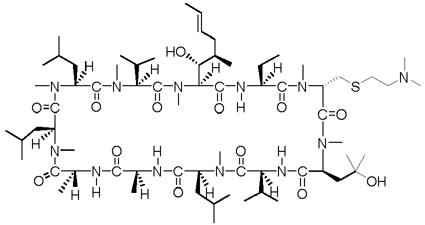 | 0% |
| [(S)-(2-(N,N-Dimethylamino)ethylthio)methyl-Sar]-3-[(γ-hydroxy)-NMeLeu]-4-cyclosporin (Example 28, isomer B) | |
| 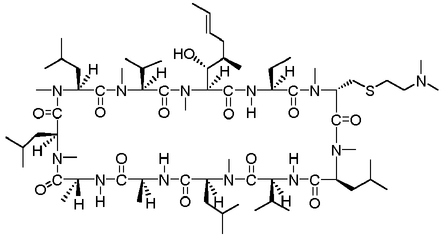 | 0% |
| [(S)-(2-(N,N-Dimethylamino)ethylthio)methyl-Sar]-3-cyclosporin (Example 6, isomer B) | |
| 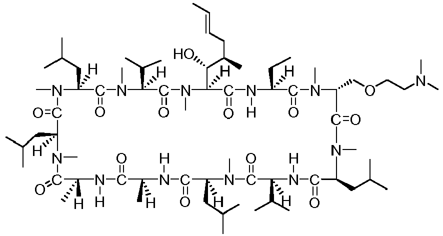 | 0% |
| [(R)-(2-(N,N-Dimethylamino)ethoxy)methyl-Sar]-3-cyclosporin (Example 13) | |
| 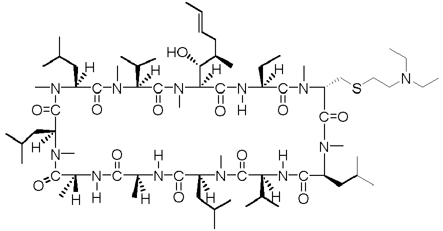 | 0% |
| [(S)-(2-(N,N-Diethylamino)ethylthio)methyl-Sar]-3-cyclosporin (Example 7, isomer B) | |
| 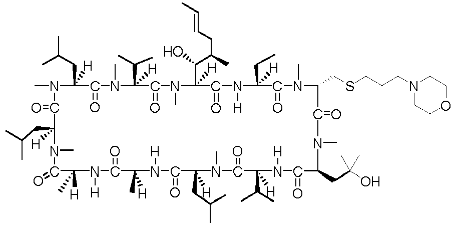 | 0% |
| [(S)-(3-(N-Morpholino)propylthio)methyl-Sar]-3-[(γ-hydroxy)-NMeLeu]-4-cyclosporin (Example 45) | |
| 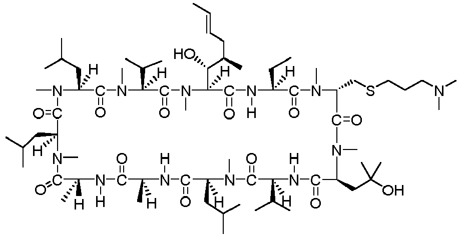 | 0% |
| [(S)-(3-(N,N-Dimethylamino)propylthio)methyl-Sar]-3-[(γ-hydroxy)-NMeLeu]-4-cyclosporin (Example 47) | |
| 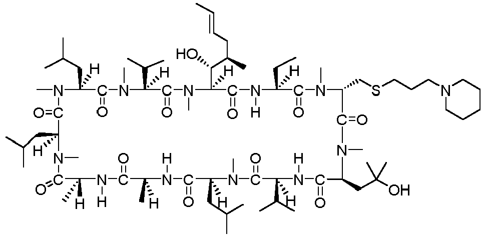 | 0% |
| [(S)-(3-(N-Piperidinyl)propylthio)methyl-Sar]-3-[(y-hydroxy)-NMeLeu]-4-cyclosporin (Example 43) | |
| 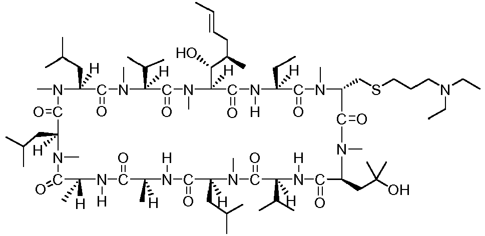 | 0% |
| [(S)-(3-(N,N-Diethylamino)propylthio)methyl-Sar]-3-[(γ-hydroxy)-N-MeLeu]-4-cyclosporin (Example 48) | |

Based on the isomerization data, the inventors suggest that the epimerization of SCY-635 occurs through the following process:

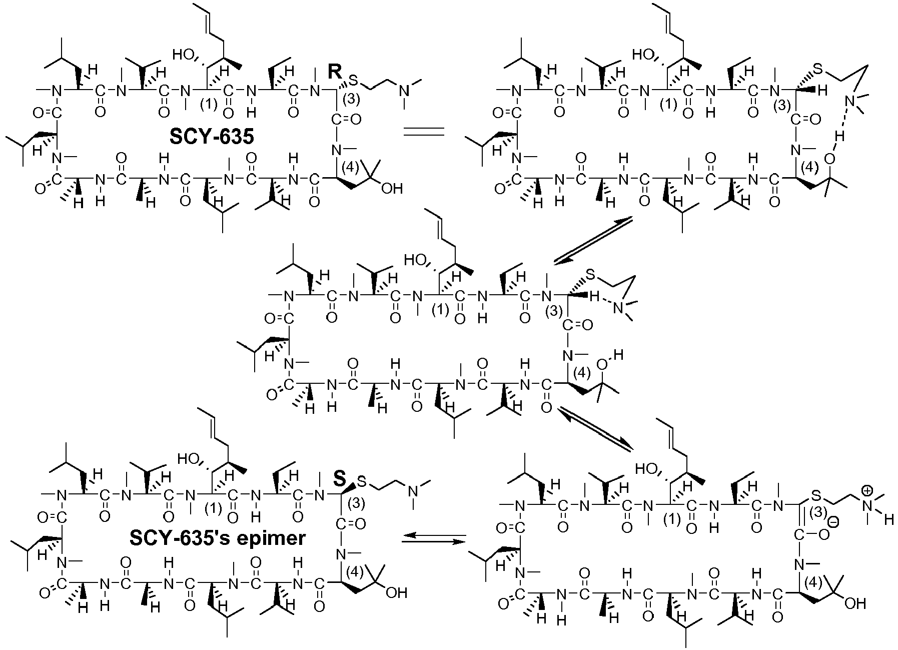

Thus, the two carbon side chain at position 3 of the sarcosine of cyclosporine contributes to the instability, because it can form a six-membered ring transation state, and stimulate the epimerization. Additionally, the epimerization is accelerated by the γ-hydroxy group at the 4-position of leucine.

Accordingly, the inventors envisioned novel cyclosporine derivatives having enhanced stability while maintaining good cyclophilin binding activity. In particular, the inventors have surprisingly found that the masking the γ-hydroxy group on leucine at position 4, elongating side carbon chain (e.g., with 3 carbons or higher), and/or substituting the amine terminal at position 3 with a bulky side chain can prevent or minimize the epimerization. Specially, when the methlene substituents are introduced on position-3, those analogs are very stable, and can prevent the epimerization.

### Example 101

### Anti HCV Activity of Cyclosporin Derivatives

Anti-HCV activity of cyclosporine derivatives were evaluated in the HCV subgenomic replicon assay. The assay use the cell line ET (luc-ubi-neo/ET), which is a Huh7 human hepatoma cell line harboring an HCV replicon with a stable luciferase (Luc) reporter. HCV RNA replication was assessed by quantifying HCV replicon-derived luciferase activity. The antiviral activity of cyclosporine analogs were evaluated after drug treatment to derive EC₅₀ and EC₉₀ values by using the luciferase end point (Krieger, N., et al., 2001, J. Virol. 75, 4614-4624; Pietschmann, T., et al., 2002, J. Virol. 76, 4008-4021). Cytotoxicity was assessed in parallel.

**Table 6, Testing results of certain representative compounds**

| Compound | Antiviral activity EC₅₀ (µM) |
|---|---|
| Cyclosporine A | 0.41 |
| [N-MeIle]-4-cyclosporin (SDZ-NIM-811) | 0.15 |
| [N-MeVal]-4-cyclosporin (SDZ 220-384) | 0.17 |
| (R)-2-(N,N-Diethylamino)ethylthio-Sar]-3-[N-MeIle]-4-cyclosporin | 0.04 |
| (S)-2-(N,N-Diethylamino)ethylthio-Sar]-3-[N-MeIle]-4-cyclosporin | 1.87 |
| (R)-2-(N,N-Diethylamino)ethylthio-Sar]-3-[N-MeVal]-4-cyclosporin | 0.04 |
| (S)-2-(N,N-Diethylamino)ethylthio-Sar]-3-[N-MeVal]-4-cyclosporin | 3.66 |
| (R)-2-(N,N-Dimethylamino)ethylthio-Sar]-3-[N-MeVal]-4-cyclosporin | 0.13 |
| (R)-2-(N-neo-Pentylamino)ethylthio-Sar]-3-cyclosporin | 0.23 |
| (S)-2-(N-neo-Pentylamino)ethylthio-Sar]-3-cyclosporin | 3.09 |
| (R)-2-(N-iso-Butyl-N-iso-propylamino)ethylthio-Sar]-3-cyclosporin | 0.48 |
| (S)-2-(N-iso-Butyl-N-iso-propylamino)ethylthio-Sar]-3-cyclosporin | 4.65 |
| (R)-2-(N-Diethylamino)ethylthio-Sar]-3-cyclosporin | 0.16 |
| [(R)-2-(N,N-Diethylamino)ethylthio-Sar]-3-[(γ-Methylthio)methoxy-NMeLeu]-4-cyclosporin | 0.11 |

**Table 7, Testing results of certain representative compounds**

| Compound | Antiviral activity EC₅₀ (µM) |
|---|---|
| [N-MeVal]-4-cyclosporin (SDZ 220-384) | 0.12 |
| (R)-2-(N,N-Dimethylamino)ethylthio-Sar]-3-[(γ-hydroxy)-N-MeLeu]-4-cyclosporin (SCY-635) | 0.08 |
| [(R)-3-(N-Morpholino)propylthio-Sar]-3-[(γ-hydroxy)-N-MeLeu]-4-cyclosporin | 0.05 |
| [(R)-3-(N-Morpholino)propylthio-Sar]-3-[(γ-hydroxy)-N-MeLeu]-4-dihydrocyclosporin | 0.15 |
| [(R)-3-(N-Morpholino)propylthio-Sar]-3-[(γ-methoxy)-N-MeLeu]-4-cyclosporin | 0.06 |
| (R)-3-(N,N-Diethylamino)propylthio-Sar]-3-[(γ-hydroxy)-N-MeLeu]-4-cyclosporin | 0.07 |
| [(R)-(3-(N-Morpholino)propylthio-Sar]-3-[(γ-ethoxy)methoxy-N-MeLeu]-4-cyclosporin | 0.16 |
| [(R)-2-(N,N-Diethylamino)ethylthio-Sar]-3-[(γ-methylthio)methoxy-N-MeLeu]-4-cyclosporin | 0.13 |
| [(R)-(3-(N-Morpholino)propylthio-Sar]-3-[(γ-methylthio)methoxy-N-MeLeu]-4-cyclosporin | 0.16 |
| [(R)-(3-(N-Morpholino)propylthio-Sar]-3-[(γ-benzyloxy)-N-MeLeu]-4-cyclosporin | 0.28 |
| [(R)-3-(N-Morpholino)propylthio-Sar]-3-[(γ-(4-Methoxy)-benzyloxy)-N-MeLeu]-4-cyclosporin | 0.28 |
| [(R)-3-(N-Morpholino)propylthio-Sar]-3-[(γ-allyloxy)-N-MeLeu]-4-cyclosporin | 0.15 |
| [(R)-(2-(N,N-Diethylamino)ethylthio)methyl-Sar]-3-[(γ-hydroxy)-N-MeLeu]-4-cyclosporin (Example 29, isomer B) | 0.03 |
| [(S)-(2-(N,N-Diethylamino)ethylthio)mathyl-Sar]-3-[(γ-Hydroxy)-N-MeLeu]-4-cyclosporin (Example 29, isomer A) | 2.12 |
| [(y-Methoxy)-N-MeLeu]-4-cyclosporin | 0.18 |
| [(γ-Methoxy)-N-MeLeu]-4-dihydrocyclosporin | 0.35 |
| [(γ-Methylthio)methoxy-N-MeLeu]-4-cyclospori | 0.40 |
| [γ-(2-Hydroxyethoxy)-N-MeLeu]-4-dihydrocyclosporin | 0.43 |
| [N-MeSer]-4-cyclosporin | 0.56 |

**Table 8, Testing results of certain representative compounds**

| Compound | Antiviral activity EC₅₀ (µM) |
|---|---|
| [(S)-(2-(N,N-Dimethylamino)ethylthio)methyl-Sar]-3-[γ-(Hydroxy)-N-MeLeu]-4-cyclosporin (Example 28, isomer B) | 0.05 |
| [(R)-(2-(N,N-Diethylamino)ethylthio)methyl-Sar]-3-[γ-(Hydroxy)-N-MeLeu]-4-cyclosporin□ (Example 29, isomer A) | 2.12 |
| [(S)-(2-(N,N-Diethylamino)ethylthio)methyl-Sar]-3-[(γ-hydroxy)-NMeLeu]-4-cyclosporin (Example 29, isomer B) | 0.03 |
| [(S)-(2-(N-Morpholino)ethylthio)methyl-Sar]-3-[(γ-hydroxy)-NMeLeu]-4-cyclosporin (Example 30) | 0.02 |
| [(S)-(2-(N-Piperidinyl)ethylthio)methyl-Sar]-3-[(γ-hydroxy)-NMeLeu]-4-cyclosporin (Example 31) | 0.04 |
| [(S)-(2-(4-Methyl-N-piperazinyl)ethylthio)methyl-Sar]-3-[(γ-hydroxy)-NMeLeu]-4-cyclosporin (Example 32) | 0.03 |
| [(S)-(3-(N-Morpholino)propylthio)methyl-Sar]-3-[(γ-hydroxy)-NMeLeu]-4-cyclosporin (Example 45) | 0.02 |
| [(S)-(3-(N,N-Dimethylamino)propylthio)methyl-Sar]-3-[(γ-hydroxy)-NMeLeu]-4-cyclosporin (Example 47) | 0.05 |
| [(S)-(3-(N,N-Diethylamino)propylthio)methyl-Sar]-3-[(γ-hydroxy)-NMeLeu]-4-cyclosporin (Example 48) | 0.03 |
| [(R)-(2-(N,N-Diethylamino)ethoxy)methyl-Sar]-3-[(γ-hydroxy)-NMeLeu]-4-cyclosporin (Example 50) | 0.05 |
| [(S)-(2-(N-Isopropyl-N-methylamino)ethylthio)methyl-Sar]-3-[(γ-hydroxy)-NMeLeu]-4-cyclosporin (Example 37) | 0.04 |
| [(R)-(2-(N,N-Diethylamino)ethoxy)methyl-Sar]-3-[(γ-methoxy)-NMeLeu]-4-cyclosporin (Example 77) | 0.02 |
| [(R)-(3-(N-Morpholino)propoxy)methyl-Sar]-3-[(γ-hydroxy)-NMeLeu]-4-cyclosporin (Example 57) | 0.04 |
| [(R)-(2-(N,N-Diethylamino)ethylthio)methyl-Sar]-3-[(γ-Methylthio)methoxy-NMeLeu]-4-cyclosporin (Example 84) | 0.02 |
| [(S)-(2-(N,N-Diethylamino)ethylthio)methyl-Sar]-3-[(γ-Ethoxy)methoxy-NMeLeu]-4-cyclosporin (Example 87) | 0.02 |
| [(S)-(3-(N-Pyrrolidinyl)propoxy)methyl-Sar]-3-[(γ-hydroxy)-N-MeLeu]-4-cyclosporin (Example 58) | 0.12 |

**Table 9, Testing results of certain representative compounds**

| Compound | Antiviral activity EC₅₀ (µM) |
|---|---|
| [N-Melle]-4-cyclosporin (SDZ-NIM-811) | 0.14 |
| [N-MeVal]-4-cyclosporin (SDZ 220-384) | 0.14 |
| [(R)-2-(N,N-Dimethylamino)ethylthio-Sar]-3-[(γ-hydroxy)-N-MeLeu]-4-cyclosporin (SCY-635) | 0.12 |
| [D-N-MeAla]-3-[N-EtVal]-4-cyclosporin (Debio-025) | 0.07 |
| [(S)-(2-(N,N-Diethylamino)ethylthio)methyl-Sar]-3-[(γ-hydroxy)-N-MeLeu]-4-cyclosporin (Example 29, isomer B) | 0.09 |
| (S)-2-(N,N-Dimethylamino)ethylthiomethyl-Sar]-3-[γ-(Hydroxy)-N-MeLeu]-4-cyclosporin (Example 28, isomer B) | 0.13 |
| [(S)-(2-(N-Morpholino)ethylthio)methyl-Sar]-3-[(γ-hydroxy)-N-MeLeu]-4-cyclosporin (Example 30) | 0.06 |
| [(S)-(3-(N-Piperidinyl)propylthio)methyl-Sar]-3-[(γ-hydroxy)-N-MeLeu]-4-cyclosporin □□ (Example 43) | 0.05 |
| [(S)-(2-(N-Piperidinyl)ethylthio)methyl-Sar]-3-[(γ-hydroxy)-N-MeLeu]-4-cyclosporin (Example 31) | 0.08 |
| [(S)-(2-(4-Methylpiperazino)ethylthio)methyl-Sar]-3-[(γ-hydroxy)-N-MeLeu]-4-cyclosporin (Example 32) | 0.09 |
| [(S)-(2-(N-Pyrrolidinyl)ethylthio)methyl-Sar]-3-[(γ-hydroxy)-N-MeLeu]-4-cyclosporin (Example 33) | 0.11 |
| [(S)-(3-(N-Pyrrolidinyl)propylthio)methyl-Sar]-3-[(γ-hydroxy)-N-MeLeu]-4-cyclosporin (Example 44) | 0.10 |
| [(S)-(3-(N-Morpholino)propylthio)methyl-Sar]-3-[(γ-hydroxy)-N-MeLeu]-4-cyclosporin (Example 45) | 0.05 |
| [(S)-(3-(4-Methyl-N-piperazinyl)propylthio)methyl-Sar]-3-[(γ-hydroxy)-N-MeLeu]-4-cyclosporin (Example 46) | 0.09 |
| [(S)-(3-(N,N-Dimethylamino)propylthio)methyl-Sar]-3-[(γ-hydroxy)-N-MeLeu]-4-cyclosporin (Example 47) | 0.11 |
| [(S)-(3-(N,N-Diethylamino)propylthio)methyl-Sar]-3-[(γ-hydroxy)-N-MeLeu]-4-cyclosporin (Example 48) | 0.08 |
| [(S)-(3-(N-Ethyl-N-isopropylamino)propylthio)methyl-Sar]-3-[(γ-hydroxy)-N-MeLeu]-4-cyclosporin (Example 49) | 0.06 |
| [(S)-(2-(N-Ethyl-N-isopropylamino)ethylthio)methyl-Sar]-3-[(γ-hydroxy)-N-MeLeu]-4-cyclosporin (Example 36) | 0.07 |
| [(R)-(2-(N,N-Diethylamino)ethoxy)methyl-Sar]-3-[(γ-hydroxy)-N-MeLeu]-4-cyclosporin (Example 50) | 0.12 |
| [(S)-(2-(N-Methyl-N-iso-propylamino)ethylthio)methyl-Sar]-3-[(γ-hydroxy)-N-MeLeu]-4-cyclosporin (Example 37) | 0.10 |
| [(S)-a-(2-(N-Isopropylamino)ethylthio)methyl-Sar]-3-[(γ-hydroxy)-N-MeLeu]-4-cyclosporin (Example 35) | 0.19 |
| [(S)-(2-(N,N-Diisobutylamino)ethylthio)methyl-Sar]-3-[(γ-hydroxy)-N-MeLeu]-4-cyclosporin (Example 38) | 0.12 |
| [(S)-(2-(N-Neopentylamino)ethylthio)methyl-Sar]-3-[(γ-hydroxy)-N-MeLeu]-4-cyclosporin (Example 40) | 0.06 |
| [(S)-(2-(N-Ethyl-N-neopentylamino)ethylthio)methyl-Sar]-3-[(γ-hydroxy)-N-MeLeu]-4-cyclosporin (Example 42) | 0.06 |
| [(S)-(2-(N-Methyl-N-neopentylamino)ethylthio)methyl-Sar]-3-[(γ-hydroxy)-N-MeLeu]-4-cyclosporin (Example 41) | 0.07 |
| [(R)-α-(Ethoxycarbonylmethoxy)methyl-Sar]-3-cyclosporin (Example 10) | 0.18 |
| [(R)-(3-(N, N-Diethylamino)propoxy)methyl-Sar]-3-cyclosporin (Example 20) | 0.12 |
| [(R)-(3-(N,N-Dimethylamino)propoxy)methyl-Sar]-3-cyclosporin (Example 19) | 0.13 |
| [(R)-(2-(N,N-Diethylamino)ethoxy)methyl-Sar]-3-[(γ-methoxy)-N-MeLeu]-4-cyclosporin (Example 77) | 0.06 |
| [(R)-(3-(N-Morpholino)propoxy)methyl-Sar]-3-[(γ-hydroxy)-N-MeLeu]-4-cyclosporin (Example 57) | 0.09 |
| [(R)-3-(N,N-Diethylamino)propylthio-Sar]-3-[N-MeIle]-4-cyclosporin | 0.08 |
| [(R)-(3-(N-Pyrrolidinyl)propoxy)methyl-Sar]-3-[(γ-hydroxy)-N-MeLeu]-4-cyclosporin (Example 58) | 0.22 |
| [(R)-3-(N,N-Diethylamino)propylthio-Sar]-3-[N-MeVal]-4-cyclosporin | 0.07 |
| [(R)-(3-(N-Morpholino)propylthio-Sar]-3-[N-MeVal]-4-cyclosporin | 0.11 |
| [(S)-(2-(N,N-Diethylamino)ethylthio)methyl-Sar]-3-[N-MeVal]-4-cyclosporin (Example 96) | 0.14 |
| [(S)-(2-(N,N-Diethylamino)ethylthio)methyl-Sar]-3-[N-MeIle]-4-cyclosporin (Example 92) | 0.10 |
| [(R)-(2-(N,N-Diethylamino)ethoxy)methyl-Sar]-3-[N-MeIle]-4-cyclosporin (Example 93) | 0.16 |
| [(R)-(3-(N-Piperidinyl)propoxy)methyl-Sar]-3-[(γ-hydroxy)-N-MeLeu]-4-cyclosporin (Example 59) | 0.15 |
| [(R)-(2-(N,N-Dimethylamino)ethoxy)methyl-Sar]-3-cyclosporin (Example 13) | 0.15 |
| [(S)-(2-(N,N-Dimethylamino)ethylthio)methyl-Sar]-3-cyclosporin (Example 6, isomer B) | 0.17 |
| [(S)-(2-(N,N-Diethylamino)ethylthio)methyl-Sar]-3-cyclosporin (Example 7, isomer B) | 0.16 |
| [(R)-(2-(N-Piperidinyl)ethoxy)methyl-Sar]-3-[(γ-hydroxy)-N-MeLeu]-4-cyclosporin (Example 51) | 0.14 |
| [(R)-(2-(N-Pyrrolidino)ethoxy)methyl-Sar]-3-[(γ-hydroxy)-N-MeLeu]-4-cyclosporin (Example 54) | 0.20 |
| [(S)-(2-(N,N-Dimethylamino)ethylthio)methyl-Sar]-3-[(γ-methoxy)-N-MeLeu]-4-cyclosporin (Example 68) | 0.10 |
| [(S)-(3-(N,N-Dimethylamino)propylthio)methyl-Sar]-3-[(γ-methoxy)-N-MeLeu]-4-cyclosporin (Example 72) | 0.09 |
| [(S)-(3-(N,N-Diethylamino)propylthio)methyl-Sar]-3-[(γ-methoxy)-N-MeLeu]-4-cyclosporin (Example 73) | 0.07 |
| [(S)-(2-(N,N-Diethylamino)ethylthio)methyl-Sar]-3-[(γ-methoxy)-N-MeLeu]-4-cyclosporin (Example 69) | 0.09 |
| [(S)-(2-(N-Pyrrolidinyl)ethylthio)methyl-Sar]-3-[(γ-methoxy)-N-MeLeu]-4-cyclosporin (Example 70) | 0.09 |
| [(S)-(2-(N-Morpholino)ethylthio)methyl-Sar]-3-[(γ-methoxy)-N-MeLeu]-4-cyclosporin (Example 71) | 0.11 |
| [(R)-(2-(N,N-Dimethylamino)ethoxy)methyl-Sar]-3-[(γ-methoxy)-N-MeLeu]-4-cyclosporin (Example 78) | 0.15 |
| [(S)-(3-(N-Morpholino)propylthio)methyl-Sar]-3-[(γ-methoxy)-N-MeLeu]-4-cyclosporin (Example 76) | 0.10 |
| [(R)-(3-(N,N-Diethylamino)propoxy)methyl-Sar]-3-[(γ-hydroxy)-N-MeLeu]-4-cyclosporin (Example 61) | 0.18 |
| [(S)-(3-(N-Pyrrolidinyl)propylthio)methyl-Sar]-3-[(γ-methoxy)-N-MeLeu]-4-cyclosporin (Example 75) | 0.10 |
| [(S)-[(3-(N-piperidinyl)propylthiomethyl-Sar]-3-[(γ-methoxy)-N-MeLeu]-4-cyclosporin (Example 74) | 0.06 |
| [(R)-(2-(N,N-Dimethylamino)ethoxy)methyl-Sar]-3-[(γ-hydroxy)-N-MeLeu]-4-cyclosporin (Example 53) | 0.24 |
| [(R)-(3-(N,N-Dimethylamino)propoxy)methyl-Sar]-3-[(γ-hydroxy)-N-MeLeu]-4-cyclosporin (Example 60) | 0.26 |
| [(R)-(3-(N,N-Diethylamino)propoxy)methyl-Sar]-3-[(γ-methoxy)-N-MeLeu]-4-cyclosporine (Example 81) | 0.10 |
| [(R)-(2-(N-Morpholino)ethoxy)methyl-Sar]-3-[(γ-methoxy)-N-MeLeu]-4-cyclosporin (Example 79) | 0.15 |

### Example 102

### Anti HIV Activity of Cyclosporin Derivatives

Anti-HIV activity of cyclosporine derivatives were evaluated by cytoprotection assay in an acute infection model using CEM-SS cells and either HIV-1_{IIIB} or HIV-1 RF. Antiviral activity was determined as a reduction in virus-caused cytopathic effects when compounds prevent virus replication. Cytoprotection and compound cytotoxicity were evaluated using the tetrazollium dye MTS (Promega) to calculate cell viability following virus infection after 6-day incubation (Zhou G., et al., 2011, J. Med. Chem. 27, 7220-31).

**Table 10, Testing results of certain representative compounds against HIV-1IIIB in CEM-SS cells (MTS Endpoint)**

| Compound | Antiviral activity EC₅₀ (nM) |
|---|---|
| AZT | 9.0 |
| [(S)-(2-(N,N-Dimethylamino)ethylthiomethyl-Sar]-3-[(γ-Hydroxy)-NMeLeu]-4-cyclosporin (Example 28, isomer B) | 118.0 |
| [(S)-(2-(N-Morpholino)ethylthio)methyl-Sar]-3-[(γ-hydroxy)-NMeLeu]-4-cyclosporin (Example 30) | 94.9 |

### Examples 103-705

### Antiviral Cyclosporin Derivatives

The following compounds can be prepared according to a method analogous to those described herein.

**Table 11**

| | | | |
|---|---|---|---|
| 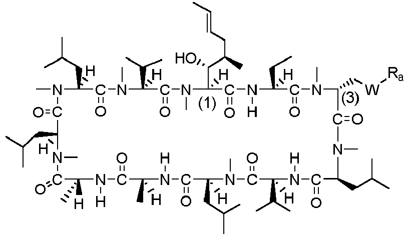 | | | |

| Ex. No. | W | Rₐ | Name |
|---|---|---|---|
| 103 | S | 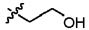 | [(S)-(2-Hydroxyethylthio)methyl-Sar]-3-cyclosporin |
| 104 | S | 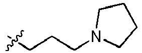 | [(S)-(2-(N-Ethyl-N-methylamino)ethylthio)methyl-Sar]-3-cyclosporin |
| 105 | S | 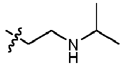 | [(S)-(2-(N-Isopropylamino)ethylthio)methyl-Sar]-3-cyclosporin |
| 106 | S | 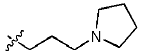 | [(S)-(2-(N-Isopropyl-N-methylamino)ethylthio)methyl-Sar]-3-cyclosporin |
| 107 | S | 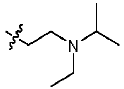 | [(S)-(2-(N-Ethyl-N-isopropylamino)ethylthio)methyl-Sar]-3-cyclosporin |
| 108 | S | 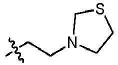 | [(S)-(2-(N-Thiazolidinyl)ethylthio)methyl-Sar]-3-cyclosporin |
| 109 | S | 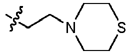 | [(S)-(2-(N-Thiomorpholino)ethylthio)methyl-Sar]-3-cyclosporin |
| 110 | S | 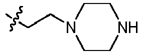 | [(S)-(2-(N-Piperazinyl)ethylthio)methyl-Sar]-3-cyclosporin |
| 111 | S | 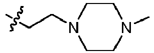 | [(S)-(2-(4-Methyl-N-piperazinyl)ethylthio)methyl-Sar]-3-cyclosporin |
| 112 | S | 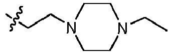 | [(S)-(2-(4-Ethyl-N-piperazinyl)ethylthio)methyl-Sar]-3-cyclo sporin |
| 113 | S | 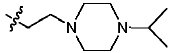 | [(S)-(2-(4-Isopropyl-N-piperazinyl)ethylthio)methyl-Sar]-3-cyclo sporin |
| 114 | S | 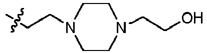 | [(S)-(2-(4-(2-Hydroxyethyl)-N-piperazinyl)ethylthio)methyl-Sar]-3-cyclosporin |
| 115 | S | 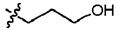 | [(S)-(3-Hydroxypropylthio)methyl-Sar]-3-cyclosporin |
| 116 | S | 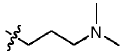 | [(S)-(3-(N,N-Dimethylamino)propylthio)methyl-Sar]-3-cyclo sporin |
| 117 | S | 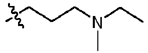 | [(S)-(3-(N-Ethyl-N-methylamino)propylthio)methyl-Sar]-3-cyclo sporin |
| 118 | S | 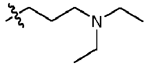 | [(S)-(3-(N,N-Diethylamino)propylthio)methyl-Sar]-3-cyclo sporin |
| 119 | S | 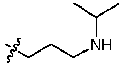 | [(S)-(3-(N-Isopropylamino)propylthio)methyl-Sar]-3-cyclo sporin |
| 120 | S | 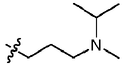 | [(S)-(3-(N-Isopropyl-N-methylamino)propylthio)methyl-Sar]-3-cyclosporin |
| 121 | S | 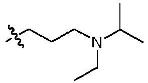 | [(S)-(3-(N-Ethyl-N-isopropylamino)propylthio)methyl-Sar]-3-cyclo sporin |
| 122 | S | 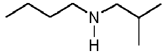 | [(S)-(3-(N-Isobutylamino)propylthio)methyl-Sar]-3-cyclosporin |
| 123 | S | 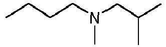 | [(S)-(3-(N-Isobutyl-N-methylamino)propylthio)methyl-Sar]-3-cyclo sporin |
| 124 | S | 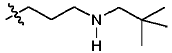 | [(S)-(3-(N-Neopentylamino)propylthio)methyl-Sar]-3-cyclo sporin |
| 125 | S | 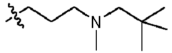 | [(S)-(3-(N-Methyl-N-neopentylamino)propylthio)methyl-Sar]-3-cyclosporin |
| 126 | S | 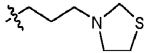 | [(S)-(3-(N-Thiazolidinyl)propylthio)methyl-Sar]-3-cyclosporin |
| 127 | S | 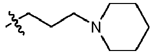 | [(S)-(3-(N-Piperidinyl)propylthio)methyl-Sar]-3-eyelosporin |
| 128 | S | 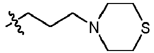 | [(S)-(3-(N-Thiomorpholino)propylthio)methyl-Sar]-3-cyclo sporin |
| 129 | S | 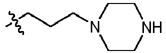 | [(S)-(3-(N-Piperazinyl)propylthio)methyl-Sar]-3-cyclosporin |
| 130 | S | 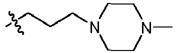 | [(S)-(3-(4-Methyl-N-piperazinyl)propylthio)methyl-Sar]-3-cyclo sporin |
| 131 | S | 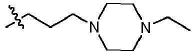 | [(S)-(3-(4-Ethyl-N-piperazinyl)propylthio)methyl-Sar]-3-cyclosporin |
| 132 | S | 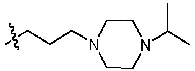 | [(S)-(3-(4-Isopropyl-N-piperazinyl)propylthio)methyl-Sar]-3-cyclo sporin |
| 133 | S | 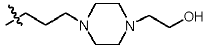 | [(S)-(3-(4-(2-Hydroxyethyl)-N-piperazinyl)propylthio)methyl-Sar]-3-cyclosporin |
| 134 | S | 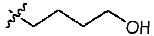 | [(S)-(4-Hydroxybutylthio)methyl-Sar]-3-cyclosporin |
| 135 | S | 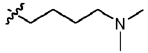 | [(S)-(4-(N,N-Dimethylamino)butylthio)methyl-Sar]-3-cyclo sporin |
| 136 | S | 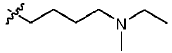 | [(S)-(4-(N-Ethyl-N-methylamino)butylthio)methyl-Sar]-3-cyclo sporin |
| 137 | S | 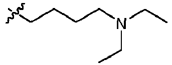 | [(S)-(4-(N,N-Diethylamino)butylthio)methyl-Sar]-3-cyclosporin |
| 138 | S | 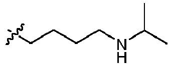 | [(S)-(4-(N-Isopropylamino)butylthio)methyl-Sar]-3-cyclosporin |
| 139 | S | 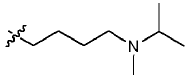 | [(S)-(4-(N-Isopropyl-N-methylamino)butylthio)methyl-Sar]-3-cyclosporin |
| 140 | S | 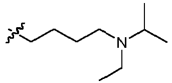 | [(S)-(4-(N-Ethyl-N-isopropylamino)butylthio)methyl-Sar]-3-cyclosporin |
| 141 | S | 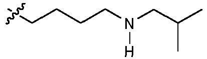 | [(S)-(4-(N-Isobutylamino)butylthio)methyl-Sar]-3-cyclosporin |
| 142 | S | 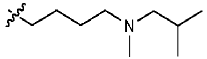 | [(S)-(4-(N-isopropyl-N-methylamino)butylthio)methyl-Sar]-3-cyclosporin |
| 143 | S | 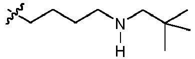 | [(S)-(4-(N-Neopentylamino)butylthio)methyl-Sar]-3-cyclosporin |
| 144 | S | 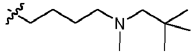 | [(S)-(4-(N-Methyl-N-Neopentylamino)butylthio)methyl-Sar]-3-cyclosporin |
| 145 | S | 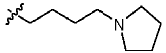 | [(S)-(4-(N-Pyrrolidinyl)butylthio)methyl-Sar]-3-cyclosporin |
| 146 | S | 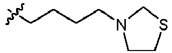 | [(S)-(4-(N-Thiazolidinyl)butylthio)methyl-Sar]-3-cyclosporin |
| 147 | S | 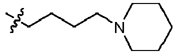 | [(S)-(4-(N-Piperidinyl)butylthio)methyl-Sar]-3-[(γ-hydroxy)-NMeLeu]-4-cyclosporin |
| 148 | S | 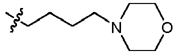 | [(S)-(4-(N-Morpholino)butylthio)methyl-Sar]-3-cyclosporin |
| 149 | S | 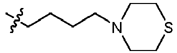 | [(S)-(4-(N-Thiomorpholino)butylthio)methyl-Sar]-3-cyclosporin |
| 150 | S | 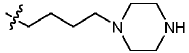 | [(S)-(4-(N-Piperazinyl)butylthio)methyl-Sar]-3-cyclosporin |
| 151 | S | 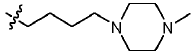 | [(S)-(4-(4-Methyl-N-piperazinyl)butylthio)methyl-Sar]-3-cyclosporin |
| 152 | S | 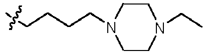 | [(S)-(4-(4-Ethyl-N-piperazinyl)butylthio)methyl-Sar]-3-cyclosporin |
| 153 | S | 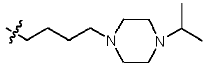 | [(S)-(4-(4-Isopropyl-N-piperazinyl)butylthio)methyl-Sar]-3-cyclosporin |
| 154 | S | 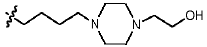 | [(S)-(4-(4-(2-Hydroxyethyl)-N-piperazinyl)butylthio)methyl-Sar]-3-cyclosporin |
| 155 | O | 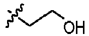 | [(R)-(2-Hydroxyethoxy)methyl-Sar]-3-cyclosporin |
| 156 | O | 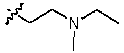 | [(R)-(2-(N-Ethyl-N-methylamino)ethoxy)methyl-Sar]-3-cyclosporin |
| 157 | O | 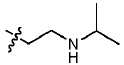 | [(R)-(2-(N-Isopropylamino)ethoxy)methyl-Sar]-3-cyclosporin |
| 158 | O | 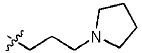 | [(R)-(2-(N-Isopropyl-N-methylamino)ethoxy)methyl-Sar]-3-cyclosporin |
| 159 | O | 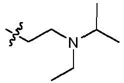 | [(R)-(2-(N-Ethyl-N-isopropylamino)ethoxy)methyl-Sar]-3-cyclosporin |
| 160 | O | 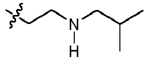 | [(R)-(2-(N-Isobutylamino)ethoxy)methyl-Sar]-3-cyclosporin |
| 161 | O | 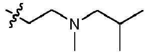 | [(R)-(2-(N-Isobutyl-N-methylamino)ethoxy)methyl-Sar]-3-cyclosporin |
| 162 | O | 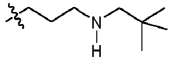 | [(R)-(2-(N-Isopentylamino)ethoxy)methyl-Sar]-3-cyclosporin |
| 163 | O | 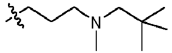 | [(R)-(2-(N-Isopentyl-N-methylamino)ethoxy)methyl-Sar]-3-cyclosporin |
| 164 | O | 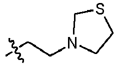 | [(R)-(2-(N-Thiazolidinyl)ethoxy)methyl-Sar]-3-cyclosporin |
| 165 | O | 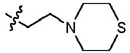 | [(R)-(2-(N-Thiomorpholino)ethoxy)methyl-Sar]-3-cyclosporin |
| 166 | O | 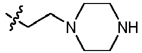 | [(R)-(2-(N-Piperazinyl)ethoxy)methyl-Sar]-3-cyclosporin |
| 167 | O | 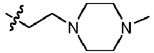 | [(R)-(2-(4-Methyl-N-piperazinyl)ethoxy)methyl-Sar]-3-cyclosporin |
| 168 | O | 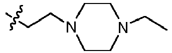 | [(R)-(2-(4-Ethyl-N-piperazinyl)ethoxy)methyl-Sar]-3-cyclosporin |
| 169 | O | 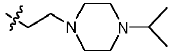 | [(R)-(2-(4-Isopropyl-N-piperazinyl)ethoxy)methyl-Sar]-3-cyclosporin |
| 170 | O | 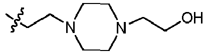 | [(R)-(2-(4-(2-Hydroxyethyl)-N-piperazinyl)ethoxy)methyl-Sar]-3-cyclosporin |
| 171 | O | 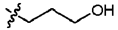 | [(R)-(3-Hydroxypropoxy)methyl-Sar]-3-cyclosporin |
| 172 | O | 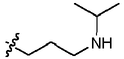 | [(R)-(3-(N-Isopropylamino)propoxy)methyl-Sar]-3-cyclosporin |
| 173 | O | 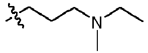 | [(R)-(3-(N-Ethyl-N-methylamino)propoxy)methyl-Sar]-3-cyclosporin |
| 174 | O | 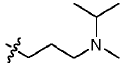 | [(R)-(3-(N-Isopropyl-N-methylamino)propoxy)methyl-Sar]-3-cyclosporin |
| 175 | O | 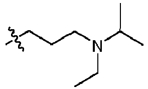 | [(R)-(3-(N-Ethyl-N-isopropylamino)propoxy)methyl-Sar]-3-cyclosporin |
| 176 | O | 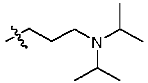 | [(R)-(3-(N,N-Diisopropylamino)propoxy)methyl-Sar]-3-cyclosporin |
| 177 | O | 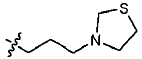 | [(R)-(3-(N-Thiazolidinyl)propoxymethyl-Sar]-3-cyclosporin |
| 178 | O | 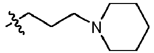 | [(R)-(3-(N-Piperidinyl)propoxy)methyl-Sar]-3-cyclosporin |
| 179 | O | 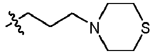 | [(R)-(3-(N-Thiomorpholino)propoxy)methyl-Sar]-3-cyclosporin |
| 180 | O | 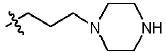 | [(R)-(3-(N-Piperazinyl)propoxy)methyl-Sar]-3-cyclosporin |
| 181 | O | 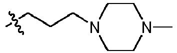 | [(R)-(3-(4-Methyl-N-piperazinyl)propoxy)methyl-Sar]-3-cyclosporin |
| 182 | O | 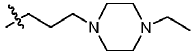 | [(R)-(3-(4-Ethyl-N-piperazinyl)propoxy)methyl-Sar]-3-cyclosporin |
| 183 | O | 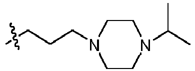 | [(R)-(3-(4-Isopropyl-N-piperazinyl)propoxy)methyl-Sar]-3-cyclosporin |
| 184 | O | 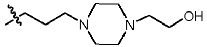 | [(R)-(3-(4-(2-Hydroxyethyl)-N-piperazinyl)propoxy)methyl-Sar]-3-cyclosporin |
| 185 | O | 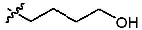 | [(R)-(4-Hydroxybutoxy)methyl-Sar]-3-cyclosporin |
| 186 | O | 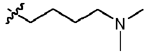 | [(R)-(4-(N, N-Dimethylamino)butoxy)methyl-Sar]-3-cyclosporin |
| 187 | O | 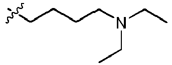 | [(R)-(4-(N-Ethyl-N-methylamino)butoxy)methyl-Sar]-3-cyclosporin |
| 188 | O | 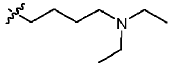 | [(R)-(4-(N,N-Diethylamino)butoxy)methyl-Sar]-3-cyclosporin |
| 189 | O | 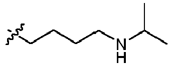 | [(R)-(4-(N-Isopropylamino)butoxy)methyl-Sar]-3-cyclosporin |
| 190 | O | 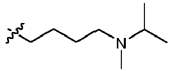 | [(R)-(4-(N-Isopropyl-N-methylamino)butoxy)methyl-Sar]-3-cyclosporin |
| 191 | O | 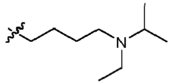 | [(R)-(4-(N-Ethyl-N-isopropylamino)butoxy)methyl-Sar]-3-cyclosporin |
| 192 | O | 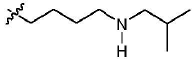 | [(R)-(4-(N-Isobutylamino)butoxy)methyl-Sar]-3-cyclosporin |
| 193 | O | 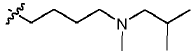 | [(R)-(4-(N-Isobutyl-N-methylamino)butoxy)methyl-Sar]-3-cyclosporin |
| 194 | O | 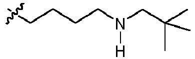 | [(R)-(4-(N-Isopentylamino)butoxy)methyl-Sar]-3-cyclosporin |
| 195 | O | 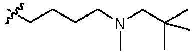 | [(R)-(4-(N-Isopentyl-N-methylamino)butoxy)methyl-Sar]-3-cyclosporin |
| 196 | O | 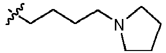 | [(R)-(4-(N-Pyrrolidinyl)butoxy)methyl-Sar]-3-cyclosporin |
| 197 | O | 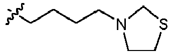 | [(R)-(4-(N-Thiazolidinyl)butoxy)methyl-Sar]-3-cyclosporin |
| 198 | O | 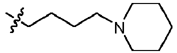 | [(R)-(4-(N-Piperidinyl)butoxy)methyl-Sar]-3-cyclosporin |
| 199 | O | 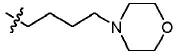 | [(R)-(4-(N-Morpholino)butoxy)methyl-Sar]-3-cyclosporin |
| 200 | O | 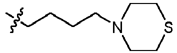 | [(R)-(4-(N-Thiomorpholino)butoxy)methyl-Sar]-3-cyclosporin |
| 201 | O | 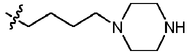 | [(R)-(4-(N-Piperazinyl)butoxy)methyl-Sar]-3-cyclosporin |
| 202 | O | 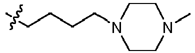 | [(R)-(4-(4-Methyl-N-piperazinyl)butoxy)methyl-Sar]-3-cyclosporin |
| 203 | O | 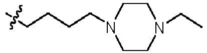 | [(R)-(4-(4-Ethyl-N-piperazinyl)butoxy)methyl-Sar]-3-cyclosporin |
| 204 | O | 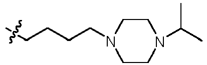 | [(R)-(4-(4-Isopropyl-N-piperazinyl)butoxy)methyl-Sar]-3-cyclosporin |
| 205 | O | 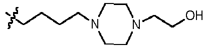 | [(R)-(4-(4-(2-Hydroxyethyl)-N-piperazinyl)butoxy)methyl-Sar]-3-cyclosporin |

**Table 12**

| | | | |
|---|---|---|---|
| 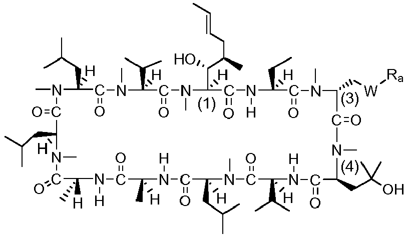 | | | |

| Ex. No. | W | Rₐ | Name |
|---|---|---|---|
| 206 | S | 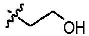 | [(S)-(2-Hydroxyethylthio)methyl-Sar]-3-[(γ-hydroxy)-N-MeLeu]-4-cyclosporin |
| 207 | S | 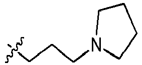 | [(S)-(2-(N-Ethyl-N-methylamino)ethylthio)methyl-Sar]-3-[(γ-hydroxy)-N-MeLeu]-4-cyclosporin |
| 208 | S | 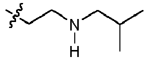 | [(S)-(2-(N-Isobutylamino)ethylthio)methyl-Sar]-3-[(γ-hydroxy)-N-MeLeu]-4-cyclosporin |
| 209 | S | 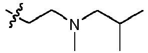 | [(S)-(2-(N-Isobutyl-N-methylamino)ethylthio)methyl-Sar]-3-[(γ-hydroxy)-N-MeLeu]-4-cyclosporin |
| 210 | S | 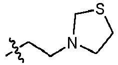 | [(S)-(2-(N-Thiazolidinyl)ethylthio)methyl-Sar]-3-[(γ-hydroxy)-N-MeLeu]-4-cyclosporin |
| 211 | S | 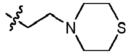 | [(S)-(2-(N-Thiomorpholino)ethylthio)methyl-Sar]-3-[(γ-hydroxy)-N-MeLeu]-4-cyclosporin |
| 212 | S | 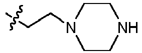 | [(S)-(2-(N-Piperazinyl)ethylthio)methyl-Sar]-3-[(γ-hydroxy)-N-MeLeu]-4-cyclosporin |
| 213 | S | 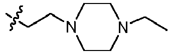 | [(S)-(2-(4-Ethyl-N-piperazinyl)ethylthio)methyl-Sar]-3-[(γ-hydroxy)-N-MeLeu]-4-cyclosporin |
| 214 | S | 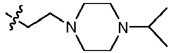 | [(S)-(2-(4-Isopropyl-N-piperazinyl)ethylthio)methyl-Sar-3-[(γ-hydroxy)-N-MeLeu]-4-cyclosporin |
| 215 | S | 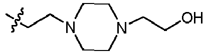 | [(S)-(2-(4-(2-Hydroxyethyl)-N-piperazinyl)ethylthio)methyl-Sar]-3-[(γ-hydroxy)-N-MeLeu]-4-cyclosporin |
| 216 | S | 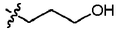 | [(S)-(3-Hydroxypropylthio)methyl-Sar]-3-[(γ-hydroxy)-N-MeLeu]-4-cyclosporin |
| 217 | S | 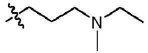 | [(S)-(3-(N-Ethyl-N-methylamino)propylthio)methyl-Sar]-3-[(γ-hydroxy)-N-MeLeu]-4-cyclosporin |
| 218 | S | 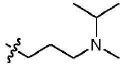 | [(S)-(3-(N-Isopropyl-N-methylamino)propylthio)methyl-Sar]-3-[(γ-hydroxy)-N-MeLeu]-4-cyclosporin |
| 219 | S | 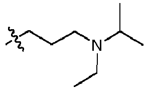 | [(S)-(3-(N-Ethyl-N-isopropylamino)propylthio)methyl-Sar]-3-[(γ-hydroxy)-N-MeLeu]-4-cyclosporin |
| 220 | S | 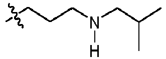 | [(S)-(3-(N-Isobutylamino)propylthio)methyl-Sar]-3-[(γ-hydroxy)-N-MeLeu]-4-cyclosporin |
| 221 | S | 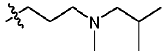 | [(S)-(3-(N-Isobutyl-N-methylamino)propylthio)methyl-Sar]-3-[(γ-hydroxy)-N-MeLeu]-4-cyclosporin |
| 222 | S | 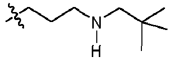 | [(S)-(3-(N-Neopentylamino)propylthio)methyl-Sar]-3-[(y-hydroxy)-N-MeLeu]-4-cyclosporin |
| 223 | S | 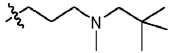 | [(S)-(3-(N-Methyl-N-Neopentyl)propylthio)methyl-Sar]-3-[(γ-hydroxy)-N-MeLeu]-4-cyclosporin |
| 224 | S | 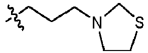 | [(S)-(3-(N-Thiazolidinyl)propylthio)methyl-Sar]-3-[(γ-hydroxy)-N-MeLeu]-4-cyclosporin |
| 225 | S | 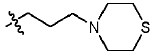 | [(S)-(3-(N-Thiomorpholino)propylthio)methyl-Sar]-3-[(γ-hydroxy)-N-MeLeu]-4-cyclosporin |
| 226 | S | 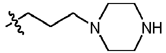 | [(S)-(3-(N-Piperazinyl)propylthio)methyl-Sar]-3-[(γ-hydroxy)-N-MeLeu]-4-cyclosporin |
| 227 | S | 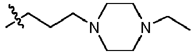 | [(S)-(3-(4-Ethyl-N-piperazinyl)propylthio)methyl-Sar]-3-[(γ-hydroxy)-N-MeLeu]-4-cyclosporin |
| 228 | S | 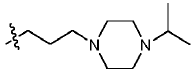 | [(S)-(3-(4-Isopropyl-N-piperazinyl)propylthio)methyl-Sar]-3-[(γ-hydroxy)-N-MeLeu]-4-cyclosporin |
| 229 | S | 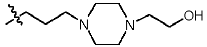 | [(S)-(3-(4-(2-Hydroxyethyl)-N-piperazinyl)propylthio)methyl-Sar]-3-[(γ-hydroxy)-N-MeLeu]-4-cyclosporin |
| 230 | S | 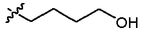 | [(S)-(4-Hydroxybutylthio)methyl-Sar]-3-[(γ-hydroxy)-N-MeLeu]-4-cyclosporin |
| 231 | S | 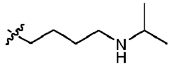 | [(S)-(4-(N-Isopropylamino)butylthio)methyl-Sar]-3-[(γ-hydroxy)-N-MeLeu]-4-cyclosporin |
| 232 | S | 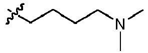 | [(S)-(4-(N,N-Dimethylamino)butylthio)methyl-Sar]-3-[(γ-hydroxy)-N-MeLeu]-4-cyclosporin |
| 233 | S | 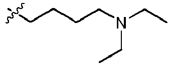 | [(S)-(4-(N-Ethyl-N-methylamino)butylthio)methyl-Sar]-3-[(γ-hydroxy)-N-MeLeu]-4-cyclosporin |
| 234 | S | 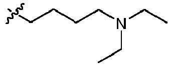 | [(S)-(4-(N,N-Diethylamino)butylthio)methyl-Sar]-3-[(γ-hydroxy)-N-MeLeu]-4-cyclosporin |
| 235 | S | 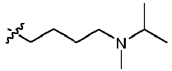 | [(S)-(4-(N-Isopropyl-N-methylamino)butylthio)methyl-Sar]-3-[(γ-hydroxy)-N-MeLeu]-4-cyclosporin |
| 236 | S | 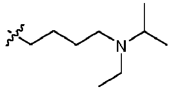 | [(S)-(4-(N-Ethyl-N-isopropylamino)butylthio)methyl-Sar]-3-[(γ-hydroxy)-N-MeLeu]-4-cyclosporin |
| 237 | S | 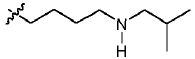 | [(S)-(4-(N-Isobutylamino)butylthio)methyl-Sar]-3-[(γ-hydroxy)-N-MeLeu]-4-cyclosporin |
| 238 | S | 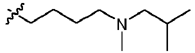 | [(S)-(4-(N-Isobutyl-N-methylamino)butylthio)methyl-Sar]-3-[(γ-hydroxy)-N-MeLeu]-4-cyclosporin |
| 239 | S | 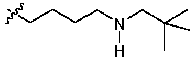 | [(S)-(4-(N-Neopentylamino)butylthio)methyl-Sar]-3-[(γ-hydroxy)-N-MeLeu]-4-cyclosporin |
| 240 | S | 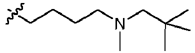 | [(S)-(4-(N-Methyl-N-Neopentylamino)butylthio)methyl-Sar]-3-[(γ-hydroxy)-N-MeLeu]-4-cyclosporin |
| 241 | S | 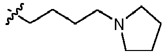 | [(S)-(4-(N-Pyrrolidinyl)butylthio)methyl-Sar]-3-[(γ-hydroxy)-N-MeLeu]-4-cyclosporin |
| 242 | S | 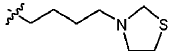 | [(S)-(4-(N-Thiazolidinyl)butylthio)methyl-Sar]-3-[(γ-hydroxy)-N-MeLeu]-4-cyclosporin |
| 243 | S | 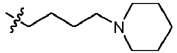 | [(S)-(4-(N-Piperidinyl)butylthio)methyl-Sar]-3-[(γ-hydroxy)-N-MeLeu]-4-cyclosporin |
| 244 | S | 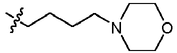 | [(S)-(4-(N-Morpholino)butylthio)methyl-Sar]-3-[(γ-hydroxy)-N-MeLeu]-4-cyclosporin |
| 245 | S | 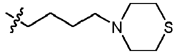 | [(S)-(4-(N-Thiomorpholino)butylthio)methyl-Sar]-3-[(γ-hydroxy)-N-MeLeu]-4-cyclosporin |
| 246 | S | 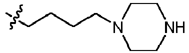 | [(S)-(4-(N-Piperazinyl)butylthio)methyl-Sar]-3-[(γ-hydroxy)-N-MeLeu]-4-cyclosporin |
| 247 | S | 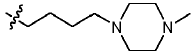 | [(S)-(4-(4-Methyl-N-piperazinyl)butylthio)methyl-Sar]-3-[(γ-hydroxy)-N-MeLeu]-4-cyclosporin |
| 248 | S | 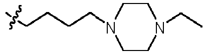 | [(S)-(4-(4-Ethyl-N-piperazinyl)butylthio)methyl-Sar]-3-[(γ-hydroxy)-N-MeLeu]-4-cyclosporin |
| 249 | S | 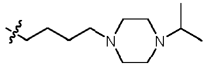 | [(S)-(4-(4-Isopropyl-N-piperazinyl)butylthio)methyl-Sar]-3-[(y-hydroxy)-N-MeLeu]-4-cyclosporin |
| 250 | S | 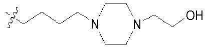 | [(S)-(4-(4-(2-Hydroxyethyl)-N-piperazinyl)butylthio)methyl-Sar]-3-[(γ-hydroxy)-N-MeLeu]-4-cyclosporin |
| 251 | O | 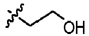 | [(R)-(2-Hydroxyethoxy)methyl-Sar]-3-[(γ-hydroxy)-N-MeLeu]-4-cyclosporin |
| 252 | O | 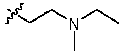 | [(R)-(2-(N-Ethyl-N-methylamino)ethoxy)methyl-Sar]-3-[(γ-hydroxy)-N-MeLeu]-4-cyclosporin |
| 253 | O | 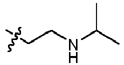 | [(R)-(2-(N-Isopropylamino)ethoxy)methyl-Sar]-3-[(γ-hydroxy)-N-MeLeu]-4-cyclosporin |
| 254 | O | 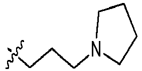 | [(R)-(2-(N-Isopropyl-N-methylamino)ethoxy)methyl-Sar]-3-[(γ-hydroxy)-N-MeLeu]-4-cyclosporin |
| 255 | O | 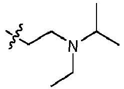 | [(R)-(2-(N-Ethyl-N-isopropylamino)ethoxy)methyl-Sar]-3-[(γ-hydroxy)-N-MeLeu]-4-cyclosporin |
| 256 | O | 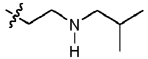 | [(R)-(2-(N-Isobutylamino)ethoxy)methyl-Sar]-3-[(γ-hydroxy)-N-MeLeu]-4-cyclosporin |
| 257 | O | 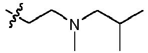 | [(R)-(2-(N-Isobutyl-N-methylamino)ethoxy)methyl-Sar]-3-[(γ-hydroxy)-N-MeLeu]-4-cyclosporin |
| 258 | O | 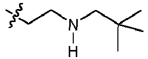 | [(R)-(2-(N-Neopentylamino)ethoxy)methyl-Sar]-3-[(γ-hydroxy)-N-MeLeu]-4-cyclosporin |
| 259 | O | 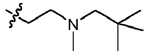 | [(R)-(2-(N-Methyl-N-neopentylamino)ethoxy)methyl-Sar]-3-[(γ-hydroxy)-N-MeLeu]-4-cyclosporin |
| 260 | O | 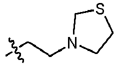 | [(R)-(2-(N-Thiazolidinyl)ethoxy)methyl-Sar]-3-[(γ-hydroxy)-N-MeLeu]-4-cyclosporin |
| 261 | O | 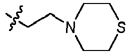 | [(R)-(2-(N-Thiomorpholino)ethoxy)methyl-Sar]-3-[(γ-hydroxy)-N-MeLeu]-4-cyclosporin |
| 262 | O | 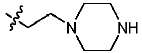 | [(R)-(2-(N-Piperazinyl)ethoxy)methyl-Sar]-3-[(γ-hydroxy)-N-MeLeu]-4-cyclosporin |
| 263 | O | 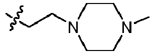 | [(R)-(2-(4-Methyl-N-piperazinyl)ethoxy)methyl-Sar]-3-[(γ-hydroxy)-N-MeLeu]-4-cyclosporin |
| 264 | O | 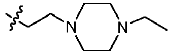 | [(R)-(2-(4-Ethyl-N-piperazinyl)ethoxy)methyl-Sar]-3-[(γ-hydroxy)-N-MeLeu]-4-cyclosporin |
| 265 | O | 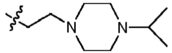 | [(R)-(2-(4-Isopropyl-N-piperazinyl)ethoxy)methyl-Sar]-3-[(γ-hydroxy)-N-MeLeu]-4-cyclosporin |
| 266 | O | 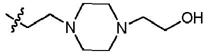 | [(R)-(2-(4-(2-Hydroxyethyl)-N-piperazinyl)ethoxy)methyl-Sar]-3-[(γ-hydroxy)-N-MeLeu]-4-cyclosporin |
| 267 | O | 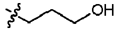 | [(R)-(3-Hydroxypropoxy)methyl-Sar]-3-[(γ-hydroxy)-N-MeLeu]-4-cyclosporin |
| 268 | O | 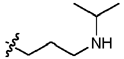 | [(R)-(3-(N-Isopropylamino)propoxylmethyl-Sar]-3-[(γ-hydroxy)-N-MeLeu]-4-cyclosporin |
| 269 | O | 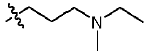 | [(R)-(3-(N-Ethyl-N-methylamino)propoxy)methyl-Sar]-3-[(γ-hydroxy)-N-MeLeu]-4-cyclosporin |
| 270 | O | 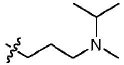 | [(R)-(3-(N-Isopropyl-N-methylamino)propoxy)methyl-Sar]-3-[(γ-hydroxy)-N-MeLeu]-4-cyclosporin |
| 271 | O | 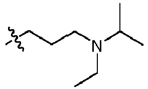 | [(R)-(3-(N-Ethyl-N-isopropylamino)propoxy)methyl-Sar]-3-[(γ-hydroxy)-N-MeLeu]-4-cyclosporin |
| 272 | O | 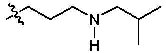 | [(R)-(3-(N-Isobutylamino)propoxy)methyl-Sar]-3-[(γ-hydroxy)-N-MeLeu]-4-cyclosporin |
| 273 | O | 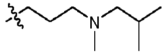 | [(R)-(3-(N-Isobutyl-N-methylamino)propoxy)methyl-Sar]-3-[(γ-hydroxy)-N-MeLeu]-4-cyclosporin |
| 274 | O | 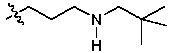 | [(R)-(3-(N-Neopentylamino)propoxy)methyl-Sar]-3-[(γ-hydroxy)-N-MeLeu]-4-cyclosporin |
| 275 | O | 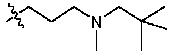 | [(R)-(3-(N-Methyl-N-Neopentylamino)propoxy)methyl-Sar]-3-[(γ-hydroxy)-N-MeLeu]-4-cyclosporin |
| 276 | O | 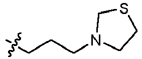 | [(R)-(3-(N-Thiazolidinyl)propoxymethyl-Sar]-3-[(γ-hydroxy)-N-MeLeu]-4-cyclosporin |
| 277 | O | 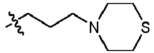 | [(R)-(3-(N-Thiomorpholino)propoxy)methyl-Sar]-3-[(γ-hydroxy)-N-MeLeu]-4-cyclosporin |
| 278 | O | 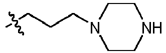 | [(R)-(3-(N-Piperazinyl)propoxy)methyl-Sar]-3-[(y-hydroxy)-N-MeLeu]-4-cyclosporin |
| 279 | O | 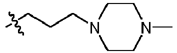 | [(R)-(3-(4-Methyl-N-piperazinyl)propoxy)methyl-Sar]-3-[(γ-hydroxy)-N-MeLeu]-4-cyclosporin |
| 280 | O | 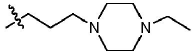 | [(R)-(3-(4-Ethyl-N-piperazinyl)propoxy)methyl-Sar]-3-[(γ-hydroxy)-N-MeLeu]-4-cyclosporin |
| 281 | O | 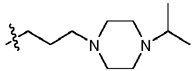 | [(R)-(3-(4-Isopropyl-N-piperazinyl)propoxy)methyl-Sar]-3-[(γ-hydroxy)-N-MeLeu]-4-cyclosporin |
| 282 | O | 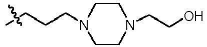 | [(R)-(3-(4-(2-Hydroxyethyl)-N-piperazinyl)propoxy)methyl-Sar]-3-[(γ-hydroxy)-N-MeLeu]-4-cyclosporin |
| 283 | O | 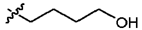 | [(R)-(4-Hydroxybutoxy)methyl-Sar]-3-[(γ-hydroxy)-N-MeLeu]-4-cyclosporin |
| 284 | O | 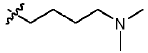 | [(R)-(4-(N,N-Dimethylamino)butoxy)methyl-Sar]-3-[(γ-hydroxy)-N-MeLeu]-4-cyclosporin |
| 285 | O | 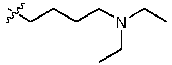 | [(R)-(4-(N-Ethyl-N-methylamino)butoxy)methyl-Sar]-3-[(γ-hydroxy)-N-MeLeu]-4-cyclo sporin |
| 286 | O | 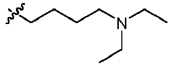 | [(R)-(4-(N,N-Diethylamino)butoxy)methyl-Sar]-3-[(γ-hydroxy)-N-MeLeu]-4-cyclosporin |
| 287 | O | 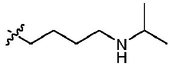 | [(R)-(4-(N-Isopropylamino)butoxy)methyl-Sar]-3-[(γ-hydroxy)-N-MeLeu]-4-cyclo sporin |
| 288 | O | 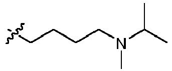 | [(R)-(4-(N-Isopropyl-N-methylamino)butoxy)methyl-Sar]-3-[(γ-hydroxy)-N-MeLeu]-4-cyclosporin |
| 289 | O | 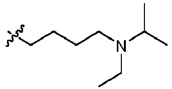 | [(R)-(4-(N-Ethyl-N-isopropylamino)butoxy)methyl-Sar]-3-[(γ-hydroxy)-N-MeLeu]-4-cyclosporin |
| 290 | O | 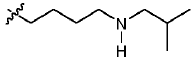 | [(R)-(4-(N-Isobutylamino)butoxy)methyl-Sar]-3-[(γ-hydroxy)-N-MeLeu]-4-cyclosporin |
| 291 | O | 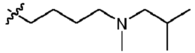 | [(R)-(4-(N-Isobutyl-N-methylamino)butoxy)methyl-Sar]-3-[(γ-hydroxy)-N-MeLeu]-4-cyclosporin |
| 292 | O | 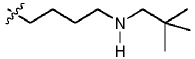 | [(R)-(4-(N-Methyl-N-neopentylamino)butoxy)methyl-Sar]-3-[(γ-hydroxy)-N-MeLeu]-4-cyclosporin |
| 293 | O | 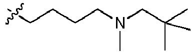 | [(R)-(4-(N-Neopentylamino)butoxy)methyl-Sar]-3-[(γ-hydroxy)-N-MeLeu]-4-cyclosporin |
| 294 | O | 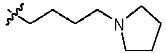 | [(R)-(4-(N-Pyrrolidinyl)butoxy)methyl-Sar]-3-[(γ-hydroxy)-N-MeLeu]-4-cyclosporin |
| 295 | O | 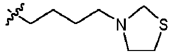 | [(R)-(4-(N-Thiazolidinyl)butoxy)methyl-Sar]-3-[(γ-hydroxy)-N-MeLeu]-4-cyclosporin |
| 296 | O | 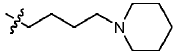 | [(R)-(4-(N-Piperidinyl)butoxy)methyl-Sar]-3-[(γ-hydroxy)-N-MeLeu]-4-cyclosporin |
| 297 | O | 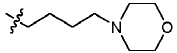 | [(R)-(4-(N-Morpholino)butoxy)methyl-Sar]-3-[(γ-hydroxy)-N-MeLeu]-4-cyclosporin |
| 298 | O | 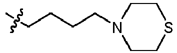 | [(R)-(4-(N-Thiomorpholino)butoxy)methyl-Sar]-3-[(γ-hydroxy)-N-MeLeu]-4-cyclosporin |
| 299 | O | 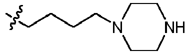 | [(R)-(4-(N-Piperazinyl)butoxy)methyl-Sar]-3-[(γ-hydroxy)-N-MeLeu]-4-cyclosporin |
| 300 | O | 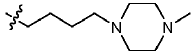 | [(R)-(4-(4-Methyl-N-piperazinyl)butoxy)methyl-Sar]-3-[(γ-hydroxy)-N-MeLeu]-4-cyclosporin |
| 301 | O | 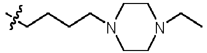 | [(R)-(4-(4-Ethyl-N-piperazinyl)butoxy)methyl-Sar]-3-[(γ-hydroxy)-N-MeLeu]-4-cyclosporin |
| 302 | O | 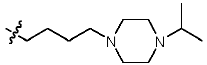 | [(R)-(4-(4-Isopropyl-N-piperazinyl)butoxy)methyl-Sar]-3-[(γ-hydroxy)-N-MeLeu]-4-cyclosporin |
| 303 | O | 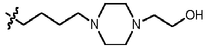 | [(R)-(4-(4-(2-Hydroxyethyl)-N-piperazinyl)butoxy)methyl-Sar]-3-[(γ-hydroxy)-N-MeLeu]-4-cyclosporin |

**Table 13**

| | | | |
|---|---|---|---|
| 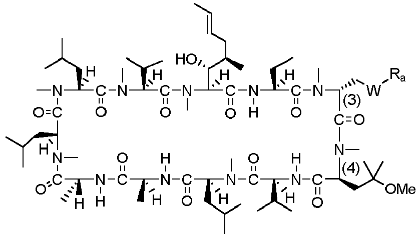 | | | |

| Ex. No. | W | Rₐ | Name |
|---|---|---|---|
| 304 | S | 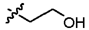 | [(S)-(2-Hydroxyethylthio)methyl-Sar]-3-[(γ-methoxy)-N-MeLeu]-4-cyclosporin |
| 305 | S | 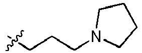 | [(S)-(2-(N-Ethyl-N-methylamino)ethylthio)methyl-Sar]-3-[(γ-methoxy)-N-MeLeu]-4-cyclosporin |
| 306 | S | 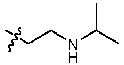 | [(S)-(2-(N-Isopropylamino)ethylthio)methyl-Sar]-3-[(γ-methoxy)-N-MeLeu]-4-cyclosporin |
| 307 | S | 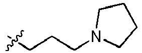 | [(S)-(2-(N-Isopropyl-N-methylamino)ethylthio)methyl-Sar]-3-[(γ-methoxy)-N-MeLeu]-4-cyclosporin |
| 308 | S | 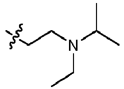 | [(S)-(2-(N-Isopropyl-N-ethylamino)ethylthio)methyl-Sar]-3-[(γ-methoxy)-N-MeLeu]-4-cyclosporin |
| 309 | S | 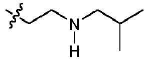 | [(S)-(2-(N-Isobutylamino)ethylthio)methyl-Sar]-3-[(γ-methoxy)-N-MeLeu]-4-cyclosporin |
| 310 | S | 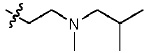 | [(S)-(2-(N-Isobutyl-N-methylamino)ethylthio)methyl-Sar]-3-[(γ-methoxy)-N-MeLeu]-4-cyclosporin |
| 311 | S | 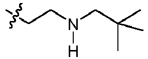 | [(S)-(2-(N-Neopentylamino)ethylthio)methyl-Sar]-3-[(γ-methoxy)-N-MeLeu]-4-cyclosporin |
| 312 | S | 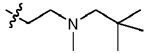 | [(S)-(2-(N-Methyl-N-neopentylamino)ethylthio)methyl-Sar]-3-[(γ-methoxy)-N-MeLeu]-4-cyclosporin |
| 313 | S | 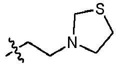 | [(S)-(2-(N-Thiazolidinyl)ethylthio)methyl-Sar]-3-[(γ-methoxy)-N-MeLeu]-4-cyclosporin |
| 314 | S | 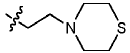 | [(S)-(2-(N-Thiomorpholino)ethylthio)methyl-Sar]-3-[(γ-methoxy)-N-MeLeu]-4-cyclosporin |
| 315 | S | 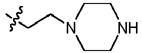 | [(S)-(2-(N-Piperazinyl)ethylthio)methyl-Sar]-3-[(γ-methoxy)-N-MeLeu]-4-cyclosporin |
| 316 | S | 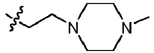 | [(S)-(2-(4-Methyl-N-piperazinyl)ethylthio)methyl-Sar]-3-[(γ-methoxy)-N-MeLeu]-4-cyclosporin |
| 317 | S | 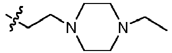 | [(S)-(2-(4-Ethyl-N-piperazinyl)ethylthio)methyl-Sar]-3-[(γ-methoxy)-N-MeLeu]-4-cyclosporin |
| 318 | S | 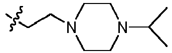 | [(S)-(2-(4-Isopropyl-N-piperazinyl)ethylthio)methyl-Sar-3-[(γ-methoxy)-N-MeLeu]-4-cyclosporin |
| 319 | S | 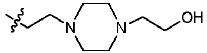 | [(S)-(2-(4-(2-Hydroxyethyl)-N-piperazinyl)ethylthio)methyl-Sar-3-[(γ-methoxy)-N-MeLeu]-4-cyclosporin |
| 320 | S | 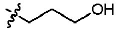 | [(S)-(3-Hydroxypropylthio)methyl-Sar]-3-[(γ-methoxy)-N-MeLeu]-4-cyclosporin |
| 321 | S | 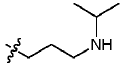 | [(S)-(3-(N-Isopropylamino)propylthio)methyl-Sar]-3-[(y-methoxy)-N-MeLeu]-4-cyclosporin |
| 322 | S | 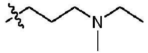 | [(S)-(3-(N-Ethyl-N-methylamino)propylthio)methyl-Sar]-3-[(γ-methoxy)-N-MeLeu]-4-cyclosporin |
| 323 | S | 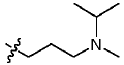 | [(S)-(3-(N-Isopropyl-N-methylamino)propylthio)methyl-Sar]-3-[(γ-methoxy)-N-MeLeu]-4-cyclosporin |
| 324 | S | 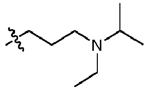 | [(S)-(3-(N-Ethyl-N-isopropylamino)propylthio)methyl-Sar]-3-[(γ-methoxy)-N-MeLeu]-4-cyclosporin |
| 325 | S | 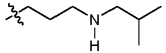 | [(S)-(3-(N-Isobutylamino)propylthio)methyl-Sar]-3-[(γ-methoxy)-N-MeLeu]-4-cyclosporin |
| 326 | S | 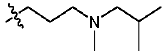 | [(S)-(3-(N-Isobutyl-N-methylamino)propylthio)methyl-Sar]-3-[(γ-methoxy)-N-MeLeu]-4-cyclosporin |
| 327 | S | 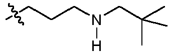 | [(S)-(3-(N-Neopentylamino)propylthio)methyl-Sar]-3-[(y-methoxy)-N-MeLeu]-4-cyclosporin |
| 328 | S | 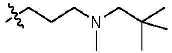 | [(S)-(3-(N-Methyl-N-neopentylamino)propylthio)methyl-Sar]-3-[(y-methoxy)-N-MeLeu]-4-cyclosporin |
| 329 | S | 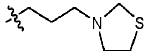 | [(S)-(3-(N-Thiazolidinyl)propylthio)methyl-Sar]-3-[(γ-methoxy)-NMeLeu]-4-cyclosporin |
| 330 | S | 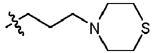 | [(S)-(3-(N-Thiomorpholino)propylthio)methyl-Sar]-3-[(γ-methoxy)-N-MeLeu]-4-cyclosporin |
| 331 | S | 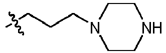 | [(S)-(3-(N-Piperazinyl)propylthio)methyl-Sar]-3-[(y-methoxy)-N-MeLeu]-4-cyclosporin |
| 332 | S | 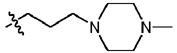 | [(S)-(3-(4-Methyl-N-piperazinyl)propylthio)methyl-Sar]-3-[(γ-methoxy)-N-MeLeu]-4-cyclosporin |
| 333 | S | 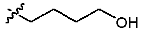 | [(S)-(4-Hydroxybutylthio)methyl-Sar]-3-[(γ-methoxy)-N-MeLeu]-4-cyclosporin |
| 334 | S | 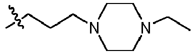 | [(S)-(3-(4-Ethyl-N-piperazinyl)propylthio)methyl-Sar]-3-[(γ-methoxy)-N-MeLeu]-4-cyclosporin |
| 335 | S | 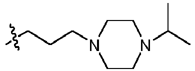 | [(S)-(3-(4-Isopropyl-N-piperazinyl)propylthio)methyl-Sar]-3-[(γ-methoxy)-N-MeLeu]-4-cyclosporin |
| 336 | S | 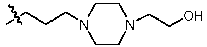 | [(S)-(3-(4-(2-Hydroxyethyl)-N-piperazinyl)propylthio)methyl-Sar]-3-[(y-methoxy)-N-MeLeu]-4-cyclosporin |
| 337 | S | 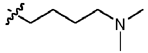 | [(S)-(4-(N,N-Dimethylamino)butylthio)methyl-Sar]-3-[(γ-methoxy)-N-MeLeu]-4-cyclosporin |
| 338 | S | 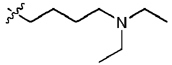 | [(S)-(4-(N-Ethyl-N-methylamino)butylthio)methyl-Sar]-3-[(γ-methoxy)-N-MeLeu]-4-cyclosporin |
| 339 | S | 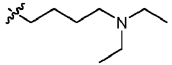 | [(S)-(4-(N,N-Diethylamino)butylthio)methyl-Sar]-3-[(γ-methoxy)-N-MeLeu]-4-cyclosporin |
| 340 | S | 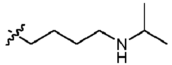 | [(S)-(4-(N-Isopropylamino)butylthio)methyl-Sar]-3-[(γ-methoxy)-N-MeLeu]-4-cyclosporin |
| 341 | S | 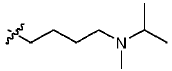 | [(S)-(4-(N-Isopropyl-N-methylamino)butylthio)methyl-Sar]-3-[(γ-methoxy)-N-MeLeu]-4-cyclosporin |
| 342 | S | 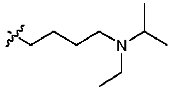 | [(S)-(4-(N-Ethyl-N-isopropylamino)butylthio)methyl-Sar]-3-[(γ-methoxy)-N-MeLeu]-4-cyclosporin |
| 343 | S | 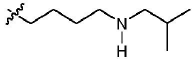 | [(S)-(4-(N-Isobutylamino)butylthio)methyl-Sar]-3-[(γ-methoxy)-N-MeLeu]-4-cyclosporin |
| 344 | S | 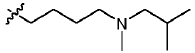 | [(S)-(4-(N-Isobutyl-N-methylamino)butylthio)methyl-Sar]-3-[(γ-methoxy)-N-MeLeu]-4-cyclosporin |
| 345 | S | 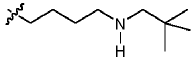 | [(S)-(4-(N-Neopentylamino)butylthio)methyl-Sar]-3-[(γ-methoxy)-N-MeLeu]-4-cyclosporin |
| 346 | S | 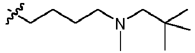 | [(S)-(4-(N-Methyl-N-neopentylamino)butylthio)methyl-Sar]-3-[(y-methoxy)-N-MeLeu]-4-cyclosporin |
| 347 | S | 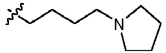 | [(S)-(4-(N-Pyrrolidinyl)butylthio)methyl-Sar]-3-[(γ-methoxy)-N-MeLeu]-4-cyclosporin |
| 348 | S | 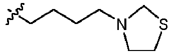 | [(S)-(4-(N-Thiazolidinyl)butylthio)methyl-Sar]-3-[(γ-methoxy)-N-MeLeu]-4-cyclosporin |
| 349 | S | 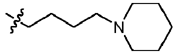 | [(S)-(4-(N-Piperidinyl)butylthio)methyl-Sar]-3-[(γ-methoxy)-NMeLeu]-4-cyclosporin |
| 350 | S | 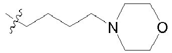 | [(S)-(4-(N-Morpholino)butylthio)methyl-Sar]-3-[(γ-methoxy)-N-MeLeu]-4-cyclosporin |
| 351 | S | 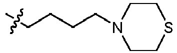 | [(S)-(4-(N-Thiomorpholino)butylthio)methyl-Sar]-3-[(γ-methoxy)-N-MeLeu]-4-cyclosporin |
| 352 | S | 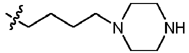 | [(S)-(4-(N-Piperazinyl)butylthio)methyl-Sar]-3-[(γ-methoxy)-N-MeLeu]-4-cyclosporin |
| 353 | S | 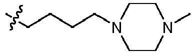 | [(S)-(4-(4-Methyl-N-piperazinyl)butylthio)methyl-Sar]-3-[(γ-methoxy)-N-MeLeu]-4-cyclosporin |
| 354 | S | 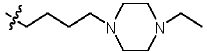 | [(S)-(4-(4-Ethyl-N-piperazinyl)butylthio)methyl-Sar]-3-[(γ-methoxy)-N-MeLeu]-4-cyclosporin |
| 355 | S | 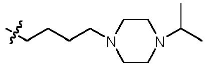 | [(S)-(4-(4-Isopropyl-N-piperazinyl)butylthio)methyl-Sar]-3-[(γ-methoxy)-N-MeLeu]-4-cyclosporin |
| 356 | S | 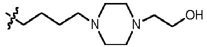 | [(S)-(4-(4-(2-Hydroxyethyl)-N-piperazinyl)butylthio)methyl-Sar]-3-[(γ-methoxy)-N-MeLeu]-4-cyclosporin |
| 357 | O | 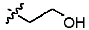 | [(R)-(2-Hydroxyethoxy)methyl-Sar]-3-[(γ-methoxy)-N-MeLeu]-4-cyclosporin |
| 358 | O | 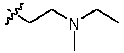 | [(R)-(2-(N-Ethyl-N-methylamino)ethoxy)methyl-Sar]-3-[(γ-methoxy)-N-MeLeu]-4-cyclosporin |
| 359 | O | 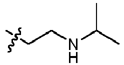 | [(R)-(2-(N-Isopropylamino)ethoxy)methyl-Sar]-3-[(γ-methoxy)-N-MeLeu]-4-cyclosporin |
| 360 | O | 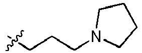 | [(R)-(2-(N-Isopropyl-N-methylamino)ethoxy)methyl-Sar]-3-[(γ-methoxy)-N-MeLeu]-4-cyclosporin |
| 361 | O | 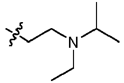 | [(R)-(2-(N-Ethyl-N-isopropylamino)ethoxy)methyl-Sar]-3-[(γ-methoxy)-N-MeLeu]-4-cyclosporin |
| 362 | O | 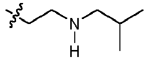 | [(R)-(2-(N-Isobutylamino)ethoxy)methyl-Sar]-3-[(γ-methoxy)-N-MeLeu]-4-cyclosporin |
| 363 | O | 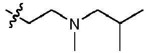 | [(R)-(2-(N-Isobutyl-N-methylamino)ethoxy)methyl-Sar]-3-[(γ-methoxy)-N-MeLeu]-4-cyclosporin |
| 364 | O | 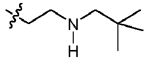 | [(R)-(2-(N-Neopentylamino)ethoxy)methyl-Sar]-3-[(γ-methoxy)-N-MeLeu]-4-cyclosporin |
| 365 | O | 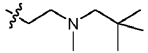 | [(R)-(2-(N-Methyl-N-neopentylamino)ethoxy)methyl-Sar]-3-[(γ-methoxy)-N-MeLeu]-4-cyclosporin |
| 366 | O | 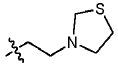 | [(R)-(2-(N-Thiazolidinyl)ethoxy)methyl-Sar]-3-[(γ-methoxy)-N-MeLeu]-4-cyclosporin |
| 367 | O | 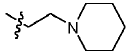 | [(R)-(2-(N-Piperidinyl)ethoxy)methyl-Sar]-3-[(γ-methoxy)-N-MeLeu]-4-cyclosporin |
| 368 | O | 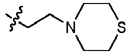 | [(R)-(2-(N-Thiomorpholino)ethoxy)methyl-Sar]-3-[(γ-methoxy)-N-MeLeu]-4-cyclosporin |
| 369 | O | 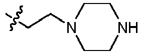 | [(R)-(2-(N-Piperazinyl)ethoxy)methyl-Sar]-3-[(γ-methoxy)-N-MeLeu]-4-cyclosporin |
| 370 | O | 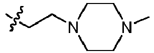 | [(R)-(2-(4-Methyl-N-piperazinyl)ethoxy)methyl-Sar]-3-[(γ-methoxy)-N-MeLeu]-4-cyclosporin |
| 371 | O | 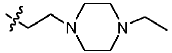 | [(R)-(2-(4-Ethyl-N-piperazinyl)ethoxy)methyl-Sar]-3-[(γ-methoxy)-N-MeLeu]-4-cyclosporin |
| 372 | O | 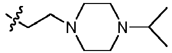 | [(R)-(2-(4-Isopropyl-N-piperazinyl)ethoxy)methyl-Sar]-3-[(γ-methoxy)-N-MeLeu]-4-cyclosporin |
| 373 | O | 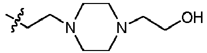 | [(R)-(2-(4-(2-Hydroxyethyl)-N-piperazinyl)ethoxy)methyl-Sar]-3-[(y-methoxy)-N-MeLeu]-4-cyclosporin |
| 374 | O | 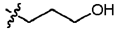 | [(R)-(3-Hydroxypropoxy)methyl-Sar]-3-[(γ-methoxy)-N-MeLeu]-4-cyclosporin |
| 375 | O | 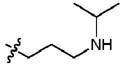 | [(R)-(3-(N-Isopropylamino)propoxy)methyl-Sar]-3-[(γ-methoxy)-N-MeLeu]-4-cyclosporin |
| 376 | O | 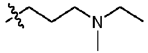 | [(R)-(3-(N-Ethyl-N-methylamino)propoxy)methyl-Sar]-3-[(γ-methoxy)-N-MeLeu]-4-cyclosporin |
| 377 | O | 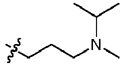 | [(R)-(3-(N-Isopropyl-N-methylamino)propoxy)methyl-Sar]-3-[(γ-methoxy)-N-MeLeu]-4-cyclosporin |
| 378 | O | 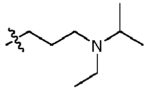 | [(R)-(3-(N-Ethyl-N-isopropylamino)propoxy)methyl-Sar]-3-[(γ-methoxy)-N-MeLeu]-4-cyclosporin |
| 379 | O | 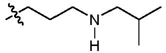 | [(R)-(3-(N-Isobutylamino)propoxy)methyl-Sar]-3-[(γ-methoxy)-N-MeLeu]-4-cyclosporin |
| 380 | O | 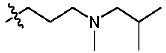 | [(R)-(3-(N-Isobutyl-N-methylamino)propoxy)methyl-Sar]-3-[(γ-methoxy)-N-MeLeu]-4-cyclosporin |
| 381 | O | 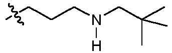 | [(R)-(3-(N-Neopentylamino)propoxy)methyl-Sar]-3-[(γ-methoxy)-N-MeLeu]-4-cyclosporin |
| 382 | O | 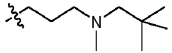 | [(R)-(3-(N-Methyl-N-neopentylamino)propoxy)methyl-Sar]-3-[(γ-methoxy)-N-MeLeu]-4-cyclosporin |
| 383 | O | 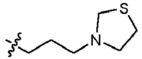 | [(R)-(3-(N-Thiazolidinyl)propoxymethyl-Sar]-3-[(γ-methoxy)-NMeLeu]-4-cyclosporin |
| 384 | O | 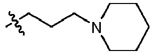 | [(R)-(3-(N-Piperidinyl)propoxy)methyl-Sar]-3-[(γ-methoxy)-N-MeLeu]-4-cyclosporin |
| 385 | O | 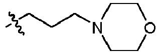 | [(R)-(3-(N-Morpholino)propoxy)methyl-Sar]-3-[(γ-methoxy)-N-MeLeu]-4-cyclosporin |
| 386 | O | 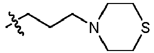 | [(R)-(3-(N-Thiomorpholino)propoxy)methyl-Sar]-3-[(γ-methoxy)-N-MeLeu]-4-cyclosporin |
| 387 | O | 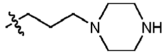 | [(R)-(3-(N-Piperazinyl)propoxy)methyl-Sar]-3-[(γ-methoxy)-N-MeLeu]-4-cyclosporin |
| 388 | O | 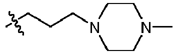 | [(R)-(3-(4-Methyl-N-piperazinyl)propoxy)methyl-Sar]-3-[(γ-methoxy)-N-MeLeu]-4-cyclosporin |
| 389 | O | 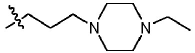 | [(R)-(3-(4-Ethyl-N-piperazinyl)propoxy)methyl-Sar]-3-[(γ-methoxy)-N-MeLeu]-4-cyclosporin |
| 390 | O | 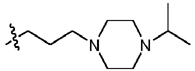 | [(R)-(3-(4-Isopropyl-N-piperazinyl)propoxy)methyl-Sar]-3-[(γ-methoxy)-N-MeLeu]-4-cyclosporin |
| 391 | O | 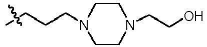 | [(R)-(3-(4-(2-Hydroxyethyl)-N-piperazinyl)propoxy)methyl-Sar]-3-[(y-methoxy)-N-MeLeu]-4-cyclosporin |
| 392 | O | 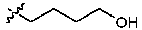 | [(R)-(4-Hydroxybutoxy)methyl-Sar]-3-[(γ-methoxy)-N-MeLeu]-4-cyclosporin |
| 393 | O | 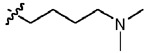 | [(R)-(4-(N,N-Dimethylamino)butoxy)methyl-Sar]-3-[(γ-methoxy)-N-MeLeu]-4-cyclosporin |
| 394 | O | 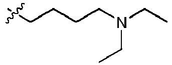 | [(R)-(4-(N-Ethyl-N-methylamino)butylthio)methyl-Sar]-3-[(γ-methoxy)-N-MeLeu]-4-cyclosporin |
| 395 | O | 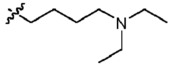 | [(R)-(4-(N,N-Diethylamino)butoxy)methyl-Sar]-3-[(γ-methoxy)-N-MeLeu]-4-cyclosporin |
| 396 | O | 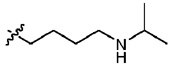 | [(R)-(4-(N-Isopropylamino)butoxy)methyl-Sar]-3-[(γ-methoxy)-N-MeLeu]-4-cyclosporin |
| 397 | O | 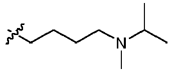 | [(R)-(4-(N-Isopropyl-N-methylamino)butoxy)methyl-Sar]-3-[(γ-methoxy)-N-MeLeu]-4-cyclosporin |
| 398 | O | 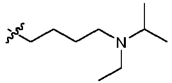 | [(R)-(4-(N-Ethyl-N-isopropylamino)butoxy)methyl-Sar]-3-[(γ-methoxy)-N-MeLeu]-4-cyclosporin |
| 399 | O | 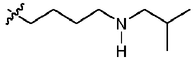 | [(R)-(4-(N-Isobutylamino)butoxy)methyl-Sar]-3-[(γ-methoxy)-N-MeLeu]-4-cyclosporin |
| 400 | O | 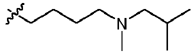 | [(R)-(4-(N-Isobutyl-N-methylamino)butoxy)methyl-Sar]-3-[(γ-methoxy)-N-MeLeu]-4-cyclosporin |
| 401 | O | 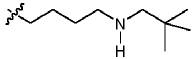 | [(R)-(4-(N-Neopentylamino)butoxy)methyl-Sar]-3-[(γ-methoxy)-N-MeLeu]-4-cyclosporin |
| 402 | O | 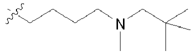 | [(R)-(4-(N-Methyl-N-neopentylamino)butoxy)methyl-Sar]-3-[(γ-methoxy)-N-MeLeu]-4-cyclosporin |
| 403 | O | 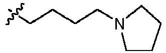 | [(R)-(4-(N-Pyrrolidinyl)butoxy)methyl-Sar]-3-[(γ-methoxy)-N-MeLeu]-4-cyclosporin |
| 404 | O | 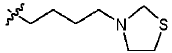 | [(R)-(4-(N-Thiazolidinyl)butoxy)methyl-Sar]-3-[(γ-methoxy)-N-MeLeu]-4-cyclosporin |
| 405 | O | 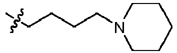 | [(R)-(4-(N-Piperidinyl)butoxy)methyl-Sar]-3-[(γ-methoxy)-N-MeLeu]-4-cyclosporin |
| 406 | O | 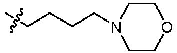 | [(R)-(4-(N-Morpholino)butoxy)methyl-Sar]-3-[(γ-methoxy)-N-MeLeu]-4-cyclosporin |
| 407 | O | 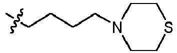 | [(R)-(4-(N-Thiomorpholino)butoxy)methyl-Sar]-3-[(γ-methoxy)-N-MeLeu]-4-cyclosporin |
| 408 | O | 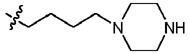 | [(R)-(4-(N-Piperazinyl)butoxy)methyl-Sar]-3-[(γ-methoxy)-N-MeLeu]-4-cyclosporin |
| 409 | O | 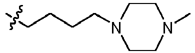 | [(R)-(4-(4-Methyl-N-piperazinyl)butoxy)methyl-Sar]-3-[(γ-methoxy)-N-MeLeu]-4-cyclosporin |
| 410 | O | 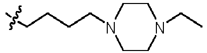 | [(R)-(4-(4-Ethyl-N-piperazinyl)butoxy)methyl-Sar]-3-[(γ-methoxy)-N-MeLeu]-4-cyclosporin |
| 411 | O | 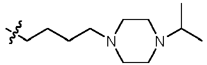 | [(R)-(4-(4-Isopropyl-N-piperazinyl)butoxy)methyl-Sar]-3-[(γ-methoxy)-N-MeLeu]-4-cyclosporin |
| 412 | O | 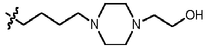 | [(R)-(4-(4-(2-Hydroxyethyl)-N-piperazinyl)butoxy)methyl-Sar]-3-[(y-methoxy)-N-MeLeu]-4-cyclosporin |

**Table 14**

| | | | |
|---|---|---|---|
| 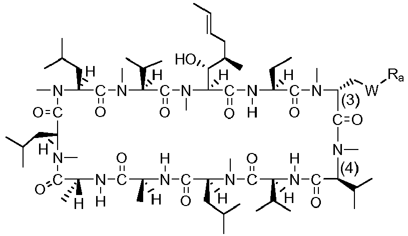 | | | |

| Ex. No. | W | Rₐ | Name |
|---|---|---|---|
| 413 | S | 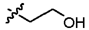 | [(S)-(2-Hydroxyethylthio)methyl-Sar]-3-[N-MeVal]-4-cyclo sporin |
| 414 | S | 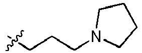 | [(S)-(2-(N-Ethyl-N-methylamino)ethylthio)methyl-Sar]-3-[N-MeVal]-4-cyclosporin |
| 415 | S | 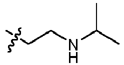 | [(S)-(2-(N-Isopropylamino)ethylthio)methyl-Sar]-3-[N-MeVal]-4-cyclosporin |
| 416 | S | 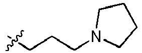 | [(S)-(2-(N-Isopropyl-N-methylamino)ethylthio)methyl-Sar]-3-[N-MeVal]-4-cyclosporin |
| 417 | S | 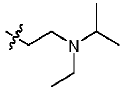 | [(S)-(2-(N-Ethyl-N-isopropylamino)ethylthio)methyl-Sar]-3-[N-MeVal]-4-cyclosporin |
| 418 | S | 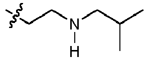 | [(S)-(2-(N-Isobutylamino)ethylthio)methyl-Sar]-3-[N-MeVal]-4-cyclosporin |
| 419 | S | 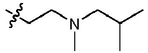 | [(S)-(2-(N-Isobutyl-N-methylamino)ethylthio)methyl-Sar]-3-[N-MeVal]-4-cyclosporin |
| 420 | S | 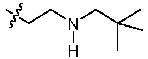 | [(S)-(2-(N-Neopentylamino)ethylthio)methyl-Sar]-3-[N-MeVal]-4-cyclosporin |
| 421 | S | 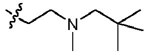 | [(S)-(2-(N-Methyl-N-neopentylamino)ethylthio)methyl-Sar]-3-[N-MeVal]-4-cyclosporin |
| 422 | S | 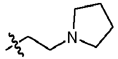 | [(S)-(2-(N-Pyrrolidinyl)ethylthio)methyl-Sar]-3-[N-MeVal]-4-cyclo sporin |
| 423 | S | 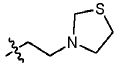 | [(S)-(2-(N-Thiazolidinyl)ethylthio)methyl-Sar]-3-[N-MeVal]-4-cyclosporin |
| 424 | S | 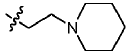 | [(S)-(2-(N-Piperidinyl)ethylthio)methyl-Sar]-3-[N-MeVal]-4-cyclo sporin |
| 425 | S | 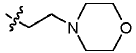 | [(S)-(2-(N-Morpholino)ethylthio)methyl-Sar]-3-[N-MeVal]-4-cyclo sporin |
| 426 | S | 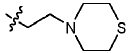 | [(S)-(2-(N-Thiomorpholino)ethylthio)methyl-Sar]-3-[N-MeVal]-4-cyclosporin |
| 427 | S | 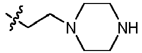 | [(S)-(2-(N-Piperazinyl)ethylthio)methyl-Sar]-3-[N-MeVal]-4-cyclosporin |
| 428 | S | 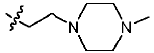 | [(S)-(2-(4-Methyl-N-piperazinyl)ethylthio)methyl-Sar]-3-[N-MeVal]-4-cyclosporin |
| 429 | S | 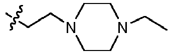 | [(S)-(2-(4-Ethyl-N-piperazinyl)ethylthio)methyl-Sar]-3-[N-MeVal]-4-cyclosporin |
| 430 | S | 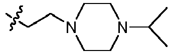 | [(S)-(2-(4-Isopropyl-N-piperazinyl)ethylthio)methyl-Sar-3-[N-MeVal]-4-cyclosporin |
| 431 | S | 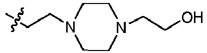 | [(S)-(2-(4-(2-Hydroxyethyl)-N-piperazinyl)ethylthio)methyl-Sar]-3-[N-MeVal]-4-cyclosporin |
| 432 | S | 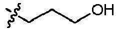 | [(S)-(3-Hydroxypropylthio)methyl-Sar]-3-[N-MeVal]-4-cyclosporin |
| 433 | S | 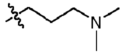 | [(S)-(3-(N,N-Dimethylamino)propylthio)methyl-Sar]-3-[N-MeVal]-4-cyclosporin |
| 434 | S | 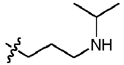 | [(S)-(3-(N-Isopropylamino)propylthio)methyl-Sar]-3-[N-MeVal]-4-cyclosporin |
| 435 | S | 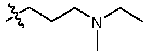 | [(S)-(3-(N-Ethyl-N-methylamino)propylthio)methyl-Sar]-3-[N-MeVal]-4-cyclosporin |
| 436 | S | 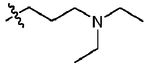 | [(S)-(3-(N,N-Diethylamino)propylthio)methyl-Sar]-3-[N-MeVal]-4-cyclosporin |
| 437 | S | 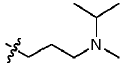 | [(S)-(3-(N-Isopropyl-N-methylamino)propylthio)methyl-Sar]-3-[N-MeVal]-4-cyclosporin |
| 438 | S | 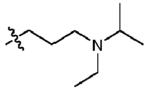 | [(S)-(3-(N-Ethyl-N-isopropylamino)propylthio)methyl-Sar]-3-[N-MeVal]-4-cyclosporin |
| 439 | S | 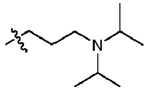 | [(S)-(3-(N,N-Diisopropylamino)propylthio)methyl-Sar]-3-[N-MeVal]-4-cyclosporin |
| 440 | S | 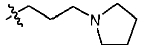 | [(S)-(3-(N-Pyrrolidinyl)propylthio)methyl-Sar]-3-[N-MeVal]-4-cyclosporin |
| 441 | S | 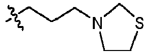 | [(S)-(3-(N-Thiazolidinyl)propylthio)methyl-Sar]-3-[N-MeVal]-4-cyclosporin |
| 442 | S | 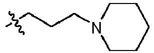 | [(S)-(3-(N-Piperidinyl)propylthio)methyl-Sar]-3-[N-MeVal]-4-cyclosporin |
| 443 | S | 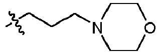 | [(S)-(3-(N-Morpholino)propylthio)methyl-Sar]-3-[N-MeVal]-4-cyclosporin |
| 444 | S | 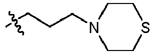 | [(S)-(3-(N-Thiomorpholino)propylthio)methyl-Sar]-3-[N-MeVal]-4-cyclosporin |
| 445 | S | 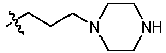 | [(S)-(3-(N-Piperazinyl)propylthio)methyl-Sar]-3-[N-MeVal]-4-cyclosporin |
| 446 | S | 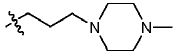 | [(S)-(3-(4-Methyl-N-piperazinyl)propylthio)methyl-Sar]-3-[N-MeVal]-4-cyclosporin |
| 447 | S | 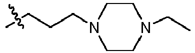 | [(S)-(3-(4-Ethyl-N-piperazinyl)propylthio)methyl-Sar]-3-[N-MeVal]-4-cyclosporin |
| 448 | S | 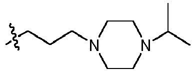 | [(S)-(3-(4-Isopropyl-N-piperazinyl)propylthio)methyl-Sar]-3-[N-MeVal]-4-cyclosporin |
| 449 | S | 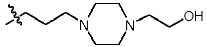 | [(S)-(3-(4-(2-Hydroxyethyl)-N-piperazinyl)propylthio)methyl-Sar]-3-[N-MeVal]-4-cyclosporin |
| 450 | S | 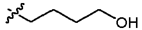 | [(S)-(4-Hydroxybutylthio)methyl-Sar]-3-[N-MeVal]-4-cyclosporin |
| 451 | S | 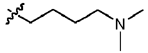 | [(S)-(4-(N,N-Dimethylamino)butylthio)methyl-Sar]-3-[N-MeVal]-4-cyclosporin |
| 452 | S | 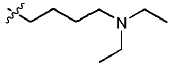 | [(S)-(4-(N-Ethyl-N-methylamino)butylthio)methyl-Sar]-3-[N-MeVal]-4-cyclosporin |
| 453 | S | 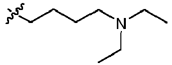 | [(S)-(4-(N,N-Diethylamino)butylthio)methyl-Sar]-3-[N-MeVal]-4-cyclosporin |
| 454 | S | 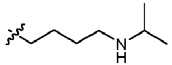 | [(S)-(4-(N-Isopropylamino)butylthio)methyl-Sar]-3-[N-MeVal]-4-cyclosporin |
| 455 | S | 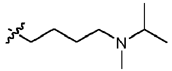 | [(S)-(4-(N-Isopropyl-N-methylamino)butylthio)methyl-Sar]-3-[N-MeVal]-4-cyclosporin |
| 456 | S | 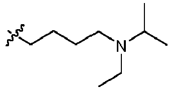 | [(S)-(4-(N-Ethyl-N-isopropylamino)butylthio)methyl-Sar]-3-[N-MeVal]-4-cyclosporin |
| 457 | S | 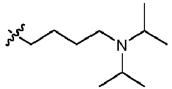 | [(S)-(4-(N,N-Diisopropylamino)butylthio)methyl-Sar]-3-[N-MeVal]-4-cyclosporin |
| 458 | S | 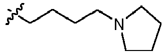 | [(S)-(4-(N-Pyrrolidinyl)butylthio)methyl-Sar]-3-[N-MeVal]-4-cyclosporin |
| 459 | S | 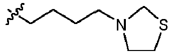 | [(S)-(4-(N-Thiazolidinyl)butylthio)methyl-Sar]-3-[N-MeVal]-4-cyclosporin |
| 460 | S | 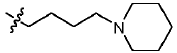 | [(S)-(4-(N-Piperidinyl)butylthio)methyl-Sar]-3-[N-MeVal]-4-cyclosporin |
| 461 | S | 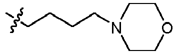 | [(S)-(4-(N-Morpholino)butylthio)methyl-Sar]-3-[N-MeVal]-4-cyclosporin |
| 462 | S | 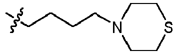 | [(S)-(4-(N-Thiomorpholino)butylthio)methyl-Sar]-3-[N-MeVal]-4-cyclosporin |
| 463 | S | 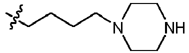 | [(S)-(4-(N-Piperazinyl)butylthio)methyl-Sar]-3-[N-MeVal]-4-cyclosporin |
| 464 | S | 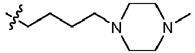 | [(S)-(4-(4-Methyl-N-piperazinyl)butylthio)methyl-Sar]-3-[N-MeVal]-4-cyclosporin |
| 465 | S | 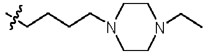 | [(S)-(4-(4-Ethyl-N-piperazinyl)butylthio)methyl-Sar]-3-[N-MeVal]-4-cyclosporin |
| 466 | S | 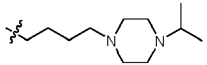 | [(S)-(4-(4-Isopropyl-N-piperazinyl)butylthio)methyl-Sar]-3-[N-MeVal]-4-cyclosporin |
| 467 | S | 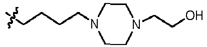 | [(S)-(4-(4-(2-Hydroxyethyl)-N-piperazinyl)butylthio)methyl-Sar]-3-[N-MeVal]-4-cyclosporin |
| 468 | O | 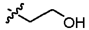 | [(R)-(2-Hydroxyethoxy)methyl-Sar]-3-[N-MeVal]-4-cyclosporin |
| 469 | O | 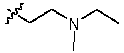 | [(R)-(2-(N-Ethyl-N-methylamino)ethoxy)methyl-Sar]-3-[N-MeVal]-4-cyclosporin |
| 470 | O | 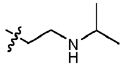 | [(R)-(2-(N-Isopropylamino)ethoxy)methyl-Sar]-3-[N-MeVal]-4-cyclosporin |
| 471 | O | 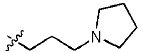 | [(R)-(2-(N-Isopropyl-N-methylamino)ethoxy)methyl-Sar]-3-[N-MeVal]-4-cyclosporin |
| 472 | O | 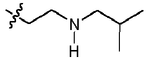 | [(R)-(2-(N-Isobutylamino)ethoxy)methyl-Sar]-3-[N-MeVal]-4-cyclosporin |
| 473 | O | 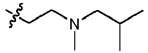 | [(R)-(2-(N-Isobutyl-N-methylamino)ethoxy)methyl-Sar]-3-[N-MeVal]-4-cyclosporin |
| 474 | O | 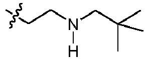 | [(R)-(2-(N-Neopentylamino)ethoxy)methyl-Sar]-3-[N-MeVal]-4-cyclosporin |
| 475 | O | 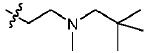 | [(R)-(2-(N-Methyl-N-neopentylamino)ethoxy)methyl-Sar]-3-[N-MeVal]-4-cyclosporin |
| 476 | O | 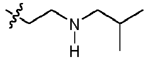 | [(R)-(2-(N-Isobutylamino)ethoxy)methyl-Sar]-3-[N-MeVal]-4-cyclosporin |
| 477 | O | 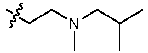 | [(R)-(2-(N-Isobutyl-N-methylamino)ethoxy)methyl-Sar]-3-[N-MeVal]-4-cyclosporin |
| 478 | O | 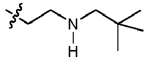 | [(R)-(2-(N-Neopentylamino)ethoxy)methyl-Sar]-3-[N-MeVal]-4-cyclosporin |
| 479 | O | 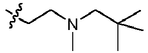 | [(R)-(2-(N-Methyl-N-neopentylamino)ethoxy)methyl-Sar]-3-[N-MeVal]-4-cyclosporin |
| 480 | O | 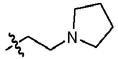 | [(R)-(2-(N-Pyrrolidinyl)ethoxy)methyl-Sar]-3-[N-MeVal]-4-cyclosporin |
| 481 | O | 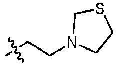 | [(R)-(2-(N-Thiazolidinyl)ethoxy)methyl-Sar]-3-[N-MeVal]-4-cyclosporin |
| 482 | O | 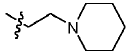 | [(R)-(2-(N-Piperidinyl)ethoxy)methyl-Sar]-3-[N-MeVal]-4-cyclosporin |
| 483 | O | 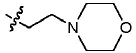 | [(R)-(2-(N-Morpholino)ethoxy)methyl-Sar]-3-[N-MeVal]-4-cyclosporin |
| 484 | O | 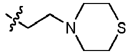 | [(R)-(2-(N-Thiomorpholino)ethoxy)methyl-Sar]-3-[N-MeVal]-4-cyclosporin |
| 485 | O | 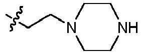 | [(R)-(2-(N-Piperazinyl)ethoxy)methyl-Sar]-3-[N-MeVal]-4-cyclosporin |
| 486 | O | 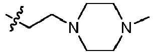 | [(R)-(2-(4-Methyl-N-piperazinyl)ethoxy)methyl-Sar]-3-[N-MeVal]-4-cyclosporin |
| 487 | O | 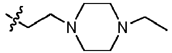 | [(R)-(2-(4-Ethyl-N-piperazinyl)ethoxy)methyl-Sar]-3-[N-MeVal]-4-cyclosporin |
| 488 | O | 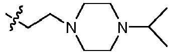 | [(R)-(2-(4-Isopropyl-N-piperazinyl)ethoxy)methyl-Sar]-3-[N-MeVal]-4-cyclosporin |
| 489 | O | 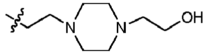 | [(R)-(2-(4-(2-Hydroxyethyl)-N-piperazinyl)ethoxy)methyl-Sar]-3-[N-MeVal]-4-cyclosporin |
| 490 | O | 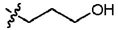 | [(R)-(3-Hydroxypropoxy)methyl-Sar]-3-[N-MeVal]-4-cyclosporin |
| 491 | O | 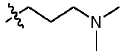 | [(R)-(3-(N,N-Dimethylamino)propoxy)methyl-Sar]-3-[N-MeVal]-4-cyclosporin |
| 492 | O | 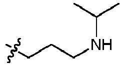 | [(R)-(3-(N-Isopropylamino)propoxy)methyl-Sar]-3-[N-MeVal]-4-cyclosporin |
| 493 | O | 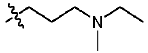 | [(R)-(3-(N-Ethyl-N-methylamino)propoxy)methyl-Sar]-3-[N-MeVal]-4-cyclosporin |
| 494 | O | 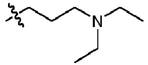 | [(R)-(3-(N,N-Diethylamino)propoxy)methyl-Sar]-3-[N-MeVal]-4-cyclosporin |
| 495 | O | 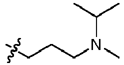 | [(R)-(3-(N-Isopropyl-N-methylamino)propoxy)methyl-Sar]-3-[N-MeVal]-4-cyclosporin |
| 496 | O | 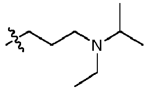 | [(R)-(3-(N-Ethyl-N-isopropylamino)propoxy)methyl-Sar]-3-[N-MeVal]-4-cyclosporin |
| 497 | O | 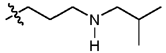 | [(R)-(3-(N-Isobutylamino)propoxy)methyl-Sar]-3-[N-MeVal]-4-cyclosporin |
| 498 | O | 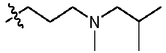 | [(R)-(3-(N-Isobutyl-N-methylamino)propoxy)methyl-Sar]-3-[N-MeVal]-4-cyclosporin |
| 499 | O | 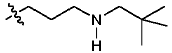 | [(R)-(3-(N-Neopentylamino)propoxy)methyl-Sar]-3-[N-MeVal]-4-cyclosporin |
| 500 | O | 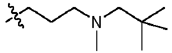 | [(R)-(3-(N-Methyl-N-neopentylamino)propoxy)methyl-Sar]-3-[N-MeVal]-4-cyclosporin |
| 501 | O | 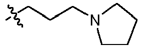 | [(R)-(3-(N-Pyrrolidinyl)propoxy)methyl-Sar]-3-[N-MeVal]-4-cyclosporin |
| 502 | O | 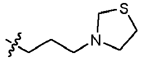 | [(R)-(3-(N-Thiazolidinyl)propoxy)methyl-Sar]-3-[N-MeVal]-4-cyclosporin |
| 503 | O | 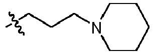 | [(R)-(3-(N-Piperidinyl)propoxy)methyl-Sar]-3-[N-MeVal]-4-cyclosporin |
| 504 | O | 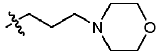 | [(R)-(3-(N-Morpholino)propoxy)methyl-Sar]-3-[N-MeVal]-4-cyclosporin |
| 505 | O | 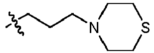 | [(R)-(3-(N-Thiomorpholino)propoxy)methyl-Sar]-3-[N-MeVal]-4-cyclosporin |
| 506 | O | 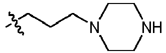 | [(R)-(3-(N-Piperazinyl)propoxy)methyl-Sar]-3-[N-MeVal]-4-cyclosporin |
| 507 | O | 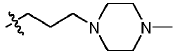 | [(R)-(3-(4-Methyl-N-piperazinyl)propoxy)methyl-Sar]-3-[N-MeVal]-4-cyclosporin |
| 508 | O | 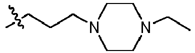 | [(R)-(3-(4-Ethyl-N-piperazinyl)propoxy)methyl-Sar]-3-[N-MeVal]-4-cyclosporin |
| 509 | O | 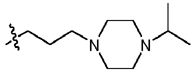 | [(R)-(3-(4-Isopropyl-N-piperazinyl)propoxy)methyl-Sar]-3-[N-MeVal]-4-cyclosporin |
| 510 | O | 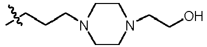 | [(R)-(3-(4-(2-Hydroxyethyl)-N-piperazinyl)propoxy)methyl-Sar]-3-[N-MeVal]-4-cyclosporin |
| 511 | O | 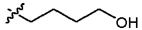 | [(R)-(4-Hydroxybutoxy)methyl-Sar]-3-[N-MeVal]-4-cyclosporin |
| 512 | O | 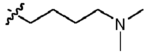 | [(R)-(4-(N,N-Dimethylamino)butoxy)methyl-Sar]-3-[N-MeVal]-4-cyclosporin |
| 513 | O | 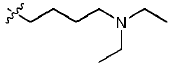 | [(R)-(4-(N-Ethyl-N-methylamino)butoxy)methyl-Sar]-3-[N-MeVal]-4-cyclosporin |
| 514 | O | 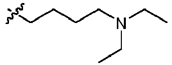 | [(R)-(4-(N,N-Diethylamino)butoxy)methyl-Sar]-3-[N-MeVal]-4-cyclosporin |
| 515 | O | 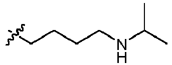 | [(R)-(4-(N-Isopropylamino)butoxy)methyl-Sar]-3-[N-MeVal]-4-cyclosporin |
| 516 | O | 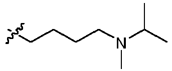 | [(R)-(4-(N-Isopropyl-N-methylamino)butoxy)methyl-Sar]-3-[N-MeVal]-4-cyclosporin |
| 517 | O | 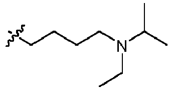 | [(R)-(4-(N-Ethyl-N-isopropylamino)butoxy)methyl-Sar]-3-[N-MeVal]-4-cyclosporin |
| 518 | O | 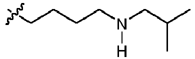 | [(R)-(4-(N-Isobutylamino)butoxy)methyl-Sar]-3-[N-MeVal]-4-cyclosporin |
| 519 | O | 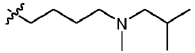 | [(R)-(4-(N-Isobutyl-N-methylamino)butoxy)methyl-Sar]-3-[N-MeVal]-4-cyclosporin |
| 520 | O | 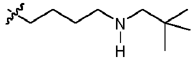 | [(R)-(4-(N-Neopentylamino)butoxy)methyl-Sar]-3-[N-MeVal]-4-cyclosporin |
| 521 | O | 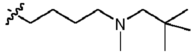 | [(R)-(4-(N-Methyl-N-neopentylamino)butoxy)methyl-Sar]-3-[N-MeVal]-4-cyclosporin |
| 522 | O | 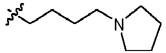 | [(R)-(4-(N-Pyrrolidinyl)butoxy)methyl-Sar]-3-[N-MeVal]-4-cyclosporin |
| 523 | O | 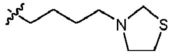 | [(R)-(4-(N-Thiazolidinyl)butoxy)methyl-Sar]-3-[N-MeVal]-4-cyclosporin |
| 524 | O | 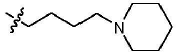 | [(R)-(4-(N-Piperidinyl)butoxy)methyl-Sar]-3-[N-MeVal]-4-cyclosporin |
| 525 | O | 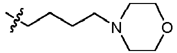 | [(R)-(4-(N-Morpholino)butoxy)methyl-Sar]-3-[N-MeVal]-4-cyclosporin |
| 526 | O | 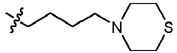 | [(R)-(4-(N-Thiomorpholino)butoxy)methyl-Sar]-3-[N-MeVal]-4-cyclosporin |
| 527 | O | 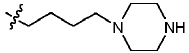 | [(R)-(4-(N-Piperazinyl)butoxy)methyl-Sar]-3-[N-MeVal]-4-cyclosporin |
| 528 | O | 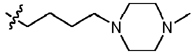 | [(R)-(4-(4-Methyl-N-piperazinyl)butoxy)methyl-Sar]-3-[N-MeVal]-4-cyclosporin |
| 529 | O | 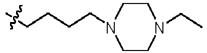 | [(R)-(4-(4-Ethyl-N-piperazinyl)butoxy)methyl-Sar]-3-[N-MeVal]-4-cyclosporin |
| 530 | O | 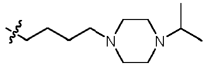 | [(R)-(4-(4-Isopropyl-N-piperazinyl)butoxy)methyl-Sar]-3-[N-MeVal]-4-cyclosporin |
| 531 | O | 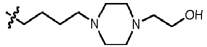 | [(R)-(4-(4-(2-Hydroxyethyl)-N-piperazinyl)butoxy)methyl-Sar]-3-[N-MeVal]-4-cyclosporin |

**Table 15**

| | | | |
|---|---|---|---|
| 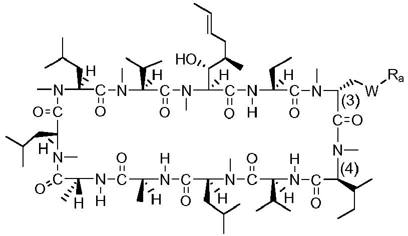 | | | |

| Ex. No. | W | Rₐ | Name |
|---|---|---|---|
| 532 | S | 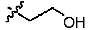 | [(S)-(2-hydroxyethylthio)methyl-Sar]-3-[N-MeIle]-4-cyclosporin |
| 533 | S | 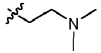 | [(S)-(2-(N,N-Dimethylamino)ethylthio)methyl-Sar]-3-[N-MeIle]-4-cyclosporin |
| 534 | S | 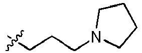 | [(S)-(2-(N-Ethyl-N-methylamino)ethylthio)methyl-Sar]-3-[N-MeIle]-4-cyclosporin |
| 535 | S | 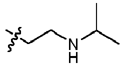 | [(S)-(2-(N-Isopropylamino)ethylthio)methyl-Sar]-3-[N-MeIle]-4-cyclosporin |
| 536 | S | 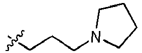 | [(S)-(2-(N-Isopropyl-N-methylamino)ethylthio)methyl-Sar]-3-[N-MeIle]-4-cyclosporin |
| 537 | S | 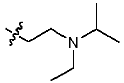 | [(S)-(2-(N-Ethyl-N-isopropylamino)ethylthio)methyl-Sar]-3-[N-MeIle]-4-cyclosporin |
| 538 | S | 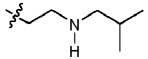 | [(S)-(2-(N-Isobutylamino)ethylthio)methyl-Sar]-3-[N-MeIle]-4-cyclosporin |
| 539 | S | 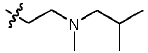 | [(S)-(2-(N-Isobutyl-N-methylamino)ethylthio)methyl-Sar]-3-[N-MeIle]-4-cyclosporin |
| 540 | S | 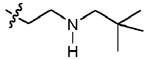 | [(S)-(2-(N-Neopentylamino)ethylthio)methyl-Sar]-3-[N-MeIle]-4-cyclosporin |
| 541 | S | 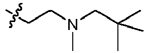 | [(S)-(2-(N-Methyl-N-neopentylamino)ethylthio)methyl-Sar]-3-[N-MeIle]-4-cyclosporin |
| 542 | S | 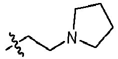 | [(S)-(2-(N-Pyrrolidinyl)ethylthio)methyl-Sar]-3-[N-MeIle]-4-cyclosporin |
| 543 | S | 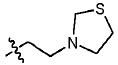 | [(S)-(2-(N-Thiazolidinyl)ethylthio)methyl-Sar]-3-[N-MeIle]-4-cyclosporin |
| 544 | S | 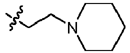 | [(S)-(2-(N-Piperidinyl)ethylthio)methyl-Sar]-3-[N-MeIle]-4-cyclosporin |
| 545 | S | 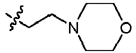 | [(S)-(2-(N-Morpholino)ethylthio)methyl-Sar]-3-[N-MeIle]-4-cyclosporin |
| 546 | S | 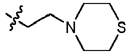 | [(S)-(2-(N-Thiomorpholino)ethylthio)methyl-Sar]-3-[N-MeIle]-4-cyclosporin |
| 547 | S | 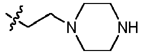 | [(S)-(2-(N-Piperazinyl)ethylthio)methyl-Sar]-3-[N-MeIle]-4-cyclosporin |
| 548 | S | 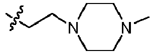 | [(S)-(2-(4-Methyl-N-piperazinyl)ethylthio)methyl-Sar]-3-[N-MeIle]-4-cyclosporin |
| 549 | S | 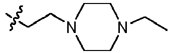 | [(S)-(2-(4-Ethyl-N-piperazinyl)ethylthio)methyl-Sar]-3-[N-MeIle]-4-cyclosporin |
| 550 | S | 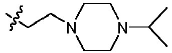 | [(S)-(2-(4-Isopropyl-N-piperazinyl)ethylthio)methyl-Sar]-3-[N-MeIle]-4-cyclosporin |
| 551 | S | 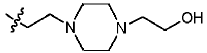 | [(S)-(2-(4-(2-Hydroxyethyl)-N-piperazinyl)ethylthio)methyl-Sar]-3-[N-MeIle]-4-cyclosporin |
| 552 | S | 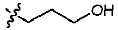 | [(S)-(3-Hydroxypropylthio)methyl-Sar]-3-[N-MeIle]-4-cyclosporin |
| 553 | S | 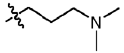 | [(S)-(3-(N,N-Dimethylamino)propylthio)methyl-Sar]-3-[N-MeIle]-4-cyclosporin |
| 554 | S | 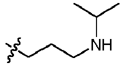 | [(S)-(3-(N-Isopropylamino)propylthio)methyl-Sar]-3-[N-MeIle]-4-cyclosporin |
| 555 | S | 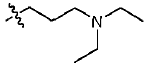 | [(S)-(3-(N,N-Diethylamino)propylthio)methyl-Sar]-3-[N-MeIle]-4-cyclosporin |
| 556 | S | 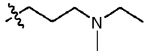 | [(S)-(3-(N-Ethyl-N-methylamino)propylthio)methyl-Sar]-3-[N-MeIle]-4-cyclosporin |
| 557 | S | 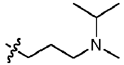 | [(S)-(3-(N-Isopropyl-N-methylamino)propylthio)methyl-Sar]-3-[N-MeIle]-4-cyclosporin |
| 558 | S | 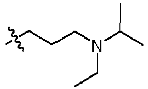 | [(S)-(3-(N-Ethyl-N-isopropylamino)propylthio)methyl-Sar]-3-[N-MeIle]-4-cyclosporin |
| 559 | S | 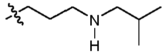 | [(S)-(3-(N-Isobutylamino)propylthio)methyl-Sar]-3-[N-MeIle]-4-cyclosporin |
| 560 | S | 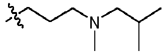 | [(S)-(3-(N-Isobutyl-N-methylamino)propylthio)methyl-Sar]-3-[N-MeIle]-4-cyclosporin |
| 561 | S | 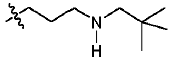 | [(S)-(3-(N-Neopentylamino)propylthio)methyl-Sar]-3-[N-MeIle]-4-cyclosporin |
| 562 | S | 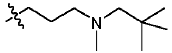 | [(S)-(3-(N-Methyl-N-neopentylamino)propylthio)methyl-Sar]-3-[N-MeIle]-4-cyclosporin |
| 563 | S | 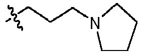 | [(S)-(3-(N-Pyrrolidinyl)propylthio)methyl-Sar]-3-[N-MeIle]-4-cyclosporin |
| 564 | S | 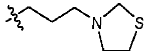 | [(S)-(3-(N-Thiazolidinyl)propylthio)methyl-Sar]-3-[N-MeIle]-4-cyclosporin |
| 565 | S | 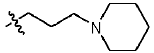 | [(S)-(3-(N-Piperidinyl)propylthio)methyl-Sar]-3-[N-MeIle]-4-cyclosporin |
| 566 | S | 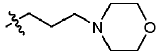 | [(S)-(3-(N-Morpholino)propylthio)methyl-Sar]-3-[N-MeIle]-4-cyclosporin |
| 567 | S | 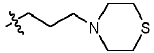 | [(S)-(3-(N-Thiomorpholino)propylthio)methyl-Sar]-3-[N-MeIle]-4-cyclosporin |
| 568 | S | 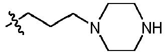 | [(S)-(3-(N-Piperazinyl)propylthio)methyl-Sar]-3-[N-MeIle]-4-cyclosporin |
| 569 | S | 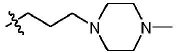 | [(S)-(3-(4-Methyl-N-piperazinyl)propylthio)methyl-Sar]-3-[N-MeIle]-4-cyclosporin |
| 570 | S | 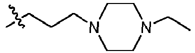 | [(S)-(3-(4-Ethyl-N-piperazinyl)propylthio)methyl-Sar]-3-[N-MeIle]-4-cyclosporin |
| 571 | S | 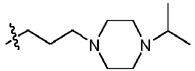 | [(S)-(3-(4-Isopropyl-N-piperazinyl)propylthio)methyl-Sar]-3-[N-MeIle]-4-cyclosporin |
| 572 | S | 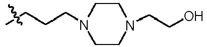 | [(S)-(3-(4-(2-Hydroxyethyl)-N-piperazinyl)propylthio)methyl-Sar]-3-[N-MeIle]-4-cyclosporin |
| 573 | S | 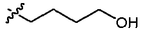 | [(S)-(4-Hydroxybutylthio)methyl-Sar]-3-[N-MeIle]-4-cyclosporin |
| 574 | S | 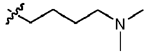 | [(S)-(4-(N,N-Dimethylamino)butylthio)methyl-Sar]-3-[N-MeIle]-4-cyclosporin |
| 575 | S | 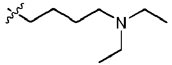 | [(S)-(4-(N-Ethyl-N-methylamino)butylthio)methyl-Sar]-3-[N-MeIle]-4-cyclosporin |
| 576 | S | 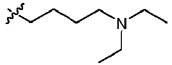 | [(S)-(4-(N,N-Diethylamino)butylthio)methyl-Sar]-3-[N-MeIle]-4-cyclosporin |
| 577 | S | 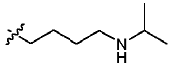 | [(S)-(4-(N-Isopropylamino)butylthio)methyl-Sar]-3-[N-MeIle]-4-cyclosporin |
| 578 | S | 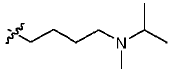 | [(S)-(4-(N-Isopropyl-N-methylamino)butylthio)methyl-Sar]-3-[N-MeIle]-4-cyclosporin |
| 579 | S | 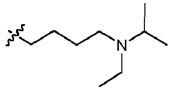 | [(S)-(4-(N-Ethyl-N-isopropylamino)butylthio)methyl-Sar]-3-[N-MeIle]-4-cyclosporin |
| 580 | S | 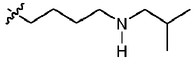 | [(S)-(4-(N-Isobutylamino)butylthio)methyl-Sar]-3-[N-MeIle]-4-cyclosporin |
| 581 | S | 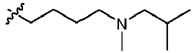 | [(S)-(4-(N-Isobutyl-N-methylamino)butylthio)methyl-Sar]-3-[N-MeIle]-4-cyclosporin |
| 582 | S | 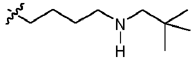 | [(S)-(4-(N-Neopentylamino)butylthio)methyl-Sar]-3-[N-MeIle]-4-cyclosporin |
| 583 | S | 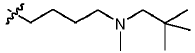 | [(S)-(4-(N-Methyl-N-neopentylamino)butylthio)methyl-Sar]-3-[N-MeIle]-4-cyclosporin |
| 584 | S | 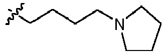 | [(S)-(4-(N-Pyrrolidinyl)butylthio)methyl-Sar]-3-[N-MeIle]-4-cyclosporin |
| 585 | S | 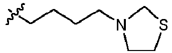 | [(S)-(4-(N-Thiazolidinyl)butylthio)methyl-Sar]-3-[N-MeIle]-4-cyclosporin |
| 586 | S | 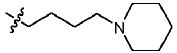 | [(S)-(4-(N-Piperidinyl)butylthio)methyl-Sar]-3-[N-MeIle]-4-cyclosporin |
| 587 | S | 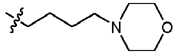 | [(S)-(4-(N-Morpholino)butylthio)methyl-Sar]-3-[N-MeIle]-4-cyclosporin |
| 588 | S | 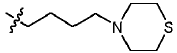 | [(S)-(4-(N-Thiomorpholino)butylthio)methyl-Sar]-3-[N-MeIle]-4-cyclosporin |
| 589 | S | 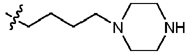 | [(S)-(4-(N-Piperazinyl)butylthio)methyl-Sar]-3-[N-MeIle]-4-cyclosporin |
| 590 | S | 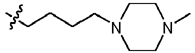 | [(S)-(4-(4-Methyl-N-piperazinyl)butylthio)methyl-Sar]-3-[N-MeIle]-4-cyclosporin |
| 591 | S | 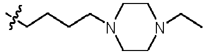 | [(S)-(4-(4-Ethyl-N-piperazinyl)butylthio)methyl-Sar]-3-[N-MeIle]-4-cyclosporin |
| 592 | S | 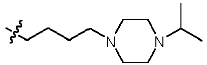 | [(S)-(4-(4-Isopropyl-N-piperazinyl)butylthio)methyl-Sar]-3-[N-MeIle]-4-cyclosporin |
| 593 | S | 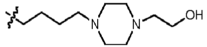 | [(S)-(4-(4-(2-Hydroxyethyl)-N-piperazinyl)butylthio)methyl-Sar]-3-[N-MeIle]-4-cyclosporin |
| 594 | O | 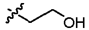 | [(R)-(2-Hydroxyethoxy)methyl-Sar]-3-[N-MeIle]-4-cyclosporin |
| 595 | O | 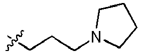 | [(R)-(2-(N,N-Dimethylamino)ethoxy)methyl-Sar]-3-[N-MeIle]-4-cyclosporin |
| 596 | O | 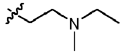 | [(R)-(2-(N-Ethyl-N-methylamino)ethoxy)methyl-Sar]-3-[N-MeIle]-4-cyclosporin |
| 597 | O | 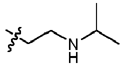 | [(R)-(2-(N-Isopropylamino)ethoxy)methyl-Sar]-3-[N-MeIle]-4-cyclosporin |
| 598 | O | 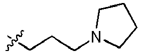 | [(R)-(2-(N-Isopropyl-N-methylamino)ethoxy)methyl-Sar]-3-[N-MeIle]-4-cyclosporin |
| 599 | O | 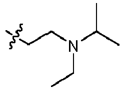 | [(R)-(2-(N-Ethyl-N-isopropylamino)ethoxy)methyl-Sar]-3-[N-MeIle]-4-cyclosporin |
| 600 | O | 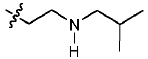 | [(R)-(2-(N-Isobutylamino)ethoxy)methyl-Sar]-3-[N-MeIle]-4-cyclosporin |
| 601 | O | 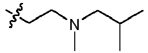 | [(R)-(2-(N-Isobutyl-N-methylamino)ethoxy)methyl-Sar]-3-[N-MeIle]-4-cyclosporin |
| 602 | O | 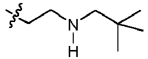 | [(R)-(2-(N-Neopentylamino)ethoxy)methyl-Sar]-3-[N-MeIle]-4-cyclosporin |
| 603 | O | 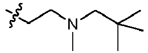 | [(R)-(2-(N-Methyl-N-neopentylamino)ethoxy)methyl-Sar]-3-[N-MeIle]-4-cyclosporin |
| 604 | O | 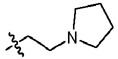 | [(R)-(2-(N-Pyrrolidinyl)ethoxy)methyl-Sar]-3-[N-MeIle]-4-cyclosporin |
| 605 | O | 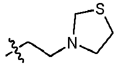 | [(R)-(2-(N-Thiazolidinyl)ethoxy)methyl-Sar]-3-[N-MeIle]-4-cyclosporin |
| 606 | O | 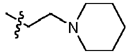 | [(R)-(2-(N-Piperidinyl)ethoxy)methyl-Sar]-3-[N-MeIle]-4-cyclosporin |
| 607 | O | 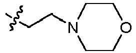 | [(R)-(2-(N-Morpholino)ethoxy)methyl-Sar]-3-[N-MeIle]-4-cyclosporin |
| 608 | O | 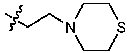 | [(R)-(2-(N-Thiomorpholino)ethoxy)methyl-Sar]-3-[N-MeIle]-4-cyclosporin |
| 609 | O | 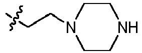 | [(R)-(2-(N-Piperazinyl)ethoxy)methyl-Sar]-3-[N-MeIle]-4-cyclosporin |
| 610 | O | 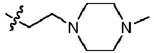 | [(R)-(2-(4-Methyl-N-piperazinyl)ethoxy)methyl-Sar]-3-[N-MeIle]-4-cyclosporin |
| 611 | O | 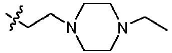 | [(R)-(2-(4-Ethyl-N-piperazinyl)ethoxy)methyl-Sar]-3-[N-MeIle]-4-cyclosporin |
| 612 | O | 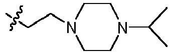 | [(R)-(2-(4-Isopropyl-N-piperazinyl)ethoxy)methyl-Sar]-3-[N-MeIle]-4-cyclosporin |
| 613 | O | 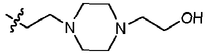 | [(R)-(2-(4-(2-Hydroxyethyl)-N-piperazinyl)ethoxy)methyl-Sar]-3-[N-MeIle]-4-cyclosporin |
| 614 | O | 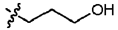 | [(R)-(3-Hydroxypropoxy)methyl-Sar]-3-[N-MeIle]-4-cyclosporin |
| 615 | O | 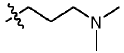 | [(R)-(3-(N,N-Dimethylamino)propoxy)methyl-Sar]-3-[N-MeIle]-4-cyclosporin |
| 616 | O | 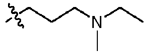 | [(R)-(3-(N-Ethyl-N-methylamino)propoxy)methyl-Sar]-3-[N-MeIle]-4-cyclosporin |
| 617 | O | 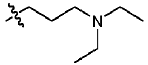 | [(R)-(3-(N,N-Diethylamino)propoxy)methyl-Sar]-3-[N-MeIle]-4-cyclosporin |
| 618 | O | 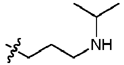 | [(R)-(3-(N-Isopropylamino)propoxy)methyl-Sar]-3-[N-MeIle]-4-cyclosporin |
| 619 | O | 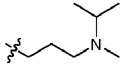 | [(R)-(3-(N-Isopropyl-N-methylamino)propoxy)methyl-Sar]-3-[N-MeIle]-4-cyclosporin |
| 670 | O | 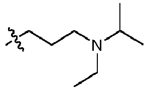 | [(R)-(3-(N-Ethyl-N-isopropylamino)propoxy)methyl-Sar]-3-[N-MeIle]-4-cyclosporin |
| 671 | O | 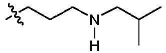 | [(R)-(3-(N-Isobutylamino)propoxy)methyl-Sar]-3-[N-MeIle]-4-cyclosporin |
| 672 | O | 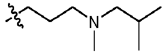 | [(R)-(3-(N-Isobutyl-N-methylamino)propoxy)methyl-Sar]-3-[N-MeIle]-4-cyclosporin |
| 673 | O | 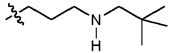 | [(R)-(3-(N-Neopentylamino)propoxy)methyl-Sar]-3-[N-MeIle]-4-cyclosporin |
| 674 | O | 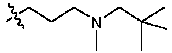 | [(R)-(3-(N-Methyl-N-neopentylamino)propoxy)methyl-Sar]-3-[N-MeIle]-4-cyclosporin |
| 675 | O | 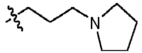 | [(R)-(3-(N-Pyrrolidinyl)propoxy)methyl-Sar]-3-[N-MeIle]-4-cyclosporin |
| 576 | O | 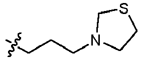 | [(R)-(3-(N-Thiazolidinyl)propoxy)methyl-Sar]-3-[N-MeIle]-4-cyclosporin |
| 677 | O | 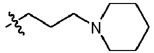 | [(R)-(3-(N-Piperidinyl)propoxy)methyl-Sar]-3-[N-MeIle]-4-cyclosporin |
| 678 | O | 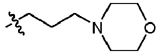 | [(R)-(3-(N-Morpholino)propoxy)methyl-Sar]-3-[N-MeIle]-4-cyclosporin |
| 679 | O | 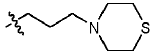 | [(R)-(3-(N-Thiomorpholino)propoxy)methyl-Sar]-3-[N-MeIle]-4-cyclosporin |
| 680 | O | 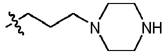 | [(R)-(3-(N-Piperazinyl)propoxy)methyl-Sar]-3-[N-MeIle]-4-cyclosporin |
| 681 | O | 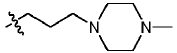 | [(R)-(3-(4-Methyl-N-piperazinyl)propoxy)methyl-Sar]-3-[N-MeIle]-4-cyclosporin |
| 682 | O | 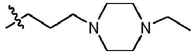 | [(R)-(3-(4-Ethyl-N-piperazinyl)propoxy)methyl-Sar]-3-[N-MeIle]-4-cyclosporin |
| 683 | O | 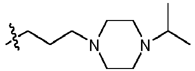 | [(R)-(3-(4-Isopropyl-N-piperazinyl)propoxy)methyl-Sar]-3-[N-MeIle]-4-cyclosporin |
| 684 | O | 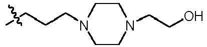 | [(R)-(3-(4-(2-Hydroxyethyl)-N-piperazinyl)propoxy)methyl-Sar]-3-[N-MeIle]-4-cyclosporin |
| 685 | O | 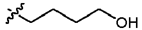 | [(R)-(4-Hydroxybutoxy)methyl-Sar]-3-[N-MeIle]-4-cyclosporin |
| 686 | O | 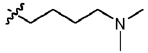 | [(R)-(4-(N,N-Dimethylamino)butoxy)methyl-Sar]-3-[N-MeIle]-4-cyclosporin |
| 687 | O | 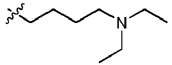 | [(R)-(4-(N-Ethyl-N-methylamino)butoxy)methyl-Sar]-3-[N-MeIle]-4-cyclosporin |
| 688 | O | 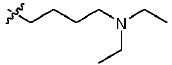 | [(R)-(4-(N,N-Diethylamino)butoxy)methyl-Sar]-3-[N-MeIle]-4-cyclosporin |
| 689 | O | 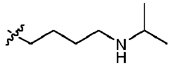 | [(R)-(4-(N-Isopropylamino)butoxy)methyl-Sar]-3-[N-MeIle]-4-cyclosporin |
| 690 | O | 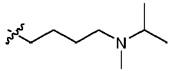 | [(R)-(4-(N-Isopropyl-N-methylamino)butoxy)methyl-Sar]-3-[N-MeIle]-4-cyclosporin |
| 691 | O | 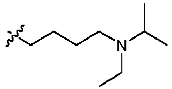 | [(R)-(4-(N-Ethyl-N-isopropylamino)butoxy)methyl-Sar]-3-[N-MeIle]-4-cyclosporin |
| 692 | O | 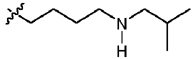 | [(R)-(4-(N-Isobutylamino)butoxy)methyl-Sar]-3-[N-MeIle]-4-cyclosporin |
| 693 | O | 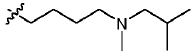 | [(R)-(4-(N-Isobutyl-N-methylamino)butoxy)methyl-Sar]-3-[N-MeIle]-4-cyclosporin |
| 694 | O | 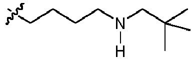 | [(R)-(4-(N-Neopentylamino)butoxy)methyl-Sar]-3-[N-MeIle]-4-cyclosporin |
| 695 | O | 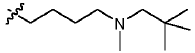 | [(R)-(4-(N-Methyl-N-neopentylamino)butoxy)methyl-Sar]-3-[N-MeIle]-4-cyclosporin |
| 696 | O | 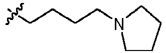 | [(R)-(4-(N-Pyrrolidinyl)butoxy)methyl-Sar]-3-[N-MeIle]-4-cyclosporin |
| 697 | O | 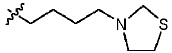 | [(R)-(4-(N-Thiazolidinyl)butoxy)methyl-Sar]-3-[N-MeIle]-4-cyclosporin |
| 698 | O | 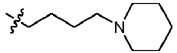 | [(R)-(4-(N-Piperidinyl)butoxy)methyl-Sar]-3-[N-MeIle]-4-cyclosporin |
| 699 | O | 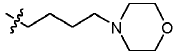 | [(R)-(4-(N-Morpholino)butoxy)methyl-Sar]-3-[N-MeIle]-4-cyclosporin |
| 700 | O | 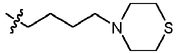 | [(R)-(4-(N-Thiomorpholino)butoxy)methyl-Sar]-3-[N-MeIle]-4-cyclosporin |
| 701 | O | 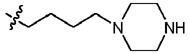 | [(R)-(4-(N-Piperazinyl)butoxy)methyl-Sar]-3-[N-MeIle]-4-cyclosporin |
| 702 | O | 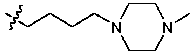 | [(R)-(4-(4-Methyl-N-piperazinyl)butoxy)methyl-Sar]-3-[N-MeIle]-4-cyclosporin |
| 703 | O | 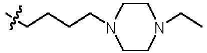 | [(R)-(4-(4-Ethyl-N-piperazinyl)butoxy)methyl-Sar]-3-[N-MeIle]-4-cyclosporin |
| 704 | O | 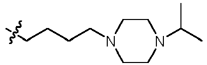 | [(R)-(4-(4-Isopropyl-N-piperazinyl)butoxy)methyl-Sar]-3-[N-MeIle]-4-cyclosporin |
| 705 | O | 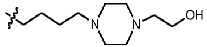 | [(R)-(4-(4-(2-Hydroxyethyl)-N-piperazinyl)butoxy)methyl-Sar]-3-[N-MeIle]-4-cyclosporin |

## Claims

1. A compound of Formula (I): 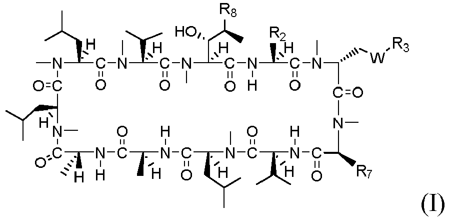 or pharmaceutically acceptable salt thereof, wherein:
R₈ is n-butyl, (*E*)-but-2-enyl, or 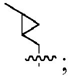
R₂ is ethyl, 1-hydroxyethyl, isopropyl or n-propyl;
W is O or S;
R₃ is:
(C₁-C₆)alkyl, optionally substituted by one or more groups R₄ which may be the same or different;
(C₂-C₆)alkenyl, optionally substituted by one or more groups which may be the same or different selected from halogen, hydroxy, amino, monoalkylamino and dialkylamino;
(C₂-C₆)alkynyl, optionally substituted by one or one or more groups which may be the same or different selected from halogen, hydroxy, amino, monoalkylamino and dialkylamino;
(C₃-C₇)cycloalkyl, optionally substituted by one or more groups which may be the same or different selected from halogen, hydroxy, amino, monoalkylamino and dialkylamino;
phenyl or CH₂-phenyl, optionally substituted by one or more groups which may be the same or different selected from halogen, hydroxy, (C₁-C₆)alkyl;
R₇ is 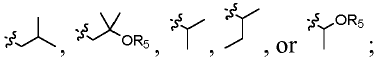
R₅ is:
H;
(C₁-C₆)alkyl, optionally substituted by one or more groups R₆ which may be the same or different;
(C₂-C₆)alkenyl, optionally substituted by one or more groups which may be the same or different selected from hydroxy, (C₁-C₆)alkyl, aryl, (CH₂)ₚOR_{A}, O(CH₂)ₘOH, O(CH₂)ₘO(CH₂)ₘO_{H}, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)ₚC(=O)OR_{A};
(C₂-C₆)alkynyl, optionally substituted by one or one or more groups which may be the same or different selected from halogen, hydroxy, amino, monoalkylamino and dialkylamino;
(C₃-C₇)cycloalkyl, optionally substituted by one or more groups which may be the same or different selected from halogen, hydroxy, amino, monoalkylamino and dialkylamino;
phenyl or CH₂-phenyl, optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₄ is independently halogen, hydroxy, aryl, O(CH₂)ₘOR_{A}, O(CH₂)ₘO(CH₂)ₘOR_{A}, C(=O)(C₁-C₆)alkyl, C(=O)OR_{A}, C(=O)NR_{A}R_{B}, -NR_{A}R_{B}, -NR_{C}CH₂(CH₂)ₚNR_{A}R_{B}, NR_{C}[CH₂(CH₂)ₚNR_{A}]ₘCH₂(CH₂)ₙNR_{A}R_{B}, O[CH₂(CH₂)ₚO]ₘCH₂(CH₂)ₙOR_{A}, OCH₂(CH₂)ₚNR_{A}R_{B}, or O[CH₂(CH₂)ₚO]ₘCH₂(CH₂)ₙNR_{A}R_{B};
each occurrence of R₆ is independently halogen, hydroxy, aryl, S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
hydrogen;
(C₁-C₆)alkyl, optionally substituted by one or more groups R_{D} which may be the same or different;
(C₂-C₆)alkenyl or (C₂-C₆)alkynyl;
(C₃-C₇)cycloalkyl optionally substituted with (C₁-C₆)alkyl;
phenyl optionally substituted with from one to five groups which may be the same or different selected from halogen, -O(C₁-C₆)alkyl, -C(=O)O(C₁-C₆)alkyl, amino,
alkylamino and dialkylamino;
or a heterocyclic ring which may be saturated or unsaturated containing five or six ring atoms and from one to three heteroatoms which may be the same or different selected from nitrogen, sulfur and oxygen;
or R_{A} and R_{B}, together with the nitrogen atom to which they are attached, form a saturated or unsaturated heterocyclic ring containing from three to seven ring atoms,
which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
p is an integer of 0, 1, 2, 3, 4, or 5; and
m is an integer of 1, 2, 3, 4 or 5.

2. A compound of claim 1, having the structure of Formulae (II) through (V): 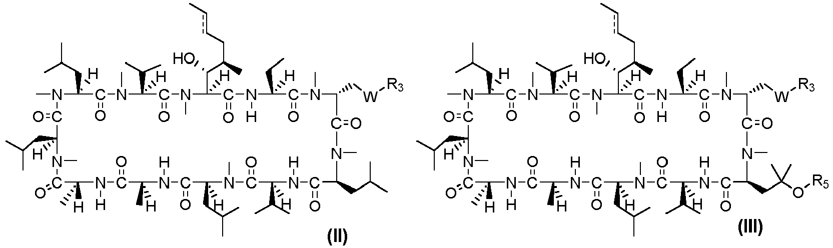 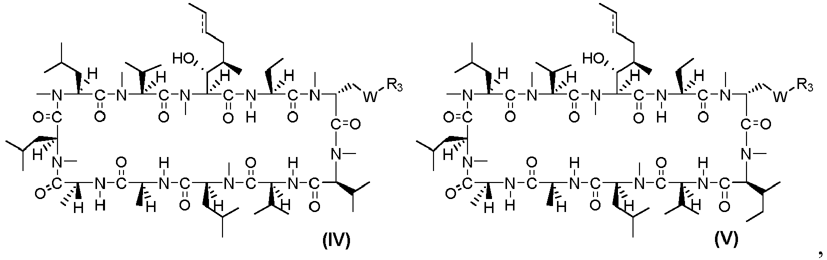 or pharmaceutically acceptable salt thereof, wherein: 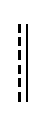
represents a single bond or double bond;
each W is independently O or S;
each R₃ is independently:
(C₁-C₆)alkyl, optionally substituted by one or more groups R₄ which may be the same or different;
(C₂-C₆)alkenyl, optionally substituted by one or more groups which may be the same or different selected from halogen, hydroxy, (C₁-C₆)alkyl, aryl, (CH₂)ₚOR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{c}(CH₂)ₘNR_{c}(CH₂)ₘNR_{A}R_{B}, (CH₂)ₚC(=O)NR_{A}R_{B}, (CH₂)ₚC(=O)OR_{A};
(C₂-C₆)alkynyl, optionally substituted by one or one or more groups which may be the same or different selected from halogen, hydroxy, amino, monoalkylamino and dialkylamino;
(C₃-C₇)cycloalkyl, optionally substituted by one or more groups which may be the same or different selected from halogen, hydroxy, amino, monoalkylamino and dialkylamino;
phenyl or CH₂-phenyl, optionally substituted by one or more groups which may be the same or different selected from halogen, hydroxy, (C₁-C₆)alkyl;
each R₅ is independently:
H;
(C₁-C₆)alkyl, optionally substituted by one or more groups R₆ which may be the same or different;
(C₂-C₆)alkenyl, optionally substituted by one or more groups which may be the same or different selected from halogen, hydroxy, (C₁-C₆)alkyl, aryl, (CH₂)ₚOR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)ₚNR_{A}R_{B}, (CH₂)ₚNR_{C}(CH₂)ₘNR_{A}P_{B}, (CH₂)ₚNR_{c}(CH₂)ₘNR_{c}(CH₂)ₘNR_{A}R_{B}, (CH₂)ₚC(=O)NR_{A}P_{B}, (CH₂)ₚC(=O)OR_{A};
(C₂-C₆)alkynyl, optionally substituted by one or one or more groups which may be the same or different selected from halogen, hydroxy, amino, monoalkylamino and dialkylamino;
(C₃-C₇)cycloalkyl, optionally substituted by one or more groups which may be the same or different selected from halogen, hydroxy, amino, monoalkylamino and dialkylamino;
phenyl or CH₂-phenyl, optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₄ is independently halogen, hydroxy, aryl, O(CH₂)ₘOR_{A}, O(CH₂)ₘO(CH₂)ₘOR_{A}, C(=O)(C₁-C₆)alkyl, C(=O)OR_{A}, C(=O)NR_{A}R_{B}, -NR_{A}R_{B}, -NR_{C}CH₂(CH₂)ₚNR_{A}RB, NR_{C}[CH₂(CH₂)ₚNR_{A}]ₘCH₂(CH₂)ₙNR_{A}R_{B}, O[CH₂(CH₂)ₚO]ₘCH₂(CH₂)ₙOR_{A}, OCH₂(CH₂)ₚNR_{A}R_{B}, or O[CH₂(CH₂)ₚO]ₘCH₂(CH₂)ₙNR_{A}R_{B};
each occurrence of R₆ is independently halogen, hydroxy, aryl, S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
hydrogen;
(C₁-C₆)alkyl, optionally substituted by one or more groups R_{D} which may be the same or different;
(C₂-C₆)alkenyl or (C₂-C₆)alkynyl;
(C₃-C₇)cycloalkyl optionally substituted with (C₁-C₆)alkyl;
phenyl optionally substituted with from one to five groups which may be the same or different selected from halogen, -O(C₁-C₆)alkyl, -C(=O)O(C₁-C₆)alkyl, amino, alkylamino and dialkylamino;
or a heterocyclic ring which may be saturated or unsaturated containing five or six ring atoms and from one to three heteroatoms which may be the same or different selected from nitrogen, sulfur and oxygen;
or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
or R_{A} and R_{B}, together with the nitrogen atom to which they are attached, form -N=CH-NR_{F}R_{F}, -N=CMe-NR_{F}R_{F'}, or -NR_{F}C(=NH)NR_{F}R_{F'};
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl;
each occurrence of R_{F} and R_{F'} is independently hydrogen, (C₁-C₆)alkyl, phenyl, benzyl, or R_{F} and R_{F'}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
p is an integer of 0, 1, 2, 3, 4, 5, or 6;
m is an integer of 1, 2, 3, 4, 5, or 6; and
n is an integer of 1, 2, 3, 4, 5 or 6.

3. The compound of claim 1 or claim 2, wherein R₃ is methyl, ethyl, n-propyl, i- propyl, n-butyl, i-butyl, t-butyl, CH₂CMe₃, phenyl, CH₂-phenyl, 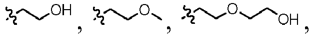 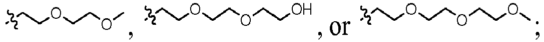 or
wherein R₃ is -(CH₂)ₙNR_{A}R_{B}, wherein n is an integer of 1, 2, 3, 4, 5 or 6; and wherein each occurrence of R_{A} and R_{B} is independently hydrogen; (C₁-C₄)alkyl, optionally substituted by one or more groups R_{D} which may be the same or different, in which each occurrence of R_{D} is independently halogen, hydroxy, O(C₁-C₄)alkyl, C(=O)(C₁-C₄)alkyl, C(=O)O(C₁-C₄)alkyl; or wherein R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from (C₁-C₄)alkyl, phenyl and benzyl; or
wherein R₃ is -(CH₂)ₙNR_{A}R_{B}, wherein n is an integer of 1, 2, 3, 4, 5 or 6; and wherein R_{A} and R_{B}, together with the nitrogen atom to which they are attached, form a saturated or unsaturated heterocyclic ring containing from three to seven ring atoms, which ring may optionally contain another heteroatom selected from nitrogen, oxygen and sulfur and may be optionally substituted by from one to four groups which may be the same or different selected from (C₁-C₄)alkyl, phenyl and benzyl; or
wherein R₃ is 2-aminoethyl, 2-aminobutyl, 3-aminobutyl, 2-monoalkylaminoethyl, 2-monoalkylaminobutyl, 3-monoalkylaminobutyl, 2-dialkylaminoethyl, 2-dialkylaminobutyl, or 3-dialkylaminobutyl, wherein said alkyl is (C₁-C₄)alkyl; or
wherein R₃ is dimethylaminoethyl, diethylaminoethyl, methylethylaminoethyl, methyl-iso-butylaminoethyl, ethyl-iso-butylaminoethyl, methyl-tert-butylaminoethyl, or ethyl-tert-butylaminoethyl; or
wherein R₃ is: 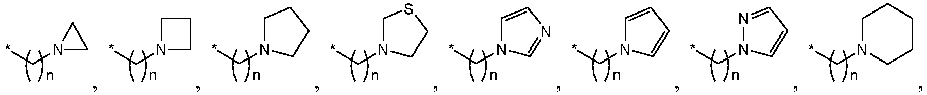 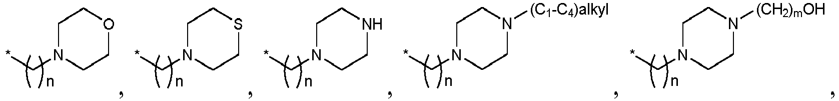 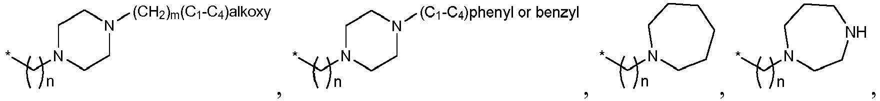 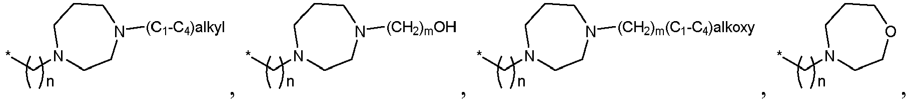 or 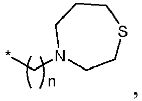 in which n is an integer of 2, 3, 4, 5, or 6, and m is an integer of 2, 3, or 4.

4. The compound of any one of claims 1-3, wherein R₅ is H, (C₁-C₆)alkyl, (C₂-C₆)alkenyl, phenyl, benzyl, CH₂-S-(C₁-C₆)alky, CH₂-O-(C₁-C₆)alkyl, (C₂-C₆)OR_{A}, (C₁-C₆)-monoalkyl amine, (C₁-C₆)-dialkyl amine, or (C₁-C₆)-cyclic amine, in which said phenyl or benzyl is optionally substituted by one to three substitutents selected from (C₁-C₄)alkyl, (C₁-C₄)alkoxy, and halogen; and R_{A} is H, (C₁-C₆)alkyl, phenyl, CH₂-phenyl, (C₁-C₆)alkylOH, (CH₂)ₚO(CH₂)ₘOH, (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl; p is an integer of 0, 1, 2, 3, 4, or 5; and m is an integer of 1, 2, 3, 4 or 5.

5. The compound of any one of claims 1-4, wherein each occurrence R_{A} and R_{B} is independently H, (C₁-C₆)alkyl, phenyl, CH₂-phenyl, (C₁-C₆)alkylOH, (CH₂)ₚO(CH₂)ₘOH, or (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl; or
wherein R_{A} and R_{B}, together with the nitrogen atom to which they are attached, form a heterocycle selected from 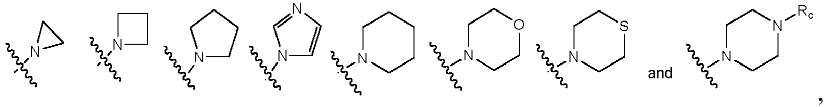 in which R_{C} is H, Me, Et, n-Pr, i-Pr, n-Bu, i-Bu, t-Bu, CH₂CMe₃, Ph, CH₂Ph, CH₂CH₂OH, or CH₂CH₂O(C₁-C₄)alkyl.

6. A compound of claim 1, having structure of Formulae (IIa)-(Va): 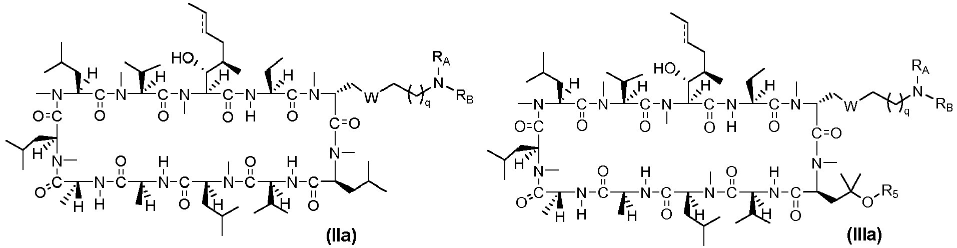 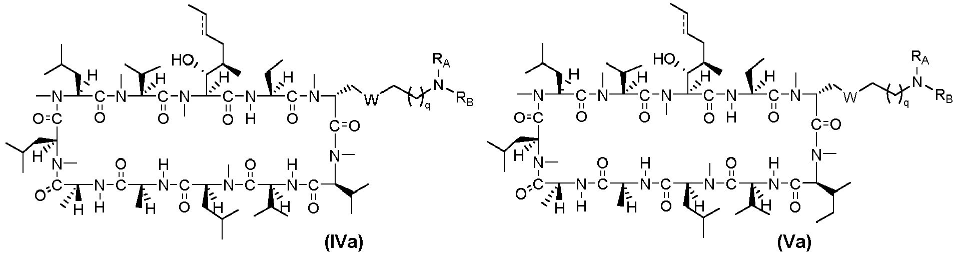 or a pharmaceutically acceptable salt thereof, wherein: 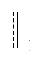
represents a single bond or double bond;
each W is independently O or S;
each R₅ is independently:
H;
(C₁-C₆)alkyl, optionally substituted by one or more groups R₆ which may be the same or different;
(C₂-C₆)alkenyl, optionally substituted by one or more groups which may be the same or different selected from halogen, hydroxy, (C₁-C₆)alkyl, aryl, (CH₂)ₚOR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{c}(CH₂)ₘNR_{A}R_{B}, (CH₂)ₚC(=O)NR_{A}R_{B}, (CH₂)ₚC(=O)OR_{A};
(C₂-C₆)alkynyl, optionally substituted by one or one or more groups which may be the same or different selected from halogen, hydroxy, amino, monoalkylamino and dialkylamino;
(C₃-C₇)cycloalkyl, optionally substituted by one or more groups which may be the same or different selected from halogen, hydroxy, amino, monoalkylamino and dialkylamino;
phenyl or CH₂-phenyl, optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B}, (CH₂)ₚC(=O)OR_{A};
each occurrence of R₆ is independently halogen, hydroxy, aryl, S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
hydrogen;
(C₁-C₆)alkyl, optionally substituted by one or more groups R_{D} which may be the same or different;
(C₂-C₆)alkenyl or (C₂-C₆)alkynyl;
(C₃-C₇)cycloalkyl optionally substituted with (C₁-C₆)alkyl;
phenyl optionally substituted with from one to five groups which may be the same or different selected from halogen, -O(C₁-C₆)alkyl, -C(=O)O(C₁-C₆)alkyl, amino, alkylamino and dialkylamino;
or a heterocyclic ring which may be saturated or unsaturated containing five or six ring atoms and from one to three heteroatoms which may be the same or different selected from nitrogen, sulfur and oxygen;
or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl;
each p is independently an integer of 0, 1, 2, 3, 4, or 5; and
each of m, n and q is independently an integer of 1, 2, 3, 4 or 5.

7. A compound of claim 1, wherein R₇ is

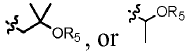

8. The compound of claim 2, wherein: 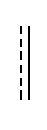
represents a single bond or double bond;
each W is independently O or S;
each R₃ is independently 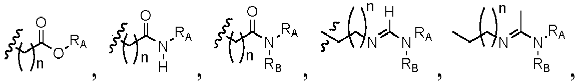 or 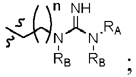
R₅ is:
H;
(C₁-C₆)alkyl, optionally substituted by one or more groups R₆ which may be the same or different;
(C₂-C₆)alkenyl, optionally substituted by one or more groups which may be the same or different selected from halogen, hydroxy, (C₁-C₆)alkyl, aryl, (CH₂)ₚOR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{c}(CH₂)ₘNR_{A}R_{B}, (CH₂)ₚC(=O)NR_{A}R_{B}, (CH₂)ₚC(=O)OR_{A};
(C₂-C₆)alkynyl, optionally substituted by one or one or more groups which may be the same or different selected from halogen, hydroxy, amino, monoalkylamino and dialkylamino;
(C₃-C₇)cycloalkyl, optionally substituted by one or more groups which may be the same or different selected from halogen, hydroxy, amino, monoalkylamino and dialkylamino;
phenyl or CH₂-phenyl, optionally substituted by one or more groups which may be the same or different selected from halogen, hydroxy, (C₁-C₆)alkyl, (CH₂)ₚOR_{A}, O(CH₂)ₚNR_{A}R_{B}, (CH₂)ₚC(=O)NR_{A}R_{B}, (CH₂)ₚC(=O)OR_{A};
each occurrence of R₆ is independently halogen, hydroxy, aryl, S(C₁-C₆)alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, or NR_{C}(CH₂)ₘNR_{C}(CH₂)ₘNR_{A}R_{B}, wherein said aryl or phenyl is optionally substituted by one or more groups which may be the same or different selected from halogen, hydroxy, (C₁-C₆)alkyl, (CH₂)ₚOR_{A}, (CH₂)ₚNR_{A}R_{B}, (CH₂)ₚC(=O)NR_{A}R_{B} and (CH₂)ₚC(=O)OR_{A};
each occurrence of R_{A} and R_{B} is independently:
hydrogen;
(C₁-C₆)alkyl, optionally substituted by one or more groups R_{D} which may be the same or different;
(C₂-C₆)alkenyl or (C₂-C₆)alkynyl;
(C₃-C₇)cycloalkyl optionally substituted with (C₁-C₆)alkyl;
phenyl optionally substituted with from one to five groups which may be the same or different selected from halogen, -O(C₁-C₆)alkyl, -C(=O)O(C₁-C₆)alkyl, amino, alkylamino and dialkylamino;
or a heterocyclic ring which may be saturated or unsaturated containing five or six ring atoms and from one to three heteroatoms which may be the same or different selected from nitrogen, sulfur and oxygen;
or R_{A} and R_{B}, together with the nitrogen atom to which they are attached, form a saturated or unsaturated heterocyclic ring containing from three to seven ring atoms, which ring may optionally contain another heteroatom selected from the group consisting of nitrogen, oxygen and sulfur and may be optionally substituted by from one to four groups which may be the same or different selected from the group consisting of alkyl, phenyl and benzyl;
each occurrence of R_{C} is independently hydrogen or (C₁-C₆)alkyl;
each occurrence of R_{D} is independently halogen, hydroxy, O(C₁-C₄)alkyl, C(=O)(C₁-C₄)alkyl, C(=O)O(C₁-C₄)alkyl;
each p is independently an integer of 0, 1, 2, 3, 4, or 5; and
each of m, n and q is independently an integer of 1, 2, 3, 4 or 5.

9. The compound of any one of claims 6 to 8, wherein:
R₅ is H, (C₁-C₆)alkyl, (C₂-C₆)alkenyl, phenyl, benzyl, CH₂-S-(C₁-C₆)alkyl, CH₂-O-(C₁-C₆)alkyl, (C₂-C₆)OR_{A}, (C₁-C₆)-monoalkyl amine, (C₁-C₆)-dialkyl amine, or (C₁-C₆)-cyclic amine, in which said phenyl or benzyl is optionally substituted by one to three substitutents selected from (C₁-C₄)alkyl, (C₁-C₄)alkoxy, and halogen; and R_{A} is H, (C₁-C₆)alkyl, phenyl, CH₂-phenyl, (C₁-C₆)alkylOH, (CH₂)ₚO(CH₂)ₘOH, (CH₂)ₚO(CH₂)ₘO(CH₂)ₘOH, (C₁-C₆)alkylO(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl; p is an integer of 0, 1, 2, 3, 4, or 5; and m is an integer of 1, 2, 3, 4 or 5; or
wherein:
R₅ is H, (C₁-C₄)alkyl, (C₂-C₄)alkenyl, phenyl, benzyl, CH₂-S-(C₁-C₄)alkyl, CH₂-O-(C₁-C₄)alkyl, (CH₂)₂OH, or (CH₂)₂O(C₁-C₄)alkyl.

10. The compound of any one of claims 6 to 9, wherein each occurrence R_{A} and R_{B} is independently H, (C₁-C₆)alkyl, phenyl, CH₂-phenyl, (C₁-C₆)alkyl-OH, (CH₂)ₚO(CH₂)ₘOH, or (CH₂)ₚO(CH₂)ₘO(CH₂)ₘOH, (C₁-C₆)alkyl-O-(C₁-C₄)alkyl, (CH₂)ₚO(CH₂)ₘO(C₁-C₄)alkyl, or (CH₂)ₚO(CH₂)ₘO(CH₂)ₘO(C₁-C₄)alkyl; or
wherein each occurrence R_{A} and R_{B} is independently H or (C₁-C₆)alkyl; or
wherein R_{A} and R_{B}, together with the nitrogen atom to which they are attached, form a heterocycle selected from 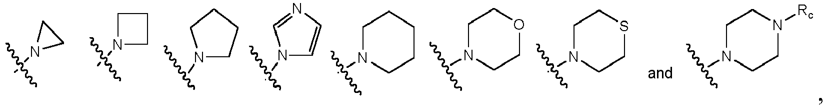 in which R_{C} is H, Me, Et, n-Pr, i-Pr, n-Bu, i-Bu, t-Bu, CH₂CMe₃, Ph, CH₂Ph, CH₂CH₂OH, or CH₂CH₂O(C₁-C₄)alkyl.

11. A compound of claim 1, selected from the group consisting of:
[(S)-(2-(N,N-Diethylamino)ethylthio)methyl-Sar]-3-[(γ-hydroxy)-N-MeLeu]-4-cyclosporin,
[(S)-(2-(N-Ethyl-N-isopropylamino)ethylthio)methyl-Sar]-3-[(γ-hydroxy)-N-MeLeu]-4-cyclosporin,
[(S)-(2-(N-Isobutylamino)ethylthio)methyl-Sar]-3-[(γ-hydroxy)-N-MeLeu]-4-cyclosporin,
[(S)-(2-(N-Ethyl-N-Isobutylamino)ethylthio)methyl-Sar]-3-[(γ-hydroxy)-N-MeLeu]-4-cyclosporin,
[(S)-(2-(N-Isobutyl-N-methylamino)ethylthio)methyl-Sar]-3-[(γ-hydroxy)-N-MeLeu]-4-cyclosporin,
[(S)-(2-(N-Neopentylamino)ethylthio)methyl-Sar]-3-[(γ-hydroxy)-N-MeLeu]-4-cyclosporin,
[(S)-(2-(N-Methyl-N-Neopentylamino)ethylthio)methyl-Sar]-3-[(γ-hydroxy)-N-MeLeu]-4-cyclosporin,
[(S)-(2-(N-Ethyl-N-neopentylamino)ethylthio)methyl-Sar]-3-[(γ-hydroxy)-N-MeLeu]-4-cyclosporin,
[(S)-(2-(N-Piperidinyl)ethylthio)methyl-Sar]-3-[(γ-hydroxy)-N-MeLeu]-4-cyclosporin,
[(S)-(2-(N-Morpholino)ethylthio)methyl-Sar]-3-[(γ-hydroxy)-N-MeLeu]-4-cyclosporin,
[(S)-(2-(N-Thiomorpholino)ethylthio)methyl-Sar]-3-[(γ-hydroxy)-N-MeLeu]-4-cyclosporin,
[(S)-(2-(4-Methyl-N-piperazinyl)ethylthio)methyl-Sar]-3-[(γ-hydroxy)-N-MeLeu]-4-cyclosporin,
[(S)-(3-(N,N-Diethylamino)propylthio)methyl-Sar]-3-[(γ-hydroxy)-N-MeLeu]-4-cyclosporin,
[(S)-(3-(N-Ethyl-N-isopropylamino)propylthio)methyl-Sar]-3-[(γ-hydroxy)-N-MeLeu]-4-cyclosporin,
[(S)-(3-(N-Neopentylamino)propylthio)methyl-Sar]-3-[(γ-hydroxy)-N-MeLeu]-4-cyclosporin,
[(S)-(3-(N-Pyrrolidinyl)propylthio)methyl-Sar]-3-[(γ-hydroxy)-N-MeLeu]-4-cyclosporin,
[(S)-(3-(N-Piperidinyl)propylthio)methyl-Sar]-3-[(γ-hydroxy)-N-MeLeu]-4-cyclosporin,
[(S)-(3-(N-Morpholino)propylthio)methyl-Sar]-3-[(γ-hydroxy)-N-MeLeu]-4-cyclosporin,
[(S)-(3-(N-Thiomorpholino)propylthio)methyl-Sar]-3-[(γ-hydroxy)-N-MeLeu]-4-cyclosporin,
[(S)-(3-(4-Methyl-N-piperazinyl)propylthio)methyl-Sar]-3-[(γ-hydroxy)-N-MeLeu]-4-cyclosporin,
[(S)-(4-(N,N-Diethylamino)butylthio)methyl-Sar]-3-[(γ-hydroxy)-N-MeLeu]-4-cyclosporin,
[(S)-(4-(N-Ethyl-N-isopropylamino)butylthio)methyl-Sar]-3-[(γ-hydroxy)-N-MeLeu]-4-cyclosporin,
[(S)-(4-(N-Isobutylamino)butylthio)methyl-Sar]-3-[(γ-hydroxy)-N-MeLeu]-4-cyclosporin,
[(S)-(4-(N-Isobutyl-N-methylamino)butylthio)methyl-Sar]-3-[(γ-hydroxy)-N-MeLeu]-4-cyclosporin,
[(S)-(4-(N-Ethyl-N-isobutylamino)butylthio)methyl-Sar]-3-[(γ-hydroxy)-N-MeLeu]-4-cyclosporin,
[(S)-(4-(N-Neopentylamino)butylthio)methyl-Sar]-3-[(γ-hydroxy)-N-MeLeu]-4-cyclosporin,
[(S)-(4-(N-Methyl-N-neopentylamino)butylthio)methyl-Sar]-3-[(γ-hydroxy)-N-MeLeu]-4-cyclosporin,
[(S)-(4-(N-Piperidinyl)butylthio)methyl-Sar]-3-[(γ-hydroxy)-N-MeLeu]-4-cyclosporin,
[(S)-(4-(N-Morpholino)butylthio)methyl-Sar]-3-[(γ-hydroxy)-N-MeLeu]-4-cyclosporin,
[(S)-(4-(N-Thiomorpholino)butylthio)methyl-Sar]-3-[(γ-hydroxy)-N-MeLeu]-4-cyclosporin,
[(S)-(4-(4-Methyl-N-piperazinyl)butylthio)methyl-Sar]-3-[(γ-hydroxy)-N-MeLeu]-4-cyclosporin,
[(R)-(3-(N-Morphlino)propoxy)methyl-Sar]-3-[(γ-hydroxy)-N-MeLeu]-4-cyclosporin,
[(S)-(2-(N,N-Diethylamino)ethylthio)methyl-Sar]-3-[(γ-methoxy)-N-MeLeu]-4-cyclosporin,
[(S)-(2-(N-Pyrrolidinyl)ethylthio)methyl-Sar]-3-[(γ-methoxy)-N-MeLeu]-4-cyclosporin,
[(S)-(3-(N,N-Dimethylamino)propylthio)methyl-Sar]-3-[(γ-methoxy)-N-MeLeu]-4-cyclosporin,
[(S)-(3-(N,N-Diethylamino)propylthio)methyl-Sar]-3-[(γ-methoxy)-N-MeLeu]-4-cyclosporin,
[(S)-(3-(N-Piperidinyl)propylthio)methyl-Sar]-3-[(γ-methoxy)-N-MeLeu]-4-cyclosporin,
[(S)-(4-(N-Piperidinyl)butylthio)methyl-Sar]-3-[(γ-methoxy)-N-MeLeu]-4-cyclosporin,
[(R)-(2-(N,N-Diethylamino)ethoxy)methyl-Sar]-3-[(γ-methoxy)-N-MeLeu]-4-cyclosporin, and
[(R)-(3-(N,N-Diethylamino)propoxy)methyl-Sar]-3-[(γ-methoxy)-N-MeLeu]-4-cyclo sporin.

12. A compound of claim 1, having the structure of: 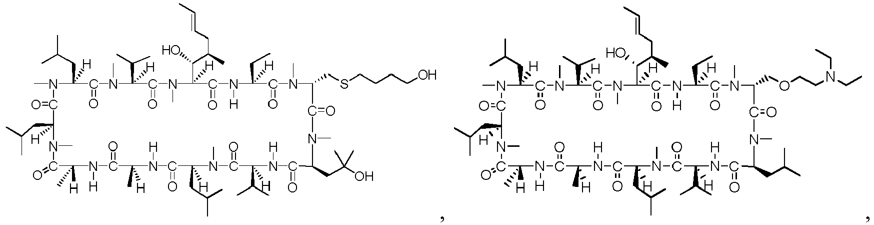 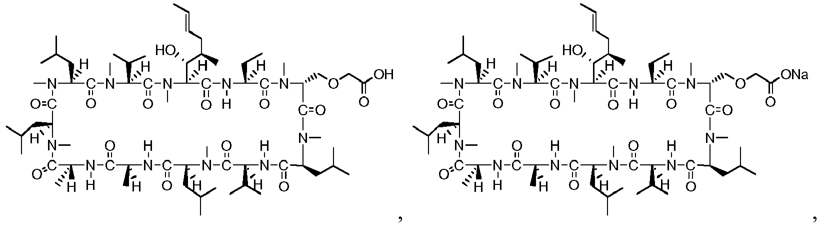 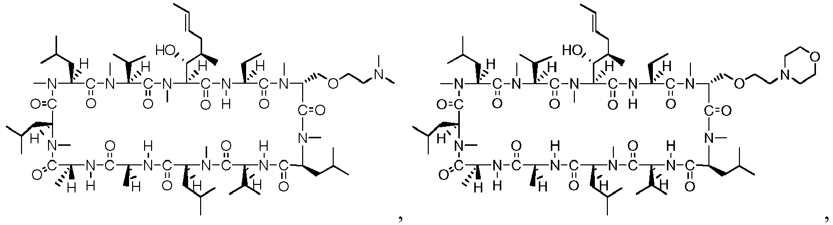 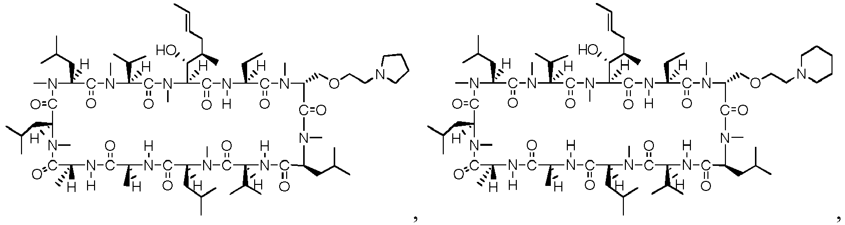 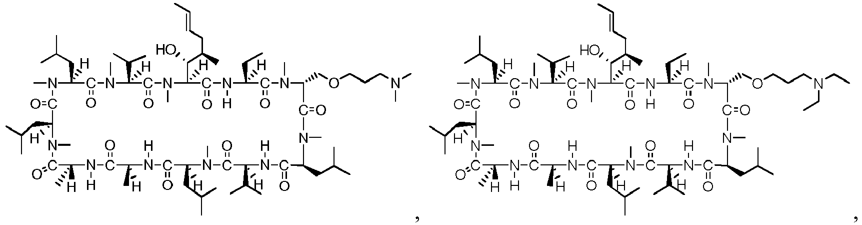 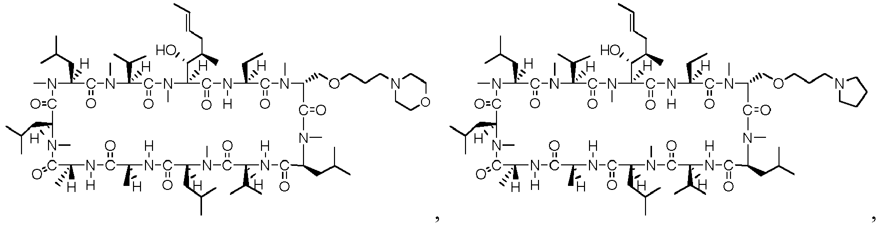 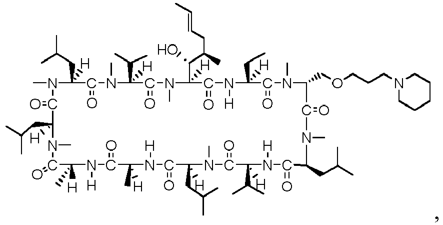 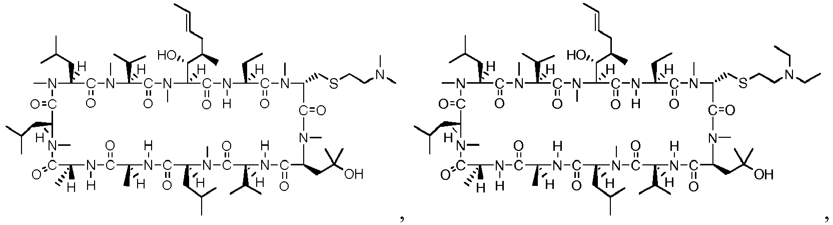 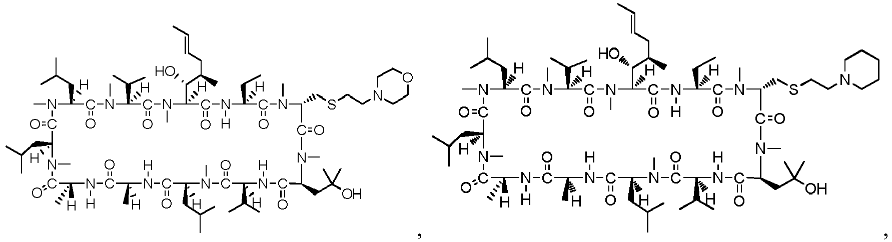 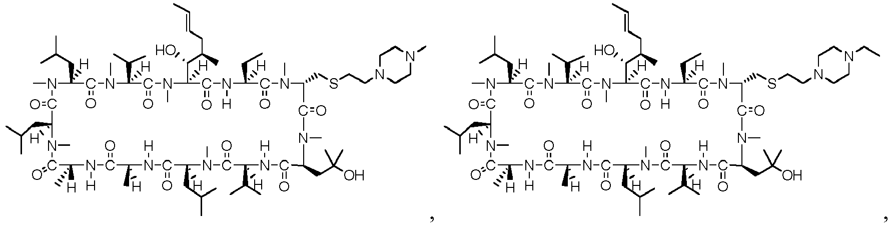 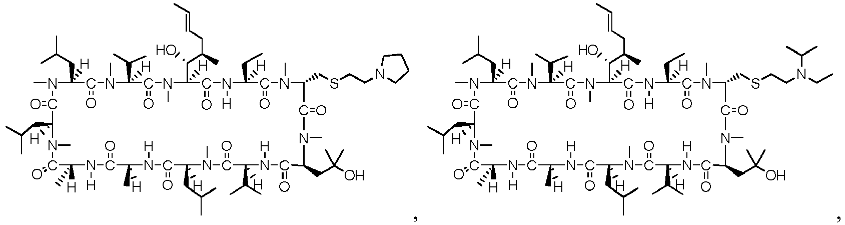 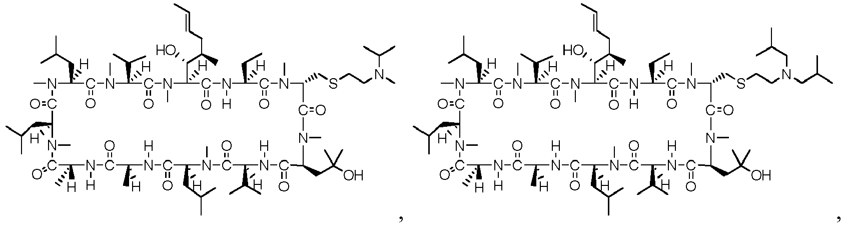 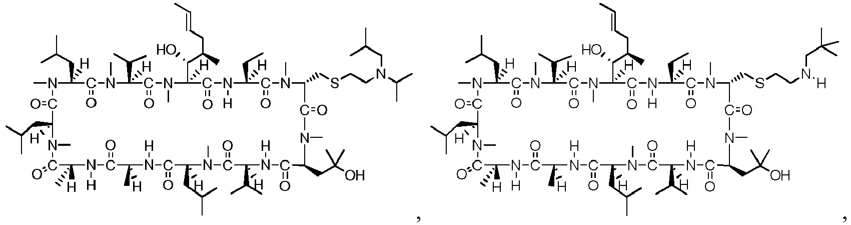 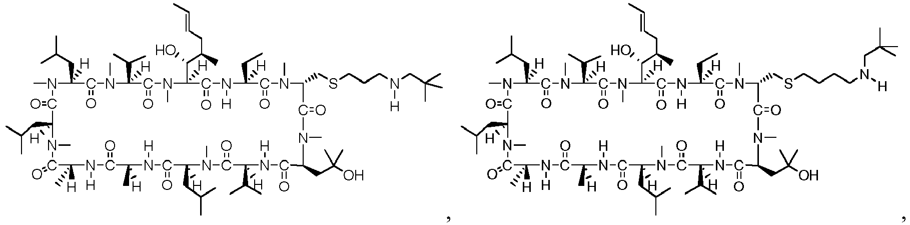 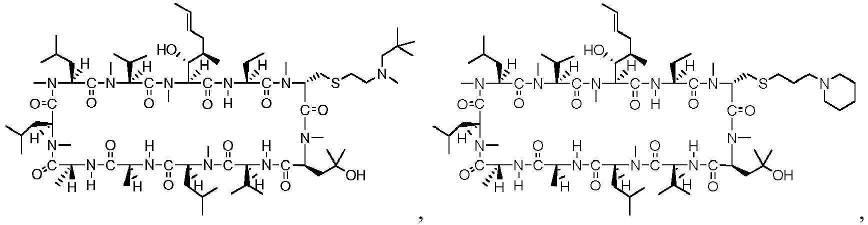 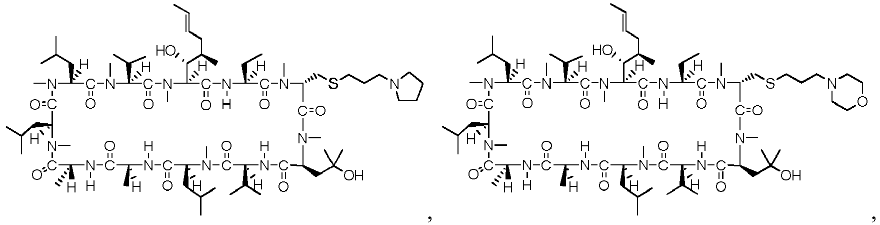 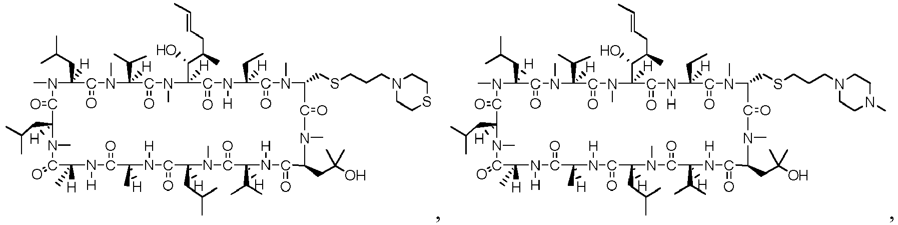 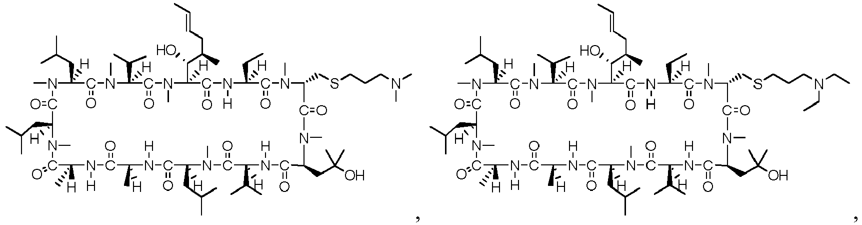 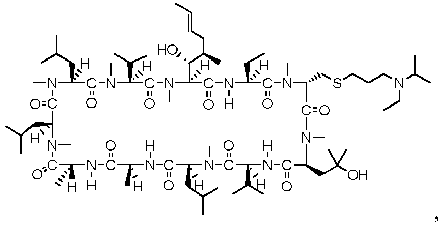 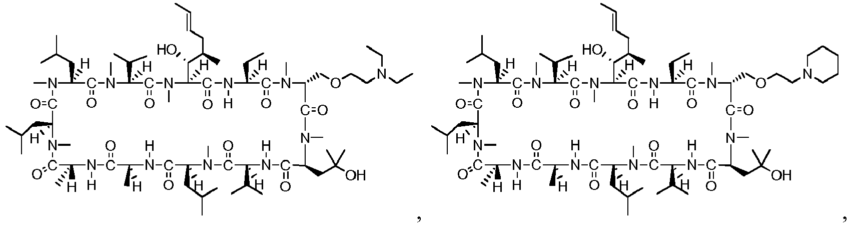 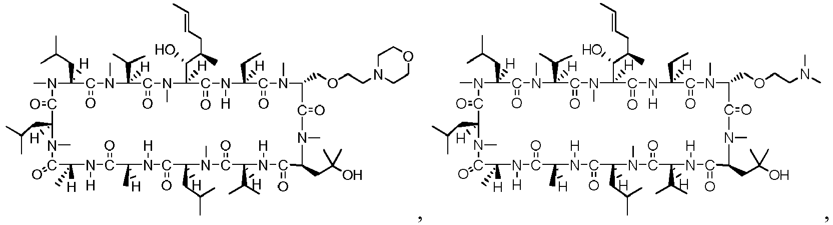 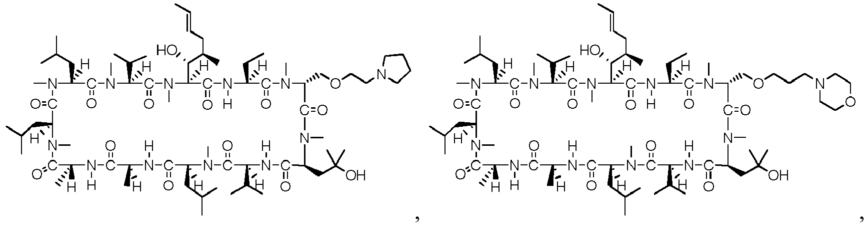 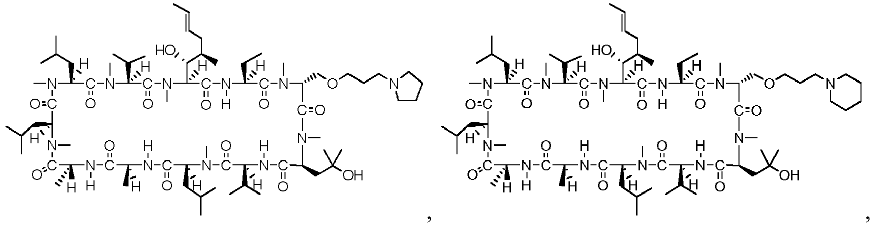 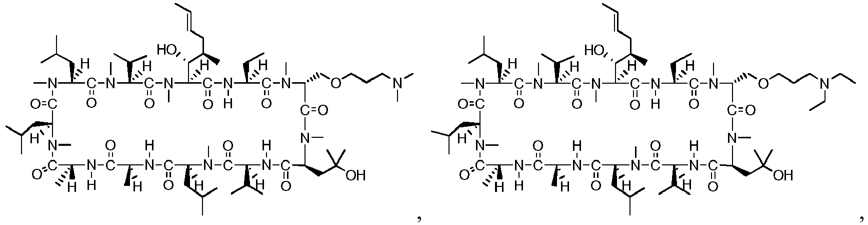 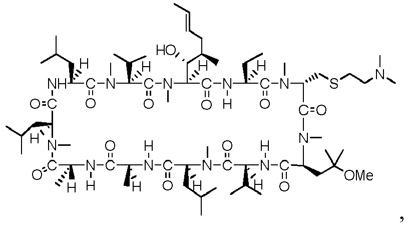 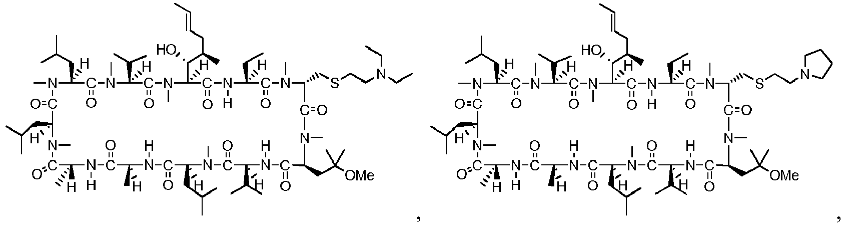 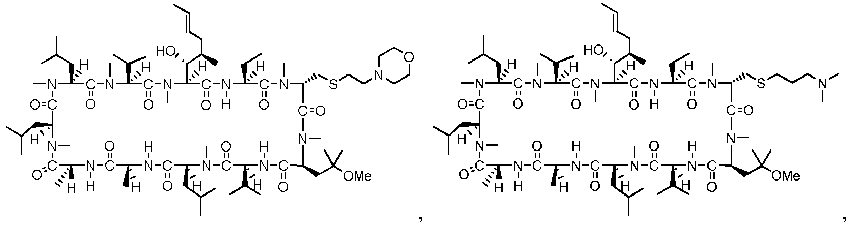 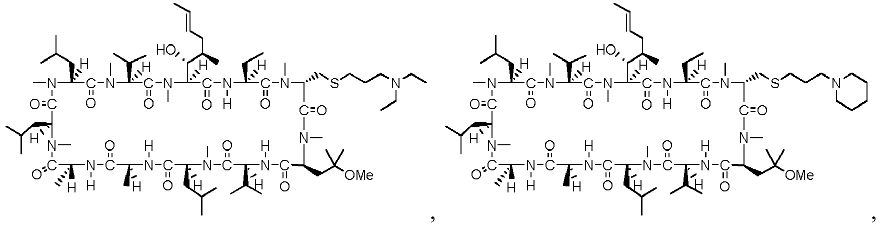 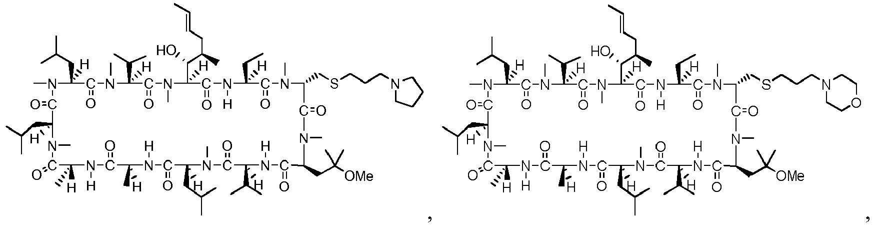 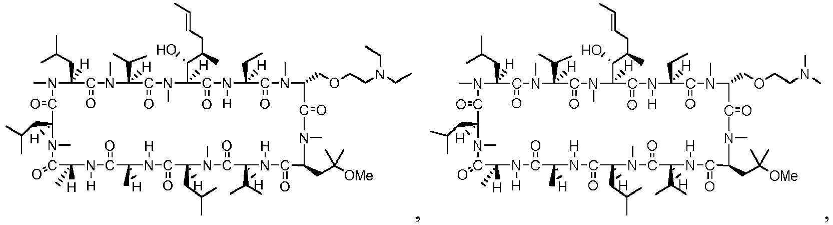 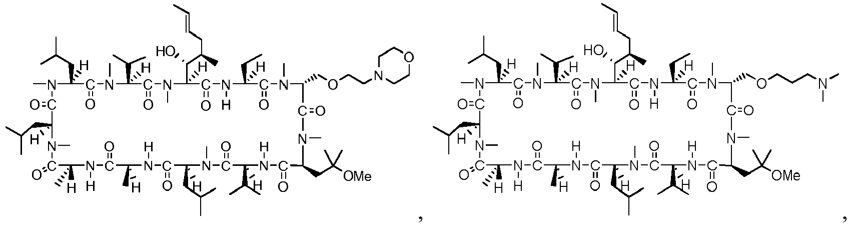 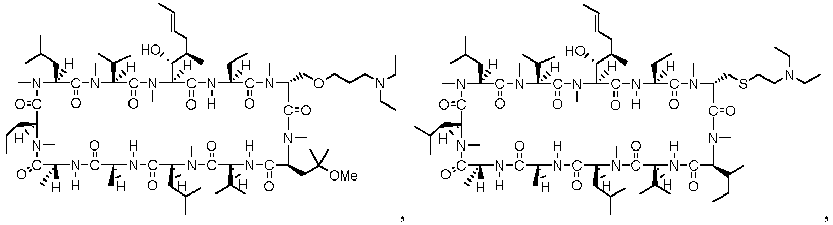 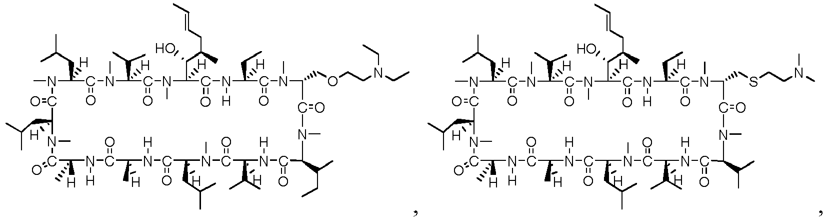 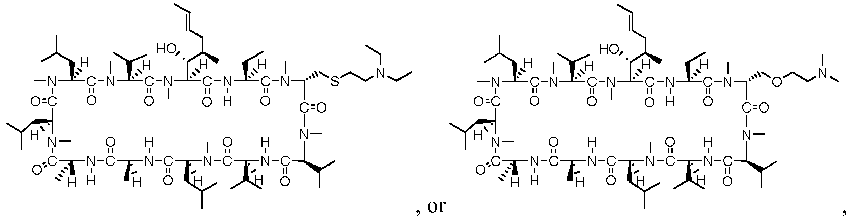 or a pharmaceutically acceptable salt thereof.

13. A compound of claim 1, having the following structure: 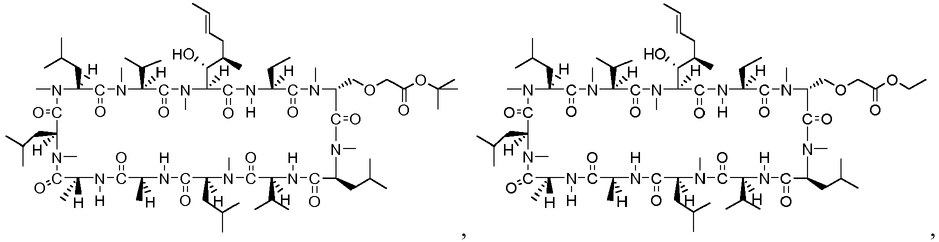 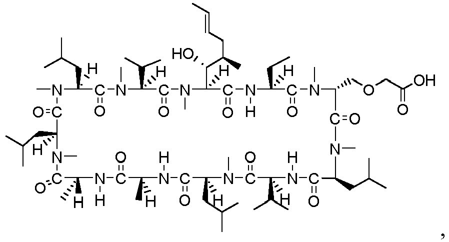 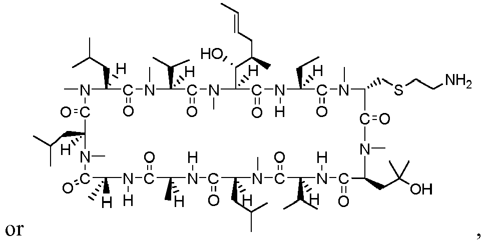 or a pharmaceutically acceptable salt thereof.

14. A pharmaceutical composition comprising at least one compound according to any one of claims 1-10 and a pharmaceutically-acceptable carrier or diluent.

15. A compound of any of claims 1-10 for use in treating or preventing hepatitis B virus infection, hepatitis C virus infection, or HIV infection in a mammalian species.

## Patentansprüche

1. Verbindung der Formel (I): 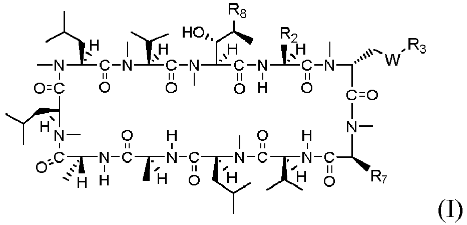 oder pharmazeutisch unbedenkliches Salz davon, wobei Folgendes gilt:
R₈ ist n-Butyl, (*E*)-But-2-enyl, oder 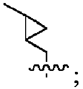
R₂ ist Ethyl, 1-Hydroxyethyl, Isopropyl oder n-Propyl;
W ist O oder S;
R₃ ist:
(C₁-C₆)Alkyl, optional ersetzt durch eine oder mehrere Gruppen R₄, die gleich oder verschieden sein können;
(C₂-C₆)Alkenyl, optional ersetzt durch eine oder mehrere Gruppen, die gleich oder verschieden sein können, ausgewählt aus Halogen, Hydroxy, Amino, Monoalkylamino und Dialkylamino;
(C₂-C₆)Alkinyl, optional ersetzt durch eine oder eine oder mehrere Gruppen, die gleich oder verschieden sein können, ausgewählt aus Halogen, Hydroxy, Amino, Monoalkylamino und Dialkylamino;
(C₃-C₇)Cycloalkyl, optional ersetzt durch eine oder mehrere Gruppen, die gleich oder verschieden sein können, ausgewählt aus Halogen, Hydroxy, Amino, Monoalkylamino und Dialkylamino;
Phenyl oder CH₂-Phenyl, optional ersetzt durch eine oder mehrere Gruppen, die gleich oder verschieden sein können, ausgewählt aus Halogen, Hydroxy, (C₁-C₆)Alkyl;
R₇ ist 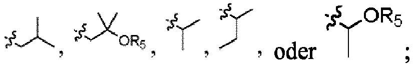
R₅ ist:
H;
(C₁-C₆)Alkyl, optional ersetzt durch eine oder mehrere Gruppen R₆, die gleich oder verschieden sein können;
(C₂-C₆)Alkenyl, optional ersetzt durch eine oder mehrere Gruppen, die gleich oder verschieden sein können, ausgewählt aus Hydroxy, (C₁-C₆)Alkyl, Aryl, (CH₂)_{P}OR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)_{P}C(=O)NR_{A}R_{B}, (CH₂)_{P}C(=O)OR_{A};
(C₂-C₆)Alkinyl, optional ersetzt durch eine oder eine oder mehrere Gruppen, die gleich oder verschieden sein können, ausgewählt aus Halogen, Hydroxy, Amino, Monoalkylamino und Dialkylamino;
(C₃-C₇)Cycloalkyl, optional ersetzt durch eine oder mehrere Gruppen, die gleich oder verschieden sein können, ausgewählt aus Halogen, Hydroxy, Amino, Monoalkylamino und Dialkylamino;
Phenyl oder CH₂-Phenyl, optional ersetzt durch eine oder mehrere Gruppen, die gleich oder verschieden sein können, ausgewählt aus Halogen, Hydroxy, (C₁-C₆)Alkyl, (CH₂)_{P}OR_{A}, (CH₂)_{P}NR_{A}R_{B}, (CH₂)_{P}C(=O)NR_{A}R_{B}, (CH₂)_{P}C(=O)OR_{A};
jedes Auftreten von R₄ ist unabhängig Halogen, Hydroxy, Aryl, O(CH₂)ₘOR_{A}, O(CH₂)ₘO(CH₂)ₘOR_{A}, C(=O)(C₁-C₆)Alkyl, C(=O)OR_{A}, C(=O)NR_{A}R_{B}, -NR_{A}R_{B}, -NR_{C}CH₂(CH₂)_{P}NR_{A}P_{B}, NR_{C}[CH₂(CH₂)ₚNR_{A}]ₘCH₂(CH₂)ₙNR_{A}R_{B}, O[CH₂(CH₂)ₚO]ₘCH₂(CH₂)ₙOR_{A}, OCH₂(CH₂)_{P}NR_{A}R_{B} oder O[CH₂(CH₂)ₚO]ₘCH₂(CH₂)ₙNR_{A}R_{B};
jedes Auftreten von R₆ ist unabhängig Halogen, Hydroxy, Aryl, S(C₁-C₆)Alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B} oder NR_{C}(CH₂)ₘNR_{C}(CH₂)ₘNR_{A}R_{B}, wobei das Aryl oder Phenyl optional durch eine oder mehrere Gruppen ersetzt wird, die gleich oder verschieden sein können, ausgewählt aus Halogen, Hydroxy, (C₁-C₆)Alkyl, (CH₂)_{P}OR_{A}, (CH₂)_{P}NR_{A}R_{B}, (CH₂)_{P}C(=O)NR_{A}R_{B} und (CH₂)ₚC(=O)OR_{A};
jedes Auftreten von R_{A} und R_{B} ist unabhängig:
Wasserstoff;
(C₁-C₆)Alkyl, optional ersetzt durch eine oder mehrere Gruppen R_{D}, die gleich oder verschieden sein können;
(C₂-C₆)Alkenyl oder (C₂-C₆)Alkinyl;
(C₃-C₇)Cycloalkyl optional ersetzt durch (C₁-C₆)Alkyl;
Phenyl optional ersetzt durch eine bis fünf Gruppen, die gleich oder verschieden sein können, ausgewählt aus Halogen, -O(C₁-C₆)Alkyl, -C(=O)O(C₁-C₆)Alkyl, Amino, Alkylamino und Dialkylamino;
oder ein heterozyklischer Ring, der gesättigt oder ungesättigt sein kann, der fünf oder sechs Ringatome und einen bis drei Heteroatome enthält, die gleich oder verschieden sein können, ausgewählt aus Stickstoff, Schwefel und Sauerstoff;
oder R_{A} und R_{B} bilden zusammen mit dem Stickstoffatom, an das sie gebunden sind, einen gesättigten oder ungesättigten heterozyklischen Ring, der drei bis sieben Ringatome enthält, wobei der Ring optional ein weiteres Heteroatom enthalten kann, ausgewählt aus der Gruppe, die aus Stickstoff, Sauerstoff und Schwefel besteht, und optional durch eine bis vier Gruppen ersetzt werden kann, die gleich oder verschieden sein können, ausgewählt aus der Gruppe, die aus Alkyl, Phenyl und Benzyl besteht;
jedes Auftreten von R_{C} ist unabhängig Wasserstoff oder (C₁-C₆)Alkyl;
p ist eine ganze Zahl aus 0, 1, 2, 3, 4, oder 5; und
m ist eine ganze Zahl aus 1, 2, 3, 4 oder 5.

2. Verbindung nach Anspruch 1, mit der Struktur der Formeln (II) bis (V): 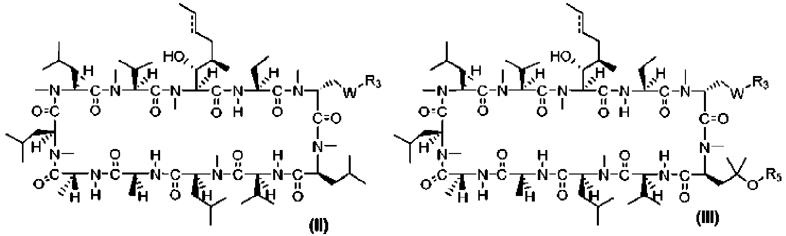 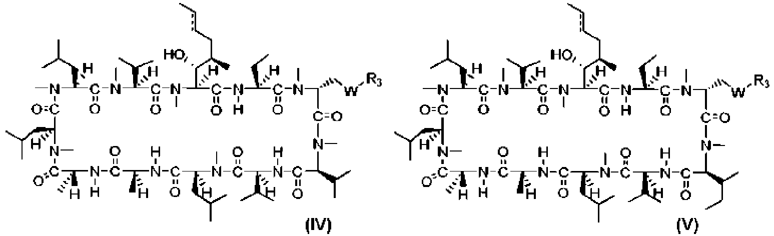 oder pharmazeutisch unbedenkliches Salz davon, wobei Folgendes gilt: 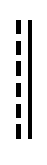
stellt eine Einfachbindung oder Doppelbindung dar;
jedes W ist unabhängig O oder S;
jedes R₃ ist unabhängig:
(C₁-C₆)Alkyl, optional ersetzt durch eine oder mehrere Gruppen R₄, die gleich oder verschieden sein können;
(C₂-C₆)Alkenyl, optional ersetzt durch eine oder mehrere Gruppen, die gleich oder verschieden sein können, ausgewählt aus Halogen, Hydroxy, (C₁-C₆)Alkyl, Aryl, (CH₂)ₚOR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)_{P}C(=O)OR_{A};
(C₂-C₆)Alkinyl, optional ersetzt durch eine oder eine oder mehrere Gruppen, die gleich oder verschieden sein können, ausgewählt aus Halogen, Hydroxy, Amino, Monoalkylamino und Dialkylamino;
(C₃-C₇)Cycloalkyl, optional ersetzt durch eine oder mehrere Gruppen, die gleich oder verschieden sein können, ausgewählt aus Halogen, Hydroxy, Amino, Monoalkylamino und Dialkylamino;
Phenyl oder CH₂-Phenyl, optional ersetzt durch eine oder mehrere Gruppen, die gleich oder verschieden sein können, ausgewählt aus Halogen, Hydroxy, (C₁-C₆)Alkyl;
jedes R₅ ist unabhängig:
H;
(C₁-C₆)Alkyl, optional ersetzt durch eine oder mehrere Gruppen R₆, die gleich oder verschieden sein können;
(C₂-C₆)Alkenyl, optional ersetzt durch eine oder mehrere Gruppen, die gleich oder verschieden sein können, ausgewählt aus Halogen, Hydroxy, (C₁-C₆)Alkyl, Aryl, (CH₂)_{P}OR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)_{P}NR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)_{P}C(=O)OR_{A};
(C₂-C₆)Alkinyl, optional ersetzt durch eine oder eine oder mehrere Gruppen, die gleich oder verschieden sein können, ausgewählt aus Halogen, Hydroxy, Amino, Monoalkylamino und Dialkylamino;
(C₃-C₇)Cycloalkyl, optional ersetzt durch eine oder mehrere Gruppen, die gleich oder verschieden sein können, ausgewählt aus Halogen, Hydroxy, Amino, Monoalkylamino und Dialkylamino;
Phenyl oder CH2-Phenyl, optional ersetzt durch eine oder mehrere Gruppen, die gleich oder verschieden sein können, ausgewählt aus Halogen, Hydroxy, (C₁-C₆)Alkyl, (CH₂)_{P}OR_{A}, (CH₂)_{P}NR_{A}R_{B}, (CH₂)_{P}C(=O)NR_{A}R_{B}, (CH₂)_{P}C(=O)OR_{A};
jedes Auftreten von R₄ ist unabhängig Halogen, Hydroxy, Aryl, O(CH₂)ₘOR_{A}, O(CH₂)ₘO(CH₂)ₘOR_{A}, C(=O)(C₁-C₆)Alkyl C(=O)OR_{A}, C(=O)NR_{A}R_{B}, -NR_{A}R_{B}, -NR_{C}CH₂(CH₂)_{P}NR_{A}R_{B}, NR_{C}[CH₂(CH₂)pNR_{A}]ₘCH₂(CH₂)ₙNR_{A}R_{B}, O[CH₂(CH₂)ₚO]ₘCH₂(CH₂)ₙOR_{A}, OCH₂(CH₂)_{P}NR_{A}R_{B}, oder O[CH₂(CH₂)ₚO]ₘCH₂(CH₂)ₙNR_{A}R_{B};
jedes Auftreten von R₆ ist unabhängig Halogen, Hydroxy, Aryl, S(C₁-C₆)Alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B} oder NR_{C}(CH₂)ₘNR_{C}(CH₂)ₘNR_{A}R_{B}, wobei das Aryl oder Phenyl optional durch eine oder mehrere Gruppen ersetzt wird, die gleich oder verschieden sein können, ausgewählt aus Halogen, Hydroxy, (C₁-C₆)Alkyl, (CH₂)_{P}OR_{A}, (CH₂)_{P}NR_{A}R_{B}, (CH₂)_{P}C(=O)NR_{A}R_{B} und (CH₂)ₚC(=O)OR_{A};
jedes Auftreten von R_{A} und R_{B} ist unabhängig:
Wasserstoff;
(C₁-C₆)Alkyl, optional ersetzt durch eine oder mehrere Gruppen R_{D}, die gleich oder verschieden sein können;
(C₂-C₆)Alkenyl oder (C₂-C₆)Alkinyl;
(C₃-C₇)Cycloalkyl optional ersetzt durch (C₁-C₆)Alkyl;
Phenyl optional ersetzt durch eine bis fünf Gruppen, die gleich oder verschieden sein können, ausgewählt aus Halogen, -O(C₁-C₆)Alkyl, -C(=O)O(C₁-C₆)Alkyl, Amino, Alkylamino und Dialkylamino;
oder ein heterozyklischer Ring, der gesättigt oder ungesättigt sein kann, der fünf oder sechs Ringatome und einen bis drei Heteroatome enthält, die gleich oder verschieden sein können, ausgewählt aus Stickstoff, Schwefel und Sauerstoff;
oder R_{A} und R_{B} bilden zusammen mit dem Stickstoffatom, an das sie gebunden sind, einen gesättigten oder ungesättigten heterozyklischen Ring, der drei bis sieben Ringatome enthält, wobei der Ring optional ein weiteres Heteroatom enthalten kann, ausgewählt aus der Gruppe, die aus Stickstoff, Sauerstoff und Schwefel besteht, und optional durch eine bis vier Gruppen ersetzt werden kann, die gleich oder verschieden sein können, ausgewählt aus der Gruppe, die aus Alkyl, Phenyl und Benzyl besteht;
oder R_{A} und R_{B} bilden zusammen mit dem Stickstoffatom, an das sie gebunden sind, -N=CH-NR_{F}R_{F}, -N=CMe-NR_{F}R_{F} oder -NR_{F}C(=NH)NR_{F}R_{F};
jedes Auftreten von R_{C} ist unabhängig Wasserstoff oder (C₁-C₆)Alkyl;
jedes Auftreten von R_{D} ist unabhängig Halogen, Hydroxy, O(C₁-C₄)Alkyl, C(=O)(C₁-C₄)Alkyl, C(=O)O(C₁-C₆)Alkyl;
jedes Auftreten von R_{F} und R_{F'} ist unabhängig Wasserstoff, (C₁-C₆)Alkyl, Phenyl, Benzyl oder R_{F} und R_{F'} bilden zusammen mit dem Stickstoffatom, an das sie gebunden sind, einen gesättigten oder ungesättigten heterozyklischen Ring, der drei bis sieben Ringatome enthält, wobei der Ring optional ein weiteres Heteroatom enthalten kann, ausgewählt aus der Gruppe, die aus Stickstoff, Sauerstoff und Schwefel besteht, und optional durch eine bis vier Gruppen ersetzt werden kann, die gleich oder verschieden sein können, ausgewählt aus der Gruppe, die aus Alkyl, Phenyl und Benzyl besteht;
p ist eine ganze Zahl aus 0, 1, 2, 3, 4, 5 oder 6;
m ist eine ganze Zahl aus 1, 2, 3, 4, 5 oder 6; und
n ist eine ganze Zahl aus 1, 2, 3, 4, 5 oder 6.

3. Verbindung nach Anspruch 1 oder Anspruch 2, wobei R₃ Methyl, Ethyl, n-Propyl, i-Propyl, n-Butyl, i-Butyl, t-Butyl, CH₂CMe₃, Phenyl, CH₂-Phenyl, 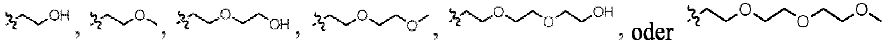 ist; oder
wobei R3 -(CH₂)ₙNR_{A}R_{B} ist, wobei n eine ganze Zahl aus 1, 2, 3, 4, 5 oder 6 ist; und wobei jedes Auftreten von R_{A} und R_{B} unabhängig Wasserstoff ist; (C₁-C₄)Alkyl, optional ersetzt durch eine oder mehrere Gruppen R_{D}, die gleich oder verschieden sein können, wobei jedes Auftreten von R_{D} unabhängig Halogen, Hydroxy, O(C₁-C₄)Alkyl, C(=O)(C₁-C₄)Alkyl, C(=O)O(C₁-C₄)Alkyl ist; oder wobei R_{A} und R_{B} zusammen mit dem Stickstoffatom, an das sie gebunden sind, einen gesättigten oder ungesättigten heterozyklischen Ring bilden, der drei bis sieben Ringatome enthält, wobei der Ring optional ein weiteres Heteroatom enthalten kann, ausgewählt aus der Gruppe, die aus Stickstoff, Sauerstoff und Schwefel besteht, und optional durch eine bis vier Gruppen ersetzt werden kann, die gleich oder verschieden sein können, ausgewählt aus (C₁-C₄)Alkyl, Phenyl und Benzyl; oder
wobei R₃ -(CH₂)ₙNR_{A}R_{B} ist, wobei n eine ganze Zahl aus 1, 2, 3, 4, 5 oder 6 ist; und wobei R_{A} und R_{B} zusammen mit dem Stickstoffatom, an das sie gebunden sind, einen gesättigten oder ungesättigten heterozyklischen Ring bilden, der drei bis sieben Ringatome enthält, wobei der Ring optional ein weiteres Heteroatom enthalten kann, ausgewählt aus Stickstoff, Sauerstoff und Schwefel, und optional durch eine bis vier Gruppen ersetzt werden kann, die gleich oder verschieden sein können, ausgewählt aus (C₁-C₄)Alkyl, Phenyl und Benzyl; oder
wobei R₃ 2-Aminoethyl, 2-Aminobutyl, 3-Aminobutyl, 2-Monoalkylaminoethyl, 2-Monoalkylaminobutyl, 3-Monoalkylaminobutyl, 2-Dialkylaminoethyl, 2-Dialkylaminobutyl, oder 3-Dialkylaminobutyl ist, wobei das Alkyl (C₁-C₄)Alkyl ist; oder
wobei R₃ Dimethylaminoethyl, Diethylaminoethyl, Methylethylaminoethyl, Methyl-iso-butylaminoethyl, Ethyl-iso-butylaminoethyl, Methyl-tert-butylaminoethyl oder Ethyl-tert-butylaminoethyl ist; oder wobei R₃ Folgendes ist: 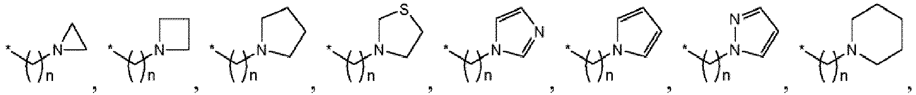 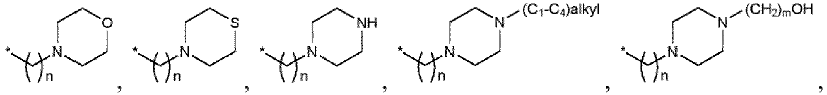 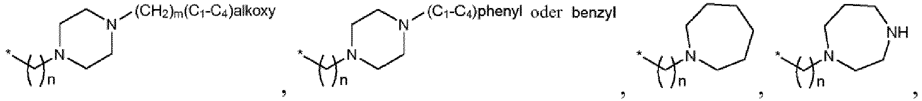 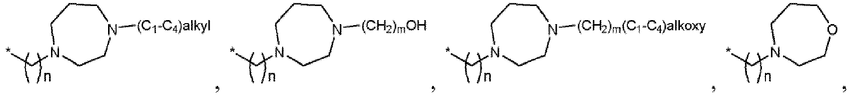 oder 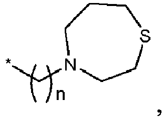 wobei n eine ganze Zahl aus 2, 3, 4, 5, oder 6 ist und m eine ganze Zahl aus 2, 3 oder 4 ist.

4. Verbindung nach einem der Ansprüche 1-3, wobei R₅ H, (C₁-C₆)Alkyl, (C₂-C₆)Alkenyl, Phenyl, Benzyl, CH₂-S-(C₁-C₆)Alkyl, CH₂-O-(C₁-C₆)Alkyl, (C₂-C₆)OR_{A}, (C₁-C₆)-Monoalkylamin, (C₁-C₆)-Dialkylamin oder (C₁-C₆)-zyklisches Amin ist, wobei das Phenyl oder Benzyl optional durch einen bis drei Ersatzstoffe ersetzt wird, ausgewählt aus (C₁-C₄)Alkyl, (C₁-C₄)Alkoxy und Halogen; und R_{A} H, (C₁-C₆)Alkyl, Phenyl, CH₂-Phenyl, (C₁-C₆)Alkyl-OH, (CH₂)ₚO(CH₂)ₘOH, (CH₂)ₚO(CH₂)ₘO(CH₂)ₘOH, (C₁-C₆)Alkyl-O-(C₁-C₄)Alkyl, (CH₂)ₚO(CH₂)ₘO(C₁-C₄)Alkyl oder (CH₂)ₚO(CH₂)ₘO(CH₂)ₘO(C₁-C₄)Alkyl ist; wobei p eine ganze Zahl aus 0, 1, 2, 3, 4 oder 5 ist; und m eine ganze Zahl aus 1, 2, 3, 4 oder 5 ist.

5. Verbindung nach einem der Ansprüche 1-4, wobei jedes Auftreten von R_{A} und R_{B} unabhängig H, (C₁-C₆)Alkyl, Phenyl, CH₂-Phenyl, (C₁-C₆)Alkyl-OH, (CH₂)ₚO(CH₂)ₘOH ist, oder (CH₂)ₚO(CH₂)ₘO(CH₂)ₘOH, (C₁-C₆)Alkyl-O-(C₁-C₄)Alkyl, (CH₂)ₚO(CH₂)ₘO(C₁-C₄)Alkyl ist, oder (CH₂)ₚO(CH₂)ₘO(CH₂)ₘO(C₁-C₄)Alkyl ist; oder
wobei R_{A} und R_{B} zusammen mit dem Stickstoffatom, an das sie gebunden sind, einen Heterozyklus bilden, ausgewählt aus 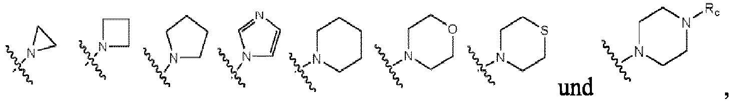 wobei R_{C} H, Me, Et, n-Pr, i-Pr, n-Bu, i-Bu, t-Bu, CH₂CMe₃, Ph, CH₂Ph, CH₂CH₂OH oder CH₂CH₂O(C₁-C₄)Alkyl ist.

6. Verbindung nach Anspruch 1, mit der Struktur der Formeln (IIa)-(Va): 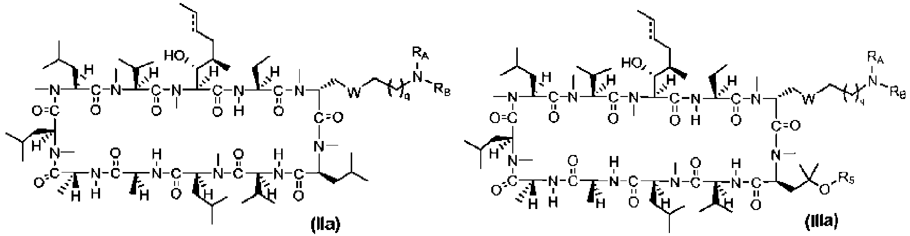 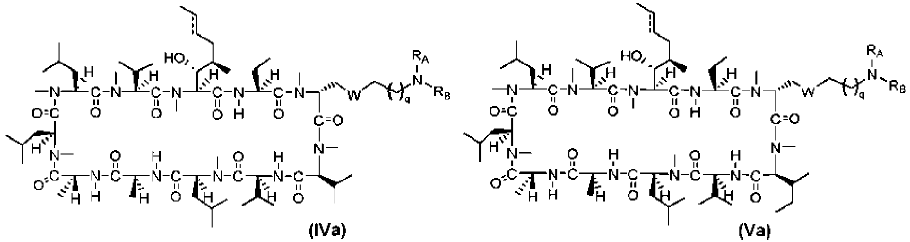 oder ein pharmazeutisch unbedenkliches Salz davon, wobei Folgendes gilt: 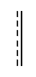
stellt eine Einfachbindung oder Doppelbindung dar; jedes W ist unabhängig O oder S; jedes R₅ ist unabhängig:
H;
(C₁-C₆)Alkyl, optional ersetzt durch eine oder mehrere Gruppen R₆, die gleich oder verschieden sein können;
(C₂-C₆)Alkenyl, optional ersetzt durch eine oder mehrere Gruppen, die gleich oder verschieden sein können, ausgewählt aus Halogen, Hydroxy, (C₁-C₆)Alkyl, Aryl, (CH₂)_{P}OR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)_{P}NR_{A}R_{B}, (CH₂)pNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)ₚC(=O)OR_{A};
(C₂-C₆)Alkinyl, optional ersetzt durch eine oder eine oder mehrere Gruppen, die gleich oder verschieden sein können, ausgewählt aus Halogen, Hydroxy, Amino, Monoalkylamino und Dialkylamino;
(C₃-C₇)Cycloalkyl, optional ersetzt durch eine oder mehrere Gruppen, die gleich oder verschieden sein können, ausgewählt aus Halogen, Hydroxy, Amino, Monoalkylamino und Dialkylamino;
Phenyl oder CH₂-Phenyl, optional ersetzt durch eine oder mehrere Gruppen, die gleich oder verschieden sein können, ausgewählt aus Halogen, Hydroxy, (C₁-C₆)Alkyl, (CH₂)_{P}OR_{A}, (CH₂)_{P}NR_{A}R_{B}, (CH₂)_{P}C(=O)NR_{A}R_{B}, (CH₂)_{P}C(=O)OR_{A};
jedes Auftreten von R₆ ist unabhängig Halogen, Hydroxy, Aryl, S(C₁-C₆)Alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, oder NR_{C}(CH₂)ₘNR_{C}(CH₂)ₘNR_{A}R_{B}, wobei das Aryl oder Phenyl optional durch eine oder mehrere Gruppen ersetzt wird, die gleich oder verschieden sein können, ausgewählt aus Halogen, Hydroxy, (C₁-C₆)Akyl, (CH₂)_{P}OR_{A}, (CH₂)_{P}NR_{A}R_{B}, (CH₂)_{P}C(=O)NR_{A}R_{B} und (CH₂)ₚC(=O)OR_{A};
jedes Auftreten von R_{A} und R_{B} ist unabhängig:
Wasserstoff;
(C₁-C₆)Alkyl, optional ersetzt durch eine oder mehrere Gruppen R_{D}, die gleich oder verschieden sein können;
(C₂-C₆)Alkenyl oder (C₂-C₆)Alkinyl;
(C₃-C₇)Cycloalkyl optional ersetzt durch (C₁-C₆)Alkyl;
Phenyl optional ersetzt durch eine bis fünf Gruppen, die gleich oder verschieden sein können, ausgewählt aus Halogen, -O(C₁-C₆)Alkyl, -C(=O)O(C₁-C₆)Alkyl, Amino, Alkylamino und Dialkylamino;
oder ein heterozyklischer Ring, der gesättigt oder ungesättigt sein kann, der fünf oder sechs Ringatome und einen bis drei Heteroatome enthält, die gleich oder verschieden sein können, ausgewählt aus Stickstoff, Schwefel und Sauerstoff;
oder R_{A} und R_{B} bilden zusammen mit dem Stickstoffatom, an das sie gebunden sind, einen gesättigten oder ungesättigten heterozyklischen Ring, der drei bis sieben Ringatome enthält, wobei der Ring optional ein weiteres Heteroatom enthalten kann, ausgewählt aus der Gruppe, die aus Stickstoff, Sauerstoff und Schwefel besteht, und optional durch eine bis vier Gruppen ersetzt werden kann, die gleich oder verschieden sein können, ausgewählt aus der Gruppe, die aus Alkyl, Phenyl und Benzyl besteht;
jedes Auftreten von R_{C} ist unabhängig Wasserstoff oder (C₁-C₆)Alkyl;
jedes Auftreten von R_{D} ist unabhängig Halogen, Hydroxy, O(C₁-C₄)Alkyl, C(=O)(C₁-C₄)Alkyl, C(=O)O(C₁-C₄)Alkyl;
jedes p ist unabhängig eine ganze Zahl aus 0, 1, 2, 3, 4 oder 5; und
jedes m, n und q ist unabhängig eine ganze Zahl aus 1, 2, 3, 4 oder 5.

7. Verbindung nach Anspruch 1, wobei R7 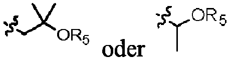 ist.

8. Verbindung nach Anspruch 2, wobei Folgendes gilt: 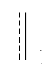
stellt eine Einfachbindung oder Doppelbindung dar; jedes W ist unabhängig O oder S; jedes R₃ ist unabhängig 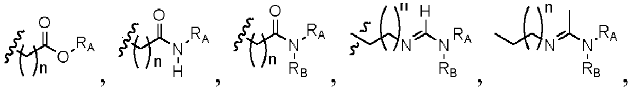 oder 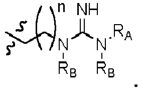
R5 ist:
H;
(C₁-C₆)Alkyl, optional ersetzt durch eine oder mehrere Gruppen R₆, die gleich oder verschieden sein können;
(C₂-C₆)Alkenyl, optional ersetzt durch eine oder mehrere Gruppen, die gleich oder verschieden sein können, ausgewählt aus Halogen, Hydroxy, (C₁-C₆)Alkyl, Aryl, (CH₂)_{P}OR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)ₚC(=O)OR_{A};
(C₂-C₆)Alkinyl, optional ersetzt durch eine oder eine oder mehrere Gruppen, die gleich oder verschieden sein können, ausgewählt aus Halogen, Hydroxy, Amino, Monoalkylamino und Dialkylamino;
(C₃-C₇)Cycloalkyl, optional ersetzt durch eine oder mehrere Gruppen, die gleich oder verschieden sein können, ausgewählt aus Halogen, Hydroxy, Amino, Monoalkylamino und Dialkylamino;
Phenyl oder CH₂-Phenyl, optional ersetzt durch eine oder mehrere Gruppen, die gleich oder verschieden sein können, ausgewählt aus Halogen, Hydroxy, (C₁-C₆)Alkyl, (CH₂)_{P}OR_{A}, (CH₂)_{P}NR_{A}R_{B}, (CH₂)_{P}C(=O)NR_{A}R_{B}, (CH₂)_{P}C(=O)OR_{A};
jedes Auftreten von R₆ ist unabhängig Halogen, Hydroxy, Aryl, S(C₁-C₆)Alkyl, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B}, oder NR_{C}(CH₂)ₘNR_{C}(CH₂)ₘNR_{A}R_{B}, wobei das Aryl oder Phenyl optional durch eine oder mehrere Gruppen ersetzt wird, die gleich oder verschieden sein können, ausgewählt aus Halogen, Hydroxy, (C₁-C₆)Alkyl, (CH₂)_{P}OR_{A}, (CH₂)_{P}NR_{A}R_{B}, (CH₂)_{P}C(=O)NR_{A}R_{B} und (CH₂)_{P}C(=O)OR_{A};
jedes Auftreten von R_{A} und R_{B} ist unabhängig:
Wasserstoff;
(C₁-C₆)Alkyl, optional ersetzt durch eine oder mehrere Gruppen R_{D}, die gleich oder verschieden sein können;
(C₂-C₆)Alkenyl oder (C₂-C₆)Alkinyl;
(C₃-C₇)Cycloalkyl optional ersetzt durch (C₁-C₆)Alkyl;
Phenyl optional ersetzt durch eine bis fünf Gruppen, die gleich oder verschieden sein können, ausgewählt aus Halogen, -O(C₁-C₆)Alkyl, -C(=O)O(C₁-C₆)Alkyl, Amino, Alkylamino und Dialkylamino;
oder ein heterozyklischer Ring, der gesättigt oder ungesättigt sein kann, der fünf oder sechs Ringatome und einen bis drei Heteroatome enthält, die gleich oder verschieden sein können, ausgewählt aus Stickstoff, Schwefel und Sauerstoff;
oder R_{A} und R_{B} bilden zusammen mit dem Stickstoffatom, an das sie gebunden sind, einen gesättigten oder ungesättigten heterozyklischen Ring, der drei bis sieben Ringatome enthält, wobei der Ring optional ein weiteres Heteroatom enthalten kann, ausgewählt aus der Gruppe, die aus Stickstoff, Sauerstoff und Schwefel besteht, und optional durch eine bis vier Gruppen ersetzt werden kann, die gleich oder verschieden sein können, ausgewählt aus der Gruppe, die aus Alkyl, Phenyl und Benzyl besteht;
jedes Auftreten von R_{C} ist unabhängig Wasserstoff oder (C₁-C₆)Alkyl;
jedes Auftreten von R_{D} ist unabhängig Halogen, Hydroxy, O(C₁-C₆)Alkyl, C(=O)(C₁-C₄)Alkyl, C(=O)O(C₁-C₄)Alkyl;
jedes p ist unabhängig eine ganze Zahl aus 0, 1, 2, 3, 4 oder 5; und
jedes m, n und q ist unabhängig eine ganze Zahl aus 1, 2, 3, 4 oder 5.

9. Verbindung nach einem der Ansprüche 6 bis 8, wobei Folgendes gilt:
R₅ ist H, (C₁-C₆)Alkyl, (C₂-C₆)Alkenyl, Phenyl, Benzyl, CH₂-S-(C₁-C₆)Alkyl, CH₂-O-(C₁-C₆)Alkyl, (C₂-C₆)OR_{A}, (C₁-C₆)-Monoalkylamin, (C₁-C₆)-Dialkylamin oder (C₁-C₆)-zyklisches Amin, wobei das Phenyl oder Benzyl optional durch einen bis drei Ersatzstoffe ersetzt wird, ausgewählt aus (C₁-C₄)Alkyl, (C₁-C₄)Alkoxy und Halogen; und R_{A} ist H, (C₁-C₆)Alkyl, Phenyl, CH₂-Phenyl, (C₁-C₆)Alkyl-OH, (CH₂)ₚO(CH₂)ₘOH, (CH₂)ₚO(CH₂)ₘO(CH₂)ₘOH, (C₁-C₆)Alkyl-O-(C₁-C₄)Alkyl, (CH₂)ₚO(CH₂)ₘO(C₁-C₄)Alkyl oder (CH₂)ₚO(CH₂)ₘO(CH₂)ₘO(C₁-C₄)Alkyl; p ist eine ganze Zahl aus 0, 1, 2, 3, 4 oder 5; und m ist eine ganze Zahl aus 1, 2, 3, 4 oder 5; oder
wobei Folgendes gilt:
R₅ ist H, (C₁-C₄)Alkyl, (C₂-C₄)Alkenyl, Phenyl, Benzyl, CH₂-S-(C₁-C₄)Alkyl, CH₂-O-(C₁-C₄)Alkyl, (CH₂)₂OH, oder (CH₂)₂O(C₁-C₄)Alkyl.

10. Verbindung nach einem der Ansprüche 6 bis 9, wobei jedes Auftreten von R_{A} und R_{B} unabhängig H, (C₁-C₆)Alkyl, Phenyl, CH₂-Phenyl, (C₁-C₆)Alkyl-OH, (CH₂)ₚO(CH₂)ₘOH ist, oder (CH₂)ₚO(CH₂)ₘO(CH₂)ₘOH, (C₁-C₆)Alkyl-O-(C₁-C₄)Alkyl, (CH₂)ₚO(CH₂)ₘO(C₁-C₄)Alkyl ist, oder (CH₂)ₚO(CH₂)ₘO(CH₂)ₘO(C₁-C₄)Alkyl ist; oder
wobei jedes Auftreten von R_{A} und R_{B} unabhängig H oder (C₁-C₆)Alkyl ist; oder
wobei R_{A} und R_{B} zusammen mit dem Stickstoffatom, an das sie gebunden sind, einen Heterozyklus bilden, ausgewählt aus 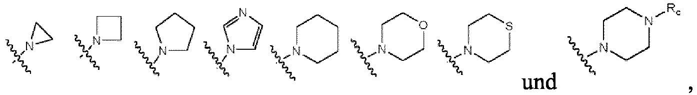 wobei R_{C} H, Me, Et, n-Pr, i-Pr, n-Bu, i-Bu, t-Bu, CH₂CMe₃, Ph, CH₂Ph, CH₂CH₂OH oder CH₂CH₂O(C₁-C₄)Alkyl ist.

11. Verbindung nach Anspruch 1, ausgewählt aus der Gruppe, die aus Folgendem besteht:
[(S)-(2-(N,N-Diethylamino)ethylthio)methyl-Sar]-3-[(γ-hydroxy)-N-MeLeu]-4-Cyclospo rin,
[(S)-(2-(N-Ethyl-N-isopropylamino)ethylthio)methyl-Sar]-3-[(γ-hydroxy)-N-MeLeu]-4-Cyclosporin,
[(S)-(2-(N-Isobutylamino)ethylthio)methyl-Sar]-3-[(γ-hydroxy)-N-MeLeu]-4-Cyclospori n,
[(S)-(2-(N-Ethyl-N-Isobutylamino)ethylthio)methyl-Sar]-3-[(γ-hydroxy)-N-MeLeu]-4-C yclosporin,
[(S)-(2-(N-Isobutyl-N-methylamino)ethylthio)methyl-Sar]-3-[(γ-hydroxy)-N-MeLeu]-4-Cyclosporin,
[(S)-(2-(N-Neopentylamino)ethylthio)methyl-Sar]-3-[(γ-hydroxy)-N-MeLeu]-4-Cyclospo rin,
[(S)-(2-(N-Methyl-N-Neopentylamino)ethylthio)methyl-Sar]-3-[(γ-hydroxy)-N-MeLeu]-4-Cyclosporin,
[(S)-(2-(N-Ethyl-N-neopentylamino)ethylthio)methyl-Sar]-3-[(γ-hydroxy)-N-MeLeu]-4-Cyclosporin,
[(S)-(2-(N-Piperidinyl)ethylthio)methyl-Sar]-3-[(γ-hydroxy)-N-MeLeu]-4-Cyclosporin, [(S)-(2-(N-Morpholino)ethylthio)methyl-Sar]-3-[(γ-hydroxy)-N-MeLeu]-4-Cyclosporin,
[(S)-(2-(N-Thiomorpholino)ethylthio)methyl-Sar]-3-[(γ-hydroxy)-N-MeLeu]-4-Cyclospo rin,
[(S)-(2-(4-Methyl-N-piperazinyl)ethylthio)methyl-Sar]-3-[(γ-hydroxy)-N-MeLeu]-4-Cycl osporin,
[(S)-(3-(N,N-Diethylamino)propylthio)methyl-Sar]-3-[(γ-hydroxy)-N-MeLeu]-4-Cyclosp orin,
[(S)-(3-(N-Ethyl-N-isopropylamino)propylthio)methyl-Sar]-3-[(γ-hydroxy)-N-MeLeu]-4-Cyclosporin,
[(S)-(3-(N-Neopentylamino)propylthio)methyl-Sar]-3-[(γ-hydroxy)-N-MeLeu]-4-Cyclos porin,
[(S)-(3-(N-Pyrrolidinyl)propylthio)methyl-Sar]-3-[(γ-hydroxy)-N-MeLeu]-4-Cyclosporin ,
[(S)-(3-(N-Piperidinyl)propylthio)methyl-Sar]-3-[(γ-hydroxy)-N-MeLeu]-4-Cyclosporin,
[(S)-(3-(N-Morpholino)propylthio)methyl-Sar]-3-[(γ-hydroxy)-N-MeLeu]-4-Cyclosporin ,
[(S)-(3-(N-Thiomorpholino)propylthio)methyl-Sar]-3-[(γ-hydroxy)-N-MeLeu]-4-Cyclosp orin,
[(S)-(3-(4-Methyl-N-piperazinyl)propylthio)methyl-Sar]-3-[(γ-hydroxy)-N-MeLeu]-4-Cy closporin,
[(S)-(4-(N,N-Diethylamino)butylthio)methyl-Sar]-3-[(γ-hydroxy)-N-MeLeu]-4-Cyclospo rin,
[(S)-(4-(N-Ethyl-N-isopropylamino)butylthio)methyl-Sar]-3-[(γ-hydroxy)-N-MeLeu]-4-Cyclosporin,
[(S)-(4-(N-Isobutylamino)butylthio)methyl-Sar]-3-[(γ-hydroxy)-N-MeLeu]-4-Cyclospori n,
[(S)-(4-(N-Isobutyl-N-methylamino)butylthio)methyl-Sar]-3-[(γ-hydroxy)-N-MeLeu]-4-Cyclosporin,
[(S)-(4-(N-Ethyl-N-isobutylamino)butylthio)methyl-Sar]-3-[(γ-hydroxy)-N-MeLeu]-4-C yclosporin,
[(S)-(4-(N-Neopentylamino)butylthio)methyl-Sar]-3-[(γ-hydroxy)-N-MeLeu]-4-Cyclosp orin,
[(S)-(4-(N-Methyl-N-neopentylamino)butylthio)methyl-Sar]-3-[(γ-hydroxy)-N-MeLeu]-4 -Cyclosporin,
[(S)-(4-(N-Piperidinyl)butylthio)methyl-Sar]-3-[(γ-hydroxy)-N-MeLeu]-4-Cyclosporin,
[(S)-(4-(N-Morpholino)butylthio)methyl-Sar]-3-[(γ-hydroxy)-N-MeLeu]-4-Cyclosporin,
[(S)-(4-(N-Thiomorpholino)butylthio)methyl-Sar]-3-[(γ-hydroxy)-N-MeLeu]-4-Cyclospo rin,
[(S)-(4-(4-Methyl-N-piperazinyl)butylthio)methyl-Sar]-3-[(γ-hydroxy)-N-MeLeu]-4-Cyc losporin,
[(R)-(3-(N-Morpholino)propoxy)methyl-Sar]-3-[(γ-hydroxy)-N-MeLeu]-4-Cyclosporin, [(S)-(2-(N,N-Diethylamino)ethylthio)methyl-Sar]-3-[(γ-methoxy)-N-MeLeu]-4-Cyclospo rin,
[(S)-(2-(N-Pyrrolidinyl)ethylthio)methyl-Sar]-3-[(γ-methoxy)-N-MeLeu]-4-Cyclosporin, [(S)-(3-(N,N-Dimethylamino)propylthio)methyl-Sar]-3-[(γ-methoxy)-N-MeLeu]-4-Cyclo sporin,
[(S)-(3-(N,N-Diethylamino)propylthio)methyl-Sar]-3-[(γ-methoxy)-N-MeLeu]-4-Cyclos porin,
[(S)-(3-(N-Piperidinyl)propylthio)methyl-Sar]-3-[(γ-methoxy)-N-MeLeu]-4-Cyclosporin, [(S)-(4-(N-Piperidinyl)butylthio)methyl-Sar]-3-[(γ-methoxy)-N-MeLeu]-4-Cyclosporin, [(R)-(2-(N,N-Diethylamino)ethoxy)methyl-Sar]-3-[(γ-methoxy)-N-MeLeu]-4-Cyclospori n, und
[(R)-(3-(N,N-Diethylamino)propoxy)methyl-Sar]-3-[(γ-methoxy)-N-MeLeu]-4-Cyclospo rin.

12. Verbindung nach Anspruch 1 mit der Struktur: 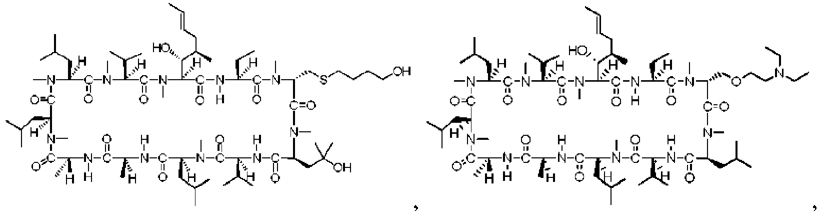 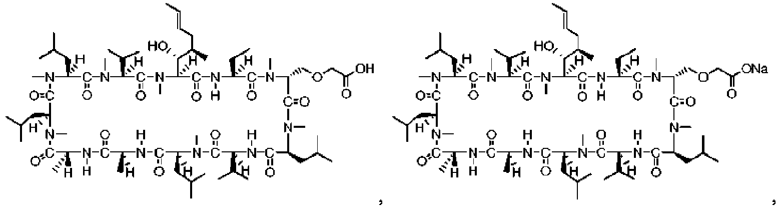 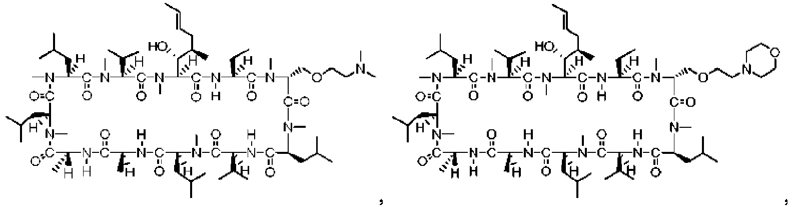 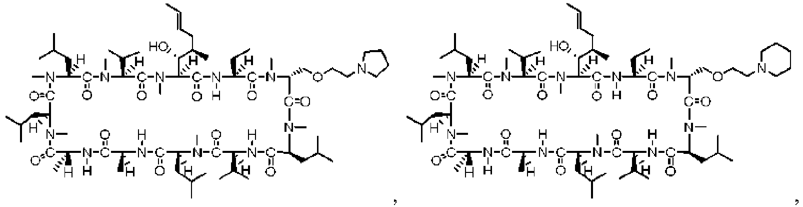 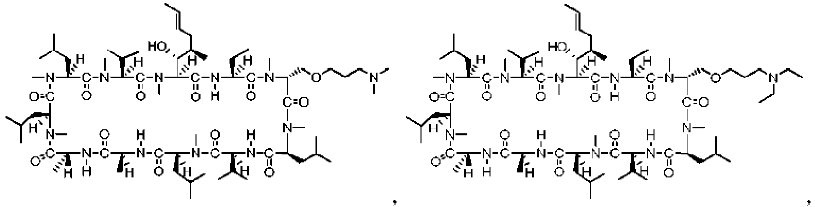 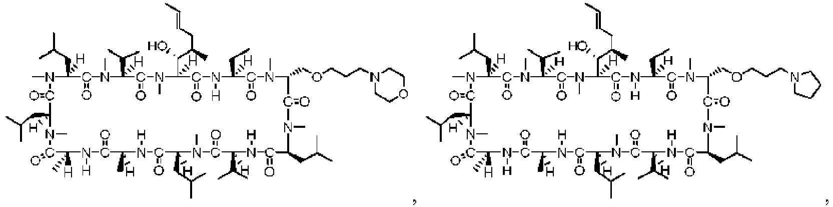 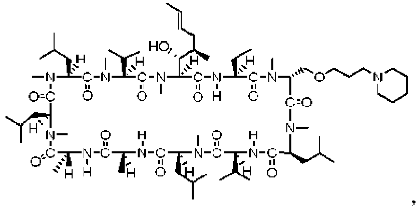 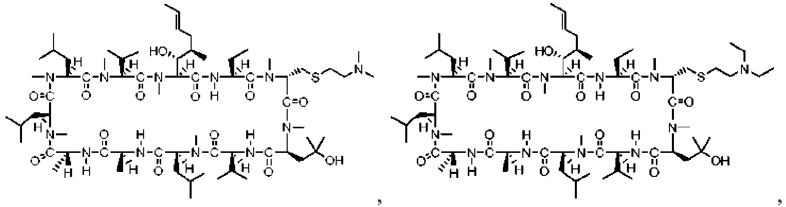 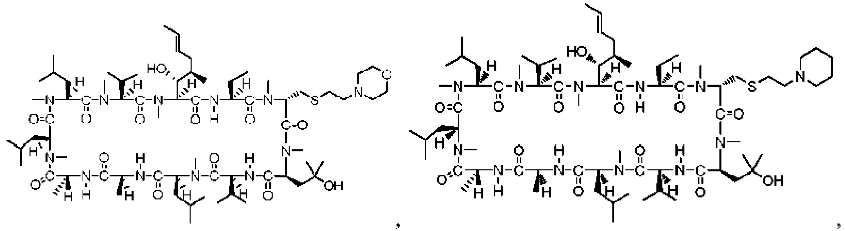 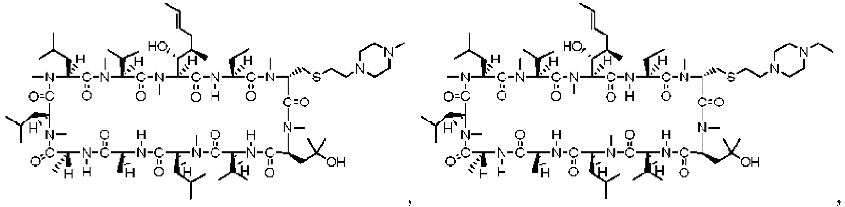 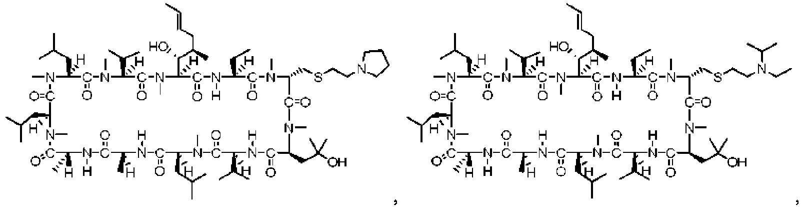 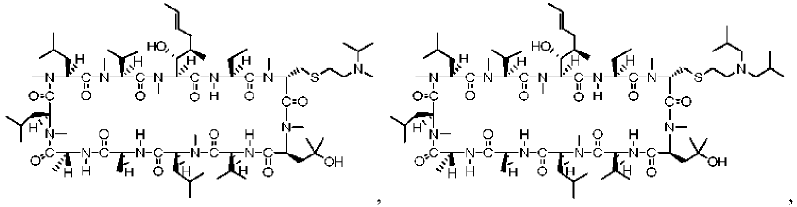 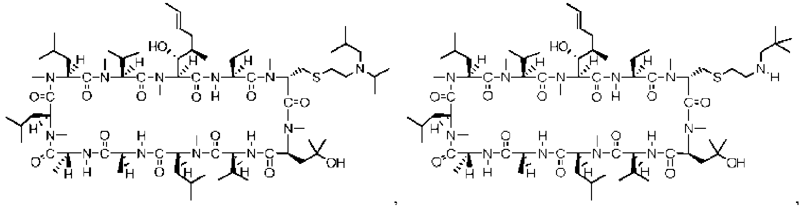 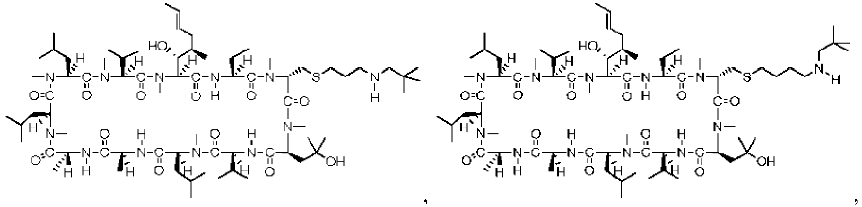 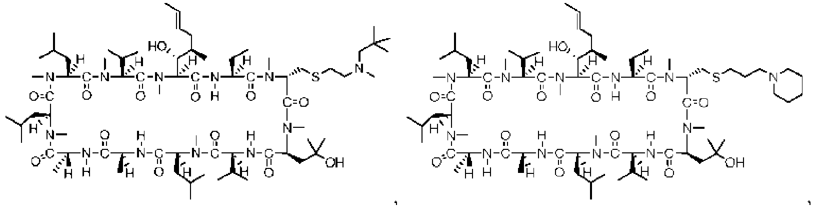 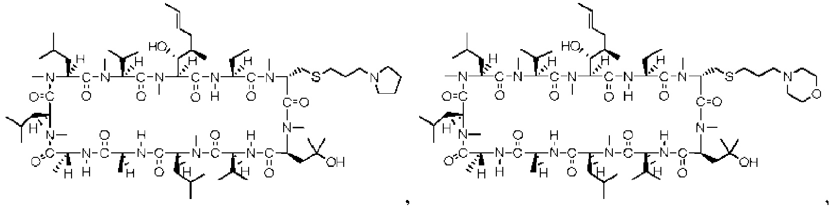 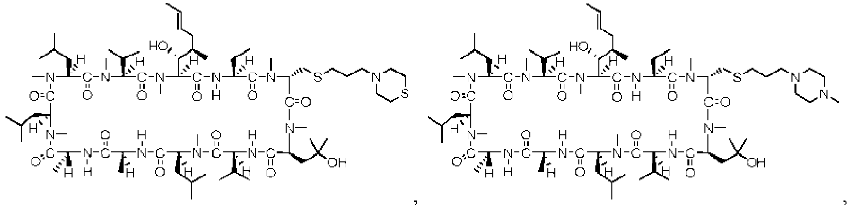 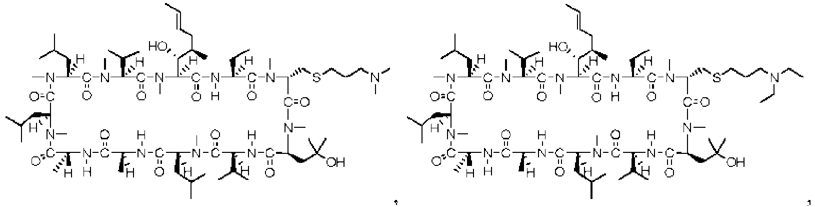 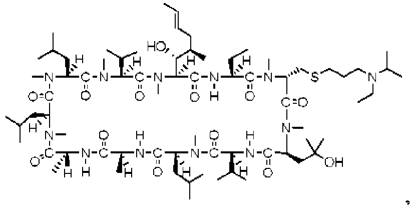 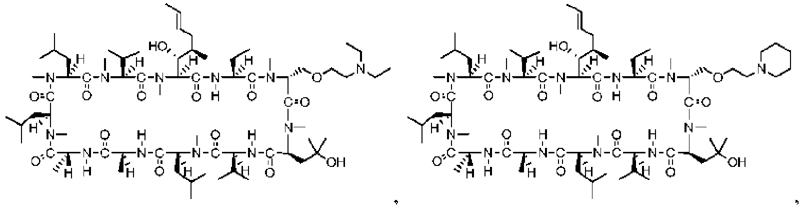 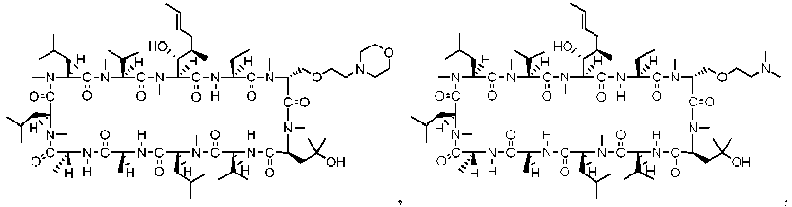 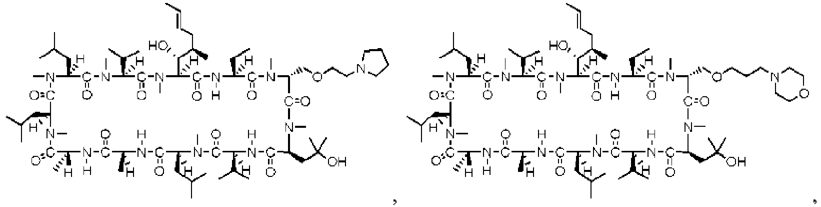 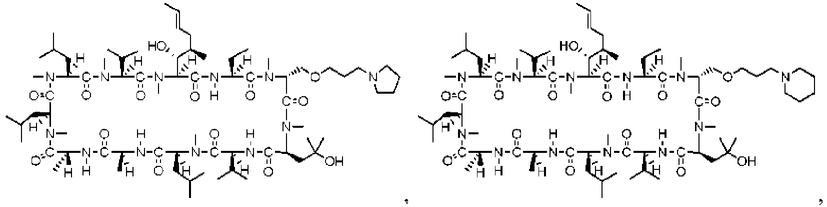 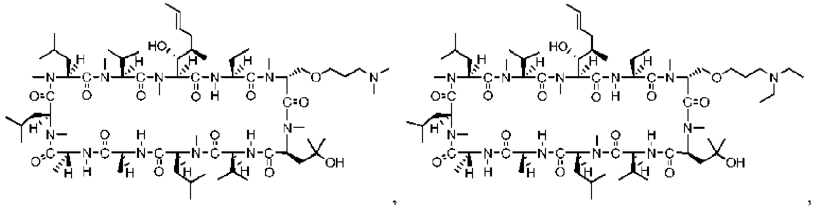 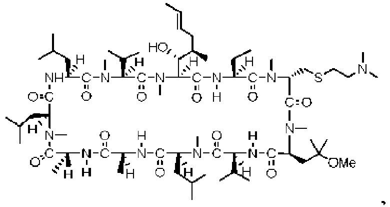 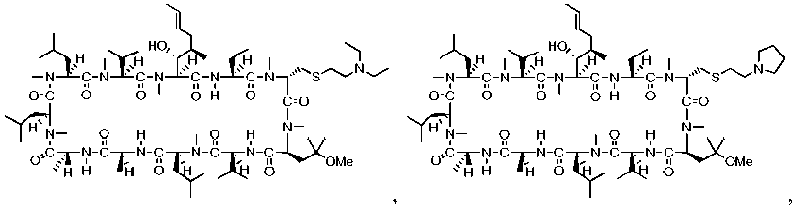 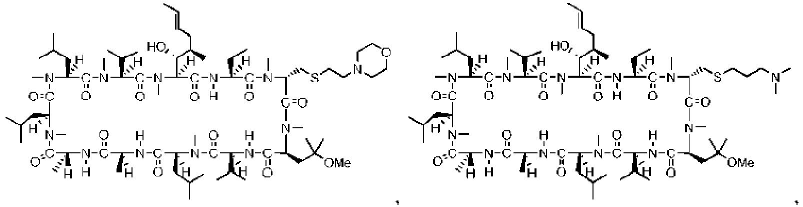 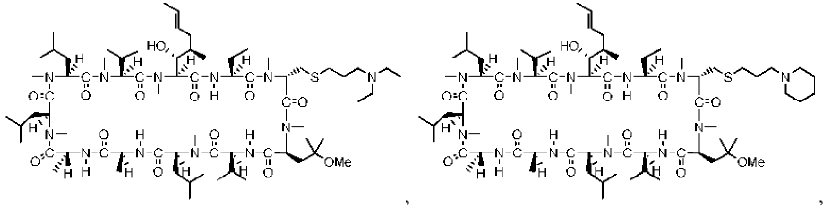 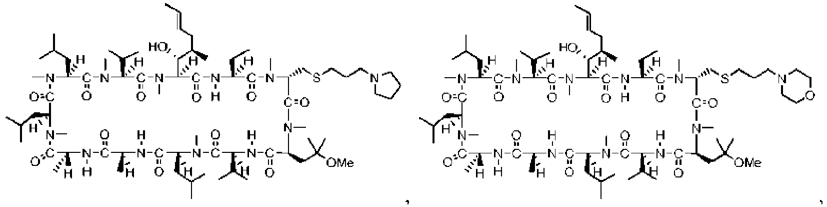 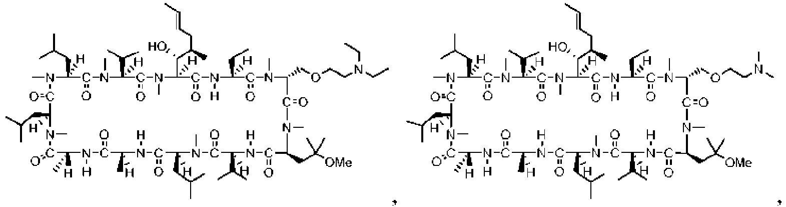 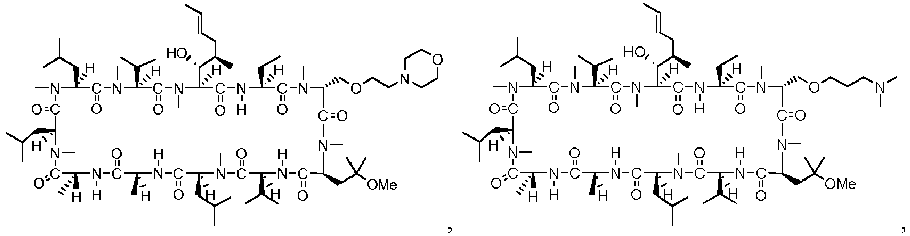 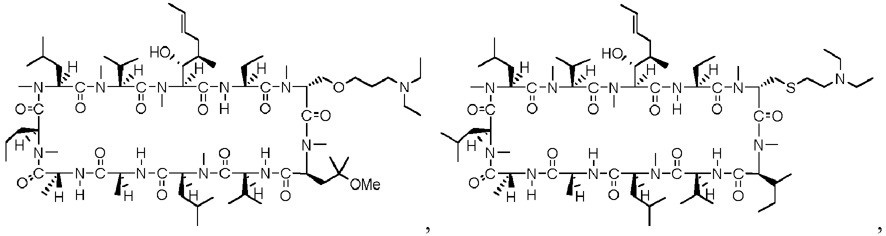 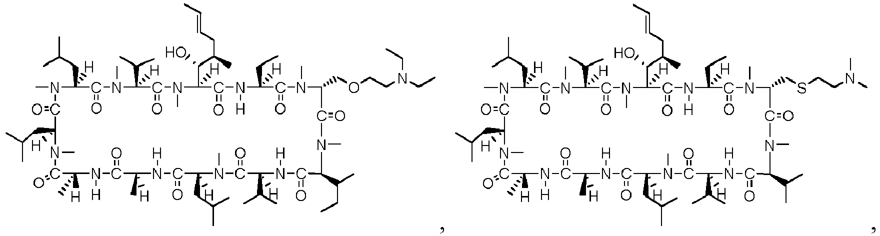 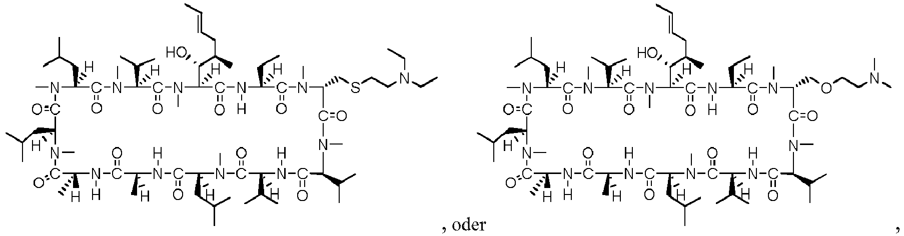 oder ein pharmazeutisch annehmbares Salz davon.

13. Verbindung nach Anspruch 1 mit der folgenden Struktur: 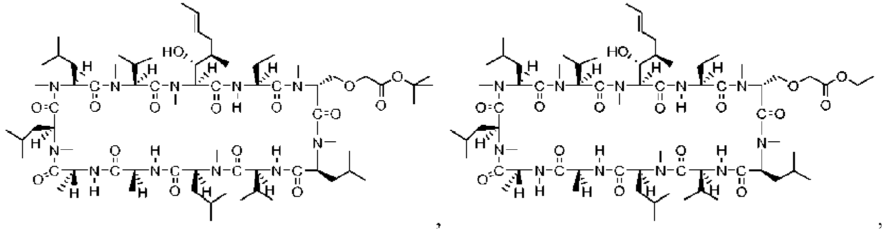 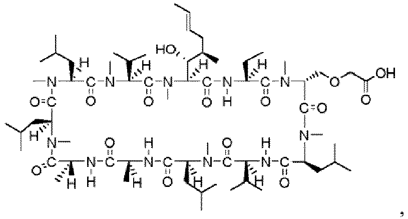 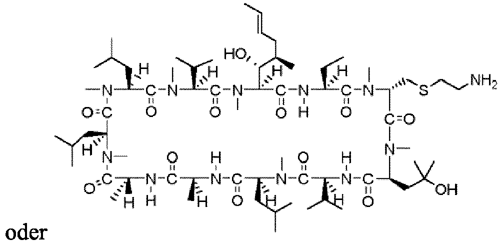 oder ein pharmazeutisch annehmbares Salz davon.

14. Pharmazeutische Zusammensetzung, die mindestens eine Verbindung nach einem der Ansprüche 1-10 und einen pharmazeutisch annehmbaren Träger oder ein pharmazeutisch annehmbares Verdünnungsmittel umfasst.

15. Verbindung nach einem der Ansprüche 1-10 zur Verwendung bei der Behandlung oder Vorbeugung von Hepatitis-B-Virusinfektionen, Hepatitis-C-Virusinfektionen oder HIV-Infektionen in einer Säugerspezies.

## Revendications

1. Composé de formule (I) : 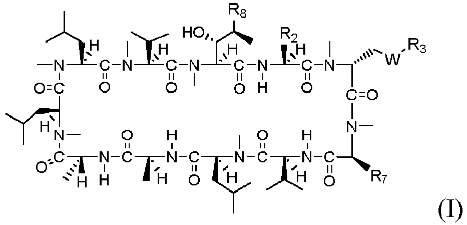 ou sel pharmaceutiquement acceptable de celui-ci, dans laquelle :
R₈ représente un groupe n-butyle, (*E*)-but-2-ényle, ou 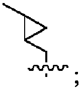
R₂ représente un groupe éthyle, 1-hydroxyéthyle, isopropyle ou n-propyle ;
W représente un atome O ou S ;
R₃ représente :
un groupe alkyle en C₁ à C₆ éventuellement substitué par un ou plusieurs groupes R₄ qui peuvent être identiques ou différents ;
un groupe alcényle en C₂ à C₆ éventuellement substitué par un ou plusieurs groupes qui peuvent être identiques ou différents choisis parmi un atome d'halogène, un groupe hydroxy, amino, monoalkylamino et dialkylamino ;
un groupe alcynyle en C₂ à C₆ éventuellement substitué par un ou un ou plusieurs groupes qui peuvent être identiques ou différents choisis parmi un atome d'halogène, un groupe hydroxy, amino, monoalkylamino et dialkylamino ;
un groupe cycloalkyle en C₃ à C₇ éventuellement substitué par un ou plusieurs groupes qui peuvent être identiques ou différents choisis parmi un atome d'halogène, un groupe hydroxy, amino, monoalkylamino et dialkylamino ;
un groupe phényle ou CH₂-phényle éventuellement substitué par un ou plusieurs groupes qui peuvent être identiques ou différents choisis parmi un atome d'halogène, un groupe hydroxy, alkyle en C₁ à C₆ ;
R₇ représente 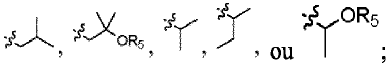
R₅ représente :
un atome H ;
un groupe alkyle en C₁ à C₆ éventuellement substitué par un ou plusieurs groupes R₆ qui peuvent être identiques ou différents ;
un groupe alcényle en C₂ à C₆ éventuellement substitué par un ou plusieurs groupes qui peuvent être identiques ou différents choisis parmi un groupe hydroxy, alkyle en C₁ à C₆, aryle, (CH₂)_{P}OR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)_{P}C(=O)NR_{A}R_{B}, (CH₂)_{P}C(=O)OR_{A} ;
un groupe alcynyle en C₂ à C₆ éventuellement substitué par un ou un ou plusieurs groupes qui peuvent être identiques ou différents choisis parmi un atome d'halogène, un groupe hydroxy, amino, monoalkylamino et dialkylamino ;
un groupe cycloalkyle en C₃ à C₇ éventuellement substitué par un ou plusieurs groupes qui peuvent être identiques ou différents choisis parmi un atome d'halogène, un groupe hydroxy, amino, monoalkylamino et dialkylamino ;
un groupe phényle ou CH₂-phényle éventuellement substitué par un ou plusieurs groupes qui peuvent être identiques ou différents choisis parmi un atome d'halogène, un groupe hydroxy, alkyle en C₁ à C₆, (CH₂)_{P}OR_{A}, (CH₂)_{P}NR_{A}R_{B}, (CH₂)_{P}C(=O)NR_{A}R_{B}, (CH₂)_{P}C(=O)OR_{A} ;
chaque occurrence de R₄ représente indépendamment un atome d'halogène, un groupe hydroxy, aryle, O(CH₂)ₘOR_{A}, O(CH₂)ₘO(CH₂)ₘOR_{A}, C(=O)(C₁ à C₆)alkyle, C(=O)OR_{A}, C(=O)NR_{A}R_{B}, -NR_{A}R_{B}, - NR_{C}CH₂(CH₂)_{P}NR_{A}R_{B}, NR_{C}[CH₂(CH₂)ₚNR_{A}]ₘCH₂(CH₂)ₙNR_{A}R_{B}, O[CH₂(CH₂)ₚO]ₘCH₂(CH₂)ₙOR_{A}, OCH₂(CH₂)_{P}NR_{A}R_{B} ou O[CH₂(CH₂)ₚO]ₘCH₂(CH₂)ₙNR_{A}R_{B} ;
chaque occurrence de R₆ représente indépendamment un atome d'halogène, un groupe hydroxy, aryle, S-alkyle en C₁ à C₆, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B} ou NR_{C}(CH₂)ₘNR_{C}(CH₂)ₘNR_{A}R_{B}, ledit groupe aryle ou phényle étant éventuellement substitué par un ou plusieurs groupes qui peuvent être identiques ou différents choisis parmi un atome d'halogène, un groupe hydroxy, alkyle en C₁ à C₆, (CH₂)_{P}OR_{A}, (CH₂)_{P}NR_{A}R_{B}, (CH₂)_{P}C(=O)NR_{A}R_{B} et (CH₂)ₚC(=O)OR_{A} ;
chaque occurrence de R_{A} et R_{B} représente indépendamment :
un atome d'hydrogène ;
un groupe alkyle en C₁ à C₆ éventuellement substitué par un ou plusieurs groupes R_{D} qui peuvent être identiques ou différents ;
un groupe alcényle en C₂ à C₆ ou alcynyle en C₂ à C₆ ;
un groupe cycloalkyle en C₃ à C₇ éventuellement substitué par un groupe alkyle en C₁ à C₆;
un groupe phényle éventuellement substitué par un à cinq groupes qui peuvent être identiques ou différents choisis parmi un atome d'halogène, un groupe -O-alkyle en C₁ à C₆, -C(=O)O(C₁ à C₆)alkyle, amino, alkylamino et dialkylamino ;
ou un noyau hétérocyclique qui peut être saturé ou insaturé contenant cinq ou six atomes de noyau et d'un à trois hétéroatomes qui peuvent être identiques ou différents choisis parmi un atome d'azote, de soufre et d'oxygène ;
ou R_{A} et R_{B}, ensemble avec l'atome d'azote auquel ils sont fixés, forment un noyau hétérocyclique saturé ou insaturé contenant de trois à sept atomes de noyau, lequel noyau peut éventuellement contenir un autre hétéroatome choisi dans le groupe constitué par l'atome d'azote, d'oxygène et de soufre et peut être éventuellement substitué par un à quatre groupes qui peuvent être identiques ou différents choisis dans le groupe constitué par un groupe alkyle, phényle et benzyle ;
chaque occurrence de R_{C} représente indépendamment un atome d'hydrogène ou un groupe alkyle en C₁ à C₆ ;
p est un entier valant 0, 1, 2, 3, 4 ou 5 ; et
m est un entier valant 1, 2, 3, 4 ou 5.

2. Composé selon la revendication 1 ayant la structure des formules (II) à (V) : 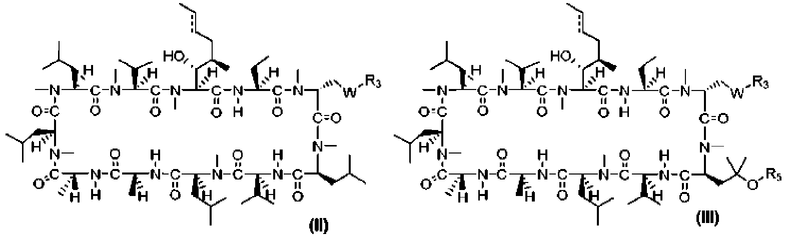 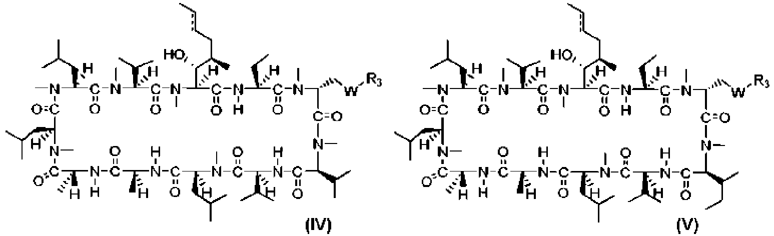 ou sel pharmaceutiquement acceptable de celui-ci, dans lesquelles : 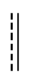
représente une liaison simple ou une double liaison ;
chaque W représente indépendamment un atome O ou S ;
chaque R₃ représente indépendamment :
un groupe alkyle en C₁ à C₆ éventuellement substitué par un ou plusieurs groupes R₄ qui peuvent être identiques ou différents ;
un groupe alcényle en C₂ à C₆ éventuellement substitué par un ou plusieurs groupes qui peuvent être identiques ou différents choisis parmi un atome d'halogène, un groupe hydroxy, un groupe alkyle en C₁ à C₆, aryle, (CH₂)ₚOR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)_{P}C(=O)OR_{A} ;
un groupe alcynyle en C₂ à C₆ éventuellement substitué par un ou un ou plusieurs groupes qui peuvent être identiques ou différents choisis parmi un atome d'halogène, un groupe hydroxy, amino, monoalkylamino et dialkylamino ;
un groupe cycloalkyle en C₃ à C₇ éventuellement substitué par un ou plusieurs groupes qui peuvent être identiques ou différents choisis parmi un atome d'halogène, un groupe hydroxy, amino, monoalkylamino et dialkylamino ;
un groupe phényle ou CH₂-phényle éventuellement substitué par un ou plusieurs groupes qui peuvent être identiques ou différents choisis parmi un atome d'halogène, un groupe hydroxy, alkyle en C₁ à C₆ ;
chaque R₅ représente indépendamment :
un atome H ;
un groupe alkyle en C₁ à C₆ éventuellement substitué par un ou plusieurs groupes R₆ qui peuvent être identiques ou différents ;
un groupe alcényle en C₂ à C₆ éventuellement substitué par un ou plusieurs groupes qui peuvent être identiques ou différents choisis parmi un atome d'halogène, un groupe hydroxy, un groupe alkyle en C₁ à C₆, aryle, (CH₂)_{P}OR_{A}, (CH₂)ₘOH, (CH₂)ₘO(CH₂)ₘOH, (CH₂)ₘNR_{A}R_{B}, (CH₂)ₘO(CH₂)ₘNR_{A}R_{B}, (CH₂)_{P}NR_{A}R_{B}, (CH₂)ₚNR_{C}(CH₂)ₘNR_{A}R_{B}, (CH₂)ₚNR_{c}(CH₂)ₘNR_{c}(CH₂)ₘNR_{A}R_{B}, (CH₂)ₚC(=O)NR_{A}R_{B}, (CH₂)_{P}C(=O)OR_{A} ;
un groupe alcynyle en C₂ à C₆ éventuellement substitué par un ou un ou plusieurs groupes qui peuvent être identiques ou différents choisis parmi un atome d'halogène, un groupe hydroxy, amino, monoalkylamino et dialkylamino ;
un groupe cycloalkyle en C₃ à C₇ éventuellement substitué par un ou plusieurs groupes qui peuvent être identiques ou différents choisis parmi un atome d'halogène, un groupe hydroxy, amino, monoalkylamino et dialkylamino ;
un groupe phényle ou CH₂-phényle éventuellement substitué par un ou plusieurs groupes qui peuvent être identiques ou différents choisis parmi un atome d'halogène, un groupe hydroxy, alkyle en C₁ à C₆, (CH₂)_{P}OR_{A}, (CH₂)_{P}NR_{A}R_{B}, (CH₂)_{P}C(=O)NR_{A}R_{B}, (CH₂)_{P}C(=O)OR_{A} ;
chaque occurrence de R₄ représente indépendamment un atome d'halogène, un groupe hydroxy, aryle, O(CH₂)ₘOR_{A}, O(CH₂)ₘO(CH₂)ₘOR_{A}, C(=O)(C₁ à C₆)alkyle, C(=O)OR_{A}, C(=O)NR_{A}R_{B}, -NR_{A}R_{B}, - NR_{C}H₂(CH₂)_{P}NR_{A}R_{B}, NR_{C}[CH₂(CH₂)ₚNR_{A}]ₘCH₂(CH₂)ₙNR_{A}R_{B}, O[CH₂(CH₂)ₚO]ₘCH₂(CH₂)ₙOR_{A}, OCH₂(CH₂)_{P}NR_{A}R_{B} ou O[CH₂(CH₂)ₚO]ₘCH₂(CH₂)ₙNR_{A}R_{B};
chaque occurrence de R₆ représente indépendamment un atome d'halogène, un groupe hydroxy, aryle, S-alkyle en C₁ à C₆, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B},NR_{C}(CH₂)ₘNR_{A}R_{B} ou NR_{C}(CH₂)ₘNR_{C}(CH₂)ₘNR_{A}R_{B}, ledit groupe aryle ou phényle étant éventuellement substitué par un ou plusieurs groupes qui peuvent être identiques ou différents choisis parmi un atome d'halogène, un groupe hydroxy, alkyle en C₁ à C₆, (CH₂)_{P}OR_{A}, (CH₂)_{P}NR_{A}R_{B}, (CH₂)_{P}C(=O)NR_{A}R_{B} et (CH₂)ₚC(=O)OR_{A} ;
chaque occurrence de R_{A} et R_{B} représente indépendamment :
un atome d'hydrogène ;
un groupe alkyle en C₁ à C₆ éventuellement substitué par un ou plusieurs groupes R_{D} qui peuvent être identiques ou différents ;
un groupe alcényle en C₂ à C₆ ou alcynyle en C₂ à C₆ ;
un groupe cycloalkyle en C₃ à C₇ éventuellement substitué par un groupe alkyle en C₁ à C₆;
un groupe phényle éventuellement substitué par un à cinq groupes qui peuvent être identiques ou différents choisis parmi un atome d'halogène, un groupe -O-alkyle en C₁ à C₆, -C(=O)O(C₁ à C₆)alkyle, amino, alkylamino et dialkylamino ;
ou un noyau hétérocyclique qui peut être saturé ou insaturé contenant cinq ou six atomes de noyau et d'un à trois hétéroatomes qui peuvent être identiques ou différents choisis parmi un atome d'azote, de soufre et d'oxygène ;
ou R_{A} et R_{B}, ensemble avec l'atome d'azote auquel ils sont fixés, forment un noyau hétérocyclique saturé ou insaturé contenant de trois à sept atomes de noyau, lequel noyau peut éventuellement contenir un autre hétéroatome choisi dans le groupe constitué par l'atome d'azote, d'oxygène et de soufre et peut être éventuellement substitué par un à quatre groupes qui peuvent être identiques ou différents choisis dans le groupe constitué par un groupe alkyle, phényle et benzyle ;
ou R_{A} et R_{B}, ensemble avec l'atome d'azote auquel ils sont fixés, forment un groupe -N=CH-NR_{F}R_{F'}, -N=CMe-NR_{F}R_{F'} ou -NR_{F}C(=NH)NR_{F}R_{F'} ;
chaque occurrence de R_{C} représente indépendamment un atome d'hydrogène ou un groupe alkyle en C₁ à C₆ ;
chaque occurrence de R_{D} représente indépendamment un atome d'halogène, un groupe hydroxy, O-alkyle en C₁ à C₄, C(=O)(C₁ à C₄)alkyle, C(=O)O(C₁ à C₄)alkyle ;
chaque occurrence de R_{F} and R_{F'} représente indépendamment un atome d'hydrogène, un groupe alkyle en C₁ à C₆, phényle, benzyle,
ou R_{F} et R_{F'}, ensemble avec l'atome d'azote auquel ils sont fixés, forment un noyau hétérocyclique saturé ou insaturé contenant de trois à sept atomes de noyau, lequel noyau peut éventuellement contenir un autre hétéroatome choisi dans le groupe constitué par l'atome d'azote, d'oxygène et de soufre et peut être éventuellement substitué par un à quatre groupes qui peuvent être identiques ou différents choisis dans le groupe constitué par un groupe alkyle, phényle et benzyle ;
p est un entier valant 0, 1, 2, 3, 4, 5 ou 6 ;
m est un entier valant 1, 2, 3, 4, 5 ou 6 ; et
n est un entier valant 1, 2, 3, 4, 5 ou 6.

3. Composé selon la revendication 1 ou la revendication 2, R₃ représentant un groupe méthyle, éthyle, n-propyle, i- propyle, n- butyle, i-butyle, t-butyle, CH₂CMe₃, phényle, CH₂-phényle, 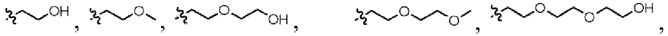 ou 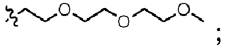 ou
R₃ représentant un groupe -(CH₂)ₙNR_{A}R_{B}, n étant un entier valant 1 ,2, 3, 4, 5 ou 6 ; et chaque occurrence de R_{A} et R_{B} représentant indépendamment un atome d'hydrogène ; un groupe alkyle en C₁ à C₄ éventuellement substitué par un ou plusieurs groupes R_{D} qui peuvent être identiques ou différents, dans lequel chaque occurrence de R_{D} représente indépendamment un atome d'halogène, un groupe hydroxy, O-alkyle en C₁ à C₄, C(=O)(C₁ à C₄)alkyle, C(=O)O(C₁ à C₄)alkyle ; ou R_{A} et R_{B}, ensemble avec l'atome d'azote auquel ils sont fixés, formant un noyau hétérocyclique saturé ou insaturé contenant de trois à sept atomes de noyau, lequel noyau peut éventuellement contenir un autre hétéroatome choisi dans le groupe constitué par l'atome d'azote, d'oxygène et de soufre et peut être éventuellement substitué par un à quatre groupes qui peuvent être identiques ou différents choisis parmi un groupe alkyle en C₁ à C₄, phényle et benzyle ; ou
R₃ représentant un groupe -(CH₂)ₙNR_{A}R_{B}, n étant un entier valant 1, 2, 3, 4, 5 ou 6 ; et R_{A} et R_{B}, ensemble avec l'atome d'azote auquel ils sont fixés, formant un noyau hétérocyclique saturé ou insaturé contenant de trois à sept atomes de noyau, lequel noyau peut éventuellement contenir un autre hétéroatome choisi parmi l'atome d'azote, d'oxygène et de soufre et peut être éventuellement substitué par un à quatre groupes qui peuvent être identiques ou différents choisis parmi un groupe alkyle en C₁ à C₄, phényle et benzyle ; ou
R₃ représentant un groupe 2-aminoéthyle, 2-aminobutyle, 3-aminobutyle, 2-monoalkylaminoéthyle, 2-monoalkylaminobutyle, 3-monoalkylaminobutyle, 2-dialkylaminoéthyle, 2-dialkylaminobutyle ou 3-dialkylaminobutyle, ledit groupe alkyle étant un groupe alkyle en C₁ à C₄ ; ou
R₃ représentant un groupe diméthylaminoéthyle, diéthylaminoéthyle, méthyléthylaminoéthyle, méthyl-iso-butylaminoéthyle, éthyle-iso-butylaminoéthyle, méthyle-tert-butylaminoéthyle ou éthyl-tert-butylaminoéthyle ; ou
R₃ représentant : 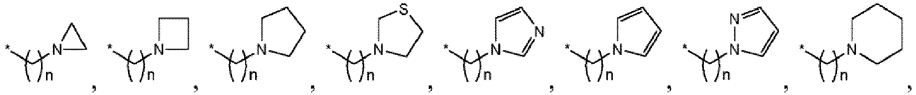 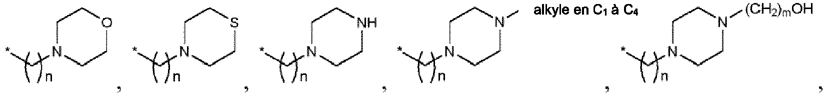 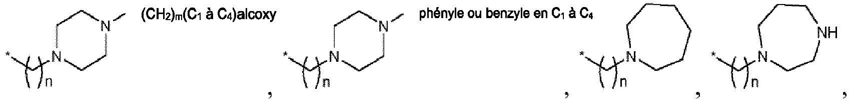 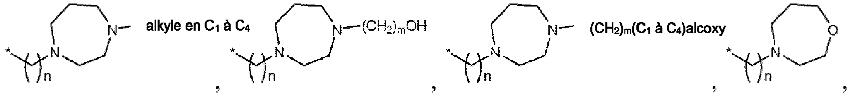 ou 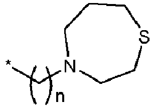 dans lesquels n est un entier valant 2, 3, 4, 5 ou 6, et m est un entier valant 2, 3 ou 4.

4. Composé selon l'une quelconque des revendications 1 à 3, R₅ représentant un atome H, un groupe alkyle en C₁ à C₆, alcényle en C₂ à C₆, phényle, benzyle, CH₂-S-(C₁ à C₆)alkyle, CH₂-O-(C₁ à C₆)alkyle, (C₂ à C₆)OR_{A}, monoalkylamine en C₁ à C₆, dialkylamine en C₁ à C₆, ou amine cyclique en C₁ à C₆, dans lequel ledit groupe phényle ou benzyle est éventuellement substitué par un à trois substituants choisis parmi un groupe alkyle en C₁ à C₄, alcoxy en C₁ à C₄, et un atome d'halogène ; et R_{A} représentant un atome H, un groupe alkyle en C₁ à C₆, phényle, CH₂-phényle, alkyl-OH en C₁ à C₆, (CH₂)ₚO(CH₂)ₘOH, (CH₂)ₚO(CH₂)ₘO(CH₂)ₘOH, (C₁ à C₆)alkylO(C₁ à C₄)alkyle, (CH₂)ₚO(CH₂)ₘO(C₁ à C₄)alkyle ou (CH₂)ₚO(CH₂)ₘO(CH₂)ₘO(C₁-C₄)alkyle ; p étant un entier valant 0, 1, 2, 3, 4 ou 5 ; et m étant un entier valant 1, 2, 3, 4 ou 5.

5. Composé selon l'une quelconque des revendications 1 à 4, chaque occurrence de R_{A} et R_{B} représentant indépendamment un atome H, un groupe alkyle en C₁ à C₆, phényle, CH₂-phényle, alkyl-OH en C₁ à C₆, (CH₂)ₚO(CH₂)ₘOH ou (CH₂)ₚO(CH₂)ₘO(CH₂)ₘOH, (C₁ à C₆)alkylO(C₁ à C₄)alkyle, (CH₂)ₚO(CH₂)ₘO(C₁ à C₄)alkyle ou (CH₂)ₚO(CH₂)ₘO(CH₂)ₘO(C₁-C₄)alkyle ; ou
R_{A} et R_{B}, ensemble avec l'atome d'azote auquel ils sont fixés, formant un hétérocycle choisi parmi 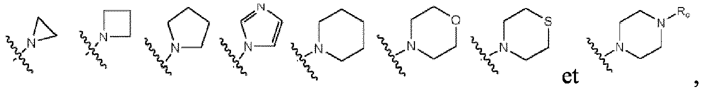 dans lequel R_{C} représente un atome H, un groupe Me, Et, n-Pr, i-Pr, n-Bu, i-Bu, t-Bu, CH₂CMe₃, Ph, CH₂Ph, CH₂CH₂OH ou CH₂CH₂O(C₁ à C₄)alkyle.

6. Composé selon la revendication 1 ayant la structure des formules (IIa) à (Va) : 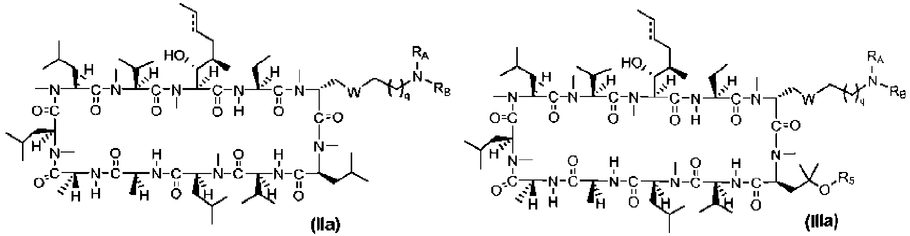 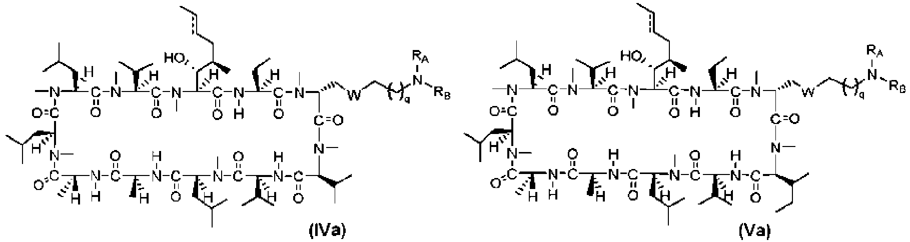 ou sel pharmaceutiquement acceptable de celui-ci, dans lesquelles : 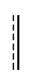
représente une liaison simple ou une double liaison ;
chaque W représente indépendamment un atome O ou S ;
chaque R₅ représente indépendamment :
un atome H ;
un groupe alkyle en C₁ à C₆ éventuellement substitué par un ou plusieurs groupes R₆ qui peuvent être identiques ou différents ;
un groupe alcényle en C₂ à C₆ éventuellement substitué par un ou plusieurs groupes qui peuvent être identiques ou différents choisis parmi un atome d'halogène, un groupe hydroxy, alkyle en C₁ à C₆, aryle, (CH₂)_{P}OR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)_{P}NR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)ₚC(=O)OR_{A} ;
un groupe alcynyle en C₂ à C₆ éventuellement substitué par un ou un ou plusieurs groupes qui peuvent être identiques ou différents choisis parmi un atome d'halogène, un groupe hydroxy, amino, monoalkylamino et dialkylamino ;
un groupe cycloalkyle en C₃ à C₇ éventuellement substitué par un ou plusieurs groupes qui peuvent être identiques ou différents choisis parmi un atome d'halogène, un groupe hydroxy, amino, monoalkylamino et dialkylamino ;
un groupe phényle ou CH₂-phényle éventuellement substitué par un ou plusieurs groupes qui peuvent être identiques ou différents choisis parmi un atome d'halogène, un groupe hydroxy, alkyle en C₁ à C₆, (CH₂)_{P}OR_{A}, (CH₂)_{P}NR_{A}R_{B}, (CH₂)_{P}C(=O)NR_{A}R_{B}, (CH₂)_{P}C(=O)OR_{A};
chaque occurrence de R₆ représente indépendamment un atome d'halogène, un groupe hydroxy, aryle, S-alkyle en C₁ à C₆, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B} ou NR_{C}(CH₂)mNR_{C}(CH₂)mNR_{A}R_{B}, ledit groupe aryle ou phényle étant éventuellement substitué par un ou plusieurs groupes qui peuvent être identiques ou différents choisis parmi un atome d'halogène, un groupe hydroxy, alkyle en C₁ à C₆, (CH₂)_{P}OR_{A}, (CH₂)_{P}NR_{A}R_{B}, (CH₂)_{P}C(=O)NR_{A}R_{B} et (CH₂)_{P}C(=O)OR_{A} ;
chaque occurrence de R_{A} et R_{B} représente indépendamment :
un atome d'hydrogène ;
un groupe alkyle en C₁ à C₆ éventuellement substitué par un ou plusieurs groupes R_{D} qui peuvent être identiques ou différents ;
un groupe alcényle en C₂ à C₆ ou alcynyle en C₂ à C₆ ;
un groupe cycloalkyle en C₃ à C₇ éventuellement substitué par un groupe alkyle en C₁ à C₆;
un groupe phényle éventuellement substitué par un à cinq groupes qui peuvent être identiques ou différents choisis parmi un atome d'halogène, un groupe -O-alkyle en C₁ à C₆, -C(=O)O(C₁ à C₆)alkyle, amino, alkylamino et dialkylamino ;
ou un noyau hétérocyclique qui peut être saturé ou insaturé contenant cinq ou six atomes de noyau et d'un à trois hétéroatomes qui peuvent être identiques ou différents choisis parmi un atome d'azote, de soufre et d'oxygène ;
ou R_{A} et R_{B}, ensemble avec l'atome d'azote auquel ils sont fixés, forment un noyau hétérocyclique saturé ou insaturé contenant de trois à sept atomes de noyau, lequel noyau peut éventuellement contenir un autre hétéroatome choisi dans le groupe constitué par l'atome d'azote, d'oxygène et de soufre et peut être éventuellement substitué par un à quatre groupes qui peuvent être identiques ou différents choisis dans le groupe constitué par un groupe alkyle, phényle et benzyle ;
chaque occurrence de R_{C} représente indépendamment un atome d'hydrogène ou un groupe alkyle en C₁ à C₆ ;
chaque occurrence de R_{D} représente indépendamment un atome d'halogène, un groupe hydroxy, O-alkyle en C₁ à C₄, C(=O)(C₁ à C₄)alkyle, C(=O)O(C₁ à C₄)alkyle ;
chaque p est indépendamment un entier valant 0, 1, 2, 3, 4 ou 5 ; et
chacun de m, n et q est indépendamment un entier valant 1, 2, 3, 4 ou 5.

7. Composé selon la revendication 1, R₇ représentant

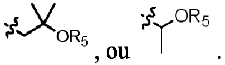

8. Composé selon la revendication 2 dans lequel : 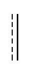
représente une liaison simple ou une double liaison ;
chaque W représente indépendamment un atome O ou S ;
chaque R₃ représente indépendamment 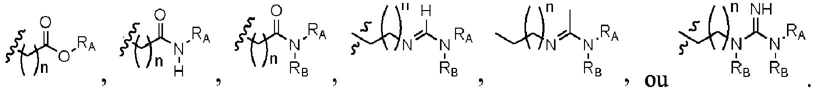
R₅ représente :
un atome H ;
un groupe alkyle en C₁ à C₆ éventuellement substitué par un ou plusieurs groupes R₆ qui peuvent être identiques ou différents ;
un groupe alcényle en C₂ à C₆ éventuellement substitué par un ou plusieurs groupes qui peuvent être identiques ou différents choisis parmi un atome d'halogène, un groupe hydroxy, alkyle en C₁ à C₆, aryle, (CH₂)_{P}OR_{A}, O(CH₂)ₘOH, O(CH₂)ₘO(CH₂)ₘOH, O(CH₂)ₘNR_{A}R_{B}, O(CH₂)ₘO(CH₂)ₘNR_{A}R_{B}, (CH₂)ₚNR_{A}R_{B}, (CH₂)ₚNR_{C}(CH₂)ₘNR_{A}R_{B}, (CH₂)ₚNR_{C}(CH₂)ₘNR_{C}(CH₂)ₘNR_{A}R_{B}, (CH₂)ₚC(=O)NR_{A}R_{B}, (CH₂)ₚC(=O)OR_{A} ;
un groupe alcynyle en C₂ à C₆ éventuellement substitué par un ou un ou plusieurs groupes qui peuvent être identiques ou différents choisis parmi un atome d'halogène, un groupe hydroxy, amino, monoalkylamino et dialkylamino ;
un groupe cycloalkyle en C₃ à C₇ éventuellement substitué par un ou plusieurs groupes qui peuvent être identiques ou différents choisis parmi un atome d'halogène, un groupe hydroxy, amino, monoalkylamino et dialkylamino ;
un groupe phényle ou CH₂-phényle éventuellement substitué par un ou plusieurs groupes qui peuvent être identiques ou différents choisis parmi un atome d'halogène, un groupe hydroxy, alkyle en C₁ à C₆, (CH₂)_{P}OR_{A}, (CH₂)_{P}NR_{A}R_{B}, (CH₂)_{P}C(=O)NR_{A}R_{B}, (CH₂)_{P}C(=O)OR_{A};
chaque occurrence de R₆ représente indépendamment un atome d'halogène, un groupe hydroxy, aryle, S-alkyle en C₁ à C₆, SR_{A}, OR_{A}, O(CH₂)ₘOR_{A}, O(CH₂)ₘO(CH₂)ₘOR_{A}, C(=O)OR_{A}, C(=O)NR_{A}R_{B}, NR_{A}R_{B}, O(CH₂)ₘNR_{A}R_{B}, O(CH₂)ₘO(CH₂)ₘNR_{A}R_{B}, NR_{C}(CH₂)ₘNR_{A}R_{B} ou NR_{C}(CH₂)ₘNR_{C}(CH₂)ₘNR_{A}R_{B}, ledit groupe aryle ou phényle étant éventuellement substitué par un ou plusieurs groupes qui peuvent être identiques ou différents choisis parmi un atome d'halogène, un groupe hydroxy, alkyle en C₁ à C₆, (CH₂)_{P}OR_{A}, (CH₂)_{P}NR_{A}R_{B}, (CH₂)_{P}C(=O)NR_{A}R_{B} et (CH₂)_{P}C(=O)OR_{A} ;
chaque occurrence de R_{A} et R_{B} représente indépendamment :
un atome hydrogène ;
un groupe alkyle en C₁ à C₆ éventuellement substitué par un ou plusieurs groupes R_{D} qui peuvent être identiques ou différents ;
un groupe alcényle en C₂ à C₆ ou alcynyle en C₂ à C₆ ;
un groupe cycloalkyle en C₃ à C₇ éventuellement substitué par un groupe alkyle en C₁ à C₆;
un groupe phényle éventuellement substitué par un à cinq groupes qui peuvent être identiques ou différents choisis parmi un atome d'halogène, un groupe -O-alkyle en C₁ à C₆, -C(=O)O(C₁ à C₆)alkyle, amino, alkylamino et dialkylamino ;
ou un noyau hétérocyclique qui peut être saturé ou insaturé contenant cinq ou six atomes de noyau et d'un à trois hétéroatomes qui peuvent être identiques ou différents choisis parmi un atome d'azote, de soufre et d'oxygène ;
ou R_{A} et R_{B}, ensemble avec l'atome d'azote auquel ils sont fixés, forment un noyau hétérocyclique saturé ou insaturé contenant de trois à sept atomes de noyau, lequel noyau peut éventuellement contenir un autre hétéroatome choisi dans le groupe constitué par l'atome d'azote, d'oxygène et de soufre et peut être éventuellement substitué par un à quatre groupes qui peuvent être identiques ou différents choisis dans le groupe constitué par un groupe alkyle, phényle et benzyle ;
chaque occurrence de R_{C} représente indépendamment un atome d'hydrogène ou un groupe alkyle en C₁ à C₆ ;
chaque occurrence de R_{D} représente indépendamment un atome d'halogène, un groupe hydroxy, O-alkyle en C₁ à C₄, C(=O)(C₁ à C₄)alkyle, C(=O)O(C₁ à C₄)alkyle ;
chaque p est indépendamment un entier valant 0, 1, 2, 3, 4 ou 5 ; et
chacun de m, n et q est indépendamment un entier valant 1, 2, 3, 4 ou 5.

9. Composé selon l'une quelconque des revendications 6 à 8, dans lequel :
R₅ représente un atome H, un groupe alkyle en C₁ à C₆, alcényle en C₂ à C₆, phényle, benzyle, CH₂-S-(C₁ à C₆)alkyle, CH₂-O-(C₁ à C₆)alkyle, (C₂ à C₆)OR_{A}, monoalkylamine en C₁ à C₆, dialkylamine en C₁ à C₆, ou amine cyclique en C₁ à C₆, dans lequel ledit groupe phényle ou benzyle est éventuellement substitué par un à trois substituants choisis parmi un groupe alkyle en C₁ à C₄, alcoxy en C₁ à C₄, et un atome d'halogène ; et R_{A} représente un atome H, un groupe alkyle en C₁ à C₆, phényle, CH₂-phényle, alkyl-OH en C₁ à C₆, (CH₂)ₚO(CH₂)ₘOH, (CH₂)ₚO(CH₂)ₘO(CH₂)ₘOH, (C₁ à C₆)alkylO(C₁ à C₄)alkyle, (CH₂)ₚO(CH₂)ₘO(C₁-C₄)alkyle ou (CH₂)ₚO(CH₂)ₘO(CH₂)ₘO(C₁-C₄)alkyle ;
p est un entier valant 0, 1, 2, 3, 4 ou 5 ; et
m est un entier valant 1, 2, 3, 4 ou 5 ; ou
dans lequel :
R₅ représente un atome H, un groupe alkyle en C₁ à C₄, alcényle en C₂ à C₄, phényle, benzyle, CH₂-S-(C₁ à C₄)alkyle, CH₂-O-(C₁ à C₄)alkyle, (CH₂)₂OH ou (CH₂)₂O(C₁ à C₄)alkyle.

10. Composé selon l'une quelconque des revendications 6 à 9, dans lequel chaque occurrence de R_{A} et R_{B} représente indépendamment un atome H, un groupe alkyle en C₁ à C₆, phényle, CH₂-phényle, alkyl-OH en C₁ à C₆, (CH₂)ₚO(CH₂)ₘOH, ou (CH₂)ₚO(CH₂)ₘO(CH₂)ₘOH, (C₁ à C₆)alkyl-O-(C₁ à C₄)alkyle, (CH₂)ₚO(CH₂)ₘO(C₁ à C₄)alkyle ou (CH₂)ₚO(CH₂)ₘO(CH₂)ₘO(C₁ à C₄)alkyle ; ou
dans lequel chaque occurrence R_{A} et R_{B} représente indépendamment un atome H ou un groupe alkyle en C₁ à C₆ ; ou
dans lequel R_{A} et R_{B}, ensemble avec l'atome d'azote auquel ils sont fixés, forment un hétérocycle choisi parmi 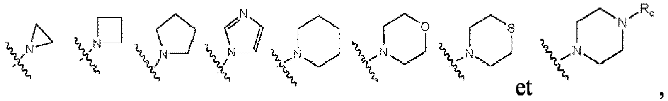 dans lequel R_{C} représente un atome H, un groupe Me, Et, n-Pr, i-Pr, n-Bu, i-Bu, t-Bu, CH₂CMe₃, Ph, CH₂Ph, CH₂CH₂OH, ou CH₂CH₂O(C₁ à C₄)alkyle.

11. Composé selon la revendication 1 choisi dans le groupe constitué par :
[(S)-(2-(N,N-diéthylamino)éthylthio)méthyl-Sar]-3-[(γ-hydroxy)-N-MeLeu]-4-cyclospori ne,
[(S)-(2-(N-éthyl-N-isopropylamino)éthylthio)méthyl-Sar]-3-[(γ-hydroxy)-N-MeLeu]-4-c yclosporine,
[(S)-(2-(N-isobutylamino)éthylthio)méthyl-Sar]-3-[(γ-hydroxy)-N-MeLeu]-4-cyclosporin e,
[(S)-(2-(N-éthyl-N-isobutylamino)éthylthio)méthyl-Sar]-3-[(γ-hydroxy)-N-MeLeu]-4-cy closporine,
[(S)-(2-(N-isobutyl-N-méthylamino)éthylthio)méthyl-Sar]-3-[(γ-hydroxy)-N-MeLeu]-4-c yclosporine,
[(S)-(2-(N-néopentylamino)éthylthio)méthyl-Sar]-3-[(γ-hydroxy)-N-MeLeu]-4-cyclospor ine,
[(S)-(2-(N-méthyl-N-néopentylamino)éthylthio)méthyl-Sar]-3-[(γ-hydroxy)-N-MeLeu]-4 -cyclosporine,
[(S)-(2-(N-éthyl-N-néopentylamino)éthylthio)méthyl-Sar]-3-[(γ-hydroxy)-N-MeLeu]-4-c yclosporine,
[(S)-(2-(N-pipéridinyl)éthylthio)méthyl-Sar]-3-[(γ-hydroxy)-N-MeLeu]-4-cyclosporine,
[(S)-(2-(N-morpholino)éthylthio)méthyl-Sar]-3-[(γ-hydroxy)-N-MeLeu]-4-cyclosporine,
[(S)-(2-(N-thiomorpholino)éthylthio)méthyl-Sar]-3-[(γ-hydroxy)-N-MeLeu]-4-cyclospori ne,
[(S)-(2-(4-méthyl-N-pipérazinyl)éthylthio)méthyl-Sar]-3-[(γ-hydroxy)-N-MeLeu]-4-cycl osporine,
[(S)-(3-(N,N-diéthylamino)propylthio)méthyl-Sar]-3-[(γ-hydroxy)-N-MeLeu]-4-cyclospo rine,
[(S)-(3-(N-éthyl-N-isopropylamino)propylthio)méthyl-Sar]-3-[(γ-hydroxy)-N-MeLeu]-4-cyclosporine,
[(S)-(3-(N-néopentylamino)propylthio)méthyl-Sar]-3-[(γ-hydroxy)-N-MeLeu]-4-cyclosp orine,
[(S)-(3-(N-pyrrolidinyl)propylthio)méthyl-Sar]-3-[(γ-hydroxy)-N-MeLeu]-4-cyclosporin e,
[(S)-(3-(N-pipéridinyl)propylthio)méthyl-Sar]-3-[(γ-hydroxy)-N-MeLeu]-4-cyclosporine,
[(S)-(3-(N-morpholino)propylthio)méthyl-Sar]-3-[(γ-hydroxy)-N-MeLeu]-4-cyclosporine ,
[(S)-(3-(N-thiomorpholino)propylthio)méthyl-Sar]-3-[(γ-hydroxy)-N-MeLeu]-4-cyclospo rine,
[(S)-(3-(4-méthyl-N-pipérazinyl)propylthio)méthyl-Sar]-3-[(γ-hydroxy)-N-MeLeu]-4-cyc losporine,
[(S)-(4-(N,N-diéthylamino)butylthio)méthyl-Sar]-3-[(γ-hydroxy)-N-MeLeu]-4-cyclospor ine,
[(S)-(4-(N-éthyl-N-isopropylamino)butylthio)méthyl-Sar]-3-[(γ-hydroxy)-N-MeLeu]-4-c yclosporine,
[(S)-(4-(N-isobutylamino)butylthio)méthyl-Sar]-3-[(γ-hydroxy)-N-MeLeu]-4-cyclosporin e,
[(S)-(4-(N-Isobutyl-N-méthylamino)butylthio)méthyl-Sar]-3-[(γ-hydroxy)-N-MeLeu]-4-c yclosporine,
[(S)-(4-(N-éthyl-N-isobutylamino)butylthio)méthyl-Sar]-3-[(γ-hydroxy)-N-MeLeu]-4-cy closporine,
[(S)-(4-(N-néopentylamino)butylthio)méthyl-Sar]-3-[(γ-hydroxy)-N-MeLeu]-4-cyclospor ine,
[(S)-(4-(N-méthyl-N-néopentylamino)butylthio)méthyl-Sar]-3-[(γ-hydroxy)-N-MeLeu]-4 -cyclosporine,
[(S)-(4-(N-pipéridinyl)butylthio)méthyl-Sar]-3-[(γ-hydroxy)-N-MeLeu]-4-cyclosporine,
[(S)-(4-(N-morpholino)butylthio)méthyl-Sar]-3-[(γ-hydroxy)-N-MeLeu]-4-cyclosporine,
[(S)-(4-(N-thiomorpholino)butylthio)méthyl-Sar]-3-[(γ-hydroxy)-N-MeLeu]-4-cyclospori ne,
[(S)-(4-(4-méthyl-N-pipérazinyl)butylthio)méthyl-Sar]-3-[(γ-hydroxy)-N-MeLeu]-4-cycl osporine,
[(R)-(3-(N-morphlino)propoxy)méthyl-Sar]-3-[(γ-hydroxy)-N-MeLeu]-4-cyclosporine, [(S)-(2-(N,N-diéthylamino)éthylthio)méthyl-Sar]-3-[(γ-méthoxy)-N-MeLeu]-4-cyclospor ine,
[(S)-(2-(N-pyrrolidinyl)éthylthio)méthyl-Sar]-3-[(γ-méthoxy)-N-MeLeu]-4-cyclosporine, [(S)-(3-(N,N-diméthylamino)propylthio)méthyl-Sar]-3-[(γ-méthoxy)-N-MeLeu]-4-cyclosporine,
[(S)-(3-(N,N-diéthylamino)propylthio)méthyl-Sar]-3-[(γ-méthoxy)-N-MeLeu]-4-cyclosporine,
[(S)-(3-(N-pipéridinyl)propylthio)méthyl-Sar]-3-[(y-méthoxy)-N-MeLeu]-4-cyclosporine ,
[(S)-(4-(N-pipéridinyl)butylthio)méthyl-Sar]-3-[(γ-méthoxy)-N-MeLeu]-4-cyclosporine, [(R)-(2-(N,N-diéthylamino)éthoxy)méthyl-Sar]-3-[(γ-méthoxy)-N-MeLeu]-4-cyclosporin e, et
[(R)-(3-(N,N-diéthylamino)propoxy)méthyl-Sar]-3-[(γ-méthoxy)-N-MeLeu]-4-cyclospor ine.

12. Composé selon la revendication 1 ayant la structure de : 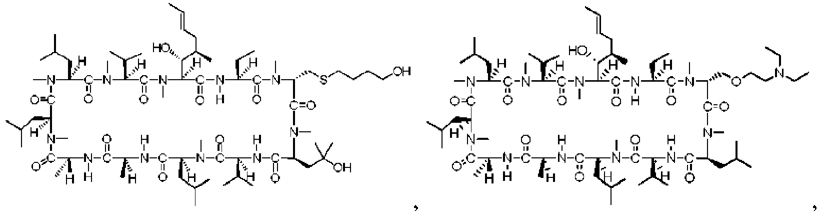 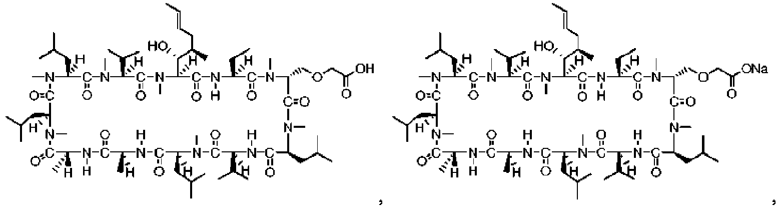 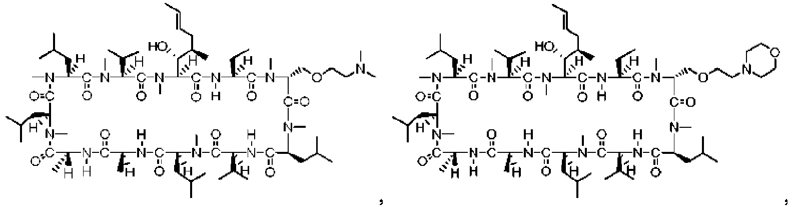 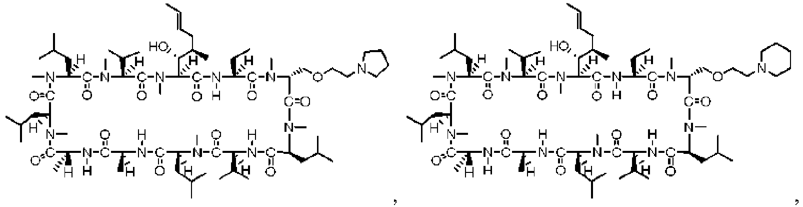 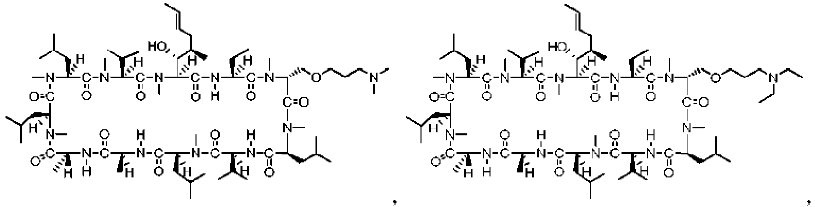 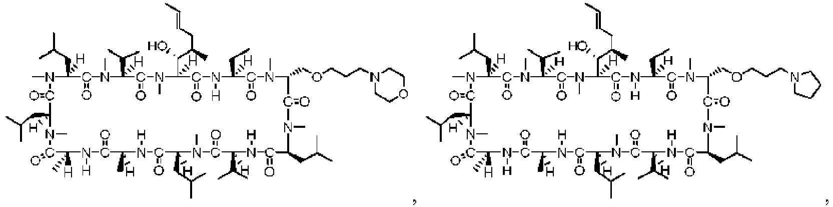 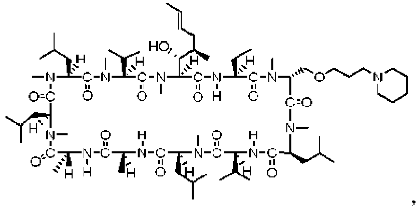 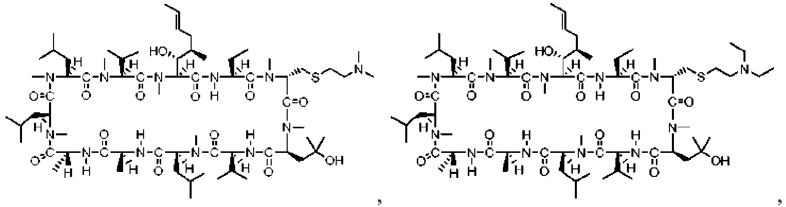 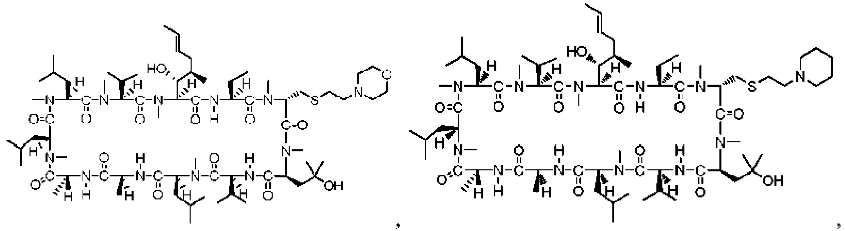 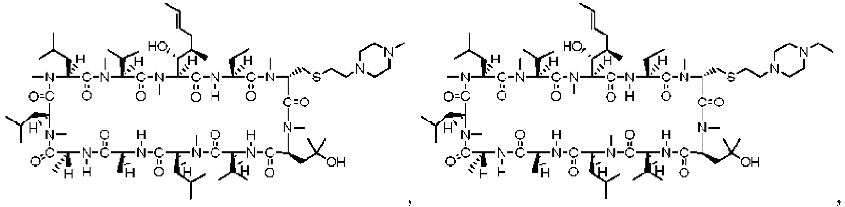 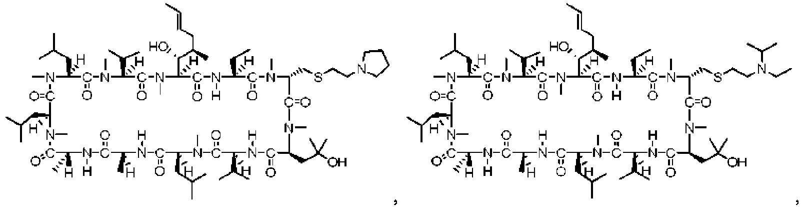 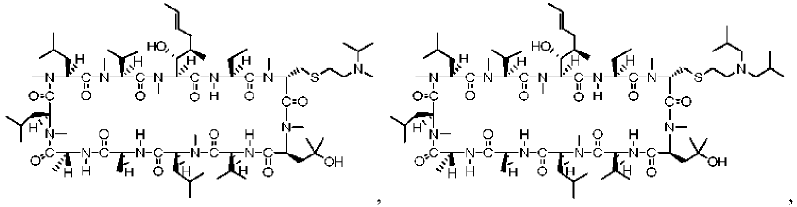 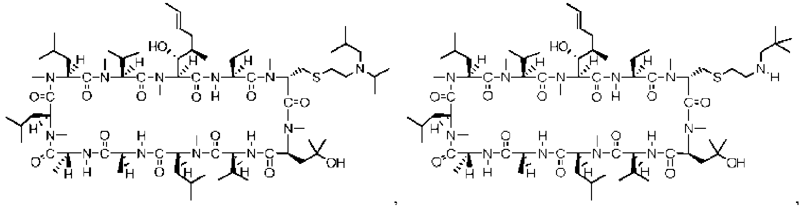 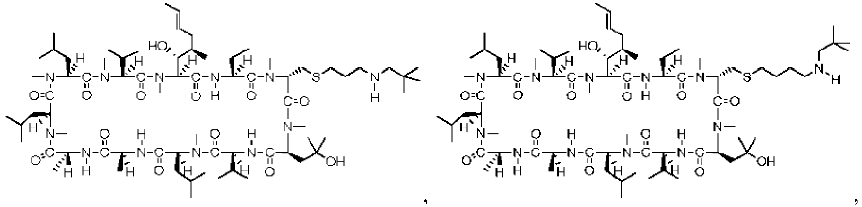 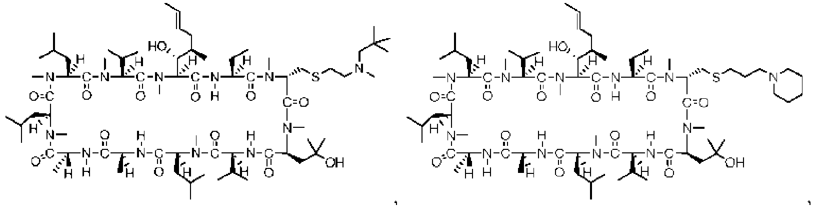 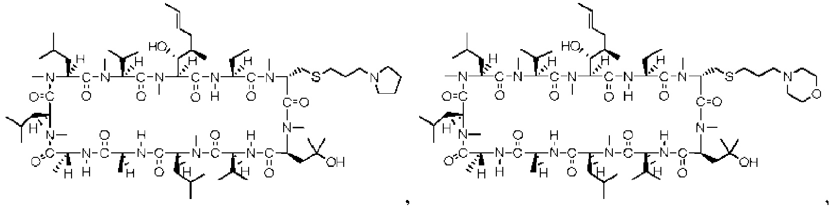 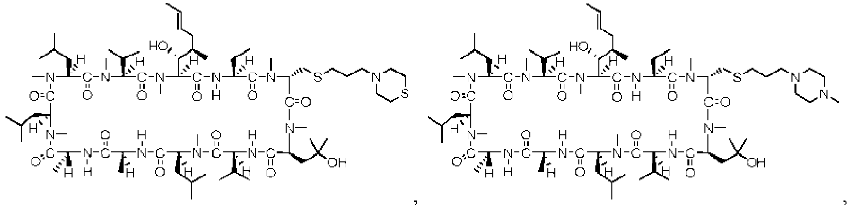 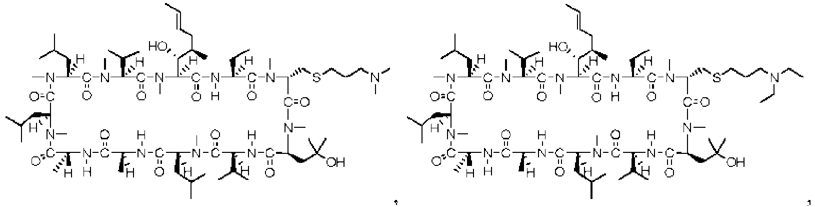 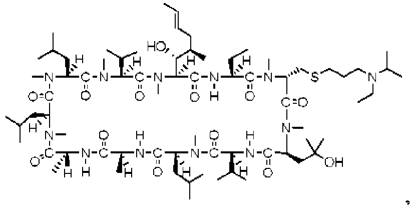 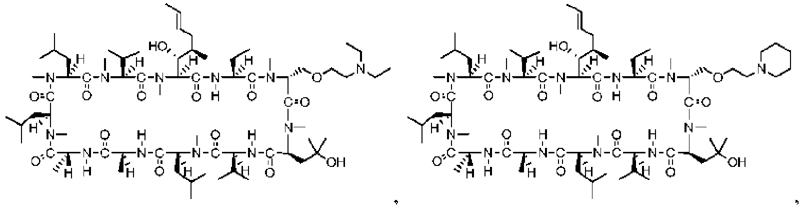 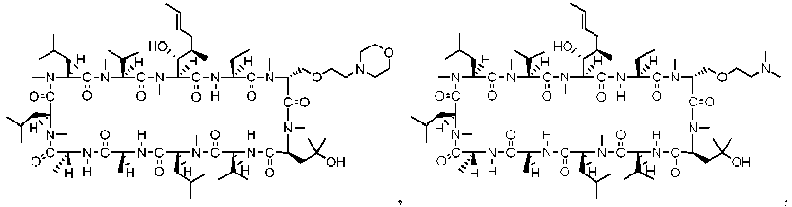 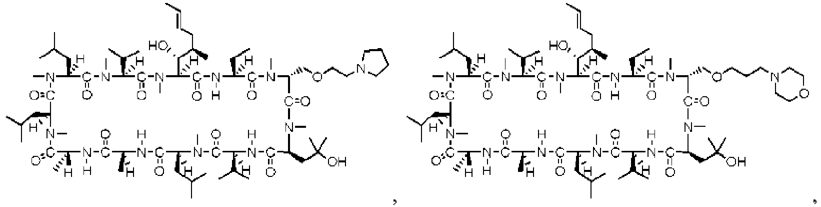 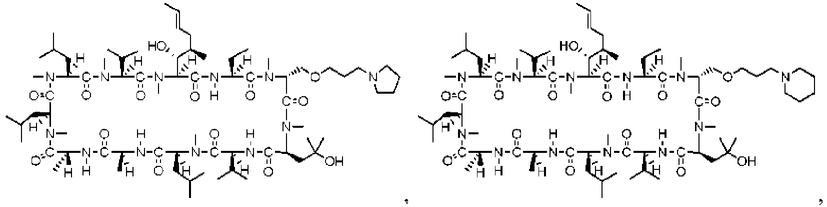 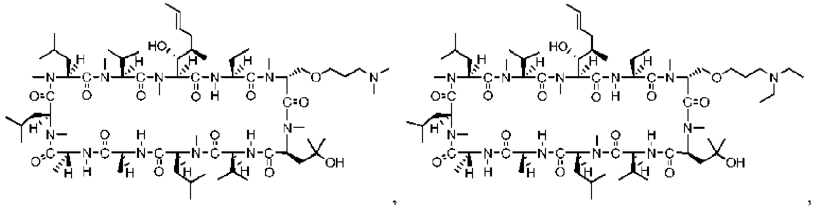 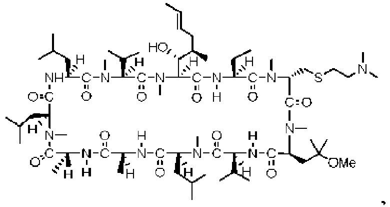 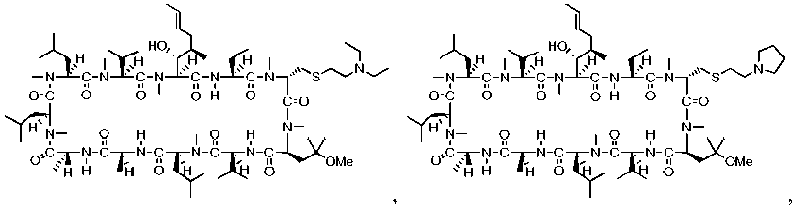 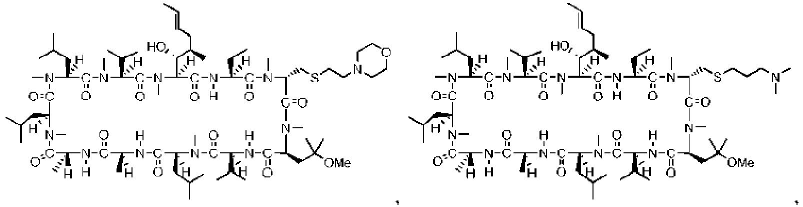 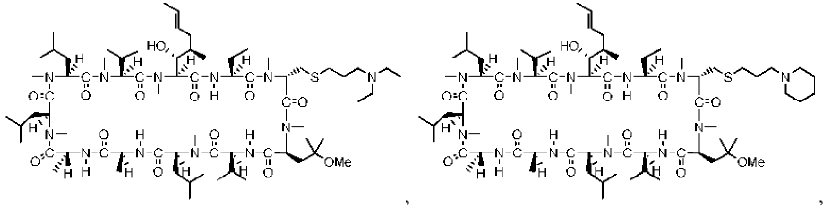 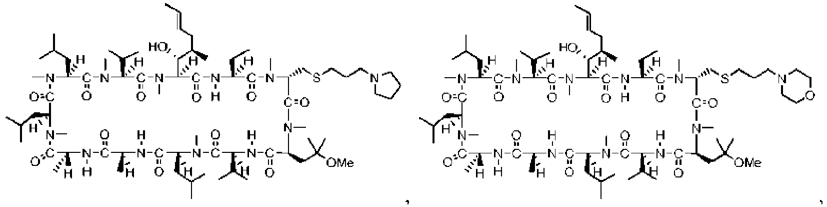 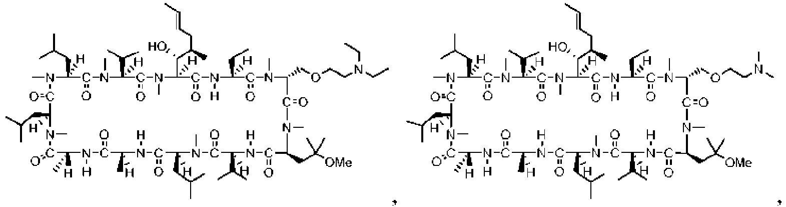 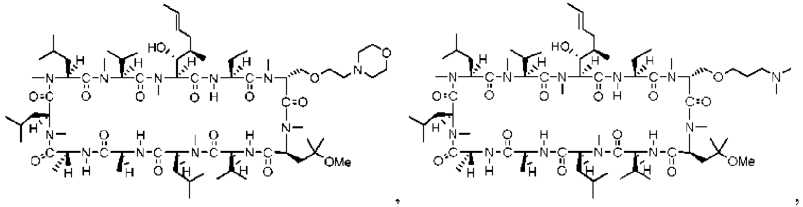 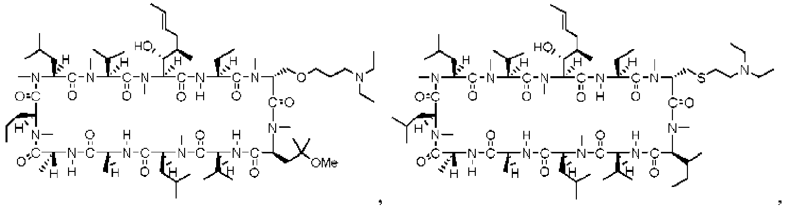 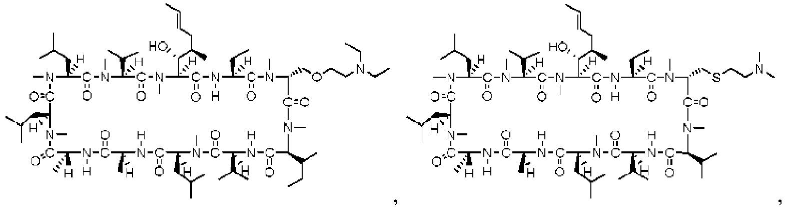 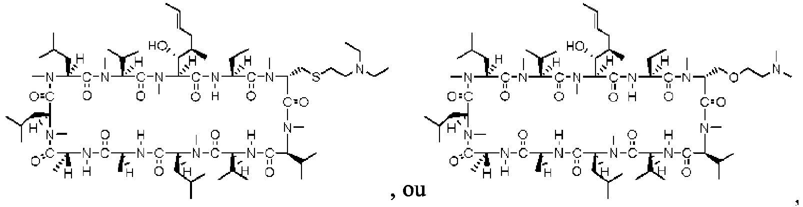 ou sel pharmaceutiquement acceptable de celui-ci.

13. Composé selon la revendication 1 ayant la structure suivante : 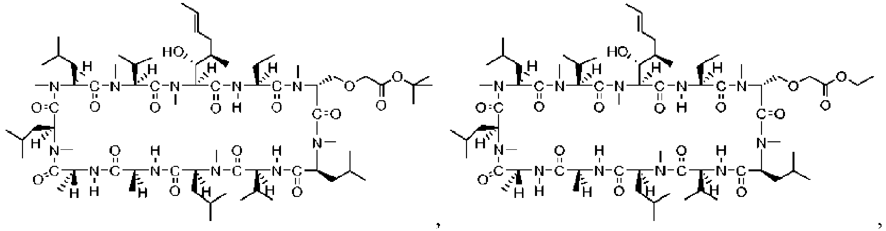 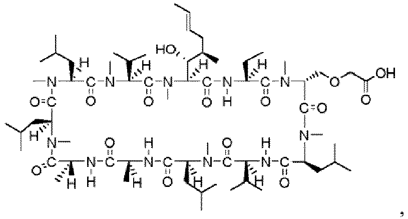 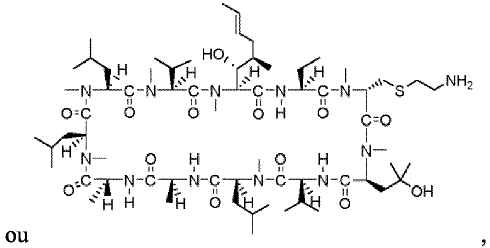 ou sel pharmaceutiquement acceptable de celui-ci.

14. Composition pharmaceutique comprenant au moins un composé selon l'une quelconque des revendications 1 à 10 et un excipient ou diluant pharmaceutiquement acceptable.

15. Composé selon l'une quelconque des revendications 1 à 10 destiné à être utilisé dans le traitement ou la prévention d'une infection par le virus de l'hépatite B, d'une infection par le virus de l'hépatite C ou d'une infection par le VIH chez une espèce mammifère.
